# EUROPEAN PATENT APPLICATION

(11) **EP 2 476 690 A1**
(43) Date of publication of application: **18.07.2012**
(21) Application number: 12153673.4
(22) Date of filing: 01.07.2009
(51) Int. Cl.: C07H 19/20, A61K 31/708, A61P 31/12

(54) **Compounds and pharmaceutical compositions for the treatment of viral infections**

(30) Priority: 02.07.2008 US 133844 P; 27.01.2009 US 147722 P
(62) Divisional of application: 09788862.2
(71) Applicant: IDENIX Pharmaceuticals, Inc., Cambridge, MA 02139 (US)
(72) Inventor: Cretton-Scott, Erika, Birmingham, AL Alabama 35242 (US); Gupta, Kusum, Lexington, MA Massachusetts 02421 (US); Hernandez-Santiago, Brenda, Arlington, MA Massachusetts 02476 (US); Larsson, Marita, Waltham, MA Massachusetts 02452 (US)
(74) Representative: Ritter, Thomas Kurt

(57) **Abstract**

Provided herein are compounds, compositions and methods for the treatment of liver disorder, including HCV infections. Specifically, compound and compositions of nucleoside derivatives are disclosed, which can be administered either alone or in combination with other anti-viral agents.

## Description

### CROSS REFERENCE TO RELATED APPLICATIONS

This application claims the benefit of the priority of U.S. Provisional Application Nos. 61/133,844, filed July 2, 2008, and 61/147,722, filed January 27, 2009, the disclosure of each of which is encorporated herein by reference in its entirety.

### FIELD

Provided herein are compounds, methods and pharmaceutical compositions, for use in treatment of viral infections, including hepatitis C virus infection, and hepatitis B virus infection in a host in need thereof.

### BACKGROUND

### Flaviviridae Viruses

The Flaviviridae family of viruses comprises at least three distinct genera: *pestiviruses,* which cause disease in cattle and pigs; *flaviviruses,* which are the primary cause of diseases such as dengue fever and yellow fever; and *hepaciviruses,* whose sole member is HCV. The flavivirus genus includes more than 68 members separated into groups on the basis of serological relatedness (Calisher et al., J. Gen. Virol, 1993, 70, 37-43). Clinical symptoms vary and include fever, encephalitis and hemorrhagic fever (Fields Virology, Editors: Fields, B. N., Knipe, D. M., and Howley, P. M., Lippincott-Raven Publishers, Philadelphia, PA, 1996, Chapter 31, 931-959). Flaviviruses of global concern that are associated with human disease include the dengue hemorrhagic fever viruses (DHF), yellow fever virus, shock syndrome and Japanese encephalitis virus (Halstead, S. B., Rev. Infect. Dis., 1984, 6, 251-264; Halstead, S. B., Science, 239:476-481, 1988; Monath, T. P., New Eng. J. Med., 1988, 319, 641-643).

The pestivirus genus includes bovine viral diarrhea virus (BVDV), classical swine fever virus (CSFV, also called hog cholera virus) and border disease virus (BDV) of sheep (Moennig, V. et al. Adv. Vir. Res. 1992, 41, 53-98). Pestivirus infections of domesticated livestock (cattle, pigs and sheep) cause significant economic losses worldwide. BVDV causes mucosal disease in cattle and is of significant economic importance to the livestock industry (Meyers, G. and Thiel, H.-J., Advances in Virus Research, 1996, 47, 53-118; Moennig V., et al, Adv. Vir. Res. 1992, 41, 53-98). Human pestiviruses have not been as extensively characterized as the animal pestiviruses. However, serological surveys indicate considerable pestivirus exposure in humans.

*Pestiviruses* and *hepaciviruses* are closely related virus groups within the Flaviviridae family. Other closely related viruses in this family include the GB virus A, GB virus A-like agents, GB virus-B and GB virus-C (also called hepatitis G virus, HGV). The hepacivirus group (hepatitis C virus; HCV) consists of a number of closely related but genotypically distinguishable viruses that infect humans. There are approximately 6 HCV genotypes and more than 50 subtypes. Due to the similarities between pestiviruses and hepaciviruses, combined with the poor ability of hepaciviruses to grow efficiently in cell culture, bovine viral diarrhea virus (BVDV) is often used as a surrogate to study the HCV virus.

The genetic organization of pestiviruses and hepaciviruses is very similar. These positive stranded RNA viruses possess a single large open reading frame (ORF) encoding all the viral proteins necessary for virus replication. These proteins are expressed as a polyprotein that is co- and post-translationally processed by both cellular and virus-encoded proteinases to yield the mature viral proteins. The viral proteins responsible for the replication of the viral genome RNA are located towards the carboxy-terminal. Two-thirds of the ORF are termed nonstructural (NS) proteins. The genetic organization and polyprotein processing of the nonstructural protein portion of the ORF for pestiviruses and hepaciviruses is very similar. For both the pestiviruses and hepaciviruses, the mature nonstructural (NS) proteins, in sequential order from the amino-terminus of the nonstructural protein coding region to the carboxy-terminus of the ORF, consist of p7, NS2, NS3, NS4A, NS4B, NS5A, and NS5B.

The NS proteins of pestiviruses and hepaciviruses share sequence domains that are characteristic of specific protein functions. For example, the NS3 proteins of viruses in both groups possess amino acid sequence motifs characteristic of serine proteinases and of helicases (Gorbalenya et al. (1988) Nature 333:22; Bazan and Fletterick (1989) Virology 171:637-639; Gorbalenya et al. (1989) Nucleic Acid Res. 17.3889-3897). Similarly, the NS5B proteins of pestiviruses and hepaciviruses have the motifs characteristic of RNA-directed RNA polymerases (Koonin, E.V. and Dolja, V.V. (1993) Crit. Rev. Biochem. Molec. Biol. 28:375-430).

The actual roles and functions of the NS proteins of pestiviruses and hepaciviruses in the lifecycle of the viruses are directly analogous. In both cases, the NS3 serine proteinase is responsible for all proteolytic processing of polyprotein precursors downstream of its position in the ORF (Wiskerchen and Collett (1991) Virology 184:341-350; Bartenschlager et al. (1993) J. Virol. 67:3835-3844; Eckart et al. (1993) Biochem. Biophys. Res. Comm. 192:399-406; Grakoui et al. (1993) J Virol. 67:2832-2843; Grakoui et al. (1993) Proc. Natl. Acad. Sci. USA 90:10583-10587; Hijikata et al. (1993) J Virol. 67:4665-4675; Tome et al. (1993) J. Virol. 67:4017-4026). The NS4A protein, in both cases, acts as a cofactor with the NS3 serine protease (Bartenschlager et al. (1994) J Virol. 68:5045-5055; Failla et al. (1994) J Virol. 68: 3753-3760; Lin et al. (1994) 68:8147-8157; Xu et al. (1997) J Virol. 71:5312-5322). The NS3 protein of both viruses also functions as a helicase (Kim et al. (1995) Biochem. Biophys. Res. Comm. 215: 160-166; Jin and Peterson (1995) Arch. Biochem. Biophys., 323:47-53; Warrener and Collett (1995) J. Virol. 69:1720-1726). Finally, the NS5B proteins of pestiviruses and hepaciviruses have predicted RNA-directed RNA polymerase activity (Behrens et al. (1996) EMBO J. 15:12-22; Lehmann et al. (1997) J Virol. 71:8416-8428; Yuan et al.(1997) Biochem. Biophys. Res. Comm. 232:231-235; Hagedorn, PCT WO 97/12033; US patent nos. 5,981,247; 6,248,589 and 6,461,845 Zhong et al.(1998) J. Virol. 72.9365-9369)

### Hepatitis C Virus

The hepatitis C virus (HCV) is a leading cause of chronic liver disease worldwide. (Boyer, N. et al. J Hepatol. 32:98-112, 2000). HCV causes a slow growing viral infection and is the major cause of cirrhosis and hepatocellular carcinoma (Di Besceglie, A. M. and Bacon, B. R., Scientific American, Oct.: 80-85, (1999); Boyer, N. et al. J Hepatol. 32:98-112, 2000). An estimated 170 million persons are infected with HCV worldwide. (Boyer, N. et al. J Hepatol. 32:98-112, 2000). Cirrhosis caused by chronic hepatitis C infection accounts for 8,000-12,000 deaths per year in the United States, and HCV infection is the leading indication for liver transplantation.

HCV is known to cause at least 80% of posttransfusion hepatitis and a substantial proportion of sporadic acute hepatitis. Preliminary evidence also implicates HCV in many cases of "idiopathic" chronic hepatitis, "cryptogenic" cirrhosis, and probably hepatocellular carcinoma unrelated to other hepatitis viruses, such as Hepatitis B Virus (HBV). A small proportion of healthy persons appear to be chronic HCV carriers, varying with geography and other epidemiological factors. The numbers may substantially exceed those for HBV, though information is still preliminary; how many of these persons have subclinical chronic liver disease is unclear. (The Merck Manual, 18th ed., (2006)).

HCV is an enveloped virus containing a positive-sense single-stranded RNA genome of approximately 9.4kb. The viral genome consists of a 5' untranslated region (UTR), a long open reading frame encoding a polyprotein precursor of approximately 3011 amino acids, and a short 3' UTR. The 5' UTR is the most highly conserved part of the HCV genome and is important for the initiation and control of polyprotein translation. Translation of the HCV genome is initiated by a cap-independent mechanism known as internal ribosome entry. This mechanism involves the binding of ribosomes to an RNA sequence known as the internal ribosome entry site (IRES). An RNA pseudoknot structure has recently been determined to be an essential structural element of the HCV IRES. Viral structural proteins include a nucleocapsid core protein (C) and two envelope glycoproteins, E1 and E2. HCV also encodes two proteinases, a zinc-dependent metalloproteinase encoded by the NS2-NS3 region and a serine proteinase encoded in the NS3 region. These proteinases are required for cleavage of specific regions of the precursor polyprotein into mature peptides. The carboxyl half of nonstructural protein 5, NS5B, contains the RNA-dependent RNA polymerase. The function of the remaining nonstructural proteins, NS4A and NS4B, and that of NS5A (the amino-terminal half of nonstructural protein 5) remain unknown.

A significant focus of current antiviral research is directed to the development of improved methods of treatment of chronic HCV infections in humans (Di Besceglie, A. M. and Bacon, B. R., Scientific American, Oct.: 80-85, (1999)).

In light of the fact that HCV infection has reached epidemic levels worldwide, and has tragic effects on the infected patient, there remains a strong need to provide new effective pharmaceutical agents to treat hepatitis C that have low toxicity to the host.

Further, given the rising threat of other flaviviridae infections, there remains a strong need to provide new effective pharmaceutical agents to treat the flaviviridae infections.

### SUMMARY

In certain embodiments, the compounds provided herein are of formula I. 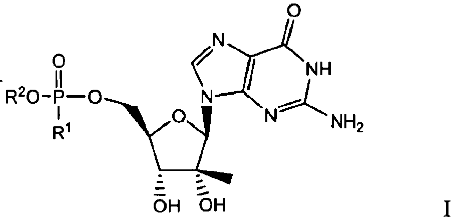 or a pharmaceutically acceptable salt, solvate, hydrate, a stereoisomeric, or tautomeric form thereof, wherein R¹ is hydroxyl, amino or benzylamino; and R² is hydrogen, 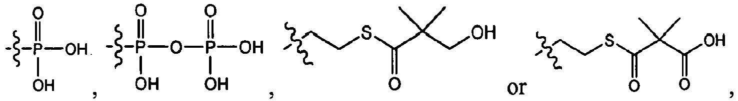 such that when R¹ is hydroxyl, R² is other than hydrogen, 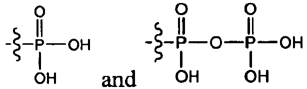 and when R¹ is benzylamino, R² is other than

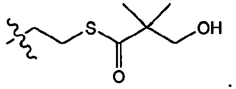

In one embodiment, provided herein is Compound 1a having formula: 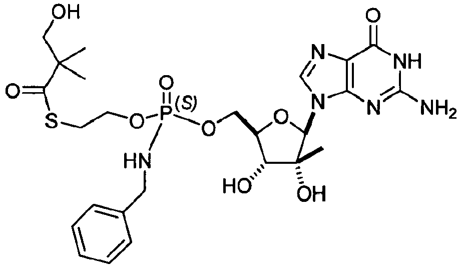 or a pharmaceutically acceptable salt, solvate or hydrate thereof.

In one embodiment, provided herein is Compound 1b having formula: 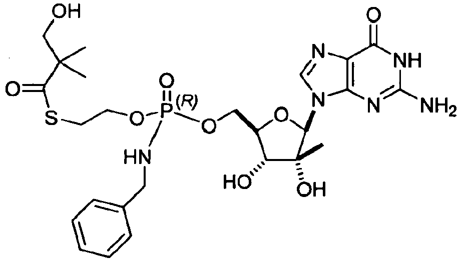 or a pharmaceutically acceptable salt, solvate or hydrate thereof.

In certain embodiments, the compounds provided herein are of formula VI. 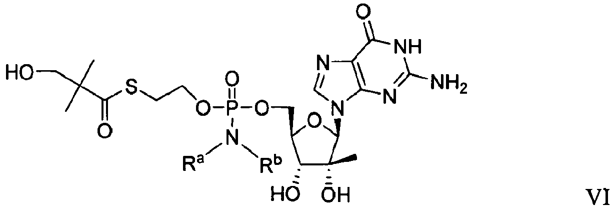 or a pharmaceutically acceptable salt, solvate, hydrate, a stereoisomeric, or tautomeric form thereof, wherein R^{a} and R^{b} are selected as follows:
i) R^{a} is hydrogen or alkyl and R^{b} is alkyl, cycloalkyl, carboxyalkyl, alkoxycarbonylalkyl or dialkylaminoalkyl; or
ii) R^{a} and R^{b} together with the nitrogen atom on which they are substituted form a 3-7 membered heterocyclic or heteroaryl ring, optionally substituted with one or two alkyl groups.

In certain embodiments compound 1 is a prodrug of parent drug 2'-C-methyl guanosine. In other words, the parent drug can be obtained from metabolism of Compound 1 in the liver, and thus the parent drug is capable of accumulating in the liver of a host.

In certain embodiments, the compounds provided herein are useful in the prevention and treatment of *Flaviviridae* infections and other related conditions such as anti-*Flaviviridae* antibody positive and *Flaviviridae*-positive conditions, chronic liver inflammation caused by HCV, cirrhosis, fibrosis, acute hepatitis, fulminant hepatitis, chronic persistent hepatitis, and fatigue. These compounds or formulations can also be used prophylactically to prevent or retard the progression of clinical illness in individuals who are anti-*Flaviviridae* antibody or *Flaviviridae*-antigen positive or who have been exposed to a *Flaviviridae.* In one embodiment, the *Flaviviridae* is hepatitis C. In certain embodiments, the compound is used to treat any virus that replicates through an RNA-dependent RNA polymerase.

In one embodiment, a method for the treatment of a *Flaviviridae* infection in a host, including a human, is provided that includes administering an effective amount of a compound provided herein, administered either alone or in combination or alternation with another anti-*Flaviviridae* agent, optionally in a pharmaceutically acceptable carrier.

In one aspect, the compounds described herein are provided or administered in combination with a second therapeutic agent, such as one useful for the treatment or prevention of HCV infections. Exemplary therapeutic agents are described in detail in the sections below.

In one embodiment, a method for the treatment of a *Flaviviridae* infection in a host, including a human, is provided that includes administering S-(2-{[(2R,3R,4R,5R)-5-(2-amino-6-oxo-1,6-dihydro-purin-9-yl)-3,4-dihydroxy-4-methyl-tetrahydro-furan-2-ylmethoxy]-benzylamino-phosphoryloxy}-ethyl) ester) in an amount from about 1 mg/day to about 150 mg/day, administered either alone or in combination or alternation with another anti-*Flaviviridae* agent, optionally in a pharmaceutically acceptable carrier.

In another embodiment, provided herein is a method for the treatment of a *Flaviviridae* infection in a host, including a human, that includes administering S-(2-{[(2R,3R,4R,5R)-5-(2-amino-6-oxo-1,6-dihydro-purin-9-yl)-3,4-dihydroxy-4-methyltetrahydro-furan-2-ylmethoxy]-benzylamino-phosphoryloxy}-ethyl) ester) in an amount from about 1 mg/day to about 150 mg/day, in combination or alternation with a therapeutically effective amount of ribavirin, optionally in a pharmaceutically acceptable carrier. In one embodiment, the amount of ribavirin administered is from about 800 mg to about 1400 mg.

In another aspect, provided are pharmaceutical compositions, single unit dosage forms, and kits suitable for use in treating or preventing disorders such as HCV infections which comprise a therapeutically or prophylactically effective amount of a compound described herein and a therapeutically or prophylactically effective amount of a second therapeutic such as one useful for the treatment or prevention of HCV infections.

In certain embodiments, a method for treatment of a liver disorder is provided comprising administering to an individual in need thereof a treatment effective amount of a compound provided herein.

In some embodiments, provided herein are:
(a) compounds as described herein, and pharmaceutically acceptable salts and compositions thereof;
(b) compounds as described herein, and pharmaceutically acceptable salts and compositions thereof for use in the treatment and/or prophylaxis of a liver disorder including *Flaviviridae* infection, especially in individuals diagnosed as having a *Flaviviridae* infection or being at risk of becoming infected by hepatitis C;
(c) processes for the preparation of compounds as described herein;
(d) pharmaceutical formulations comprising a compound as described herein, or a pharmaceutically acceptable salt thereof together with a pharmaceutically acceptable carrier or diluent;
(e) pharmaceutical formulations comprising a compound as described herein, or a pharmaceutically acceptable salt thereof together with one or more other effective anti-HCV agents, optionally in a pharmaceutically acceptable carrier or diluent;
(f) a method for the treatment and/or prophylaxis of a host infected with *Flaviviridae* that includes the administration of an effective amount of a compound as described herein, its pharmaceutically acceptable salt or composition; and
(g) a method for the treatment and/or prophylaxis of a host infected with *Flaviviridae* that includes the administration of an effective amount of a compounds as described herein, its pharmaceutically acceptable salt or composition in combination and/or alternation with one or more effective anti-HCV agent.

*Flaviviridae* which can be treated are, e.g., discussed generally in Fields Virology, Editors: Fields, B. N., Knipe, D. M., and Howley, P. M., Lippincott-Raven Publishers, Philadelphia, PA, Chapter 31, 1996. In a particular embodiment, the *Flaviviridae* is HCV. In an alternate embodiment, the *Flaviviridae* is a flavivirus or pestivirus. Specific flaviviruses include, without limitation: Absettarov, Alfuy, Apoi, Aroa, Bagaza, Banzi, Bouboui, Bussuquara, Cacipacore, Carey Island, Dakar bat, Dengue 1, Dengue 2, Dengue 3, Dengue 4, Edge Hill, Entebbe bat, Gadgets Gully, Hanzalova, Hypr, Ilheus, Israel turkey meningoencephalitis, Japanese encephalitis, Jugra, Jutiapa, Kadam, Karshi, Kedougou, Kokobera, Koutango, Kumlinge, Kunjin, Kyasanur Forest disease, Langat, Louping ill, Meaban, Modoc, Montana myotis leukoencephalitis, Murray valley encephalitis, Naranjal, Negishi, Ntaya, Omsk hemorrhagic fever, Phnom-Penh bat, Powassan, Rio Bravo, Rocio, Royal Farm, Russian spring-summer encephalitis, Saboya, St. Louis encephalitis, Sal Vieja, San Perlita, Saumarez Reef, Sepik, Sokuluk, Spondweni, Stratford, Tembusu, Tyuleniy, Uganda S, Usutu, Wesselsbron, West Nile, Yaounde, Yellow fever, and Zika.

Pestiviruses which can be treated are discussed generally in Fields Virology, Editors: Fields, B. N., Knipe, D. M., and Howley, P. M., Lippincott-Raven Publishers, Philadelphia, PA, Chapter 33, 1996. Specific pestiviruses include, without limitation: bovine viral diarrhea virus ("BVDV"), classical swine fever virus ("CSFV," also called hog cholera virus), and border disease virus ("BDV").

### BRIEF DESCRIPTION OF DRAWINGS

FIG. 1 provides an HPLC trace illustrating resolution of the two diastereomers of compound **1** by reverse phase HPLC - the two peaks in the trace, peak 1 (diastereomer 1) and peak 2 (diastereomer 2), correspond to pure diastereomers of compound **1**;

FIG. 2 provides representative HPLC chromatogram of compound **1** and metabolite standards obtained by method 1 of Example 14;

FIG. 3 provides representative HPLC chromatogram of compound **1** and metabolite standards obtained by method 2 of Example 15 ; and

FIG. 4 provides representative HPLC chromatogram of compound **1** and metabolite standards obtained by method 2 of Example 16.

FIG. 5 depicts the result of MacSynergyTM II analysis (Bliss independence) for five individual experimental data sets at the 99.9% confidence interval.

FIG. 6 depicts the differences between calculated additive and observed anti-HCV effects for all drug combinations from 5 independent experiments, obtained with the CombiTool software using the Loewe additivity model.

### DESCRIPTION OF EXEMPLARY EMBODIMENTS

Provided herein are compounds, compositions and methods useful for treating liver disorders such as HCV infection in a subject. Further provided are dosage forms useful for such methods.

### Definitions

When referring to the compounds provided herein, the following terms have the following meanings unless indicated otherwise.

"Pharmaceutically acceptable salt" includes any salt of a compound provided herein which retains its biological properties and which is not toxic or otherwise undesirable for pharmaceutical use. Such salts may be derived from a variety of organic and inorganic counter-ions well known in the art. Such salts include: (1) acid addition salts formed with organic or inorganic acids such as hydrochloric, hydrobromic, sulfuric, nitric, phosphoric, sulfamic, acetic, trifluoroacetic, trichloroacetic, propionic, hexanoic, cyclopentylpropionic, glycolic, glutaric, pyruvic, lactic, malonic, succinic, sorbic, ascorbic, malic, maleic, fumaric, tartaric, citric, benzoic, 3-(4-hydroxybenzoyl)benzoic, picric, cinnamic, mandelic, phthalic, lauric, methanesulfonic, ethanesulfonic, 1,2-ethane-disulfonic, 2-hydroxyethanesulfonic, benzenesulfonic, 4-chlorobenzenesulfonic, 2-naphthalenesulfonic, 4-toluenesulfonic, camphoric, camphorsulfonic, 4-methylbicyclo[2.2.2]-oct-2-ene-1-carboxylic, glucoheptonic, 3-phenylpropionic, trimethylacetic, *tert*-butylacetic, lauryl sulfuric, gluconic, benzoic, glutamic, hydroxynaphthoic, salicylic, stearic, cyclohexylsulfamic, quinic, muconic acid and the like acids; or (2) salts formed when an acidic proton present in the parent compound either (a) is replaced by a metal ion, *e.g*., an alkali metal ion, an alkaline earth ion or an aluminum ion, or alkali metal or alkaline earth metal hydroxides, such as sodium, potassium, calcium, magnesium, aluminum, lithium, zinc, and barium hydroxide, ammonia or (b) coordinates with an organic base, such as aliphatic, alicyclic, or aromatic organic amines, such as ammonia, methylamine, dimethylamine, diethylamine, picoline, ethanolamine, diethanolamine, triethanolamine, ethylenediamine, lysine, arginine, ornithine, choline, N,N'-dibenzylethylene-diamine, chloroprocaine, diethanolamine, procaine, N-benzylphenethylamine, *N*-methylglucamine piperazine, tris(hydroxymethyl)-aminomethane, tetramethylammonium hydroxide, and the like.

Salts further include, by way of example only, sodium, potassium, calcium, magnesium, ammonium, tetraalkylammonium and the like, and when the compound contains a basic functionality, salts of non-toxic organic or inorganic acids, such as hydrohalides, e.g. hydrochloride and hydrobromide, sulfate, phosphate, sulfamate, nitrate, acetate, trifluoroacetate, trichloroacetate, propionate, hexanoate, cyclopentylpropionate, glycolate, glutarate, pyruvate, lactate, malonate, succinate, sorbate, ascorbate, malate, maleate, fumarate, tartarate, citrate, benzoate, 3-(4-hydroxybenzoyl)benzoate, picrate, cinnamate, mandelate, phthalate, laurate, methanesulfonate (mesylate), ethanesulfonate, 1,2-ethane-disulfonate, 2-hydroxyethanesulfonate, benzenesulfonate (besylate), 4-chlorobenzenesulfonate, 2-naphthalenesulfonate, 4-toluenesulfonate, camphorate, camphorsulfonate, 4-methylbicyclo[2.2.2]-oct-2-ene-1-carboxylate, glucoheptonate, 3-phenylpropionate, trimethylacetate, *tert*-butylacetate, lauryl sulfate, gluconate, benzoate, glutamate, hydroxynaphthoate, salicylate, stearate, cyclohexylsulfamate, quinate, muconate and the like.

The term "pure" or "purified" with respect to a compound provided herein includes a composition that includes at least 75%, 80%, 85%, 90%, 95%, 96%, 97%, 98%, 99%, 99.5%, 99.8%, 99.9% to 100% by weight, of the compound, the remainder comprising other chemical species or diastereomers. As used herein the term "pure" when applied to a chiral compound, refers to an enantiomer or a diastereomer of the chiral compound substantially free from its opposite enantiomer or diastereomer (*i.e*., in enantiomeric or diastereomeric excess). For example, the pure "R" form of a compound is substantially free from the "S" form of the compound and is, thus, in enantiomeric or diastereomeric excess of the "S" form. The term "enantiomerically or diastereomerically pure" or "pure enantiomer or diastereomer" denotes that the compound comprises an excess of an enantiomer or diastereomer, e.g. more than 75% by weight, more than 80% by weight, more than 85% by weight, more than 90% by weight, more than 91 % by weight, more than 92% by weight, more than 93% by weight, more than 94% by weight, more than 95% by weight, more than 96% by weight, more than 97% by weight, more than 98% by weight, more than 98.5% by weight, more than 99% by weight, more than 99.2% by weight, more than 99.5% by weight, more than 99.6% by weight, more than 99.7% by weight, more than 99.8% by weight or more than 99.9% by weight, of the enantiomer or diastereomer. In certain embodiments, the weights are based upon total weight of the compound, i.e. all enantiomers or diastereomers of the compound. In certain embodiments, one enantiomer or diastereomer can be in excess by 30-80%, or by 30-70%, 30-60%, 30%, 35%, 40%, 45%, 50%, 55% or 60%, or any percentage in between.

Similarly, the term "isolated" with respect to a compound includes a composition that includes at least 75%, 80%, 85%, 90%, 95%, 98%, 99% to 100% by weight, of the compound, the remainder comprising other chemical species or enantiomers or diastereomers.

"Solvate" includes a compound provided herein or a salt thereof, that further includes a stoichiometric or non-stoichiometric amount of solvent bound by non-covalent intermolecular forces. Where the solvent is water, the solvate is a hydrate.

The term "host", as used herein, includes any unicellular or multicellular organism in which the virus can replicate, including cell lines and animals, and preferably a human. Alternatively, the host can be carrying a part of the *Flaviviridae* viral genome, whose replication or function can be altered by the compounds provided herein. The term host specifically includes infected cells, cells transfected with all or part of the *Flaviviridae* genome and animals, in particular, primates (including chimpanzees) and humans. In most animal applications, the host is a human patient. Veterinary applications, in certain indications, however, are clearly anticipated herein (such as chimpanzees).

As used herein, the terms "subject" and "patient" are used interchangeably herein. The terms "subject" and "subjects" refer to an animal, such as a mammal including a non-primate (*e.g*., a cow, pig, horse, cat, dog, rat, and mouse) and a primate (*e.g*., a monkey such as a cynomolgous monkey, a chimpanzee and a human), and for example, a human. In one embodiment, the subject is refractory or non-responsive to current treatments for hepatitis C infection. In another embodiment, the subject is a farm animal (*e.g*., a horse, a cow, a pig, etc.) or a pet (*e.g*., a dog or a cat). In one embodiment, the subject is a human.

As used herein, the terms "therapeutic agent" and "therapeutic agents" refer to any agent(s) which can be used in the treatment or prevention of a disorder or one or more symptoms thereof. In certain embodiments, the term "therapeutic agent" includes a compound provided herein. In one embodiment, a therapeutic agent is an agent which is known to be useful for, or has been or is currently being used for the treatment or prevention of a disorder or one or more symptoms thereof.

"Therapeutically effective amount" includes an amount of a compound or composition that, when administered to a subject for treating a disease, is sufficient to effect such treatment for the disease. A "therapeutically effective amount" can vary depending on, *inter alia,* the compound, the disease and its severity, and the age, weight, *etc.,* of the subject to be treated.

"Treating" or "treatment" of any disease or disorder refers, in one embodiment, to ameliorating a disease or disorder that exists in a subject. In another embodiment, "treating" or "treatment" includes ameliorating at least one physical parameter, which may be indiscernible by the subject. In yet another embodiment, "treating" or "treatment" includes modulating the disease or disorder, either physically (*e.g*., stabilization of a discernible symptom) or physiologically (*e.g*., stabilization of a physical parameter) or both. In yet another embodiment, "treating" or "treatment" includes delaying the onset of the disease or disorder.

As used herein, the terms "prophylactic agent" and "prophylactic agents" as used refer to any agent(s) which can be used in the prevention of a disorder or one or more symptoms thereof. In certain embodiments, the term "prophylactic agent" includes a compound provided herein. In certain other embodiments, the term "prophylactic agent" does not refer a compound provided herein. For example, a prophylactic agent is an agent which is known to be useful for, or has been or is currently being used to the prevent or impede the onset, development, progression and/or severity of a disorder.

As used herein, the phrase "prophylactically effective amount" includes the amount of a therapy (*e.g*., prophylactic agent) which is sufficient to result in the prevention or reduction of the development, recurrence or onset of one or more symptoms associated with a disorder (, or to enhance or improve the prophylactic effect(s) of another therapy (*e.g*., another prophylactic agent).

As used herein, "isotopic composition" refers to the amount of each isotope present for a given atom, and "natural isotopic composition" refers to the naturally occuring isotopic composition or abundance for a given atom. Atoms containing their natural isotopic composition may also be referred to herein as "non-enriched" atoms. Unless otherwise designated, the atoms of the compounds recited herein are meant to represent any stable isotope of that atom. For example, unless otherwise stated, when a position is designated specifically as "H" or "hydrogen", the position is understood to have hydrogen at its natural isotopic composition.

As used herein, "isotopically enriched" refers to an atom having an isotopic composition other than the natural isotopic composition of that atom. "Isotopically enriched" may also refer to a compound containing at least one atom having an isotopic composition other than the natural isotopic composition of that atom.

As used herein, "isotopic enrichment" refers to the percentage of incorporation of an amount of a specific isotope at a given atom in a molecule in the place of that atom's natural isotopic abundance. For example, deuterium enrichment of 1% at a given position means that 1% of the molecules in a given sample contain deuterium at the specified position. Because the naturally occurring distribution of deuterium is about 0.0156%, deuterium enrichment at any position in a compound synthesized using non-enriched starting materials is about 0.0156%. The isotopic enrichment of the compounds provided herein can be determined using conventional analytical methods known to one of ordinary skill in the art, including mass spectrometry and nuclear magnetic resonance spectroscopy.

### Compounds

The compounds provided herein are derivatives of 2'-C-methyl guanosine. The compounds are useful in treatment and/or prophylaxis of *Flaviviridae* and hepatitis C infections.

In one embodiment, the compounds provided herein are diastereomers or metabolites of (3-hydroxy-2,2-dimethyl-thiopropionic acid S-(2-{[(2R,3R,4R,5R)-5-(2-amino-6-oxo-1,6-dihydro-purin-9-yl)-3,4-dihydroxy-4-methyl-tetrahydro-furan-2-ylmethoxy]-benzylamino-phosphoryloxy}-ethyl) ester), designated herein as Compound 1. Compound 1 has the following structure:

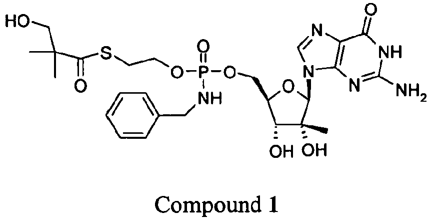

In one embodiment, provided herein is Compound 1a having formula: 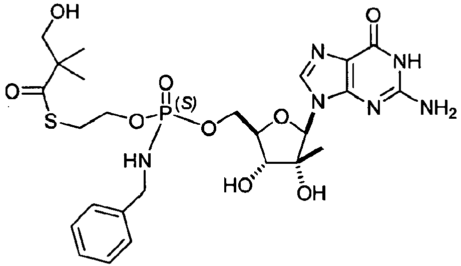 or a pharmaceutically acceptable salt, stereoisomer, solvate or hydrate thereof. In one embodiment, provided herein is pure Compound 1a. In another embodiment, provided herein is diastereomerically pure Compound 1a.

In one embodiment, provided herein is Compound 1b having formula: 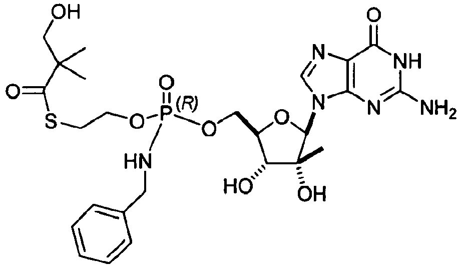 or a pharmaceutically acceptable salt, stereoisomer, solvate or hydrate thereof. In one embodiment, provided herein is pure Compound 1b. In another embodiment, provided herein is diastereomerically pure Compound 1b.

In one embodiment, provided herein is a compound of formula I: 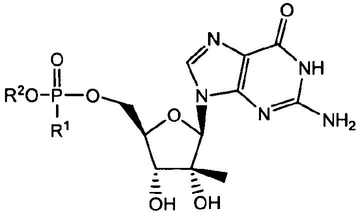 or a pharmaceutically acceptable salt, solvate, hydrate, a stereoisomeric or tautomeric form thereof, wherein R¹ is hydroxyl, amino or benzylamino; and R² is hydrogen, 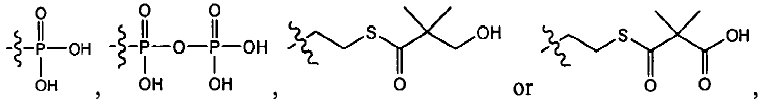 such that when R¹ is hydroxyl, R² is other than hydrogen, 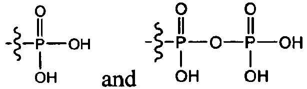 and when R¹ is benzylamino, then R² is other than

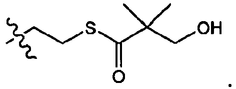

In one embodiment, provided herein is a pure compound having formula I.

In one embodiment, provided herein is a compound of formula Ia: 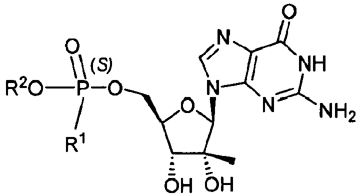 or a pharmaceutically acceptable salt, solvate, hydrate, a stereoisomeric or tautomeric form thereof.

In one embodiment, provided herein is a compound of formula Ib: 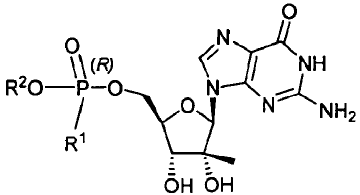 or a pharmaceutically acceptable salt, solvate, hydrate, a stereoisomeric or tautomeric form thereof. In one embodiment, provided herein is pure compound having formula Ia or Ib. In one embodiment, provided herein is an diastereomerically pure compound having formula Ia or Ib.

In one embodiment, R¹ is hydroxyl, amino or benzylamino. In another embodiment, R¹ is amino or benzylamino. In another embodiment, R¹ is hydroxy.

In one embodiment, R² is hydrogen,

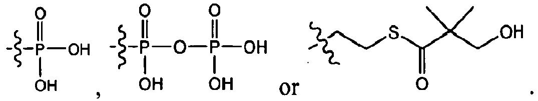

In another embodiment, R² is

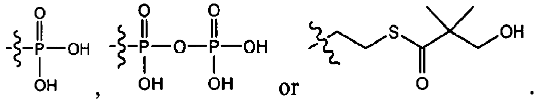

In one embodiment, R² is hydrogen.

In one embodiment, the compound provided herein has formula II: 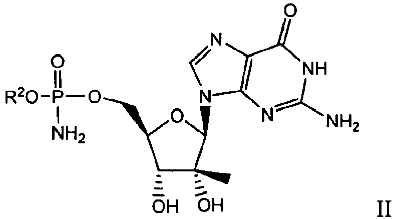 or a pharmaceutically acceptable salt, solvate, hydrate, a stereoisomeric or tautomeric form thereof, where R² is hydrogen,

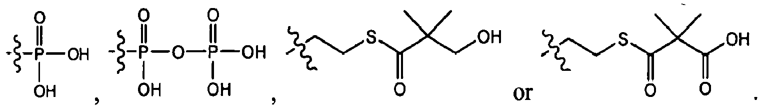

In one embodiment, provided herein is a pure compound having formula II.

In one embodiment, the compound provided herein has formula IIa: 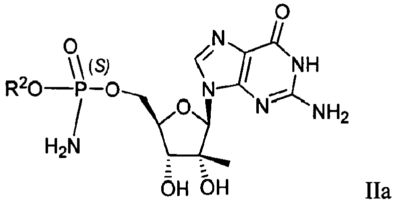 or a pharmaceutically acceptable salt, solvate, hydrate, a stereoisomeric or tautomeric form thereof.

In one embodiment, the compound provided herein has formula IIb: 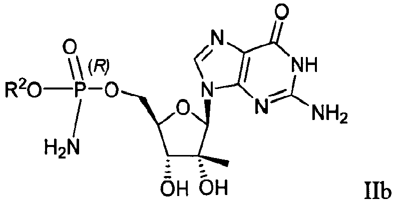 or a pharmaceutically acceptable salt, solvate, hydrate, a stereoisomeric or tautomeric form thereof. In one embodiment, provided herein is a pure compound having formula IIa or IIb. In one embodiment, provided herein is an diastereomerically pure compound having formula IIa or IIb.

In one embodiment, the compound provided herein is a compound of formula III: 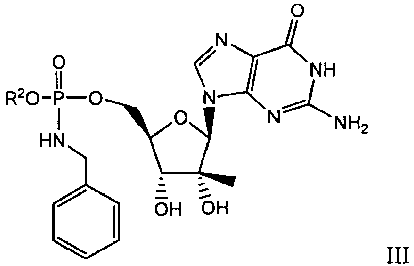 or a pharmaceutically acceptable salt, solvate, hydrate, a stereoisomeric or tautomeric form thereof, wherein R² is hydrogen,

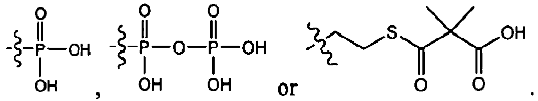

In one embodiment, provided herein is a pure compound having formula III.

In one embodiment, the compound provided herein is a compound of formula IIIa: 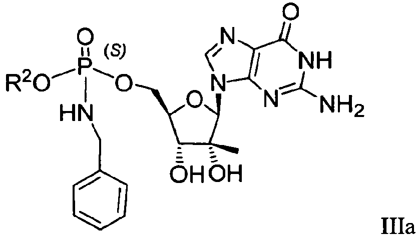 or a pharmaceutically acceptable salt, solvate, hydrate, a stereoisomeric or tautomeric form thereof.

In one embodiment, the compound provided herein is a compound of formula IIIb: 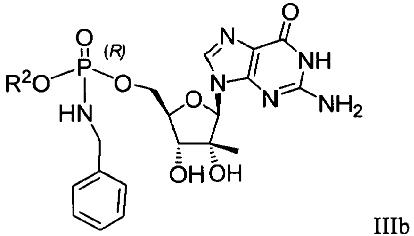 or a pharmaceutically acceptable salt, solvate, hydrate, a stereoisomeric or tautomeric form thereof. In one embodiment, provided herein is a pure compound having formula IIIa or IIIb. In one embodiment, provided herein is a diastereomerically pure compound having formula IIIa or IIIb.

In one embodiment, the compound provided herein is a compound of formula IV: 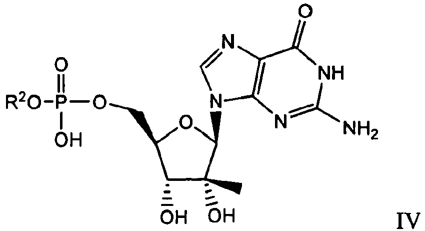 or a pharmaceutically acceptable salt, solvate, hydrate, a stereoisomeric or tautomeric form thereof, wherein R² is hydrogen,

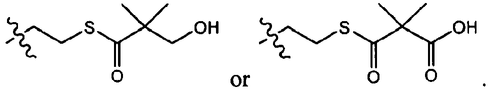

In one embodiment, provided herein is a pure compound having formula IV.

In one embodiment, the compound provided herein is a compound of formula V: 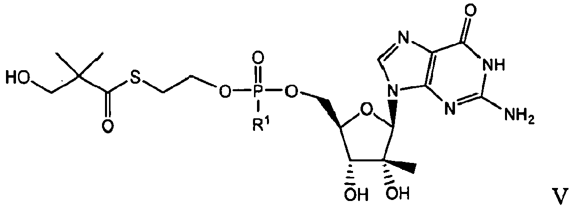 or a pharmaceutically acceptable salt, solvate, hydrate, a stereoisomeric or tautomeric form thereof, wherein R¹ is hydroxyl or amino.

In one embodiment, provided herein is a pure compound having formula V.

In one embodiment, the compound provided herein is a compound of formula Va: 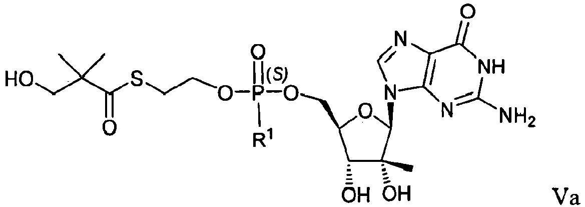 or a pharmaceutically acceptable salt, solvate, hydrate, a stereoisomeric or tautomeric form thereof.

In one embodiment, the compound provided herein is a compound of formula Vb: 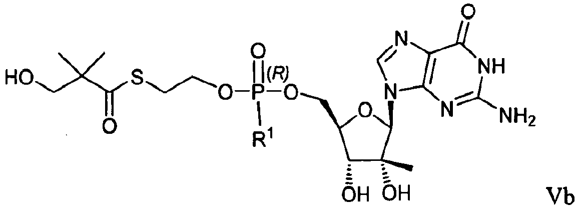 or a pharmaceutically acceptable salt, solvate, hydrate, a stereoisomeric or tautomeric form thereof. In one embodiment, provided herein is a pure compound having formula Va or Vb. In one embodiment, provided herein is a diastereomerically pure compound having formula Va or Vb.

In one embodiment, provided herein is Compound 2 having formula: 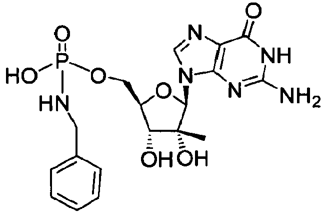 or a pharmaceutically acceptable salt, solvate, hydrate, a stereoisomeric or tautomeric form thereof. In one embodiment, provided herein is pure Compound 2.

In one embodiment, provided herein is Compound 2a having formula: 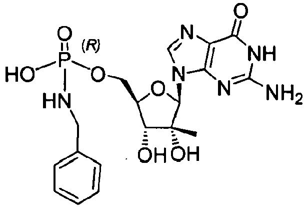 or a pharmaceutically acceptable salt, solvate, hydrate, a stereoisomeric or tautomeric form thereof.

In one embodiment, provided herein is Compound 2b having formula: 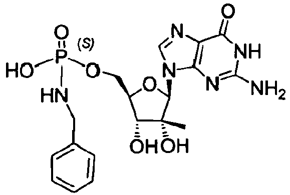 or a pharmaceutically acceptable salt, solvate, hydrate, a stereoisomeric or tautomeric form thereof. In one embodiment, provided herein is pure Compound 2a or 2b. In another embodiment, provided herein is diastereomerically pure Compound 2a or 2b.

In one embodiment, provided herein is Compound 3 having formula: 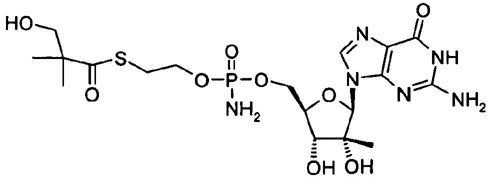 or a pharmaceutically acceptable salt, solvate, hydrate, a stereoisomeric or tautomeric form thereof. In another embodiment, provided herein is pure Compound 3.

In one embodiment, provided herein is Compound 3a having formula: 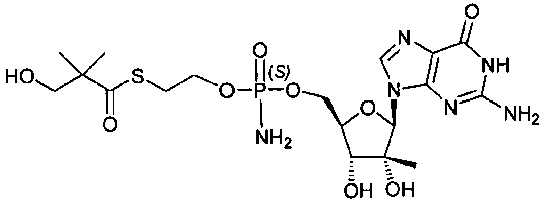 or a pharmaceutically acceptable salt, solvate, hydrate, a stereoisomeric or tautomeric form thereof.

In one embodiment, provided herein is Compound 3b having formula: 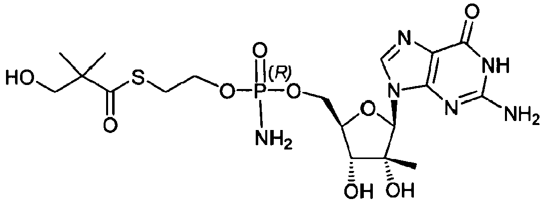 or a pharmaceutically acceptable salt, solvate, hydrate, a stereoisomeric or tautomeric form thereof. In one embodiment, provided herein is pure Compound 3a or 3b. In another embodiment, provided herein is diastereomerically pure Compound 3a or 3b.

In one embodiment, provided herein is Compound 4 having formula: 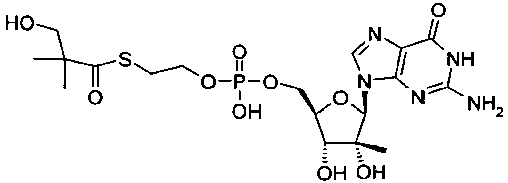 or a pharmaceutically acceptable salt, solvate, hydrate, a stereoisomeric or tautomeric form thereof. In another embodiment, provided herein is pure Compound 4.

In one embodiment, provided herein is Compound 5 having formula: 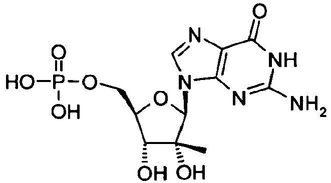 or a pharmaceutically acceptable salt, solvate, hydrate, or tautomeric form thereof. In one embodiment, provided herein is pure Compound 5.

In one embodiment, provided herein is Compound 6 having formula: 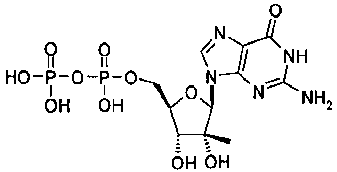 or a pharmaceutically acceptable salt, solvate, hydrate, a stereoisomeric or tautomeric form thereof. In one embodiment, provided herein is pure compound 6.

In one embodiment, provided herein is Compound 7 having formula: 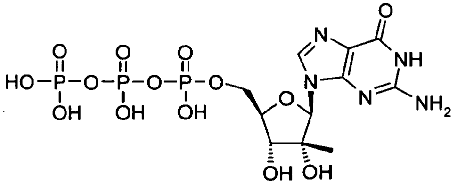 or a pharmaceutically acceptable salt, solvate, hydrate, a stereoisomeric or tautomeric form thereof. In one embodiment, provided herein is pure Compound 7.

In one embodiment, provided herein is Compound 8 having formula: 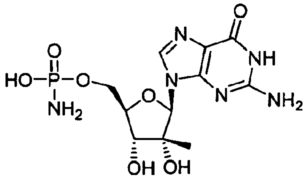 or a pharmaceutically acceptable salt, solvate, hydrate, or tautomeric form thereof. In another embodiment, provided herein is pure Compound 8.

In one embodiment, provided herein is Compound 9 having formula: 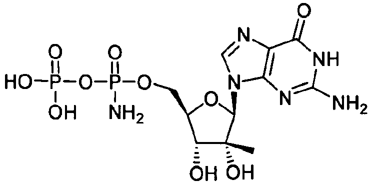 or a pharmaceutically acceptable salt, solvate, hydrate, a stereoisomeric or tautomeric form thereof. In another embodiment, provided herein is pure Compound 9.

In one embodiment, provided herein is Compound 9a having formula: 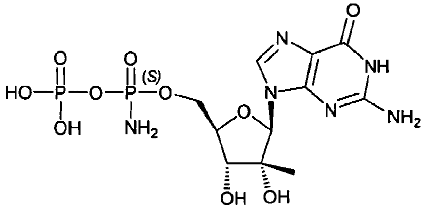 or a pharmaceutically acceptable salt, solvate, hydrate, a stereoisomeric or tautomeric form thereof. In another embodiment, provided herein is pure Compound 9a. In another embodiment, provided herein is diastereomerically pure Compound 9a.

In one embodiment, provided herein is Compound 9b having formula: 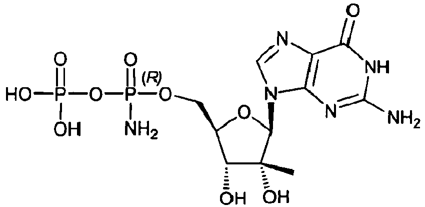 or a pharmaceutically acceptable salt, solvate, hydrate, a stereoisomeric or tautomeric form thereof. In another embodiment, provided herein is pure Compound 9b. In another embodiment, provided herein is diastereomerically pure Compound 9b.

In one embodiment, provided herein is Compound 10 of formula: 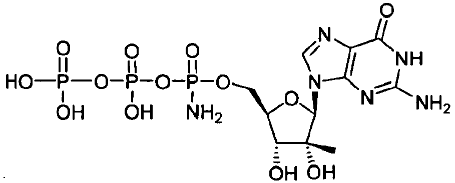 or a pharmaceutically acceptable salt, solvate, hydrate, a stereoisomeric or tautomeric form thereof. In another embodiment, provided herein is pure Compound 10.

In one embodiment, provided herein is Compound 10a having formula: 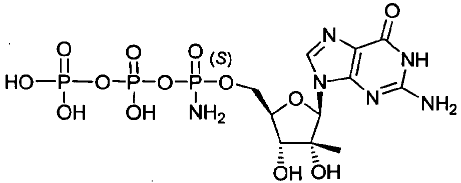 or a pharmaceutically acceptable salt, solvate, hydrate, a stereoisomeric or tautomeric form thereof. In another embodiment, provided herein is pure Compound 10a. In another embodiment, provided herein is diastereomerically pure Compound 10a.

In one embodiment, provided herein is Compound 10b having formula: 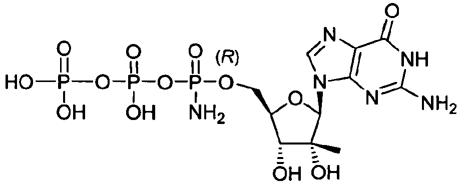 or a pharmaceutically acceptable salt, solvate, hydrate, a stereoisomeric or tautomeric form thereof. In another embodiment, provided herein is pure Compound 10b. In another embodiment, provided herein is diastereomerically pure Compound 10b.

In one embodiment, provided herein is Compound 11 having formula: 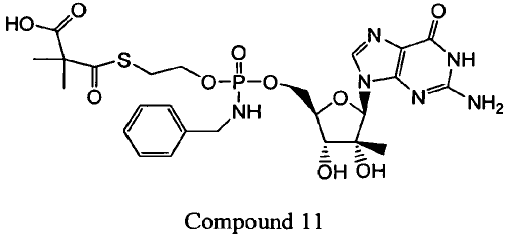 or a pharmaceutically acceptable salt, solvate, hydrate, a stereoisomeric or tautomeric form thereof. In one embodiment, provided herein is pure Compound 11.

In one embodiment, provided herein is Compound 11a having formula: 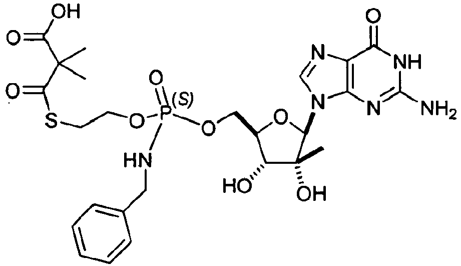 or a pharmaceutically acceptable salt, solvate, hydrate, a stereoisomeric or tautomeric form thereof.

In one embodiment, provided herein is Compound 11b having formula:

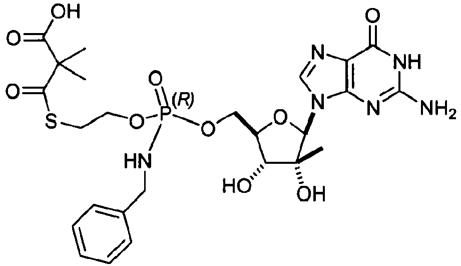

or a pharmaceutically acceptable salt, solvate, hydrate, a stereoisomeric or tautomeric form thereof. In one embodiment, provided herein is pure Compound 11a or 11 b. In another embodiment, provided herein is diastereomerically pure Compound 11a or 11b.

In certain embodiments, provided herein is an isotopically enriched compound selected from the group consisting of isotopically enriched compound of formula I, isotopically enriched compound of formula Ia, isotopically enriched compound of formula Ib, isotopically enriched compound of formula II, isotopically enriched compound of formula IIa, isotopically enriched compound of formula IIb, isotopically enriched compound of formula III, isotopically enriched compound of formula IIIa, isotopically enriched compound of formula IIIb, isotopically enriched compound of formula IV, isotopically enriched compound of formula V, isotopically enriched compound of formula Va, and isotopically enriched compound of formula Vb.

In certain embodiments, provided herein is an isotopically enriched compound selected from the group consisting of isotopically enriched compound of formula VI, isotopically enriched compound of formula VIa, and isotopically enriched compound of formula VIb.

In certain embodiments, provided herein is an isotopically enriched compound selected from the group consisting of isotopically enriched compound 1, isotopically enriched compound 1a, isotopically enriched compound 1b, isotopically enriched compound 2, isotopically enriched compound 2a, isotopically enriched compound 2b, isotopically enriched compound 3, isotopically enriched compound 3a, isotopically enriched compound 3b, isotopically enriched compound 4, isotopically enriched compound 5, isotopically enriched compound 6, isotopically enriched compound 7, isotopically enriched compound 8, isotopically enriched compound 8a, isotopically enriched compound 8b, isotopically enriched compound 9, isotopically enriched compound 9a, isotopically enriched compound 9b, isotopically enriched compound 10, isotopically enriched compound 10a, isotopically enriched compound 10b, isotopically enriched compound 11, isotopically enriched compound 11a, and isotopically enriched compound 11b.

In one embodiment, provided herein is a compound, solvate, hydrate, a stereoisomeric or tautomeric form thereof, wherein the compound is a metabolite of the compound having the formula 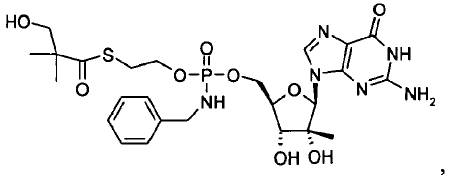 and wherein the compound is selected from the group consisting of:
(a) a compound which elutes off a C-18 (2) 4.6 x 250 mm 5 µm particle size column, at about 2.1 minutes;
(b) a compound which elutes off a C-18 (2) 4.6 x 250 mm 5 µm particle size column, at about 6.2 minutes,
(c) a compound which elutes off a C-18 (2) 4.6 x 250 mm 5 µm particle size column, at about 8.0 minutes,
(d) a compound which elutes off a C-18 (2) 4.6 x 250 mm 5 µm particle size column, at about 9.4 minutes,
(e) a compound which elutes off a C-18 (2) 4.6 x 250 mm 5 µm particle size column, at about 10.9 minutes,
(f) a compound which elutes off a C-18 (2) 4.6 x 250 mm 5 µm particle size column, at about 12.4 minutes,
(g) a compound which elutes off a C-18 (2) 4.6 x 250 mm 5 µm particle size column, at about 13.1 minutes,
(h) a compound which elutes off a C-18 (2) 4.6 x 250 mm 5 µm particle size column, at about 17.1 minutes,
(i) a compound which elutes off a C-18 (2) 4.6 x 250 mm 5 µm particle size column, at about 25.2 minutes, and
(j) a compound which elutes off a C-18 (2) 4.6 x 250 mm 5 µm particle size column, at about 26.5 minutes, where the retention times described are obtained in HPLC method 1 as described in Example 16.

In one embodiment, provided herein is a compound, solvate, hydrate, a stereoisomeric or tautomeric form thereof, wherein the compound is a metabolite of the compound having the formula 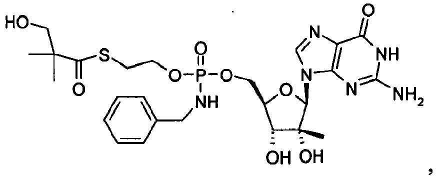 and wherein the compound elutes off a C-18 4.6 x 250 mm 5 µm particle size column, at about 16.4 minutes, in HPLC method 2 described in Example 16.

In one embodiment, provided herein is a compound, solvate, hydrate, a stereoisomeric or tautomeric form thereof, wherein the compound is a metabolite of the compound having the formula 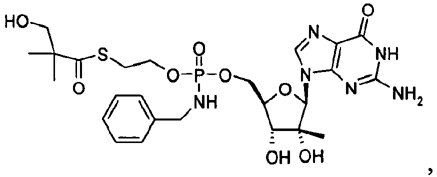 and wherein the compound is selected from the group consisting of:
(a) a compound which elutes off a C-18 4.6 x 250 mm 5 µm particle size column, at about 38.4 minutes;
(b) a compound which elutes off a C-18 4.6 x 250 mm 5 µm particle size column, at about 39.8 minutes,
(c) a compound which elutes off a C-18 4.6 x 250 mm 5 µm particle size column, at about 36.8 minutes,
(d) a compound which elutes off a C-18 4.6 x 250 mm 5 µm particle size column, at about 26.8 minutes,
(e) a compound which elutes off a C-18 4.6 x 250 mm 5 µm particle size column, at about 33.8 minutes,
(f) a compound which elutes off a C-18 4.6 x 250 mm 5 µm particle size column, at about 30.9 minutes,
(g) a compound which elutes off a C-18 4.6 x 250 mm 5 µm particle size column, at about 4.6 minutes, and
(h) a compound which elutes off a C-18 4.6 x 250 mm 5 µm particle size column, at about 28.1 minutes, where the retention times described are obtained in HPLC method 1 as described in Example 15.

In certain embodiments, the compounds provided herein are of formula VI: 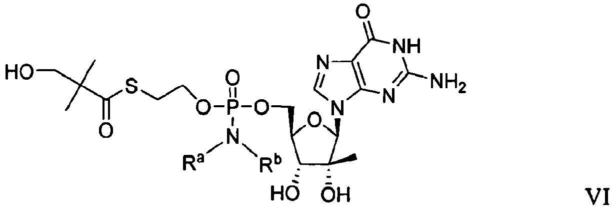 or a pharmaceutically acceptable salt, solvate, hydrate, a stereoisomeric, or tautomeric form thereof, wherein R^{a} and R^{b} are selected as follows:
i) R^{a} is hydrogen; and R^{b} is alkyl, carboxyalkyl, alkoxycarbonylalkyl or dialkylaminoalkyl; or
ii) R^{a} and R^{b} together with the nitrogen atom on which they are substituted form a 3-7 membered heterocyclic, optionally substituted with one or two alkyl groups.

In certain embodiments, the compounds provided herein are of formula VIa 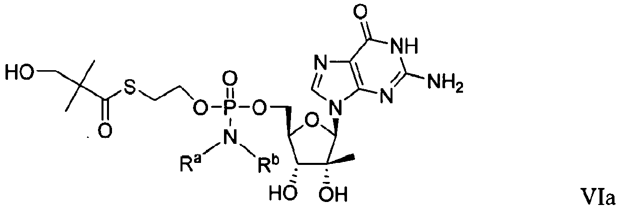 or a pharmaceutically acceptable salt, solvate, hydrate, a stereoisomeric, or tautomeric form thereof, wherein the variables are as described elsewhere herein.

In certain embodiments, the compounds provided herein are of formula VIb 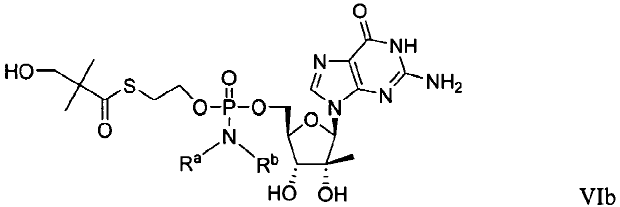 or a pharmaceutically acceptable salt, solvate, hydrate, a stereoisomeric, or tautomeric form thereof, wherein the variables are as described elsewhere herein.

In one embodiment, R^{a} is hydrogen; and R^{b} is isopropyl, *t*-butyl, cyclohexyl, ethoxycarbonylmethyl, *t*-butyloxycarbonylmethyl, carboxymethyl or dimethylaminoethyl. In one embodiment, R^{a} and R^{b} together with the nitrogen atom on which they are substituted form a 4-methylpiperazine or morpholine ring.

In certain embodiments, the compound provided herein is selected from: 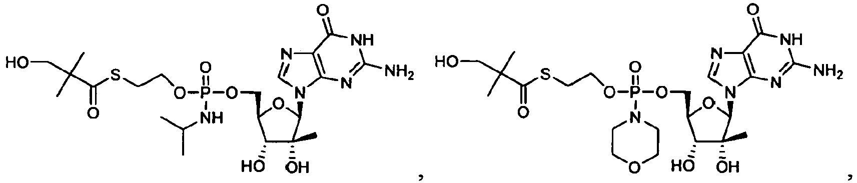 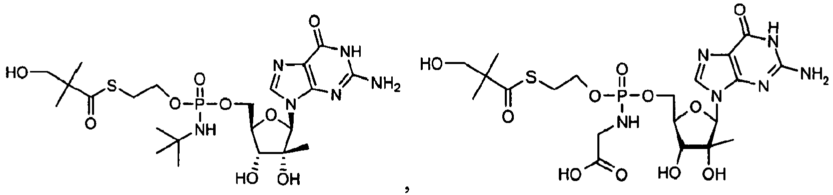 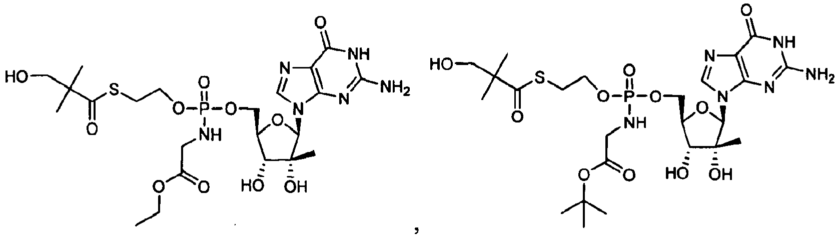 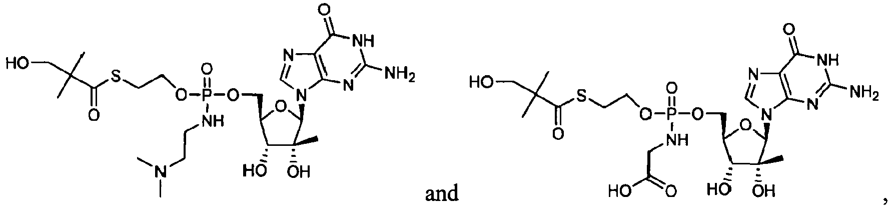 or a pharmaceutically acceptable salt, solvate, hydrate, a stereoisomeric, or tautomeric form thereof. In certain embodiments, the compound provided herein is an isotopically enriched compound according to this paragraph.

In certain embodiments, the compound provided herein is selected from 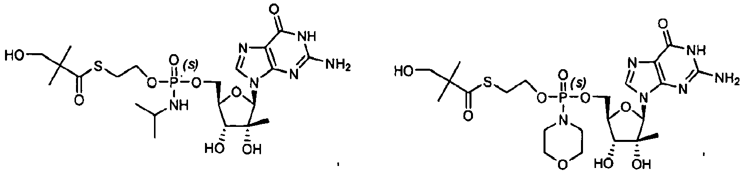 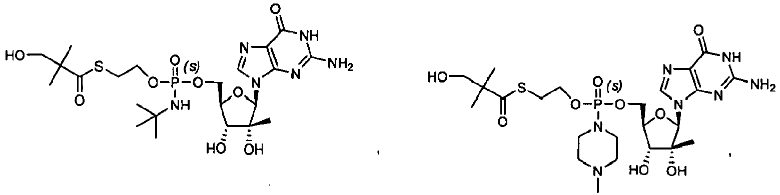 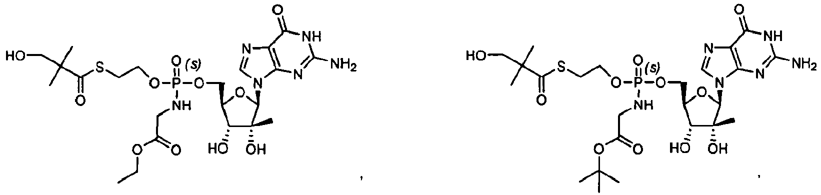 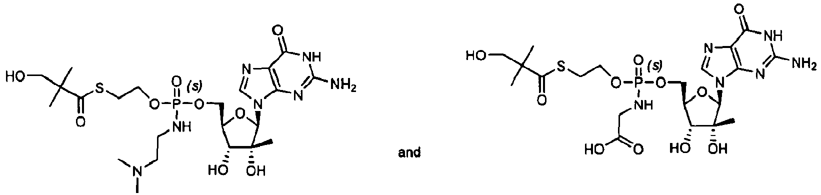 a pharmaceutically acceptable salt, solvate, hydrate, a stereoisomeric, or tautomeric form thereof according to this paragraph.

In certain embodiments, the compound provided herein is selected from 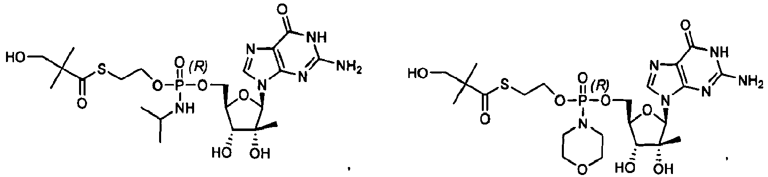 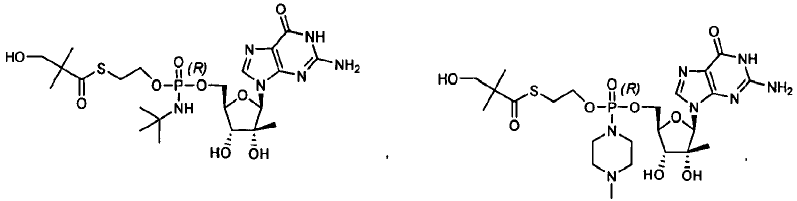 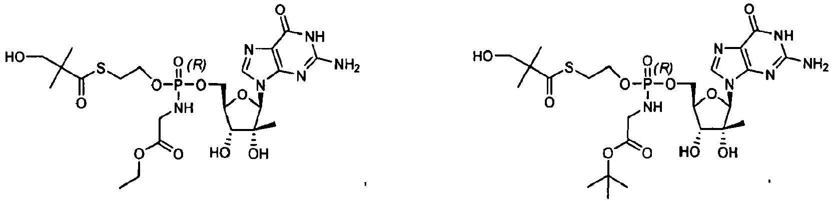 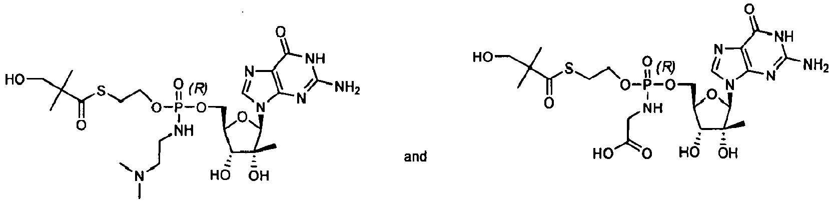 a pharmaceutically acceptable salt, solvate, hydrate, a stereoisomeric, or tautomeric form thereof according to this paragraph.

In certain embodiments, the compound provided herein is a diastereomerically pure compound or a pharmaceutically acceptable salt, solvate, hydrate, ester thereof. In certain embodiments, the compound provided herein is a diastereomerically pure compound or a pharmaceutically acceptable salt thereof. In certain embodiments, the diastereomerically pure compound comprises at least about 80% by weight of the designated diastereomer and at most about 20% by weight of the other stereoisomer(s), at least about 90% by weight of the designated diastereomer and at most about 10% by weight of the other stereoisomer(s), at least about 95% by weight of the designated diastereomer and at most about 5% by weight of the other stereoisomer(s), at least about 96.6% by weight of the designated diastereomer and at most about 3.4% by weight of the other stereoisomer(s), at least about 97% by weight of the designated diastereomer and at most about 3% by weight of the other stereoisomer(s), at least about 99% by weight of the designated diastereomer and at most about 1% by weight of the other stereoisomer(s), or at least about 99.9% by weight of the designated diastereomer and at most about 0.1% by weight of the other stereoisomer(s). In certain embodiments, the weights are based upon total weight of the compound.

In one embodiment, the compounds provided herein are present in a substantially pure form.

In certain embodiments, provided are compounds that may be given as salts, or esters that, upon administration to the recipient, provide directly or indirectly a compound provided herein or that exhibits the desired activity itself.

Also provided herein are isotopically enriched analogs of the compounds provided herein. Isotopic enrichment (for example, deuteration) of pharmaceuticals to improve pharmacokinetics ("PK"), pharmacodynamics ("PD"), and toxicity profiles, has been demonstrated previously with some classes of drugs. See, for example, Lijinsky et. al., Food Cosmet. Toxicol., 20: 393 (1982); Lijinsky et. al., J. Nat. Cancer Inst., 69: 1127 (1982); Mangold et. al., Mutation Res. 308: 33 (1994); Gordon et. al., Drug Metab. Dispos., 15: 589 (1987); Zello et. al., Metabolism, 43: 487 (1994); Gately et. al., J. Nucl. Med., 27: 388 (1986); Wade D, Chem. Biol. Interact. 117: 191 (1999).

Isotopic enrichment of a drug can be used, for example, to (1) reduce or eliminate unwanted metabolites, (2) increase the half-life of the parent drug, (3) decrease the number of doses needed to achieve a desired effect, (4) decrease the amount of a dose necessary to achieve a desired effect, (5) increase the formation of active metabolites, if any are formed, and/or (6) decrease the production of deleterious metabolites in specific tissues and/or create a more effective drug and/or a safer drug for combination therapy, whether the combination therapy is intentional or not.

Replacement of an atom for one of its isotopes often will result in a change in the reaction rate of a chemical reaction. This phenomenon is known as the Kinetic Isotope Effect ("KIE"). For example, if a C-H bond is broken during a rate-determining step in a chemical reaction (*i.e*. the step with the highest transition state energy), substitution of a deuterium for that hydrogen will cause a decrease in the reaction rate and the process will slow down. This phenomenon is known as the Deuterium Kinetic Isotope Effect ("DKIE"). (*See, e.g,* Foster et al., Adv. Drug Res., vol. 14, pp. 1-36 (1985); Kushner et al., Can. J. Physiol. Pharmacol., vol. 77, pp. 79-88 (1999)).

The magnitude of the DKIE can be expressed as the ratio between the rates of a given reaction in which a C-H bond is broken, and the same reaction where deuterium is substituted for hydrogen. The DKIE can range from about 1 (no isotope effect) to very large numbers, such as 50 or more, meaning that the reaction can be fifty, or more, times slower when deuterium is substituted for hydrogen. High DKIE values may be due in part to a phenomenon known as tunneling, which is a consequence of the uncertainty principle. Tunneling is ascribed to the small mass of a hydrogen atom, and occurs because transition states involving a proton can sometimes form in the absence of the required activation energy. Because deuterium has more mass than hydrogen, it statistically has a much lower probability of undergoing this phenomenon.

Tritium ("T") is a radioactive isotope of hydrogen, used in research, fusion reactors, neutron generators and radiopharmaceuticals. Tritium is a hydrogen atom that has 2 neutrons in the nucleus and has an atomic weight close to 3. It occurs naturally in the environment in very low concentrations, most commonly found as T₂O. Tritium decays slowly (half-life = 12.3 years) and emits a low energy beta particle that cannot penetrate the outer layer of human skin. Internal exposure is the main hazard associated with this isotope, yet it must be ingested in large amounts to pose a significant health risk. As compared with deuterium, a lesser amount of tritium must be consumed before it reaches a hazardous level. Substitution of tritium ("T") for hydrogen results in yet a stronger bond than deuterium and gives numerically larger isotope effects. Similarly, substitution of isotopes for other elements, including, but not limited to, ¹³C or ¹⁴C for carbon, ³³S, ³⁴S, or ³⁶S for sulfur, ¹⁵N for nitrogen, and ¹⁷O or ¹⁸O for oxygen, will provide a similar kinetic isotope effects.

For example, the DKIE was used to decrease the hepatotoxicity of halothane by presumably limiting the production of reactive species such as trifluoroacetyl chloride. However, this method may not be applicable to all drug classes. For example, deuterium incorporation can lead to metabolic switching. The concept of metabolic switching asserts that xenogens, when sequestered by Phase I enzymes, may bind transiently and re-bind in a variety of conformations prior to the chemical reaction (e.g., oxidation). This hypothesis is supported by the relatively vast size of binding pockets in many Phase I enzymes and the promiscuous nature of many metabolic reactions. Metabolic switching can potentially lead to different proportions of known metabolites as well as altogether new metabolites. This new metabolic profile may impart more or less toxicity.

The animal body expresses a variety of enzymes for the purpose of eliminating foreign substances, such as therapeutic agents, from its circulation system. Examples of such enzymes include the cytochrome P450 enzymes ("CYPs"), esterases, proteases, reductases, dehydrogenases, and monoamine oxidases, to react with and convert these foreign substances to more polar intermediates or metabolites for renal excretion. Some of the most common metabolic reactions of pharmaceutical compounds involve the oxidation of a carbon-hydrogen (C-H) bond to either a carbon-oxygen (C-0) or carbon-carbon (C-C) pi-bond. The resultant metabolites may be stable or unstable under physiological conditions, and can have substantially different pharmacokinetic, pharmacodynamic, and acute and long-term toxicity profiles relative to the parent compounds. For many drugs, such oxidations are rapid. These drugs therefore often require the administration of multiple or high daily doses.

Therefore, isotopic enrichment at certain positions of a compound provided herein will produce a detectable KIE that will affect the pharmacokinetic, pharmacologic, and/or toxicological profiles of a compound provided herein in comparison with a similar compound having a natural isotopic composition.

### Preparation of Compounds

The compounds provided herein can be prepared, isolated or obtained by any method apparent to those of skill in the art. Exemplary methods of preparation are described in detail in the examples below. Compound 1 can be prepared by methods described in US application no. 12/005,937, filed December 27, 2007.

It is appreciated that compounds provided herein have several chiral centers and may exist in and be isolated in optically active and diastereomeric forms. Some compounds may exhibit polymorphism. It is to be understood that any racemic, optically-active, diastereomeric, polymorphic, or stereoisomeric form, or mixtures thereof, of a compound provided herein, which possess the useful properties described herein is within the scope of the claimed subject matter. It being well known in the art how to prepare optically active forms (for example, by resolution of the racemic form by recrystallization techniques, by synthesis from optically-active starting materials, by chiral synthesis, or by chromatographic separation using a chiral stationary phase).

Examples of methods to obtain diastereomerically pure materials are known in the art, and include at least the following and any combination thereof:
i) fractional crystallization - a technique whereby the diastereomers are separated by fractional crystallization by virtue of the difference in their solubilities;
ii) fractional distillation - a technique whereby the diastereomers are separated by fractional distillation by virtue of the difference in their boiling point;
iii) chromatographic separation - a technique whereby the diastereomers are separated in a liquid mobile phase by virtue of their differing interactions with a stationary phase;
iv) chemical asymmetric synthesis - a synthetic technique whereby the desired diastereomer is synthesized from an achiral precursor under conditions that produce asymmetry (*i.e*., chirality) in the product, which may be achieved using chiral catalysts or chiral auxiliaries;

The compounds provided herein may be prepared by one of the techniques described herein or by a combination of the techniques, where necessary.

### Assay Methods

Compounds can be assayed for HCV activity according to any assay known to those of skill in the art. Further, compounds can be assayed for accumulation in liver cells of a subject according to any assay known to those of skill in the art. In certain embodiments, a compound can be administered to the subject, and a liver cell of the subject can be assayed for the compound or a derivative thereof, *e.g*. a nucleoside, nucleoside phosphate or nucleoside triphosphate derivative thereof.

In one embodiment, a compound provided herein is administered to cells, such as liver cells, *in vivo* or *in vitro,* and the nucleoside triphosphate levels delivered intracellularly are measured, to indicate delivery of the corresponding compound and triphosphorylation in the cell. The level of intracellular nucleoside triphosphate can be measured using analytical techniques known in the art.

### Methods of Use

In one embodiment, the compounds provided herein can have enhanced delivery to the liver. In some embodiments, the compounds permit delivery of an active 5'-monophosphate of a nucleoside to the liver, which can enhance the formation of active triphosphorylated compound.

In one embodiment, provided herein are methods for the treatment and/or prophylaxis of a host infected with *Flaviviridae* that includes the administration of an effective amount of a compounds provided herein, or a pharmaceutically acceptable salt, stereoisomer, solvate or hydrate thereof. In one embodiment, provided herein are methods for treating an HCV infection in a subject. In certain embodiments, the methods encompass the step of administering to the subject in need thereof an amount of a compound effective for the treatment or prevention of an HCV infection in combination with a second agent effective for the treatment or prevention of the infection. The compound can be any compound as described herein, and the second agent can be any second agent described in the art or herein. In certain embodiments, the compound is in the form of a pharmaceutical composition or dosage form, as described in the sections above.

*Flaviviridae* that can be treated are discussed generally in Fields Virology, Editors: Fields, B. N., Knipe, D. M., and Howley, P. M., Lippincott-Raven Publishers, Philadelphia, PA, Chapter 31, 1996. In a particular embodiment, the *Flaviviridae* is HCV. In an alternate embodiment, the *Flaviviridae* is a flavivirus or pestivirus. Specific flaviviruses include, without limitation: Absettarov, Alfuy, Apoi, Aroa, Bagaza, Banzi, Bouboui, Bussuquara, Cacipacore, Carey Island, Dakar bat, Dengue 1, Dengue 2, Dengue 3, Dengue 4, Edge Hill, Entebbe bat, Gadgets Gully, Hanzalova, Hypr, Ilheus, Israel turkey meningoencephalitis, Japanese encephalitis, Jugra, Jutiapa, Kadam, Karshi, Kedougou, Kokobera, Koutango, Kumlinge, Kunjin, Kyasanur Forest disease, Langat, Louping ill, Meaban, Modoc, Montana myotis leukoencephalitis, Murray valley encephalitis, Naranjal, Negishi, Ntaya, Omsk hemorrhagic fever, Phnom-Penh bat, Powassan, Rio Bravo, Rocio, Royal Farm, Russian spring-summer encephalitis, Saboya, St. Louis encephalitis, Sal Vieja, San Perlita, Saumarez Reef, Sepik, Sokuluk, Spondweni, Stratford, Tembusu, Tyuleniy, Uganda S, Usutu, Wesselsbron, West Nile, Yaounde, Yellow fever, and Zika.

Pestiviruses that can be treated are discussed generally in Fields Virology, Editors: Fields, B. N., Knipe, D. M., and Howley, P. M., Lippincott-Raven Publishers, Philadelphia, PA, Chapter 33, 1996. Specific pestiviruses include, without limitation: bovine viral diarrhea virus ("BVDV"), classical swine fever virus ("CSFV," also called hog cholera virus), and border disease virus ("BDV").

In certain embodiments, the subject can be any subject infected with, or at risk for infection with, HCV. Infection or risk for infection can be determined according to any technique deemed suitable by the practitioner of skill in the art. In one embodiment, subjects are humans infected with HCV and/or HBV.

In certain embodiments, the subject has never received therapy or prophylaxis for an HCV and/or HBV infection. In further embodiments, the subject has previously received therapy or prophylaxis for an HCV and/or HBV infection. For instance, in certain embodiments, the subject has not responded to an HCV and/or HBV therapy. For example, under current interferon therapy, up to 50% or more HCV subjects do not respond to therapy. In certain embodiments, the subject can be a subject that received therapy but continued to suffer from viral infection or one or more symptoms thereof. In certain embodiments, the subject can be a subject that received therapy but failed to achieve a sustained virologic response. In certain embodiments, the subject has received therapy for an HCV infection but has failed to show, for example, a 2 log₁₀ decline in HCV RNA levels after 12 weeks of therapy. It is believed that subjects who have not shown more than 2 log₁₀ reduction in serum HCV RNA after 12 weeks of therapy have a 97-100% chance of not responding.

In certain embodiments, the subject is a subject that discontinued an HCV therapy because of one or more adverse events associated with the therapy. In certain embodiments, the subject is a subject where current therapy is not indicated. For instance, certain therapies for HCV are associated with neuropsychiatric events. Interferon (IFN)-alfa plus ribavirin is associated with a high rate of depression. Depressive symptoms have been linked to a worse outcome in a number of medical disorders. Life-threatening or fatal neuropsychiatric events, including suicide, suicidal and homicidal ideation, depression, relapse of drug addiction/overdose, and aggressive behavior have occurred in subjects with and without a previous psychiatric disorder during HCV therapy. Interferon-induced depression is a limitation for the treatment of chronic hepatitis C, especially for subjects with psychiatric disorders. Psychiatric side effects are common with interferon therapy and responsible for about 10% to 20% of discontinuations of current therapy for HCV infection.

Accordingly, provided are methods of treating or preventing an HCV infection in subjects where the risk of neuropsychiatric events, such as depression, contraindicates treatment with current HCV therapy. In one embodiment, provided are methods of treating or preventing HCV infection in subjects where a neuropsychiatric event, such as depression, or risk of such indicates discontinuation of treatment with current HCV therapy. Further provided are methods of treating or preventing HCV infection in subjects where a neuropsychiatric event, such as depression, or risk of such indicates dose reduction of current HCV therapy.

Current therapy is also contraindicated in subjects that are hypersensitive to interferon or ribavirin, or both, or any other component of a pharmaceutical product for administration of interferon or ribavirin. Current therapy is not indicated in subjects with hemoglobinopathies (*e.g*., thalassemia major, sickle-cell anemia) and other subjects at risk from the hematologic side effects of current therapy. Common hematologic side effects include bone marrow suppression, neutropenia and thrombocytopenia. Furthermore, ribavirin is toxic to red blood cells and is associated with hemolysis. Accordingly, in one embodiment, provided are methods of treating or preventing HCV infection in subjects hypersensitive to interferon or ribavirin, or both, subjects with a hemoglobinopathy, for instance thalassemia major subjects and sickle-cell anemia subjects, and other subjects at risk from the hematologic side effects of current therapy.

In certain embodiments, the subject has received an HCV therapy and discontinued that therapy prior to administration of a method provided herein. In further embodiments, the subject has received therapy and continues to receive that therapy along with administration of a method provided herein. The methods can be co-administered with other therapy for HCV according to the judgment of one of skill in the art. In certain embodiments, the methods or compositions provided herein can be co-administered with a reduced dose of the other therapy for HCV.

In certain embodiments, provided are methods of treating a subject that is refractory to treatment with interferon. For instance, in some embodiments, the subject can be a subject that has failed to respond to treatment with one or more agents selected from the group consisting of interferon, interferon α, pegylated interferon α, interferon plus ribavirin, interferon α plus ribavirin and pegylated interferon α plus ribavirin. In some embodiments, the subject can be a subject that has responded poorly to treatment with one or more agents selected from the group consisting of interferon, interferon α, pegylated interferon α, interferon plus ribavirin, interferon α plus ribavirin and pegylated interferon α plus ribavirin. A pro-drug form of ribavirin, such as taribavirin, may also be used.

In certain embodiments, the subject has, or is at risk for, co-infection of HCV with HIV. For instance, in the United States, 30% of HIV subjects are co-infected with HCV and evidence indicates that people infected with HIV have a much more rapid course of their hepatitis C infection. Maier and Wu, 2002, World J Gastroenterol 8:577-57. The methods provided herein can be used to treat or prevent HCV infection in such subjects. It is believed that elimination of HCV in these subjects will lower mortality due to end-stage liver disease. Indeed, the risk of progressive liver disease is higher in subjects with severe AIDS-defining immunodeficiency than in those without. *See, e.g.,* Lesens et al., 1999, J Infect Dis 179:1254-1258. In one embodiment, compounds provided herein have been shown to suppress HIV in HIV subjects. Thus, in certain embodiments, provided are methods of treating or preventing HIV infection and HCV infection in subjects in need thereof.

In certain embodiments, the compounds or compositions are administered to a subject following liver transplant. Hepatitis C is a leading cause of liver transplantation in the U.S, and many subjects that undergo liver transplantation remain HCV positive following transplantation. In one embodiment, provided are methods of treating such recurrent HCV subjects with a compound or composition provided herein. In certain embodiments, provided are methods of treating a subject before, during or following liver transplant to prevent recurrent HCV infection.

In certain embodiments, the compounds of formula I provided herein are useful as markers or standards for assessing the metabolism of compound 1 in a subject, including a human.

### Second Therapeutic Agents

In certain embodiments, the compounds and compositions provided herein can be used in a method of treating a liver disorder, comprising administering an effective amount of a compound provided herein and further administering an effective amount of a second agent effective treating the disorder, such as HCV infection, to a subject in need thereof. The second agent can be any agent known to those of skill in the art to be effective for the treatment of the disorder, including those currently approved by the FDA.

In certain embodiments, a compound provided herein is administered in combination with one second agent. In further embodiments, a compound provided herein is administered in combination with two second agents. In still further embodiments, a compound provided herein is administered in combination with two or more second agents.

As used herein, the term "in combination" includes the use of more than one therapy (*e.g*., one or more prophylactic and/or therapeutic agents). The use of the term "in combination" does not restrict the order in which therapies (*e.g*., prophylactic and/or therapeutic agents) are administered to a subject in need thereof. For instance, a first therapy (*e.g.*, a prophylactic or therapeutic agent such as a compound provided herein) can be administered prior to (*e.g*., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g*., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy (*e.g*., a prophylactic or therapeutic agent) to a subject with a disorder.

As used herein, the term "synergistic" includes a combination of a compound provided herein and another therapy (*e.g*., a prophylactic or therapeutic agent) which has been or is currently being used to prevent, manage or treat a disorder, which is more effective than the additive effects of the individual therapies. A synergistic effect of a combination of therapies (*e.g*., a combination of prophylactic or therapeutic agents) can permit the use of lower dosages of one or more of the therapies and/or less frequent administration of said therapies to a subject in need thereof. The ability to utilize lower dosages of a therapy (*e.g*., a prophylactic or therapeutic agent) and/or to administer said therapy less frequently can reduce any toxicity associated with the administration of said therapy to a subject without reducing the efficacy of said therapy in the prevention or treatment of a disorder. In addition, a synergistic effect can result in improved efficacy of agents in the prevention or treatment of a disorder. Finally, a synergistic effect of a combination of therapies (*e.g*., a combination of prophylactic or therapeutic agents) can avoid or reduce adverse or unwanted side effects associated with the use of either therapy alone.

The active compounds provided herein can be administered in combination with another therapeutic agent, in particular an anti-HCV or hepatitis B agent. The active compounds can be administered at doses selected by a practitioner of skill in the art. For instance, the dosages given can depend on absorption, inactivation and excretion rates of the drug as well as other factors known to those of skill in the art. It is to be noted that dosages can also vary with the severity of the condition to be alleviated. It is to be further understood that for any particular subject, specific dosages and schedules can be adjusted over time according to individual need and the judgment of the practitioner administering or supervising the administration of the compositions. In certain embodiments, an anti-HCV (or anti-pestivirus or anti-flavivirus) compound that exhibits an EC₅₀ of 10-15 µM, or preferably less than 1-5 µM, is useful.

It has been recognized that drug-resistant variants of flaviviruses, pestiviruses or HCV can emerge after prolonged treatment with an antiviral agent. Drug resistance most typically occurs by mutation of a gene that encodes for an enzyme used in viral replication. The efficacy of a drug against viral infection can be prolonged, augmented, or restored by administering the compound in combination with a second, or perhaps third, antiviral compound that induces a different mutation from that caused by the principle drug. Alternatively, the pharmacokinetics, biodistribution or other parameters of the drug can be altered by such combination therapy. In certain embodiments, concomitant administration therapy can be used because it can induce multiple simultaneous stresses on the virus.

Any of the viral treatments described in the Background section can be used in combination with the compounds described in this specification.

### Exemplary Second Agents for Treatment of HCV

In certain embodiments, one or more compounds provided herein can be administered in combination with an anti-hepatitis C virus protease inhibitors. Useful protease inhibitors include, but are not limited to, TMC435350 (Medivir/Tibotec, Huddinge, Sweden); ITMN-191 (R-7227; InterMune Pharma., Inc., Brisbane, CA), ACH-806 (GS-9132; Achillion Pharma., Inc., New Haven, CT), ACH-1095 (Achillion Pharma., Inc.), BI 12202 (Boehringer Ingelheim, Ingelheim Germany), ciluprevir (BILN-2061; Boehringer Ingelheim), MK-7009 (Merck Pharma., Inc., Whitehouse Station, NJ), boceprevir (SCH 503034; Schering-Plough, Kenilworth, NJ), SCH 446211 (SCH6; Schering-Plough), SCH 351633 (Schering-Plough), and telaprevir (VX-950; Vertex Pharma., Inc., Cambridge, MA).

In certain embodiments, useful anti-hepatitis C virus protease inhibitors include, but are not limited to, substrate-based NS3 protease inhibitors (WO 98/22496; Attwood et al., Antiviral Chemistry and Chemotherapy 1999, 10, 259-273; German Patent Pub. DE 19914474; and WO 98/17679), including alphaketoamides and hydrazinoureas, and inhibitors that terminate in an electrophile such as a boronic acid or phosphonate (WO 99/07734); non-substrate-based NS3 protease inhibitors such as 2,4,6-trihydroxy-3-nitro-benzamide derivatives (Sudo K. et al., Biochemical and Biophysical Research Communications, 1997, 238, 643-647; Sudo K. et al. Antiviral Chemistry and Chemotherapy, 1998, 9, 186), including RD3-4082 and RD3-4078, the former substituted on the amide with a 14 carbon chain and the latter processing a para-phenoxyphenyl group; and Sch 68631, a phenanthrenequinone (Chu M. et al., Tetrahedron Letters 37:7229-7232, 1996).

In certain embodiments, useful anti-hepatitis C virus protease inhibitors include, but are not limited to, eglin c (Qasim M.A. et al., Biochemistry 36:1598-1607, 1997); cysteine protease inhibitors for inhibiting HCV endopeptidase 2 (U.S. Patent No. 6,004,933); synthetic inhibitors of hepatitis C virus NS3 protease (U.S. Patent No. 5,990,276); inhibitor tripeptides (U.S. Patent Nos. 6,534,523, 6,410,531, and 6,420,380, and WO 02/060926); diaryl peptides (WO 02/48172 and U.S. Patent No. 6,911,428); and imidazoleidinones (WO 02/08198,WO 02/48157, and U.S. Patent Nos. 6,727,366 and 6,838,475).

In certain embodiments, useful anti-hepatitis C virus serine protease inhibitors include, but are not limited to, HCV serine protease inhibitors provided in U.S. Patent No. 6,872,805; WO 2006000085; U.S. Patent No. 7,208,600; U.S. Patent Pub. No. 2006/0046956; WO 2007/001406 (Chiron); U.S. Patent Pub. No. 2005/0153877; WO 2006/119061 (Merck); WO 00/09543; U.S. Patent No. 6,323,180; WO 03/064456; U.S. Patent No. 6,642,204; WO 03/064416; U.S. Patent No. 7,091,184; WO 03/053349; U.S. Patent No. 6,867,185; WO 03/099316; U.S. Patent No. 6,869,964; WO 03/099274; U.S. Patent No. 6,995,174; WO 2004/032827; U.S. Patent No. 7,041,698; WO 2004/043339, U.S. Patent No. 5,538,865; WO 02/008251; U.S. Patent No. 7,169,760; U.S. Patent Pub. No. 2005/176648; WO 02/08187; WO 02/008256; WO 98/17679; U.S. Patent No. 6,265,380; WO 02/48116; U.S. Patent No. 6,653,295; and US 6,878,722.

In certain embodiments, one or more compounds provided herein can be administered in combination with a thiazolidine derivative which shows relevant inhibition in a reverse-phase HPLC assay with an NS3/4A fusion protein and NS5A/5B substrate (Sudo K. et al., Antiviral Research, 1996, 32, 9-18). Useful thiazolidine derivative include, but are not limited to, RD-1-6250 (possessing a fused cinnamoyl moiety substituted with a long alkyl chain), RD4 6205, and RD4 6193;

In certain embodiments, one or more compounds provided herein can be administered in combination with a thiazolidine and/or a benzanilide (Kakiuchi N. et al. J. EBS Letters 421, 217-220; Takeshita N. et al. Analytical Biochemistry, 1997, 247, 242-246).

In certain embodiments, one or more compounds provided herein can be administered in combination with a phenanthrenequinone possessing activity against protease in a SDS-PAGE and autoradiography assay isolated from the fermentation culture broth of Streptomyces sp. Useful phenanthrenequinone include, but are not limited to, SCH 68631 (Chu M. et al., Tetrahedron Letters, 1996, 37, 7229-7232) and SCH 351633 (Chu M. et al., Bioorganic and Medicinal Chemistry Letters 9, 1949-1952);

In certain embodiments, one or more compounds provided herein can be administered in combination with a helicase inhibitor (U.S. Pat. No. 5,633,358; WO 97/36554);

In certain embodiments, one or more compounds provided herein can be administered in combination with a nucleotide polymerase inhibitor. Useful nucleotide polymerase inhibitors include, but are not limited to, gliotoxin (Ferrari R. et al. Journal of Virology, 1999, 73, 1649-1654), and cerulenin (Lohmann V. et al., Virology, 1998, 249, 108-118);

In certain embodiments, one or more compounds provided herein can be administered in combination with an interfering RNA (RNAi) based antivirals. Useful RNAi based antivirals include, but are not limited to, short interfering RNA (siRNA) based antivirals, such as Sirna-034 and others described in WO/03/070750, WO 2005/012525, and US Patent Publication No. US 2004/0209831, and microRNA based antivirals, such as miR-122 (Pan Q-W. et al., World J. Gastroenterol., 2007, 13, 4431-4436).

In certain embodiments, one or more compounds provided herein can be administered in combination with an antisense phosphorothioate oligodeoxynucleotide (S-ODN) complementary to sequence stretches in the 5' non-coding region (NCR) of the virus (Alt M. et al., Hepatology, 1995, 22, 707-717), or nucleotides 326-348 comprising the 3' end of the NCR and nucleotides 371-388 located in the core coding region of the HCV RNA (Alt M. et al., Archives of Virology, 1997, 142, 589-599; Galderisi U. et al., Journal of Cellular Physiology, 1999, 181, 251-257);

In certain embodiments, one or more compounds provided herein can be administered in combination with an inhibitor of IRES-dependent translation (Japanese Patent Pub. JP-08268890; Japanese Patent Pub. JP-10101591).

In certain embodiments, one or more compounds provided herein can be administered in combination with a ribozyme. Useful ribozymes include, but are not limited to, nuclease-resistant ribozymes (Maccjak, D. J. et al., Hepatology 1999, 30, abstract 995), HEPTAZYME^{®} (Ribozyme Pharma. Inc., Boulder, CO), and ribozymes disclosed in U.S. Patent Nos. 6,043,077, 5,869,253, and 5,610,054.

In certain embodiments, the compounds provided herein can be administered in combination with any of the compounds described by Idenix Pharmaceuticals in International Publication Nos. WO 01/90121, WO 01/92282, WO 2004/003000, 2004/002422, and WO 2004/002999.

In certain embodiments, one or more compounds provided herein can be administered in combination with one or more nucleoside analogs. Useful nucleoside analogs include, but are not limited to, nucleoside analogs described in PCT/CA00/01316 (WO 01/32153) and PCT/CA01/00197 (WO 01/60315); WO 02/057425); WO 02/057287; U.S. Patent Nos. 7,202,224, 7,125,855, 7,105,499, and 6,777,395; PC17EP01/09633 (WO 02/18404); U.S. Patent Pub. Nos. 2006/0040890, 2005/0038240, and 2004/0121980; U.S. Patent Nos. 6,846,810, 6,784,166, and 6,660,721; PCT Publication Nos. WO 01/79246, WO 02/32920, and WO 02/48165; US 2005/0009737; U.S. Patent Pub. No. 2005/0009737; and U.S. Patent Nos. 7,094,770 and 6,927,291.

In certain embodiments, one or more compounds provided herein can be administered in combination with one or more second agents. Useful second agents include, but are not limited to, 2'-fluoronucleosides (WO 99/43691), 1-amino-alkylcyclohexanes (U.S. Patent No. 6,034,134), alkyl lipids (U.S. Patent No. 5,922,757), vitamin E and other antioxidants (U.S. Patent No. 5,922,757), squalene, amantadine, bile acids (U.S. Patent No. 5,846,964), N-(phosphonoacetyl)-L-aspartic acid, (U.S. Patent No. 5,830,905), benzenedicarboxamides (U.S. Patent No. 5,633,388), polyadenylic acid derivatives (U.S. Patent No. 5,496,546), 2',3'-dideoxyinosine (U.S. Patent No. 5,026,687), benzimidazoles (U.S. Patent No. 5,891,874), plant extracts (U.S. Patent Nos. 5,837,257, 5,725,859, and 6,056,961), and piperidenes (U.S. Patent No. 5,830,905).

In certain embodiments, one or more compounds provided herein can be administered in combination with an anti-hepatitis C virus interferon. Useful interferons include, but are not limited to, INTRON A^{®} (interferon alfa-2b; Schering-Plough, Inc.) and PEGASYS^{®} (Peginterferon alfa-2a; Hoffmann-LaRoche, Inc., Nutley, NJ); ROFERON A^{®} (Recombinant interferon alfa-2a; Hoffmann-LaRoche, Inc.), INFERGEN^{®} (consensus interferon; interferon alfacon-1; Three Rivers Pharma., Cranberry Township, PA PEG-INTRON^{®} (pegylated interferon alfa-2b; Schering-Plough, Inc.), and PEGASYS^{®} (pegylated interferon alfa-2a; Hoffmann-LaRoche, Inc.).

In certain embodiments, useful anti-hepatitis C virus interferons include, but are not limited to, INFERGEN^{®}, IL-29 (PEG-Interferon lambda; ZymoGenetics, Inc., Seattle, WA), ACTIMMUNE^{®} (interferon gamma-1b; Intermune, Inc.), R7025 (Maxy-alpha; Maxygen, Redwood City, CA), BELEROFON (Nautilus Biotech., Evry, France), Oral Interferon alpha (Amarillo Biosciences, Inc., Amarillo, TX), LOCTERON^{®} (BLX-883; OctoPlus, Inc., Cambridge, MA), omega interferon (Intarcia Therapeutics, Inc. Hayward, CA), MULTIFERON^{®} (Viragen, Inc., Plantation, FL), OMNIFERON^{™} (Viragen, Inc.), medusa interferon (Flamel Technologies, Inc., Venissieux Cedex, France), WELLFERON^{®} (GlaxoSmithKline, Philadelphia, PA), ALBUFERON^{®} (Human Genome Sciences, Inc., Rockville, MD), REBETRON^{®} (Schering-Plough, Inc.), and REBIF^{®} (interferon β-1a; Serono, Inc., Rockland, MA).

In certain embodiments, one or more compounds provided herein can be administered in combination with an anti-hepatitis C virus polymerase inhibitor. Useful polymerase inhibitors include, but are not limited to, REBETOL^{®} (ribavirin; Schering-Plough, Inc.), levovirin (ICN Pharma., Costa Mesa, CA), VIRAMIDINE^{®} (Valeant Pharma. International, Aliso Viejo, CA), MK-0608 (7-deaza-2'-C-methyladenosine; Merck Pharma. Inc.), 7-deaza-MK-0608 (7-deaza-7-fluoro-2'-C-methyladenosine; Merck Pharma. Inc.), NM 283 (valopicitabine; Idenix Pharma., Inc., Cambridge, MA), PSI-6130 (2'-deoxy-2'-fluoro-2'-C-methylcytidine; Hoffmann-LaRoche, Inc.), 2'-O-methylcytidine (Carroll, S.S. et al., J Biol. Chem., 2003, 278, 11979-11984), 2'-C-methyladenosine (Tomassini, J.E. et al., Antimicrob. Agents Chemother., 2005, 49, 2050-2058; Migliaccio, G. et al., J Biol. Chem., 2003, 278, 49164-49170), 2'-C-methyl guanosine (Migliaccio, G. et al., J. Biol. Chem., 2003, 278, 49164-49170; Eldrup, A.B. et al., J Med. Chem., 2004, 47, 2283-2295), R1479 (4'-azidocytidine; Hoffmann-LaRoche, Inc.), ANA598 (Anadys Pharma. Inc., San Diego, CA), R1626 (Hoffmann-LaRoche, Inc.), RO-0622 (4'-azido-2'-deoxynucleoside analog, Roche Palo Alto, LLC, Palo Alto, CA), GL-59728 (Genelabs Technologies, Inc., Redwood City, CA), GL-60667 (Genelabs Technologies, Inc.), and R7128 (Pharmasset, Inc., Princeton, NJ).

In certain embodiments, one or more compounds provided herein can be administered in combination with an anti-hepatitis C virus non-nucleoside polymerase inhibitor. Useful non-nucleoside polymerase inhibitors include, but are not limited to, BILB1941 (Boehringer Ingelheim), HCV-796 (ViroPharma, Inc., Exton, PA), DKA compound 30 (Summa, V. et al., J Med. Chem., 2004, 47, 14-17; Summa, V. et al., J Med Chem., 2004, 47, 5336-5339), a benzimidazole 5-carboxamide derivative (Beaulieu, P.L. et al., Bioorg. Med. Chem. Lett., 2004, 14, 967-971), an indole-N-acetamide derivative (Di Marco, S. et al., J. Biol. Chem., 2005, 280, 29765-29770; Harper, S. et al., J. Med Chem., 2005, 48, 1314-1317), a benzothiadiazine derivative (Dhanak, D. et al., J Biol. Chem., 2002, 277, 38322-38327; Tomei, L. et al., J. Virol., 2004, 78, 938-946), a phenylalanine derivative (Wang, M. et al., J Biol. Chem., 2003, 278, 9489-9495), a thiophene 2-carboxylic acid derivative (Chan, L. et al., Bioorg. Med. Chem. Lett., 2004, 14, 793-796; Biswal, B.K. et al., J Biol. Chem., 2005, 280, 18202-18210), a dihydropyrone derivative (De Clercq, E., Nat. Rev. Drug Dis., 2007, 6, 1001-1018), the tetrahydropyranoindolyl acetic acid derivative HCV-371 (Howe, A.Y. et al., Antimicrob. Agents Chemother., 2004, 48, 4813-4821), and a series of 5-hydroxy-3(2H)-pyridazinones (Zhou, Y. et al., Antiviral Res., 2007, 74, A38, abstract 27; Zhou, Y. et al., Antiviral Res., 2007, 74, A51-A52, abstract 59).

In certain embodiments, one or more compounds provided herein can be administered in combination with ribavarin and an anti-hepatitis C virus interferon. Useful interferons include, but are not limited to, INTRON A^{®} (interferon alfa-2b) and PEGASYS^{®} (Peginterferon alfa-2a); ROFERON A^{®} (Recombinant interferon alfa-2a), INFERGEN^{®} (consensus interferon; interferon alfacon-1), PEG-INTRON^{®} (pegylated interferon alfa-2b) and PEGASYS^{®} (pegylated interferon alfa-2a).

In certain embodiments, one or more compounds provided herein can be administered in combination with an anti-hepatitis C virus cyclophilin B inhibitor. Useful cyclophilin B inhibitors include, but are not limited to, NIM-811 (Novartis, East Hanover, NJ), cyclosporin A (CsA; Novartis), SCY-635 (Scynexis, Inc., Research Triangle Park, NC), and DEBIO-025 (Debiopharm Group, Lausanne, Switzerland).

In certain embodiments, one or more compounds provided herein can be administered in combination with an anti-hepatitis C virus α-glucosidase inhibitor. Useful α-glucosidase inhibitors include, but are not limited to, celgosivir (Migenix, Inc., Vancouver, Canada).

In certain embodiments, one or more compounds provided herein can be administered in combination with an anti-hepatitis C virus vaccine. Useful vaccines include, but are not limited to, TG4040, PeviPROTM, CGI-5005, HCV/MF59, GV1001, IC41, and INNO0101 (E1).

In certain embodiments, one or more compounds provided herein can be administered in combination with an anti-hepatitis C virus monoclonal or polyclonal antibodies. Useful monoclonal antibodies include, but are not limited to, AB68 and XTL-6865 (formerly HepX-C; XTL Biopharma., Rehovot, Israel). Useful polyclonal antibodies include, but are not limited to, cicavir.

In certain embodiments, one or more compounds provided herein can be administered in combination with an anti-hepatitis C virus immunomodulator. Useful immunomodulators include, but are not limited to, ZADAXIN^{®} (thymalfasin; SciClone Pharma. International, Foster City, CA), CEPLENE^{™} (Maxim Pharma. Inc., San Diego, CA), CELLCEPT^{®} (Hoffmann-LaRoche, Inc.), CIVACIR^{®} (Nabi Biopharma., Rockville, MD), CPG 10101 (Pfizer, Inc., New York, NY), ANA773 (Anadys Pharma. Inc.), ANA971 (Anadys Pharma. Inc.), ANA975 (Anadys Pharma. Inc.), NOV-205 (Novelos Therapeutics, Inc., Newton, MA), and Oglufanide (Implicit Bioscience, Inc., Woodside, CA).

In certain embodiments, one or more compounds provided herein can be administered in combination with other second agents. Useful second agents include, but are not limited to, Nexavar, doxorubicin, PI-88, amantadine, JBK-122, VGX-410C, MX-3253 (Ceglosivir), Suvus (BIVN-401 or virostat), PF-03491390 (formerly IDN-6556), G126270, UT-231B, EMZ702, ACH-0137171, MitoQ, ANA975, AVI-4065, Bavituxinab (Tarvacin), Alinia (nitrazoxanide), merimepodib (VX-497; Vertex Pharma., Inc.), summetrel (Endo Pharma. Holdings, Inc., Chadds Ford, PA), ISIS 14803 (Isis Pharma., Inc., Carlsbad, CA), and PYN17.

### Pharmaceutical Compositions and Methods of Administration

The compounds provided herein can be formulated into pharmaceutical compositions using methods available in the art and those disclosed herein. Such compounds can be used in some embodiments to enhance delivery of the drug to the liver.

In certain embodiments, a second agent can be formulated or packaged with the compound provided herein. Of course, the second agent will only be formulated with the compound provided herein when, according to the judgment of those of skill in the art, such co-formulation should not interfere with the activity of either agent or the method of administration. In certain embodiments, the compound provided herein and the second agent are formulated separately. They can be packaged together, or packaged separately, for the convenience of the practitioner of skill in the art.

In clinical practice the active agents provided herein may be administered by any conventional route, in particular orally, parenterally, rectally or by inhalation (e.g. in the form of aerosols). In certain embodiments, the compound provided herein is administered orally.

Use may be made, as solid compositions for oral administration, of tablets, pills, hard gelatin capsules, powders or granules. In these compositions, the active product is mixed with one or more inert diluents or adjuvants, such as sucrose, lactose or starch.

These compositions can comprise substances other than diluents, for example a lubricant, such as magnesium stearate, or a coating intended for controlled release.

Use may be made, as liquid compositions for oral administration, of solutions which are pharmaceutically acceptable, suspensions, emulsions, syrups and elixirs containing inert diluents, such as water or liquid paraffin. These compositions can also comprise substances other than diluents, for example wetting, sweetening or flavoring products.

The compositions for parenteral administration can be emulsions or sterile solutions. Use may be made, as solvent or vehicle, of propylene glycol, a polyethylene glycol, vegetable oils, in particular olive oil, or injectable organic esters, for example ethyl oleate. These compositions can also contain adjuvants, in particular wetting, isotonizing, emulsifying, dispersing and stabilizing agents. Sterilization can be carried out in several ways, for example using a bacteriological filter, by radiation or by heating. They can also be prepared in the form of sterile solid compositions which can be dissolved at the time of use in sterile water or any other injectable sterile medium.

The compositions for rectal administration are suppositories or rectal capsules which contain, in addition to the active principle, excipients such as cocoa butter, semisynthetic glycerides or polyethylene glycols.

The compositions can also be aerosols. For use in the form of liquid aerosols, the compositions can be stable sterile solutions or solid compositions dissolved at the time of use in apyrogenic sterile water, in saline or any other pharmaceutically acceptable vehicle. For use in the form of dry aerosols intended to be directly inhaled, the active principle is finely divided and combined with a water-soluble solid diluent or vehicle, for example dextran, mannitol or lactose.

In one embodiment, a composition provided herein is a pharmaceutical composition or a single unit dosage form. Pharmaceutical compositions and single unit dosage forms provided herein comprise a prophylactically or therapeutically effective amount of one or more prophylactic or therapeutic agents (*e.*g., a compound provided herein, or other prophylactic or therapeutic agent), and a typically one or more pharmaceutically acceptable carriers or excipients. In a specific embodiment and in this context, the term "pharmaceutically acceptable" means approved by a regulatory agency of the Federal or a state government or listed in the U.S. Pharmacopeia or other generally recognized pharmacopeia for use in animals, and more particularly in humans. The term "carrier" includes a diluent, adjuvant (*e.g*., Freund's adjuvant (complete and incomplete)), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water can be used as a carrier when the pharmaceutical composition is administered intravenously. Saline solutions and aqueous dextrose and glycerol solutions can also be employed as liquid carriers, particularly for injectable solutions. Examples of suitable pharmaceutical carriers are described in "Remington's Pharmaceutical Sciences" by E.W. Martin.

Typical pharmaceutical compositions and dosage forms comprise one or more excipients. Suitable excipients are well-known to those skilled in the art of pharmacy, and non limiting examples of suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Whether a particular excipient is suitable for incorporation into a pharmaceutical composition or dosage form depends on a variety of factors well known in the art including, but not limited to, the way in which the dosage form will be administered to a subject and the specific active ingredients in the dosage form. The composition or single unit dosage form, if desired, can also contain minor amounts of wetting or emulsifying agents, or pH buffering agents.

Lactose free compositions provided herein can comprise excipients that are well known in the art and are listed, for example, in the U.S. Pharmocopia (USP) SP (XXI)/NF (XVI). In general, lactose free compositions comprise an active ingredient, a binder/filler, and a lubricant in pharmaceutically compatible and pharmaceutically acceptable amounts. Exemplary lactose free dosage forms comprise an active ingredient, microcrystalline cellulose, pre gelatinized starch, and magnesium stearate.

Further encompassed herein are anhydrous pharmaceutical compositions and dosage forms comprising active ingredients, since water can facilitate the degradation of some compounds. For example, the addition of water (*e.g*., 5%) is widely accepted in the pharmaceutical arts as a means of simulating long term storage in order to determine characteristics such as shelf life or the stability of formulations over time. See, e.g., Jens T. Carstensen, Drug Stability: Principles & Practice, 2d. Ed., Marcel Dekker, NY, NY, 1995, pp. 379 80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms provided herein can be prepared using anhydrous or low moisture containing ingredients and low moisture or low humidity conditions. Pharmaceutical compositions and dosage forms that comprise lactose and at least one active ingredient that comprises a primary or secondary amine can be anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions can be packaged using materials known to prevent exposure to water such that they can be included in suitable formulary kits. Examples of suitable packaging include, but are not limited to, hermetically sealed foils, plastics, unit dose containers (*e.g*., vials), blister packs, and strip packs.

Further provided are pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers.

The pharmaceutical compositions and single unit dosage forms can take the form of solutions, suspensions, emulsion, tablets, pills, capsules, powders, sustained-release formulations and the like. Oral formulation can include standard carriers such as pharmaceutical grades of mannitol, lactose, starch, magnesium stearate, sodium saccharine, cellulose, magnesium carbonate, etc. Such compositions and dosage forms will contain a prophylactically or therapeutically effective amount of a prophylactic or therapeutic agent, in certain embodiments, in purified form, together with a suitable amount of carrier so as to provide the form for proper administration to the subject. The formulation should suit the mode of administration. In a certain embodiment, the pharmaceutical compositions or single unit dosage forms are sterile and in suitable form for administration to a subject, for example, an animal subject, such as a mammalian subject, for example, a human subject.

A pharmaceutical composition is formulated to be compatible with its intended route of administration. Examples of routes of administration include, but are not limited to, parenteral, *e.g*., intravenous, intradermal, subcutaneous, intramuscular, subcutaneous, oral, buccal, sublingual, inhalation, intranasal, transdermal, topical, transmucosal, intra-tumoral, intra-synovial and rectal administration. In a specific embodiment, the composition is formulated in accordance with routine procedures as a pharmaceutical composition adapted for intravenous, subcutaneous, intramuscular, oral, intranasal or topical administration to human beings. In an embodiment, a pharmaceutical composition is formulated in accordance with routine procedures for subcutaneous administration to human beings. Typically, compositions for intravenous administration are solutions in sterile isotonic aqueous buffer. Where necessary, the composition may also include a solubilizing agent and a local anesthetic such as lignocamne to ease pain at the site of the injection.

Examples of dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (*e.g*., nasal sprays or inhalers); gels; liquid dosage forms suitable for oral or mucosal administration to a subject, including suspensions (*e.g*., aqueous or non aqueous liquid suspensions, oil in water emulsions, or a water in oil liquid emulsions), solutions, and elixirs; liquid dosage forms suitable for parenteral administration to a subject; and sterile solids (*e.g.*, crystalline or amorphous solids) that can be reconstituted to provide liquid dosage forms suitable for parenteral administration to a subject.

The composition, shape, and type of dosage forms provided herein will typically vary depending on their use. For example, a dosage form used in the initial treatment of viral infection may contain larger amounts of one or more of the active ingredients it comprises than a dosage form used in the maintenance treatment of the same infection. Similarly, a parenteral dosage form may contain smaller amounts of one or more of the active ingredients it comprises than an oral dosage form used to treat the same disease or disorder. These and other ways in which specific dosage forms encompassed herein will vary from one another will be readily apparent to those skilled in the art. See, *e.g*., Remington's Pharmaceutical Sciences, 20th ed., Mack Publishing, Easton PA (2000).

Generally, the ingredients of compositions are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

Typical dosage forms comprise a compound provided herein, or a pharmaceutically acceptable salt, solvate or hydrate thereof lie within the range of from about 0.1 mg to about 1000 mg per day, given as a single once-a-day dose in the morning or as divided doses throughout the day taken with food. Particular dosage forms can have about 0.1, 0.2, 0.3, 0.4, 0.5, 1.0, 2.0, 2.5, 5.0, 10.0, 15.0, 20.0, 25.0, 50.0, 100, 200, 250, 500 or 1000 mg of the active compound.

### Oral Dosage Forms

Pharmaceutical compositions that are suitable for oral administration can be presented as discrete dosage forms, such as, but are not limited to, tablets (*e.g*., chewable tablets), caplets, capsules, and liquids (*e.g*., flavored syrups). Such dosage forms contain predetermined amounts of active ingredients, and may be prepared by methods of pharmacy well known to those skilled in the art. See generally, Remington's Pharmaceutical Sciences, 20th ed., Mack Publishing, Easton PA (2000).

In certain embodiments, the oral dosage forms are solid and prepared under anhydrous conditions with anhydrous ingredients, as described in detail in the sections above. However, the scope of the compositions provided herein extends beyond anhydrous, solid oral dosage forms. As such, further forms are described herein.

Typical oral dosage forms are prepared by combining the active ingredient(s) in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (*e.g*., powders, tablets, capsules, and caplets) include, but are not limited to, starches, sugars, micro crystalline cellulose, diluents, granulating agents, lubricants, binders, and disintegrating agents.

Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free flowing form such as powder or granules, optionally mixed with an excipient. Molded tablets can be made by molding in a suitable machine a mixture of the powdered compound moistened with an inert liquid diluent.

Examples of excipients that can be used in oral dosage forms include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (*e.g*., ethyl cellulose, cellulose acetate, carboxymethyl cellulose calcium, sodium carboxymethyl cellulose), polyvinyl pyrrolidone, methyl cellulose, pre gelatinized starch, hydroxypropyl methyl cellulose, (*e.g*., Nos. 2208, 2906, 2910), microcrystalline cellulose, and mixtures thereof.

Examples of fillers suitable for use in the pharmaceutical compositions and dosage forms disclosed herein include, but are not limited to, talc, calcium carbonate (*e.g*., granules or powder), microcrystalline cellulose, powdered cellulose, dextrates, kaolin, mannitol, silicic acid, sorbitol, starch, pre gelatinized starch, and mixtures thereof. The binder or filler in pharmaceutical compositions is typically present in from about 50 to about 99 weight percent of the pharmaceutical composition or dosage form.

Suitable forms of microcrystalline cellulose include, but are not limited to, the materials sold as AVICEL PH 101, AVICEL PH 103 AVICEL RC 581, AVICEL PH 105 (available from FMC Corporation, American Viscose Division, Avicel Sales, Marcus Hook, PA), and mixtures thereof. An specific binder is a mixture of microcrystalline cellulose and sodium carboxymethyl cellulose sold as AVICEL RC 581. Suitable anhydrous or low moisture excipients or additives include AVICEL PH 103™ and Starch 1500 LM.

Disintegrants are used in the compositions to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms. The amount of disintegrant used varies based upon the type of formulation, and is readily discernible to those of ordinary skill in the art. Typical pharmaceutical compositions comprise from about 0.5 to about 15 weight percent of disintegrant, specifically from about 1 to about 5 weight percent of disintegrant.

Disintegrants that can be used in pharmaceutical compositions and dosage forms include, but are not limited to, agar agar, alginic acid, calcium carbonate, microcrystalline cellulose, croscarmellose sodium, crospovidone, polacrilin potassium, sodium starch glycolate, potato or tapioca starch, pre gelatinized starch, other starches, clays, other algins, other celluloses, gums, and mixtures thereof.

Lubricants that can be used in pharmaceutical compositions and dosage forms include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (*e.g*., peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL 200, manufactured by W.R. Grace Co. of Baltimore, MD), a coagulated aerosol of synthetic silica (marketed by Degussa Co. of Plano, TX), CAB O SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated.

In an exemplary embodiment, Compound 1 is dissolved/dispersed in polyethylene glycol and sodium lauryl sulfate and filled into hypromellose hard capsules. In one embodiment, the capsule comprises about 1-150 mg of Compound 1. In another embodiment, the capsule comprises about 5-50 mg of Compound 1. In another embodiment, the capsule comprises about 5 mg of Compound 1. In another embodiment, the capsule comprises about 25 mg of Compound 1.

### Delayed Release Dosage Forms

Active ingredients such as the compounds provided herein can be administered by controlled release means or by delivery devices that are well known to those of ordinary skill in the art. Examples include, but are not limited to, those described in U.S. Patent Nos.: 3,845,770; 3,916,899; 3,536,809; 3,598,123; and 4,008,719; 5,674,533; 5,059,595; 5,591,767; 5,120,548; 5,073,543; 5,639,476; 5,354,556; 5,639,480; 5,733,566; 5,739,108; 5,891,474; 5,922,356; 5,972,891; 5,980,945; 5,993,855; 6,045,830; 6,087,324; 6,113,943; 6,197,350; 6,248,363; 6,264,970; 6,267,981; 6,376,461; 6,419,961; 6,589,548; 6,613,358; 6,699,500 each of which is incorporated herein by reference. Such dosage forms can be used to provide slow or controlled release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled release formulations known to those of ordinary skill in the art, including those described herein, can be readily selected for use with the active ingredients provided herein. Thus encompasseed herein are single unit dosage forms suitable for oral administration such as, but not limited to, tablets, capsules, gelcaps, and caplets that are adapted for controlled release.

All controlled release pharmaceutical products have a common goal of improving drug therapy over that achieved by their non controlled counterparts. Ideally, the use of an optimally designed controlled release preparation in medical treatment is characterized by a minimum of drug substance being employed to cure or control the condition in a minimum amount of time. Advantages of controlled release formulations include extended activity of the drug, reduced dosage frequency, and increased subject compliance. In addition, controlled release formulations can be used to affect the time of onset of action or other characteristics, such as blood levels of the drug, and can thus affect the occurrence of side (*e.g*., adverse) effects.

Most controlled release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled release of an active ingredient can be stimulated by various conditions including, but not limited to, pH, temperature, enzymes, water, or other physiological conditions or compounds.

In certain embodiments, the drug may be administered using intravenous infusion, an implantable osmotic pump, a transdermal patch, liposomes, or other modes of administration. In one embodiment, a pump may be used (*see,* Sefton, CRC Crit. Ref Biomed. Eng. 14:201 (1987); Buchwald et al., Surgery 88:507 (1980); Saudek et al., N. Engl. J Med. 321:574 (1989)). In another embodiment, polymeric materials can be used. In yet another embodiment, a controlled release system can be placed in a subject at an appropriate site determined by a practitioner of skill, *i.e*., thus requiring only a fraction of the systemic dose (*see, e.g.,* Goodson, Medical Applications of Controlled Release, vol. 2, pp. 115-138 (1984)). Other controlled release systems are discussed in the review by Langer (Science 249:1527-1533 (1990)). The active ingredient can be dispersed in a solid inner matrix, *e.g*., polymethylmethacrylate, polybutylmethacrylate, plasticized or unplasticized polyvinylchloride, plasticized nylon, plasticized polyethyleneterephthalate, natural rubber, polyisoprene, polyisobutylene, polybutadiene, polyethylene, ethylene-vinylacetate copolymers, silicone rubbers, polydimethylsiloxanes, silicone carbonate copolymers, hydrophilic polymers such as hydrogels of esters of acrylic and methacrylic acid, collagen, cross-linked polyvinylalcohol and cross-linked partially hydrolyzed polyvinyl acetate, that is surrounded by an outer polymeric membrane, *e.g*., polyethylene, polypropylene, ethylene/propylene copolymers, ethylene/ethyl acrylate copolymers, ethylene/vinylacetate copolymers, silicone rubbers, polydimethyl siloxanes, neoprene rubber, chlorinated polyethylene, polyvinylchloride, vinylchloride copolymers with vinyl acetate, vinylidene chloride, ethylene and propylene, ionomer polyethylene terephthalate, butyl rubber epichlorohydrin rubbers, ethylene/vinyl alcohol copolymer, ethylene/vinyl acetate/vinyl alcohol terpolymer, and ethylene/vinyloxyethanol copolymer, that is insoluble in body fluids. The active ingredient then diffuses through the outer polymeric membrane in a release rate controlling step. The percentage of active ingredient in such parenteral compositions is highly dependent on the specific nature thereof, as well as the needs of the subject.

### Parenteral Dosage Forms

In one embodiment, provided are parenteral dosage forms. Parenteral dosage forms can be administered to subjects by various routes including, but not limited to, subcutaneous, intravenous (including bolus injection), intramuscular, and intraarterial. Because their administration typically bypasses subjects' natural defenses against contaminants, parenteral dosage forms are typically, sterile or capable of being sterilized prior to administration to a subject. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water miscible vehicles such as, but not limited to, ethyl alcohol, polyethylene glycol, and polypropylene glycol; and non aqueous vehicles such as, but not limited to, corn oil, cottonseed oil, peanut oil, sesame oil, ethyl oleate, isopropyl myristate, and benzyl benzoate.

Compounds that increase the solubility of one or more of the active ingredients disclosed herein can also be incorporated into the parenteral dosage forms.

### Transdermal, Topical & Mucosal Dosage Forms

Also provided are transdermal, topical, and mucosal dosage forms. Transdermal, topical, and mucosal dosage forms include, but are not limited to, ophthalmic solutions, sprays, aerosols, creams, lotions, ointments, gels, solutions, emulsions, suspensions, or other forms known to one of skill in the art. See, *e.g*., Remington's Pharmaceutical Sciences, 16th, 18th and 20th eds., Mack Publishing, Easton PA (1980, 1990 & 2000); and Introduction to Pharmaceutical Dosage Forms, 4th ed., Lea & Febiger, Philadelphia (1985). Dosage forms suitable for treating mucosal tissues within the oral cavity can be formulated as mouthwashes or as oral gels. Further, transdermal dosage forms include "reservoir type" or "matrix type" patches, which can be applied to the skin and worn for a specific period of time to permit the penetration of a desired amount of active ingredients.

Suitable excipients (*e.g*., carriers and diluents) and other materials that can be used to provide transdermal, topical, and mucosal dosage forms encompassed herein are well known to those skilled in the pharmaceutical arts, and depend on the particular tissue to which a given pharmaceutical composition or dosage form will be applied. With that fact in mind, typical excipients include, but are not limited to, water, acetone, ethanol, ethylene glycol, propylene glycol, butane 1,3 diol, isopropyl myristate, isopropyl palmitate, mineral oil, and mixtures thereof to form lotions, tinctures, creams, emulsions, gels or ointments, which are non toxic and pharmaceutically acceptable. Moisturizers or humectants can also be added to pharmaceutical compositions and dosage forms if desired. Examples of such additional ingredients are well known in the art. See, *e.g*., Remington's Pharmaceutical Sciences, 16th, 18th and 20th eds., Mack Publishing, Easton PA (1980, 1990 & 2000).

Depending on the specific tissue to be treated, additional components may be used prior to, in conjunction with, or subsequent to treatment with active ingredients provided. For example, penetration enhancers can be used to assist in delivering the active ingredients to the tissue. Suitable penetration enhancers include, but are not limited to: acetone; various alcohols such as ethanol, oleyl, and tetrahydrofuryl; alkyl sulfoxides such as dimethyl sulfoxide; dimethyl acetamide; dimethyl formamide; polyethylene glycol; pyrrolidones such as polyvinylpyrrolidone; Kollidon grades (Povidone, Polyvidone); urea; and various water soluble or insoluble sugar esters such as Tween 80 (polysorbate 80) and Span 60 (sorbitan monostearate).

The pH of a pharmaceutical composition or dosage form, or of the tissue to which the pharmaceutical composition or dosage form is applied, may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery enhancing or penetration enhancing agent. Different salts, hydrates or solvates of the active ingredients can be used to further adjust the properties of the resulting composition.

### Dosage and Unit Dosage Forms

In human therapeutics, the doctor will determine the posology which he considers most appropriate according to a preventive or curative treatment and according to the age, weight, stage of the infection and other factors specific to the subject to be treated. In certain embodiments, doses are from about 1 to about 1000 mg per day for an adult, or from about 5 to about 250 mg per day or from about 10 to 50 mg per day for an adult. In certain embodiments, doses are from about 5 to about 400 mg per day or 25 to 200 mg per day per adult. In certain embodiments, dose rates of from about 50 to about 500 mg per day are also contemplated.

In further aspects, provided are methods of treating or preventing an HCV infection in a subject by administering, to a subject in need thereof, an effective amount of a compound provided herein, or a pharmaceutically acceptable salt thereof. The amount of the compound or composition which will be effective in the prevention or treatment of a disorder or one or more symptoms thereof will vary with the nature and severity of the disease or condition, and the route by which the active ingredient is administered. The frequency and dosage will also vary according to factors specific for each subject depending on the specific therapy (*e.g*., therapeutic or prophylactic agents) administered, the severity of the disorder, disease, or condition, the route of administration, as well as age, body, weight, response, and the past medical history of the subject. Effective doses may be extrapolated from dose-response curves derived from *in vitro* or animal model test systems.

In certain embodiments, exemplary doses of a composition include milligram or microgram amounts of the active compound per kilogram of subject or sample weight (*e.g*., about 10 micrograms per kilogram to about 50 milligrams per kilogram, about 100 micrograms per kilogram to about 25 milligrams per kilogram, or about 100 microgram per kilogram to about 10 milligrams per kilogram). For compositions provided herein, in certain embodiments, the dosage administered to a subject is 0.140 mg/kg to 3 mg/kg of the subject's body weight, based on weight of the active compound. In certain embodiments, the dosage administered to a subject is between 0.20 mg/kg and 2.00 mg/kg, or between 0.30 mg/kg and 1.50 mg/kg of the subject's body weight.

In certain embodiments, the recommended daily dose range of a composition provided herein for the conditions described herein lie within the range of from about 0.1 mg to about 1000 mg per day, given as a single once-a-day dose or as divided doses throughout a day. In one embodiment, the daily dose is administered twice daily in equally divided doses. In certain embodiments, a daily dose range should be from about 10 mg to about 200 mg per day, in other embodiments, between about 10 mg and about 150 mg per day, in further embodiments, between about 25 and about 100 mg per day. It may be necessary to use dosages of the active ingredient outside the ranges disclosed herein in some cases, as will be apparent to those of ordinary skill in the art. Furthermore, it is noted that the clinician or treating physician will know how and when to interrupt, adjust, or terminate therapy in conjunction with subject response.

Different therapeutically effective amounts may be applicable for different diseases and conditions, as will be readily known by those of ordinary skill in the art. Similarly, amounts sufficient to prevent, manage, treat or ameliorate such disorders, but insufficient to cause, or sufficient to reduce, adverse effects associated with the composition provided herein are also encompassed by the above described dosage amounts and dose frequency schedules. Further, when a subject is administered multiple dosages of a composition provided herein, not all of the dosages need be the same. For example, the dosage administered to the subject may be increased to improve the prophylactic or therapeutic effect of the composition or it may be decreased to reduce one or more side effects that a particular subject is experiencing.

In certain embodiment, the dosage of the composition provided herein, based on weight of the active compound, administered to prevent, treat, manage, or ameliorate a disorder, or one or more symptoms thereof in a subject is 0.1 mg/kg, 1 mg/kg, 2 mg/kg, 3 mg/kg, 4 mg/kg, 5 mg/kg, 6 mg/kg, 10 mg/kg, or 15 mg/kg or more of a subject's body weight. In another embodiment, the dosage of the composition or a composition provided herein administered to prevent, treat, manage, or ameliorate a disorder, or one or more symptoms thereof in a subject is a unit dose of 0.1 mg to 200 mg, 0.1 mg to 100 mg, 0.1 mg to 50 mg, 0.1 mg to 25 mg, 0.1 mg to 20 mg, 0.1 mg to 15 mg, 0.1 mg to 10 mg, 0.1 mg to 7.5 mg, 0.1 mg to 5 mg, 0.1 to 2.5 mg, 0.25 mg to 20 mg, 0.25 to 15 mg, 0.25 to 12 mg, 0.25 to 10 mg, 0.25 mg to 7.5 mg, 0.25 mg to 5 mg, 0.5 mg to 2.5 mg, 1 mg to 20 mg, 1 mg to 15 mg, 1 mg to 12 mg, 1 mg to 10 mg, 1 mg to 7.5 mg, 1 mg to 5 mg, or 1 mg to 2.5 mg.

In certain embodiments, treatment or prevention can be initiated with one or more loading doses of a compound or composition provided herein followed by one or more maintenance doses. In such embodiments, the loading dose can be, for instance, about 60 to about 400 mg per day, or about 100 to about 200 mg per day for one day to five weeks. The loading dose can be followed by one or more maintenance doses. In certain embodiments, each maintenance does is, independently, about from about 10 mg to about 200 mg per day, between about 25 mg and about 150 mg per day, or between about 25 and about 80 mg per day. Maintenance doses can be administered daily and can be administered as single doses, or as divided doses.

In certain embodiments, a dose of a compound or composition provided herein can be administered to achieve a steady-state concentration of the active ingredient in blood or serum of the subject. The steady-state concentration can be determined by measurement according to techniques available to those of skill or can be based on the physical characteristics of the subj ect such as height, weight and age. In certain embodiments, a sufficient amount of a compound or composition provided herein is administered to achieve a steady-state concentration in blood or serum of the subject of from about 300 to about 4000 ng/mL, from about 400 to about 1600 ng/mL, or from about 600 to about 1200 ng/mL. In some embodiments, loading doses can be administered to achieve steady-state blood or serum concentrations of about 1200 to about 8000 ng/mL, or about 2000 to about 4000 ng/mL for one to five days. In certain embodiments, maintenance doses can be administered to achieve a steady-state concentration in blood or serum of the subject of from about 300 to about 4000 ng/mL, from about 400 to about 1600 ng/mL, or from about 600 to about 1200 ng/mL.

In certain embodiments, administration of the same composition may be repeated and the administrations may be separated by at least 1 day, 2 days, 3 days, 5 days, 10 days, 15 days, 30 days, 45 days, 2 months, 75 days, 3 months, or 6 months. In other embodiments, administration of the same prophylactic or therapeutic agent may be repeated and the administration may be separated by at least at least 1 day, 2 days, 3 days, 5 days, 10 days, 15 days, 30 days, 45 days, 2 months, 75 days, 3 months, or 6 months.

In certain aspects, provided herein are unit dosages comprising a compound, or a pharmaceutically acceptable salt thereof, in a form suitable for administration. Such forms are described in detail above. In certain embodiments, the unit dosage comprises 1 to 1000 mg, 5 to 250 mg or 10 to 50 mg active ingredient. In particular embodiments, the unit dosages comprise about 1, 5, 10, 25, 50, 100, 125, 250, 500 or 1000 mg active ingredient. Such unit dosages can be prepared according to techniques familiar to those of skill in the art.

The dosages of the second agents are to be used in the combination therapies provided herein. In certain embodiments, dosages lower than those which have been or are currently being used to prevent or treat HCV infection are used in the combination therapies provided herein. The recommended dosages of second agents can be obtained from the knowledge of those of skill. For those second agents that are approved for clinical use, recommended dosages are described in, for example, Hardman *et al*., eds., 1996, Goodman & Gilman's The Pharmacological Basis Of Basis Of Therapeutics 9^{th} Ed, Mc-Graw-Hill, New York; Physician's Desk Reference (PDR) 57^{th} Ed., 2003, Medical Economics Co., Inc., Montvale, NJ, which are incorporated herein by reference in its entirety.

In various embodiments, the therapies (*e.g*., a compound provided herein and the second agent) are administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In various embodiments, the therapies are administered no more than 24 hours apart or no more than 48 hours apart. In certain embodiments, two or more therapies are administered within the same patient visit. In other embodiments, the compound provided herein and the second agent are administered concurrently.

In other embodiments, the compound provided herein and the second agent are administered at about 2 to 4 days apart, at about 4 to 6 days apart, at about 1 week part, at about 1 to 2 weeks apart, or more than 2 weeks apart.

In certain embodiments, administration of the same agent may be repeated and the administrations may be separated by at least 1 day, 2 days, 3 days, 5 days, 10 days, 15 days, 30 days, 45 days, 2 months, 75 days, 3 months, or 6 months. In other embodiments, administration of the same agent may be repeated and the administration may be separated by at least at least 1 day, 2 days, 3 days, 5 days, 10 days, 15 days, 30 days, 45 days, 2 months, 75 days, 3 months, or 6 months.

In certain embodiments, a compound provided herein and a second agent are administered to a patient, for example, a mammal, such as a human, in a sequence and within a time interval such that the compound provided herein can act together with the other agent to provide an increased benefit than if they were administered otherwise. For example, the second active agent can be administered at the same time or sequentially in any order at different points in time; however, if not administered at the same time, they should be administered sufficiently close in time so as to provide the desired therapeutic or prophylactic effect. In one embodiment, the compound provided herein and the second active agent exert their effect at times which overlap. Each second active agent can be administered separately, in any appropriate form and by any suitable route. In other embodiments, the compound provided herein is administered before, concurrently or after administration of the second active agent.

In certain embodiments, the compound provided herein and the second agent are cyclically administered to a patient. Cycling therapy involves the administration of a first agent (*e.g*., a first prophylactic or therapeutic agents) for a period of time, followed by the administration of a second agent and/or third agent (*e.g*., a second and/or third prophylactic or therapeutic agents) for a period of time and repeating this sequential administration. Cycling therapy can reduce the development of resistance to one or more of the therapies, avoid or reduce the side effects of one of the therapies, and/or improve the efficacy of the treatment.

In certain embodiments, the compound provided herein and the second active agent are administered in a cycle of less than about 3 weeks, about once every two weeks, about once every 10 days or about once every week. One cycle can comprise the administration of a compound provided herein and the second agent by infusion over about 90 minutes every cycle, about 1 hour every cycle, about 45 minutes every cycle. Each cycle can comprise at least 1 week of rest, at least 2 weeks of rest, at least 3 weeks of rest. The number of cycles administered is from about 1 to about 12 cycles, more typically from about 2 to about 10 cycles, and more typically from about 2 to about 8 cycles.

In other embodiments, courses of treatment are administered concurrently to a patient, i.e., individual doses of the second agent are administered separately yet within a time interval such that the compound provided herein can work together with the second active agent. For example, one component can be administered once per week in combination with the other components that can be administered once every two weeks or once every three weeks. In other words, the dosing regimens are carried out concurrently even if the therapeutics are not administered simultaneously or during the same day.

The second agent can act additively or synergistically with the compound provided herein. In one embodiment, the compound provided herein is administered concurrently with one or more second agents in the same pharmaceutical composition. In another embodiment, a compound provided herein is administered concurrently with one or more second agents in separate pharmaceutical compositions. In still another embodiment, a compound provided herein is administered prior to or subsequent to administration of a second agent. Also contemplated are administration of a compound provided herein and a second agent by the same or different routes of administration, *e.g*., oral and parenteral. In certain embodiments, when the compound provided herein is administered concurrently with a second agent that potentially produces adverse side effects including, but not limited to, toxicity, the second active agent can advantageously be administered at a dose that falls below the threshold that the adverse side effect is elicited.

### Exemplary dosages and methods of treatment

In one embodiment, provided herein are methods for the treatment of a *Flaviviridae* infection in a host, including a human, that include administering Compound 1 in an amount from about 1 mg/day to about 150 mg/day, administered either alone or in combination or alternation with another anti-*Flaviviridae* agent, optionally in a pharmaceutically acceptable carrier. In certain embodiments, the amount of Compound 1 administered is from about 5 mg/day to about 100 mg/day. In certain embodiments, the amount of Compound 1 administered is about 5, 10, 15, 20, 25, 30, 35, 40, 45, 50, 55, 60, 65, 70, 75, 80, 85, 90, 95 or 100 mg/day. In certain embodiments, the amount of Compound 1 administered is about 5, 10, 25, 50, 75, or 100 mg/day.

In certain embodiments, provided herein are methods for the treatment of a *Flaviviridae* infection in a host, including a human, that includes administering Compound 1 in an amount from about 1 mg/day to about 150 mg/day, in combination or alternation with a therapeutically effective amount of ribavirin, optionally in a pharmaceutically acceptable carrier. In one embodiment, the amount of ribavirin administered is from about 800 mg to about 1400 mg. In one embodiment, the methods comprise administering Compound 1 in an amount from about 5 mg/day to about 100 mg/day and ribavirin in an amount from about 800 mg to about 1400 mg. In certain embodiments, the amount of Compound 1 administered is about 5, 10, 15, 20, 25, 30, 35, 40, 45, 50, 55, 60, 65, 70, 75, 80, 85, 90, 95 or 100 mg/day and the amount of ribavirin is about 800 mg, 1000 mg, 1200 mg or 1400 mg. In certain embodiments, the amount of Compound 1 administered is about 5, 10, 25, 50, 75, or 100 mg/day and the amount of ribavirin is about 800 mg, 1000 mg, 1200 mg or 1400 mg.

### Kits

Also provided are kits for use in methods of treatment of a liver disorder such as HCV infections. The kits can include a compound or composition provided herein, a second agent or composition, and instructions providing information to a health care provider regarding usage for treating the disorder. Instructions may be provided in printed form or in the form of an electronic medium such as a floppy disc, CD, or DVD, or in the form of a website address where such instructions may be obtained. A unit dose of a compound or composition provided herein, or a second agent or composition, can include a dosage such that when administered to a subject, a therapeutically or prophylactically effective plasma level of the compound or composition can be maintained in the subject for at least 1 days. In some embodiments, a compound or composition can be included as a sterile aqueous pharmaceutical composition or dry powder (*e.g*., lyophilized) composition.

In some embodiments, suitable packaging is provided. As used herein, "packaging" includes a solid matrix or material customarily used in a system and capable of holding within fixed limits a compound provided herein and/or a second agent suitable for administration to a subject. Such materials include glass and plastic (*e.g*., polyethylene, polypropylene, and polycarbonate) bottles, vials, paper, plastic, and plastic-foil laminated envelopes and the like. If e-beam sterilization techniques are employed, the packaging should have sufficiently low density to permit sterilization of the contents.

The following Examples illustrate the synthesis of representative compounds provided herein. These examples are not intended, nor are they to be construed, as limiting the scope of the claimed subject matter. It will be clear that the scope of claimed subject matter may be practiced otherwise than as particularly described herein. Numerous modifications and variations of the subject matter are possible in view of the teachings herein and, therefore, are within the scope the claimed subject matter.

### EXAMPLES

### EXAMPLE 1

### Preparation of HydroxytBuSate-phosphoramidate derivative of 2'-C-methyl guanosine(Compound 3)

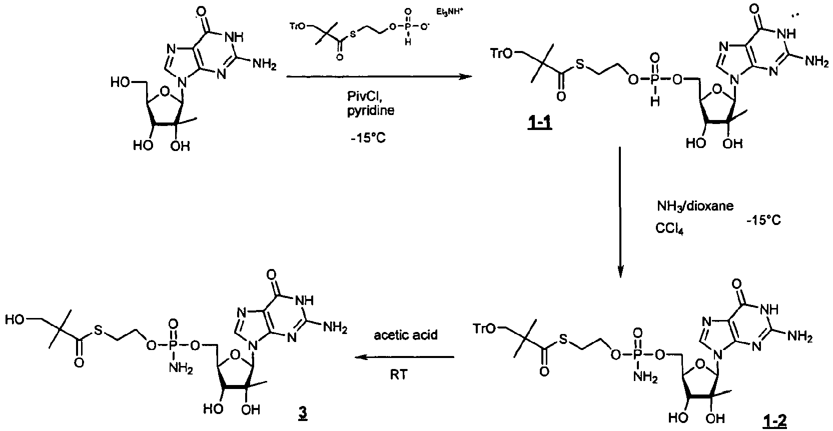

### Step 1: {9-[(2R)2-C-methyl-β-D-ribo-furanosyl]-guanin}-5'-yl-O-(triphenylmethyloxy-tert-butyl-S-acyl-2-thioethyl) H-phosphonate

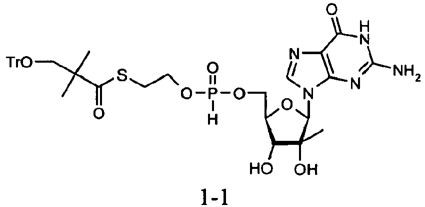

To a stirred solution of 9-(2-C-methyl-β-D-ribofuranosyl)guanine (4.87 mmol) and S-(2-phosphite-ethyl) 2,2-dimethyl-3-triphenylmethyloxy-thiopropionate triethylamine salt (6.34 mmol) in pyridine (75ml) at -15 °C was added dropwise pivaloyl chloride (9.74 mmol) under nitrogen. The reaction mixture was stirred at -15°C for 2 hours. Dichloromethane and NH₄Cl solution were added. Organic phase was separated washed with NH₄Cl solution, dried over Na₂SO₄, filtered and concentrated under reduced pressure. The crude material was purified by silica gel chromatography (DCM/MeOH) to yield the title compound.

**Molecular Formula C₃₇H₄₂N₅O₉PS. ¹H NMR** (DMSO-*d*₆, 400 MHz) δ (ppm) 0.80 (s, 3H), 1.13 (s, 6H), 3.04 (s, 2H), 3.14 (m, 2H), 3.97-4.08 (m, 4H), 4.28-4.38 (m, 2H), 5.35-5.10 (m, 2H), 5.77 (s, 1H), 6.52 (brs, 2H), 6.87-6.89 (m, 2H), 7.11-7.43 (m, 15H), 7.75 (s, 1H), **³¹P NMR** (DMSO-d₆, 162 MHz) δ (ppm) 9.20 (s) 9.47 (s). **Scan ES** ⁺764 (M+H)⁺

### Step 2: {9-[(2R)2-C-methyl-β-D-ribo-furanosyl]-guanin}-5'-yl-O-(triphenylmethyloxy-tert-butyl-S-acyl-2-thioethyl) phosphoramidate

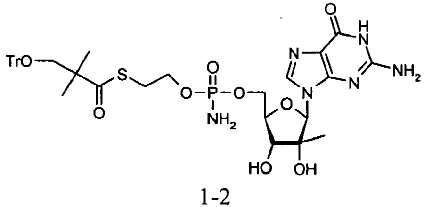

To a cooled solution (-45°C) of compound **1-1** (0.76 mmol) in carbon tetrachloride (8ml) was added a 0.5 M solution of NH₃ in dioxane (3.80 mmol, 8 ml). The reaction mixture was stirred for 3 hours between -15°C and -10°C. Volatiles were evaporated in vacuo. The residue obtained was co-evaporated with dichloromethane and used without purification for the next step. **Beige solid. Molecular Formula C₃₇H₄₃N₆O₉PS. Scan ES ⁺** 779 (M+H)⁺

### Step 3: HydroxytBuSate-phosphoramidate derivative of 2'-C-methyl guanosine

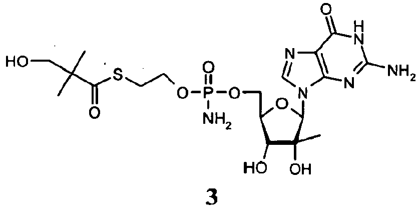

Compound **1-2** (0.78 mmol) was stirred at room temperature in aq AcOH 80% (31 ml) for 8 hours. EtOAc was added (20ml) and aqueous layer was concentrated under reduced pressure. The crude material was purified by C₁₈ chromatography (Water/Acetonitrile) to yield the title compound. **White powder. Molecular Formula C₁₈H₂₉N₆O₉PS. ¹H NMR** (D₂O-*d₆*, 400 MHz) δ (ppm) 0.96 (s, 3H), 1.02 (2s, 6H), 3.00-3.07 (m, 2H), 3.47 (2s, 2H), 3.98-4.03 (m, 2H), 4.14-4.18 (m, 1H), 4.24-4.38 (m, 3H), 5.88 (2s, 1H), 7.85 (s, 1H) **³¹P NMR** (D₂O-*d₆*, 162 MHz) δ (ppm) 12.93-13.04 (2s, 1P). **Scan ES⁺** 537 (M+H)⁺

### EXAMPLE 2

### Preparation of {9-[(2R)2-C-methyl-β-D-ribo-furanosyl]-guanin}-5'-yl phosphoramidate (Compound 4)

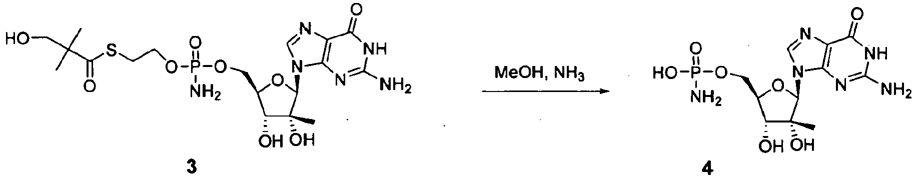

{9-[(2R)2-C-methyl-β-D-ribo-furanosyl]-guanin}-5'-yl-*O*-(hydroxy-tert-butyl-*S-*acyl-2-thioethyl) phosphoramidate (Compound **3**) (0.20 mol) was dissolved in a solution of methanol saturated with NH₃ (4 ml), at 0°C. The reaction mixture was stirred at 0°C for 30 min and at room temperature for 2 hrs. Solvent was evaporated and the crude material was purified by reverse phase (C 18) silica gel column chromatography eluting with a gradient 0-3 % methanol to yield the title compound(Compound **4**) as a **white solid. Molecular Formula C₁₁H₁₇N₆O₇P. ¹H NMR** (*d₆*-DMSO, 400 MHz) δ (ppm) 0.79 (s, 3H), 3.82-3.88 (m, 2H), 4.25-3.35 (m, 2H), 4.81 (brs, 1H), 5.63 (s, 1H), 6.85 (brs, 2H), 7.80 (s, 1H). **³¹P NMR** (DMSO-*d₆*, 162 MHz) δ (ppm) 7.58 (s, 1P).

### EXAMPLE 3

### Preparation of [(2R)-2-meth-yl-β-D-ribofuranosyl]guanine 5'-monophosphate (Compound 5)

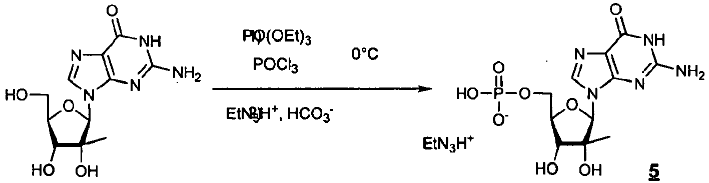

To a stirred solution of 9-(2-C-methyl-β-D-ribofuranosyl)guanine (0.33 mmol) in triethylphosphate (825 µL), phosphoryl chloride (75 µL, 1.07 mmol) was added at 0 °C. This reaction mixture was stirred overnight at 5 °C. The reaction was carefully quenched with TEAB 1M (pH = 7,5, 15 mL), stirred 20 min at 0 °C, then diluted with water and ethyl acetate. The aqueous phase was concentrated under reduced pressure. The crude material was subjected to DEAE-Sephadex chromatography eluting with TEAB). The desired fractions were combined, concentrated under reduced pressure and co-evaporated with a mixture of water/methanol, and finally co-evaporated with water. The resulting residue was purified on semi-preparative HPLC. Fractions containing the expected product were concentrated under reduced pressure, co-evaporated with a mixture of water/methanol and lyophilized from water. The triethylammonium salt monophosphate was eluted with water on a Dowex Na⁺ resin column to yield after lyophilisation the sodium salt. **White solid. Molecular Formula C₁₁H₁₅ N₅O₈P Na. ¹H NMR** (D₂O, 400 MHz) δ(ppm) 0.83 (s, 3H), 4.05-4.11 (m, 3H), 4.21-4.24 (m, 1H), 5.79 (m, 1H), 7.94 (m, 1H). **³¹P NMR** (D₂O, 121 MHz) δ(ppm) -0.42 (s, 1P). **LRFAB-MS (GT):** 422 (M+Na)⁺

### EXAMPLE 4

### Preparation of {9-[(2R)2-C-methyl-β-D-erythro-furanosyl]-guanin}-5'-yl-O-(carboxy -tert-butyl-S-acyl-2-thioethyl) benzylamine phosphoramidate

### (Compound 11)

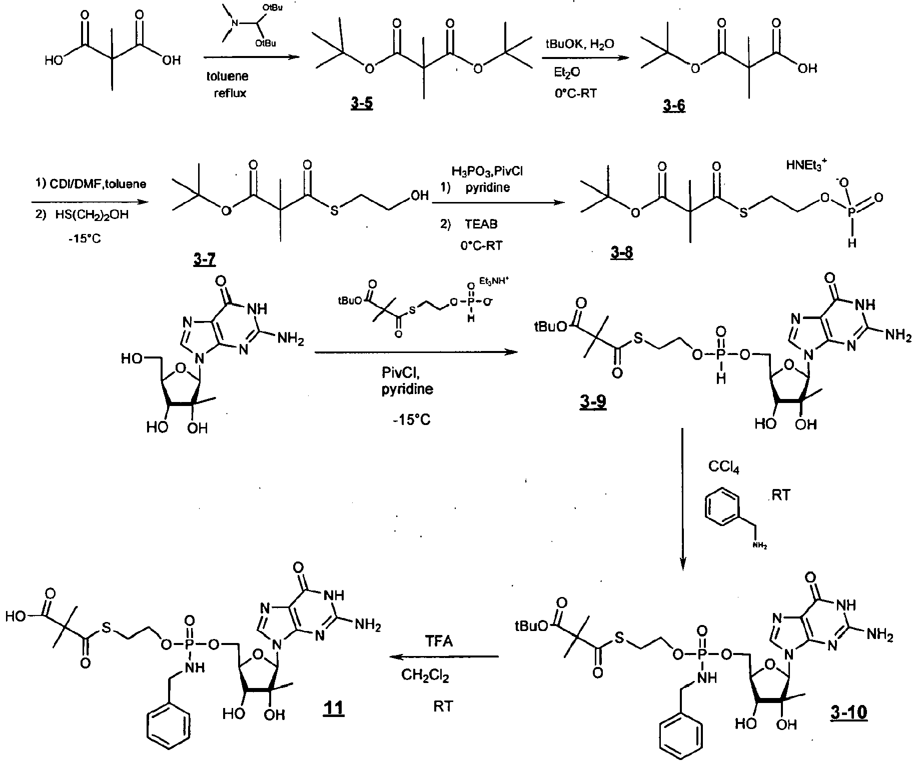

### Step 1: 2,2-dimethylmalonic acid- di- tert- butyl ester

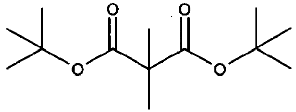

Compound **3-5** was synthesized from dimethylmalonic acid following the procedure described in Synthesis, 1983, 135*.* **Yellow oil. Molecular Formula C₁₃H₂₄O₄. ¹H NMR** (DMSO-*d₆*, 400 MHz) δ (ppm) 1.26 (s, 6H), 2.89 (s, 18H).

### Step 2: 2,2-dimethylmalonic acid- tert- butyl ester

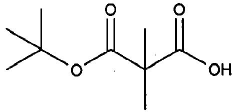

Compound **3-6** was synthesized from compound **5** following the procedure described in J.Org. Chem, 2004, 69, 6185*.* **White solid. Molecular Formula C₉H₁₆O₄. ¹H NMR** (DMSO-*d₆*, 400 MHz) δ (ppm) 1.22 (s, 6H), 1.37 (s, 9H).

### Step 3: 2-(2-hydroxy-ethylsulfanylcarbonyl)-2-methyl-propionic acid-tert-butyl ester

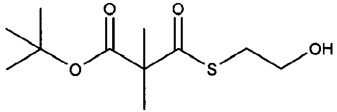

To a stirred solution of compound **3-6** (1.30 mmol) in an anh mixture of toluene (6ml) and DMF (455 µl) was added 1,1'-carbonyldiimidazole (1.69 mmol). The reaction mixture was stirred at room temperature for 30 min, diluted with toluene/DMF (93/7, v/v), cooled down to -16°C and treated with 2-mercaptoethanol (1.69 mmol). The mixture was stirred for 3 hours between -15°C and -5°C. Solvents were removed under reduced pressure. The residue was taken up in DCM and washed with water. Organics were separated, dried over Na₂SO₄ and concentrated under reduced pressure to yield the title compound. **Yellow oil. Molecular Formula C₁₁H₂₀O₄ S. ¹H NMR** (DMSO-*d₆*, 400 MHz) δ (ppm) 1.22 (s, 6H), 1.37 (s, 9H), 2.60 (m, 2H), 2.80 (m, 2H).

### Step 4: S-(2-phosphite-ethyl)-2,2-dimethyl-sulfanylearbonyl propionic acid tert-butylester triethylamine

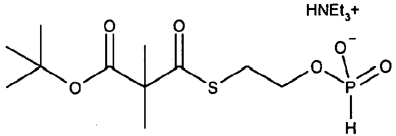

Compound **3-7** (1.3 mmol) dissolved in pyridine (6 ml) was added to phosphorous acid (13 mmol). The reaction mixture was cooled down to 0°C and pivaloyl chloride (7.15 mmol) was added. The mixture was allowed to warm up to room temperature and was stirred for 3 hours. The reaction was carefully quenched with 1M aq TEAB (3ml), diluted with EtOAc (50 ml) and washed with 0.5M TEAB (15 ml). Organics were separated, dried over Na₂SO₄, concentrated under reduced pressure, co-evaporated with toluene and purified by silica gel chromatography (DCM+1%TEA/DCM,MeOH10%,TEA1%) to yield the title compound. **Yellow oil. Molecular Formula C₁₁H₂₀NO₆ PS. ¹H NMR** (CDCl₃, 400 MHz) δ (ppm) 1.19 (s, 9H), 1.30 (s, 6H), 3.62 (m, 2H), 3.94 (m, 2H). **³¹P NMR** (CDCl₃, 162 MHz) δ (ppm) 4.25 (s, 1P).

### Step 5: {9-[(2R)2-C-methyl-β-D-erythro-furanosyl]-guanin}-5'-yl-O-(tert-butylcarboxy-tert-butyl-S-acyl-2-thioethyl) H-phosphonate

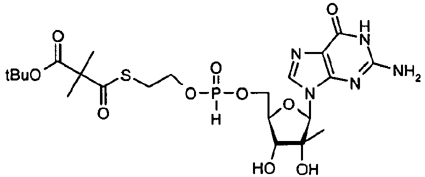

Compound **3-9** was synthesized from 9-(2-C-methyl-β-D-ribofuranosyl)guanine and S-(2-phosphite-ethyl)-2,2-dimethyl-sulfanylcarbonyl propionic acid tert-butyl ester triethylamine salt following the procedure as described for compound **1-1. Pale yellow oil. Molecular Formula C₂₂H₃₄N₅O₁₀PS- Scan ES⁺** 592 (M+H)⁺

### Step 6: {9-[(2R)2-C-methyl-B-D-erythro-furanosyl]-guanin}-5'-yl-O-(tert-butylcarboxy-tert-butyl-S-acyl-2-thioethyl) benzylamine phosphoramidate

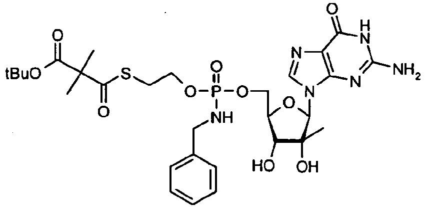

To a stirred solution of compound **3-9** (0.84 mmol) in carbon tetrachloride (8,4 ml) was added dropwise benzylamine (8.4 mmol). The reaction mixture was stirred for 3 hours at room temperature. The reaction mixture was diluted with EtOAc (100 ml) and washed with 1N HCl (50 ml). Organics were separated, dried over Na₂SO₄ and concentrated under reduced pressure. The crude material was purified by silica gel chromatography (Methanol/Dichloromethane) to yield the title compound. **White solid. Molecular Formula C₂₉H₄₁N₆O₁₀PS. Scan ES⁺** 697 (M+H)⁺

### Step 7: {9-[(2R)2-C-methyl-βD-erythro-furanosyl]-guanin}-5'-yl-O-(carboxy-tert-butyl-S-acyl-2-thioethyl) benzylamine phosphoramidate

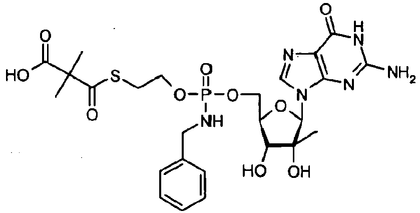

To a stirred solution of compound **3-10** (0.07 mmol) in DCM (1ml) was added TFA (2.24 mmol). The reaction mixture was stirred at room temperature for 1 hour, TFA was then added (1.12 mmol) and the mixture was let to stirred for 1 more hour. Solvent was evaporated. The crude material was purified by preparative HPLC to yield the title compound. **White solid. Molecular Formula C₂₅H₃₃N₆O₁₀PS. ³¹P NMR** (DMSO-*d₆*, 162 MHz) δ (ppm) 9.64-9.90 (2s, 1P).**Scan ES⁺** 642 (M+H)⁺

### EXAMPLE 5

### [(2R)-2-methyl-β-D-ribofuranosyl]guanine-N-benzylaminyl-5'-monophosphate (salt) (Compound 2, sodium salt)

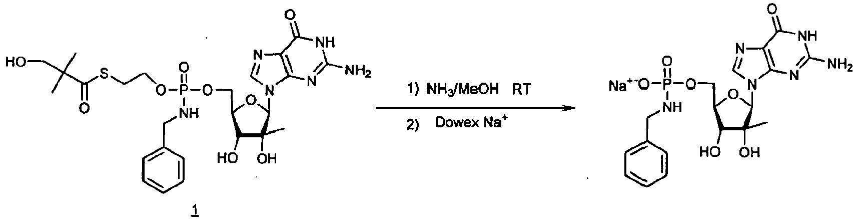

Compound 1 (0.24 mmol) was stirred in NH₃/MeOH (7N) (10 ml) at room temperature for 5 hours. The mixture was evaporated, the residue obtained was purified by silica gel chromatography (Water/Acetonitrile) and was eluted with water on a Dowex Na⁺ resin column to yield after lyophilisation the sodium salt. **White solid. Molecular Formula C₁₈H₂₃N₆O₇P. ¹H NMR** (DMSO-*d₆* +D₂O, 400 MHz) δ (ppm) 0.79 (s, 3H), 3.75-4.15 (m, 6H), 5.70 (s, 1H), 7.09-7.15 (m, 5H), 8.07 (s, 1H). **³¹P NMR** (DMSO-*d₆* + D₂O, 162 MHz) δ (ppm) 6.31 (s, 1P). **Scan ES⁺** 489 (M+Na)⁺

### EXAMPLE 6

### [(2R)-2-methyl-β-D-ribofuranosyl]guanine 5'-diphosphate

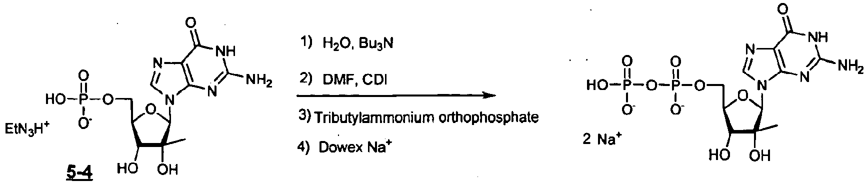

Compound **5-4** (0.276 mmol) was dissolved in water (0.7 ml) with Bu₃N (1.1 mmol). The mixture was concentrated under reduced pressure, co-evaporated thrice with pyridine and twice with toluene. DMF (2.25 ml) was added followed by 1,1-carbodiimidazole (1.68 mmol). The mixture was stirred for 16 hours and TEAB (7 ml) was added. The reaction mixture was stirred at room temperature for 24 hours, hydrolysed with water (neutral pH) and concentrated under reduced pressure. The crude material was eluted with TEAB though a Sephadex -DEAE-A25 5 (Fluka) column and was purified by preparative HPLC. The white powder obtained was eluted with water on a Dowex Na⁺ resin column to yield after lyophilisation the sodium salt. **White solid. Molecular Formula C₁₁H₁₅N₅Na₂O₁₁P₂. ¹H** NMR (D₂O, 400 MHz) δ (ppm) 0.90 (s, 3H), 4.13-4.24 (m, 4H), 5.87 (s, 1H), 7.99 (s, 1H). **³¹P NMR** (D₂O, 162 MHz) δ (ppm) -11.15,-10.45 (2d, 2P).

### EXAMPLE 7

### [(2R)-2-methyl-β-D-ribofuranosyl]guanine 5'-triphosphate (sodium salt)

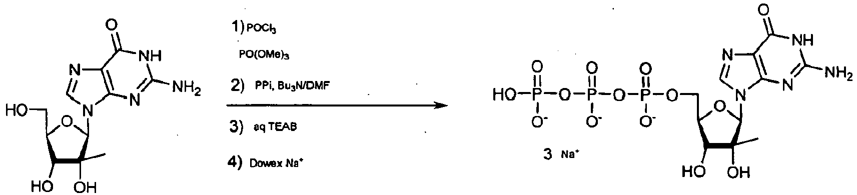

To a solution of 9-(2-C-methyl-β-D-ribofuranosyl)guanine (0.40 mmol) in triethylphosphate (1 ml), phosphoryle chloride (1.08 mmol) was added at 0 °C. This reaction mixture was stirred overnight at 5 °C. Tributylammonium pyrophosphate (PPi/Bu₃N 1/1,5, 1g, 2.19 mmol) was dissolved in anhydrous DMF (2 mL). 2.4 mL of this solution were added to the reaction mixture. The mixture was then stirred at 0 °C for 1 min. The reaction was carefully quenched with TEAB 1M (pH = 7,5, 5 mL), stirred 20 min at 0 °C, then diluted with water and ethyl acetate. The aqueous phase was concentrated under reduced pressure. The crude material was subjected to DEAE-Sephadex chromatography (eluted TEAB). The desired fractions were combined, concentrated under reduced pressure and co-evaporated with a mixture of water/methanol, and finally co-evaporated with water. The resulting residue was purified on semi-preparative HPLC. The triethylammonium salt triphosphate was eluted three times with water on a Dowex Na⁺ resin column to yield after lyophilisation the sodium salt. **White powder. Molecular Formula C₂₉H₆₃N₈Na₃O₁₄P₃.¹H NMR** (D₂O, 400 MHz) δ (ppm) 0.91 (s, 3H), 4.14-4.36 (m, 4H), 5.88 (s, 1H), 8.01 (s, 1H). **³¹P NMR** (D₂O, 162 MHz) δ (ppm) -11.20,-10.65 (2d, 2P), -22.74 (t, 1P).

### EXAMPLE 8

### O-(Hydroxyl-tert-butyl-S-acyl-2-thioethy)-2'-C-methylguanosin-5'-yl phosphate (sodium salt)

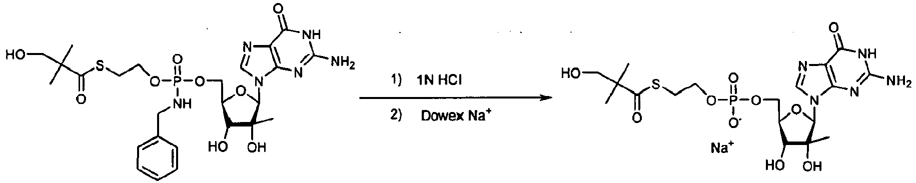

**Compound 1** (0.11 mmol) was dissolved in aq 1N HCl and stirred for 16 hours at room temperature. The reaction mixture was concentrated under reduced pressure. The residue obtained was purified by silica gel chromatography C18 (eluted with water/acetonitrile) and was eluted with water on a Dowex Na⁺ resin column to yield the sodium salt after lyophilisation. **White powder. ¹H NMR** (D₂O, 400 MHz) δ (ppm) 0.94-0.98 (m, 9H), 2.94 (t, *J*= 6.8 Hz, 2H), 3.43 (s, 2H), 3.77 (t, *J*= 7.2 Hz, 2H), 4.12-4.30 (m, 4H), 5.88 (s, 1H), 7.94 (s, 1H). **³¹P NMR** (D₂O, 126 MHz) δ (ppm) -0.08 (s, P).

### EXAMPLE 9

### Purification of crude Compound 1 with P diastereomer ratio > 1.00:1.00

Crude Compound **1** was purified by reverse-phase chromatography (prepared Bakerbond 40 µm C-18 RP-silica - washed with 100% acetonitrile gradient to 100% H₂O). The crude was dissolved in tetrahydrofuran, H₂O and saturated aqueous sodium bicarbonate solution.

Elution under gentle vacuum with a stepwise gradient*:
1.5% MeCN/H₂O
5% MeCN/H₂O
10% MeCN/H₂O
15% MeCN/H₂O
18% MeCN/H₂O
20% MeCN/H₂O
25% MeCN/H₂O

Pure Compound 1 was obtained after several fractions of 25% MeCN. The chemically pure fractions (diasteroisomeric mixture of > 1.00:1.00) were stored at 2-6°C for 15h after which time the precipitated solids (predominantly diastereomer 2) were filtered and dried under vacuum at 32-38°C, leading to pure diastereomer 2,(diasteroisomeric purity: 98%).

The chemical purity of the solid was checked by HPLC using Method A, described below. The diasteroisomeric purity was checked by chiral HPLC and ³¹P NMR.

### EXAMPLE 10

### Isolation of Compound 1, diastereomer 1:

Compound 1, diastereomer 1 was separated using reverse phase column chromatography (C18 silica gel) on a CombiFlash purification system. A mixture of diastereomers (1g, 83/17 diastereomer 2/1, chemical purity =97.3%) was dissolved in THF/water (3:1, 4ml). The solution was loaded on a pre-equilibrated C₁₈ column (Reusable RediSep Rf C₁₈, 130g) and eluted with gradient methanol/water (40/60 to 50/50, flow rate= 50ml/min). The first eluted isomer was diastereomer 1. The fractions were checked by HPLC for chemical and diastereomeric purities of diastereomer 1. Clean fractions were combined and evaporated *in vacuo* to afford diastereomer 1. Net =290mg; both chemical and diastereomer purities >99% (AUC, HPLC Method A and HPLC Method B, respectively).

### HPLC Method A: Chemical purity

Column: Zorbax Eclipse XDB-C8; 4.6x75mm 3.5-Micron
Mobile Phase A: Acetonitrile
Mobile Phase B: 0.0 1 M ammonium acetate buffer, PH=4.4
Column Temperature: 28°C
Flow Rate: 1.4ml/min
Detection: UV 254nm, UV272nm
Gradient:

| Time | Mobile Phase A | Mobile Phase B |
|---|---|---|
| 0 | 5 | 95 |
| 5.5 | 80 | 20 |
| 10 | 80 | 20 |

### Method B: HPLC Method for resolving Compound 1 diastereomers

Column: Agilent Eclipse XDB C₁₈; 4.6x150mm 5-Micron
Mobile Phase A: Methanol
Mobile Phase B: Water
Flow Rate: 1.0ml/min
Detection: UV272nm
Gradient:

| Time | Mobile Phase A | Mobile Phase B |
|---|---|---|
| 0 | 40 | 60 |
| 20 | 55 | 45 |

Retention time:
Compound **1,** diastereomer 2: 16.5±0.5min
Compound **1,** diastereomer 1: 14.4±0.5min

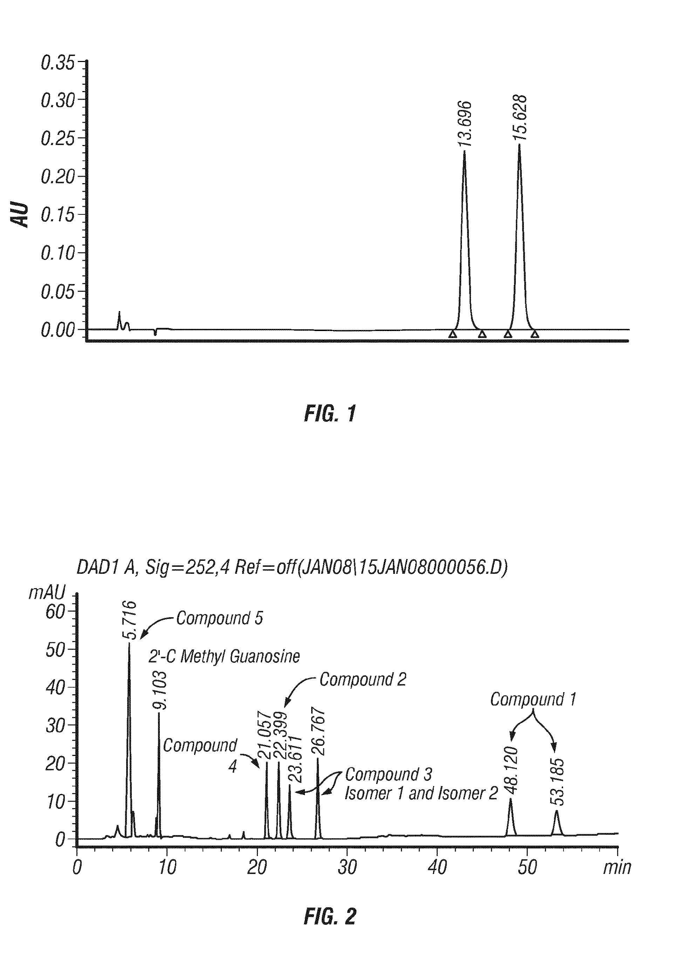

Figure 1 provides an HPLC trace illustrating resolution of the two diastereomers of Compound **1-** the two peaks in the trace, peak 1 and peak 2, correspond to diastereomers 1 and 2 of compound **1.**

### EXAMPLE 11

### A. Preparation of Compound 1

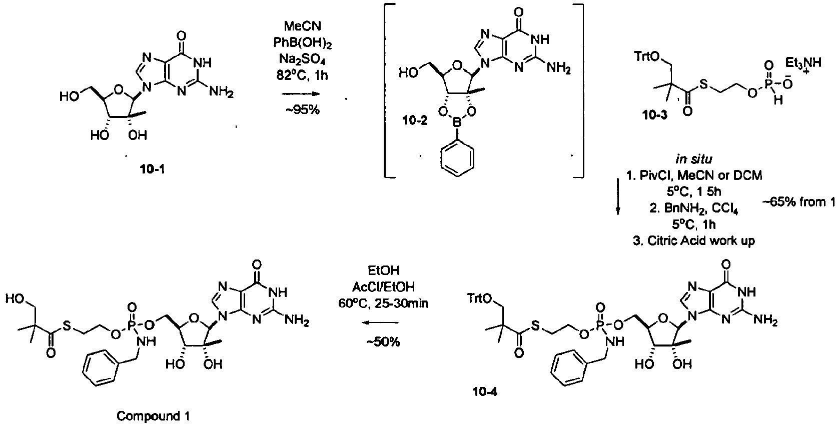

Compound 1 is synthesized as a mixture of phosphorous diastereomers in 1:1 ratio. Isolated overall yield from 2'-C-methyl guanosine to Compound 1 is typically 30-35%.

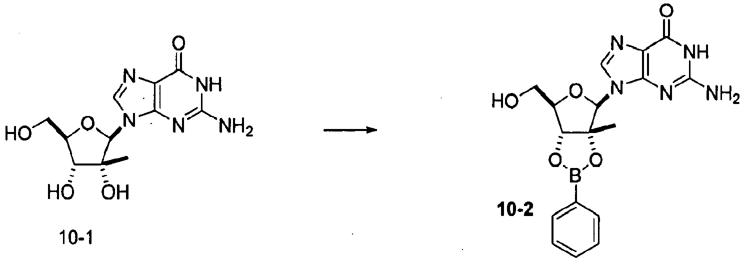

| **Material** | **Grade** | **FW gmol⁻¹** | **Quantity** | **Density gml⁻¹** | **Amount mol** | **Eq** |
|---|---|---|---|---|---|---|
| 2'-C-methyl guanosine | 97% | 297.1 | 242g | - | 0.791 | 1.0 |
| PhB(OH)₂ | 98% | 122.1 | 103.4g | - | 0.831 | 1.05 |
| Acetonitrile anhydrous | 98% | - | 1.47L | - | - | - |
| Na₂SO₄ anhydrous | 99% | 142.0 | 281g | - | 1.980 | 2.5 |
| Na₂SO₄ anhydrous | 99% | 142.0 | 112g | - | 0.791 | 1.0 |

2'-C-methyl guanosine was suspended in acetonitrile under argon and anhydrous sodium sulfate (2.5eq) was added. After stirring for five minutes, benzeneboronic acid was added in one portion and the mixture was refluxed for 1h. Analysis of the reaction mixture by ¹H-NMR (NMR sample preparation: ca. 0.1 ml of reaction aliquot was blown dry under argon flow to remove acetonitrile, the residue was dissolved in d6-DMSO) indicated a ratio of >96:4 product : starting material.

After 2h at reflux, the mixture was cooled to 25°C under argon and additional anhydrous sodium sulfate (1.0eq) was added. The resulting mixture was used directly for the next step.

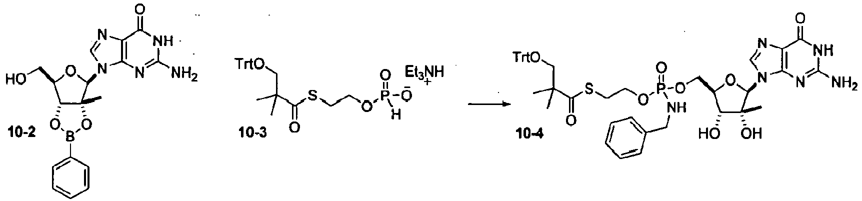

| **Material** | **Grade** | **FW gmol⁻¹** | **Quantity** | **Density gml⁻¹** | **Amount mol** | **Eq** |
|---|---|---|---|---|---|---|
| 2,3-PhB-2'-C-methyl guanosine | - | - | Mixture | - | ∼0.791 | 1 |
| Phosphonate 10-**3** | - | 585.7 | 695g | - | 1.186 | 1.5 |
| Acetonitrile anhydrous | 98% | - | 526ml | - | - | - |
| Pyridine anhydrous | 99.5% | 79.1 | 690mL | 0.978 | 8.54 | 10.8 |
| Pivaloyl chloride | 98% | 120.6 | 497mL | 0.98 | 3.95 | 5.0 |
| Benzylamine | 98% | 107.2 | 1.30L | 0.98 | 11.86 | 15 |
| Carbon tetrachloride | 99.5% | 153.8 | 918mL | 1.59 | 9.49 | 12 |

Phosphonate **10-3** was dissolved in acetonitrile (526mL) and added to the crude mixture of 2'-C-methyl guanosine-boronate **10-2** under argon. This mixture was then cooled to 5°C under argon. Separately, pyridine was treated with pivaloyl chloride under argon and the resulting mixture was added dropwise (over 1.5h) to the boronate-phosphonate reaction flask, keeping the internal temperature below 8°C. After stirring at 8°C for 30min, analysis by HPLC (Test20; 254nm) indicated ∼1.5:1 ratio of P-H product to 2'-C-methyl guanosine. The reaction mixture was allowed to warm gradually to 14°C over the subsequent 50min at which point analysis by HPLC (Test20; 254nm) indicated ∼6.5:1 ratio of P-H product to 2'-C-methyl guanosine. No additional PivCl was added.

The internal temperature of the reaction was reduced and then maintained below 8°C before benzylamine was added dropwise (1h) giving a thick suspension. Carbon tetrachloride was then added over 15min ensuring the internal temperature remained below 15°C. The reaction was slightly exothermic. Analysis by HPLC (Test20; 272nm) after 15min indicated complete consumption of the P-H intermediate (Rt 5.26min) and formation of product (Rt 5.73min). The reaction mixture was stored overnight at 4°C under argon.

TBME (3L) was added and the resultant mixture was poured into aqueous citric acid solution (22% w/v, 9.3L). Additional TBME (4.4L) was used to rinse the reaction vessel. The biphasic mixture was stirred for 45min at room temperature to effect cleavage of the boronate. Residual solids were observed in the aqueous phase therefore a further 1L citric acid solution was added to effect suitable phase separation.

The two phases were separated and the aqueous was observed to be pH 4 with no product by HPLC analysis. The organic layer was basified to pH 8 with aqueous sodium bicarbonate (5% w/v; 4.7L) and the layers were separated after addition of saturated brine (2L). No product was observed in the aqueous bicarbonate layer by HPLC analysis.

Additional TBME (7.4L) was added to the organic layer to induce further precipitation of product and the mixture was stirred for 2h at room temperature.

Filtration under vacuum and subsequent HPLC analysis indicated a small amount of product in the TBME filtrate. The cake was washed with water (8L, no product in filtrate), TBME (4L, negligible product in filtrate) and ethanol (2L, product observed in filtrate).

The solid was dried in a vacuum oven (<35°C) using Drierite absorbent to give 465g of pale yellow phosphoramidate **10-4.**

HPLC AUC Test20 @ 272nm: 92% - two major impurities Rt 4.5min (2.5%) and Rt 5.1min (3.5%) derived from the batch of phosphonate **10-3.**

Yield: 67%. ³¹P NMR ratio 9.93ppm:9.78ppm =1.1:1.0.

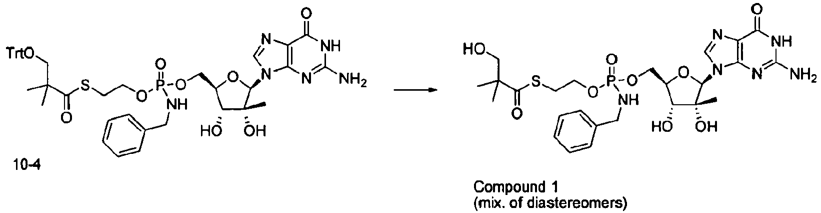

| **Material** | **Grade** | **FW gmol⁻¹** | **Quantity** | **Density gml⁻¹** | **Amount mol** | **Eq** |
|---|---|---|---|---|---|---|
| Phosphoramidate 10-4 | - | 868.9 | 317g | - | 0.365 | 1 |
| AcCl | 99% | 78.5 | 53mL | 1.105 | 0.730 | 2.0 |
| EtOH anhydrous | 98% | - | 4.7L | - | - | - |

Phosphoramidate **10-4** was dissolved in anhydrous ethanol (4.0L) under argon. Separately acetyl chloride was added carefully to anhydrous ethanol (600mL) - highly exothermic - under argon. The solution of HCl in ethanol thus produced was added to the phosphoramidate solution whereupon the internal temperature rose from 18°C to 20°C. A further 100mL of anhydrous ethanol was used to rinse the remainder of the HCl solution into the reaction mixture.

The reaction mixture was heated to 60°C for 30min after which time HPLC analysis (Test20 @ 254 or 272nm) indicated complete conversion of starting material (Rt 5.7min) to a major product (Rt 3.32min).

After a total of 45min reaction time the mixture was cooled to 25-30°C and solid sodium bicarbonate (2.3Kg) was added, keeping the internal temperature at 25-30°C whilst stirring for 1h. The pH was monitored using pH Colorfast indicator strips and found to be pH 5-6.

The mixture was filtered through Celite and washed with ethanol (4L) and tetrahydrofuran (1.5L). The filtrate was concentrated under vacuum at 35°C to give a solid (356g). Trituration with TBME (1.5L) for 15min at 35°C to remove the trityl by-product yielded a solid which was filtered, washed with TBME (750ml) and dried to give crude 10-5 (268g; 78% HPLC Test20 AUC @ 272nm). No product was observed in the filtrate by HPLC analysis.

268g of the crude Compound 1 was purified by reverse-phase chromatography (3Kg of prepared Bakerbond 40 µm C-18 RP-silica - washed with 100% acetonitrile gradient to 100% H₂O) The crude was dissolved in tetrahydrofuran (225mL), H₂O (75mL) and saturated aqueous sodium bicarbonate solution (75mL). Obtained 135g, >98% purity HPLC Test20 AUC @ 272nm (59% yield).
Typical analytical data is shown below:
**Compound 1**: C₂₅H₃₅N₆O₉PS 626.62gmol⁻¹
**HPLC AUC (Method Test20):** 99% @ 272nm, Rt 3.32min
**m/z (ESI +):** 627.05 [M+H]⁺ 100%; 1253.55 [2M+H]⁺ 20%
   **¹H NMR δ_{H} (400 MHz, d6-DMSO):** 0.85 (3H, s, C*H*₃), 1.11 (6H, s, (C*H*₃)₂C), 3.05 (2H, m, C*H*₂S), 3.44 (2H, d, *J* 5.5Hz, C*H*₂OH), 3.89-4.02 (6H, m, *H*-3', *H*-4', C*H*₂O, C*H*₂Ph), 4.14-4.20 (1H, m, *H*-5'), 4.22-4.30 (1H, m, *H*-5"), 4.97 (1H, t, *J* 5.5Hz, CH₂O*H*) 5.22 (1H, s, O*H*-2'), 5.43, 5.46 (2 x 0.5H, 2 x d, 2 x *J* 6.4Hz, O*H*-3'), 5.68 (1H, m, P-N-*H*), 5.78 (1H, s, *H*-1'), 6.56 (2H, br-s, N*H*₂), 7.20-7.24 (1H, m, Ar-*H*), 7.28-7.34 (4H, m, 4 x Ar-*H*), 7.80, 7.81 (2 x 0.5H, 2 x s, *H*-8), 10.69 (1H, br-s, N-*H*)
³¹P **NMR δ_{P} (162 MHz, d6-DMSO):** 9.78, 9.92 (1P, 2 x s, ratio 1.00:1.09)

### B. Preparation of Compound 1, diastereomer 2

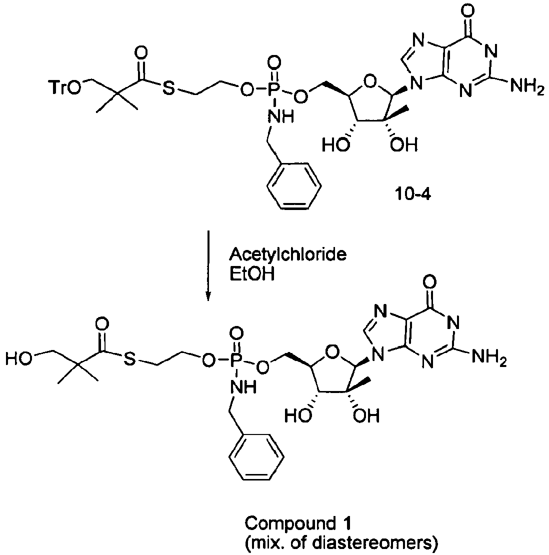

### Procedure 1

### Synthesis of Compound 1 (mixture of diastereomers) - Acetyl chloride/Ethanol mediated de-tritylation reaction:

| **Chemicals** | **Vendor/Grade** | **FW** | **Amount** | **mmol** | **Eq** |
|---|---|---|---|---|---|
| Compound **4-2** | Prepared by known procedures | 868 | 229.0g | 264 | 1.0 |
| Acetyl chloride (d= 1.105) | Fluka/ 98+% | 78.5 | 37.5 ml | 527 | 1.5 |
| Ethanol | Aldrich/ anhydrous | 46 | 3.3L | solvent | 14.5ml/ g |
| Sodium sulfate | J.T. Baker anhydrous | 142 | 65g | 458 | 1.7 |
| Sodium bicarbonate | J.T. Baker/ powder | 84 | 1.2 kg | For neutralization | |

To a suspension of compound **10-4** (229.0g, 264mmol, 89% pure, diastereomer ratio 2:1 TrO-A: TrO-B) in anhydrous ethanol (3.3L) was added acetylchloride (37.5ml, 527mmol) through an addition funnel over a period of 5 minutes. The resulting mixture was heated to 60°C on a heating mantle and was kept at 60°C for 1 hour. HPLC indicated the reaction was completed (Method A, AUC, 272nm, only less than 0.5% of compound **10-4** was detected).

Work-up: The reaction mixture was cooled on an ice bath to 28°C; anhydrous sodium sulfate (65g) was added. The cooling bath was then removed. Sodium bicarbonate powder (1.2kg) was added in portions over a period of 1 hour. The mixture was stirred at RT for 30 minutes; pH of mixture was ∼7. Insoluble solid was then removed by vacuum filtration. The filter cake was washed with ethanol (700ml) and THF (700ml). The filtrate was concentrated on a rotavap to a volume of ca. 200ml. To the residue was charged *tert-*butyl methyl ether (TBME) (1 L). A sticky solid fell out of solution and attached to the side of the flask. The mixture was then allowed to sit in the cold room (4°C) overnight.

TBME was decanted from the above mixture. Additional TBME (200ml) was added to wash the residue solid by swirling for 5 minutes. TBME was decanted to afford the crude.

### Isolation/purification of Compound 1, diastereomer 2:

The above crude material was suspended in THF (520ml) and water (30ml). Small amount (ca. 5ml) of saturated sodium bicarbonate solution was added to adjust pH to 7.5 (starting pH=6, pH paper). The mixture was swirling around in the round bottom flask for 15mintes. More THF (280ml) was added to the mixture. After stirring for 5 minutes, solid was collected by vacuum filtration. The filter cake was washed with THF (120ml) and water (140ml) and was dried in a vacuum oven at 40°C. Compound 1, diastereomer 2 was obtained as white solid. Net = 55.0g, Chemical purity = 97% (AUC, HPLC Method A, see Example 10); diasteremer purity = 97.7%. (AUC, HPLC).

### Procedure 2

### Synthesis of Compound 1 (mixture of diastereomers) - Acetyl chloride/Ethanol mediated de-tritylation reaction:

| **Chemicals** | **Vendor/Grade** | **FW** | **Amount** | **mmol** | **Eq** |
|---|---|---|---|---|---|
| Compound **10-4** | Prepared by known procedures | 868 | 231.0g | 266 | 1.0 |
| Acetyl chloride (d=1.105) | Fluka/ 98+% | 78.5 | 37.8 ml | 530 | 1.5 |
| Ethanol | Aldrich/ anhydrous | 46 | 2.46L | solvent | 10 ml/g |
| Sodium sulfate | J.T. Baker anhydrous | 142 | 70g | 492 | 1.8 |
| Sodium bicarbonate | J.T. Baker/ powder | 84 | 2.0 kg | For neutralization | |

To a suspension of compound **10-4** (231.0g, 266mmol, 90% pure, diastereomer ratio 2.3:1 TrO-A: TrO-B) in anhydrous ethanol (2.46L) was added acetylchloride (37.8ml, 530mmol) through an addition funnel over a period of 5 minutes. The resulting mixture was heated to 60°C on a heating mantle and was kept at 60°C for 1 hour. HPLC result indicated the reaction was completed (Method A, AUC, 272nm, only less than 1.5% of compound **10-4** was detected).

Work-up: The reaction mixture was cooled on an ice bath to 30°C; anhydrous sodium sulfate (70g) was added. The cooling bath was then removed. Sodium bicarbonate powder (2.0kg) was added in portions over a period of 1 hour. The mixture was stirred at RT for 30 minutes, pH of mixture was 6∼7. Insoluble solid was then removed by vacuum filtration. The filter cake was washed with ethanol (600ml) and THF (600ml). The filtrate was concentrated on a rotavap to a volume of ca. 200ml. To the residue was charged TBME (1 L), the mixture was rotated on a water bath at 35°C for 30minutes. The sticky solid fell out of solution and attached to the side of the flask. The mixture was then allowed to sit in the cold room (4°C) overnight.

TBME was decanted from the above mixture. Additional TBME (400ml) was added to wash the residue solid by swirling for 5 minutes. TBME was decanted to afford the crude.

### Isolation/purification of Compound 1, diastereomer 2:

The above crude material was suspended in THF (400ml) and water (40ml). A small amount (ca. 7ml) of saturated sodium bicarbonate solution was added to adjust pH to 7.5 (starting pH=6, pH paper). The mixture was stirred in the round bottom flask for 30mintes. Solid was collected by vacuum filtration. More THF (200ml) was added to the mixture to facilitate transferring material. The filter cake was washed with THF (40ml) and water (100ml) and was dried in a vacuum oven at 40°C. Compound 1, diastereomer 2 was obtained as white solid. Net = 50.0g, Chemical purity = 97%, (AUC, HPLC Method A, see Example 10); diastereomer purity= 94%. The material was further purified as following: triturated with Acetonitrile/water (50ml/450ml) to improve diastereomer purity; dissolved in THF/water (3:1, 360ml) and filtered through a celite pad to remove a polar impurity (RT= 0.7min, HPLC method A); 2^{nd} trituration with Acetonitrile/water (50ml/450ml) to remove an unknown impurity observed by ¹H-NMR (δ = 11.7 ppm). Final clean diastereomer 2 was obtained with both chemical and diastereomer purity >99%. Net = 38.5g.

### EXAMPLE 12

### {9-[(2R)2-C-methyl-β-D-ribo-furanosyl]-guanin}-5'-yl-O-(triphenylmethyloxy-tert-butyl-S-acyl-2-thioethyl) H-phosphonate, Compound 11-1

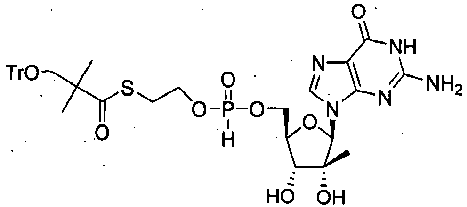

To a stirred solution of 9-(2-C-methyl-β-D-ribofuranosyl)guanine (4.87 mmol) and *S*-(2-phosphite-ethyl) 2,2-dimethyl-3-triphenylmethyloxy-thiopropionate triethylamine salt (6.34 mmol) in pyridine (75ml) at -15 °C was added dropwise pivaloyl chloride (9.74 mmol) under nitrogen. The reaction mixture was stirred at -15°C for 2 hours. Dichloromethane and NH₄Cl solution were added. Organic phase was separated washed with NH₄Cl solution, dried over Na₂SO₄, filtered and concentrated under reduced pressure. The crude material was purified by silica gel chromatography (DCM/MeOH) to yield the title compound 11-1. **Molecular Formula: C₃₇H₄₂N₅O₉PS. ¹H NMR** (DMSO-*d₆*, 400 MHz) δ (ppm) 0.80 (s, 3H), 1.13 (s, 6H), 3.04 (s, 2H), 3.14 (m, 2H), 3.97-4.08 (m, 4H), 4.28-4.38 (m, 2H), 5.35-5.10 (m, 2H), 5.77 (s, 1H), 6.52 (brs, 2H), 6.87-6.89 (m, 2H), 7.11-7.43 (m, 15H), 7.75 (s, 1H),10.67 (brs, 1H). **³¹P NMR** (DMSO-*d₆*, 162 MHz) δ (ppm) 9.20 (s) 9.47 (s). **Scan ES⁺** 764 (M+H)⁺

### Compound 11-2a

### {9-[(2R)2-C-methyl-β-D-ribo-furanosyl]-guanin}-5'-yl-O-(triphenylmethyloxy-tert-butyl-S-acyl-2-thioethyl) isopropyl phosphoramidate

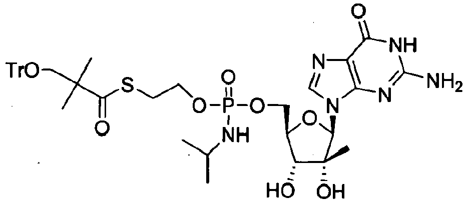

To a solution of compound **11-1** (0.98 mmol) in carbon tetrachloride (10 ml) was added isopropylamine (4.9 mmol). The reaction mixture was stirred for 3 hours at room temperature. Volatiles were evaporated in vacuo. The residue obtained was purified by silica gel chromatography (DCM/MeOH) to yield the title compound. **Beige solid. Molecular Formula: C₄₀H₄₉N₆O₉PS. Scan ES⁺** 821 (M+H)⁺

### Compound 11-3a

### {9-[(2R)2-C-methyl-β-D-ribo-furanosyl]-guanin}-5'-yl-O-(hydroxy-tert-butyl-S-acyl-2-thioethyl)-N-isopropyl phosphoramidate

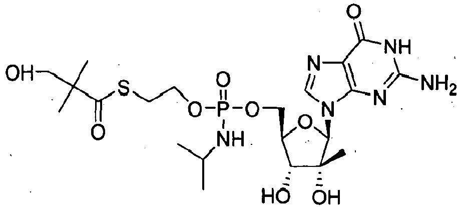

A solution of compound **11-2a** (0.5 mmol) in dichloromethane (2 ml) was stirred with trifluoro acetic acid (160 µl) for 15 minutes at room temperature. The mixture was then purified by silica gel chromatography (DCM/MeOH) to yield the title compound. **White powder. Molecular Formula: C₂₁H₃₅N₆O₉PS. ¹H NMR** (DMSO-*d₆*, 400 MHz) δ (ppm) 0.81 (s, 3H), 1.04 (m, 6H), 1.09 (s, 6H), 3.07 (t, *J*= 6.49 Hz, 2H), 3.42 (m, 2H), 3.87-3.91 (m, 2H), 3.95-3.98 (m, 2H), 4.17-4.20 (m, 2H), 4.92-4.98 (m, 2H), 5.17 (s, 1H), 5.38-5.43 (m, 1H), 6.53 (s, 2H), 7.76 (s, 1H), 10.65 (brs, 1H). **³¹P NMR** (DMSO-*d₆*, 162 MHz) δ (ppm) 8.79-8.91 (2s, 1P). **Scan ES⁺** 579 (M+H)⁺

### Compound 11-2b

### {9-[(2R)2-C-methyl-β-D-ribo-furanosyl]-guanin}-5'-yl-O-(triphenylmethyloxy-tert-butyl-S-acyl-2-thioethyl) morpholinyl phosphoramidate

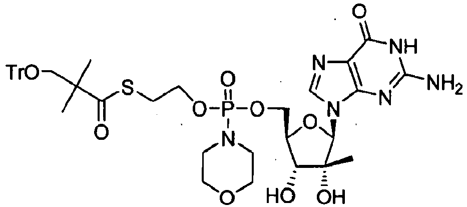

Compound **11-2b** was synthesized from compound **11-1** and morpholine following the procedure as described for compound **11-2a. Beige solid. Molecular Formula: C₄₁H₄₉N₆O₁₀PS. Scan ES⁺** 849 (M+H)⁺

### Compound 11-3b

### 3-hydroxy-2,2-dimethyl-thiopropionic acid-S-{[9-[(2R)2-C-methyl-β-D-ribo-furanosyl]-guanin-5'-yl]-[(morpholin-1-yl)-phosphinoyloxy]-ethyl}ester

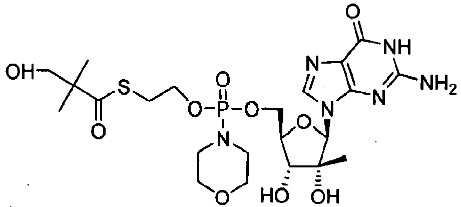

Compound **11-3b** was synthesized from compound **11-2b** following the procedure as described for compound **11-3a. White powder. Molecular Formula: C₂₂H₃₅N₆O₁₀PS.. ¹H NMR** (DMSO-*d₆*, 400 MHz) δ (ppm) 0.82 (s, 3H), 1.10 (s, 6H), 3.00 (m, 4H), 3.08-3.11 (m, 2H), 3.42 (m, 2H), 3.48-3.58 (m, 4H), 3.95-4.00 (m, 4H), 4.20 (m, 2H), 4.91-4.94 (m, 1H), 5.20 (s, 1H), 5.46 (m, 1H), 6.52 (s, 2H), 7.75 (2s, 1H), 10.64 (brs, 1H). **³¹P NMR** (DMSO-d₆, 162 MHz) δ (ppm) 7.61-7.75 (2s, 1P). **Scan ES⁺** 607 (M+H)⁺

### Compound 11-2c

### {9-[(2R)2-C-methyl-β-D-ribo-furanosyl]-guanin}-5'-yl-O-(triphenylmethyloxy-tert-butyl-S-acyl-2-thioethy) tert-butyl phosphoramidate

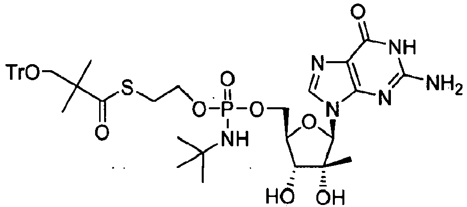

Compound **11-2c** was synthesized from compound **11-1** and tert-butylamine following the procedure as described for compound **11-2a. Beige solid. Molecular Formula: C₄₁H₅₁N₆O₉PS. Scan ES⁺** 835 (M+H)⁺

### Compound 11-3c

### {9-[(2R)2-C-methyl-β-D-ribo-furanosyl]-guanin}-5'-yl-O-(hydroxy-tert-butyl-S-acyl-2-thioethyl)-N-tert-butyl phosphoramidate

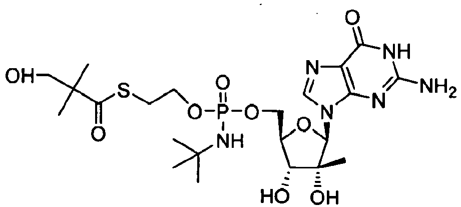

Compound **11-3c** was synthesized from compound **11-2c** following the procedure as described for compound **11-3c.White powder. Molecular Formula: C₂₂H₃₇N₆O₉PS. ¹H NMR** (DMSO-*d₆*, 400 MHz) δ (ppm) 0.81 (s, 3H), 1.09 (s, 6H), 1.17 (s, 9H), 3.06 (t, *J* = 6.45 Hz, 2H), 3.41 (m, 2H), 3.86-3.90 (m, 2H), 3.99 (m, 2H), 4.12 (m, 1H), 4.82-4.92 (m, 2H), 5.15 (s, 1H), 5.35-5.39 (m, 1H), 6.52 (s, 2H), 7.77 (s, 1H), 10.42 (brs, **1H).³¹P NMR** (DMSO-*d₆*, 162 MHz) δ (ppm) 7.42-7.46 (2s, 1P). **Scan ES⁺** 593 (M+H)⁺

### Compound 11-2d

### {9-[(2R)2-C-methyl-β-D-ribo-furanosyl]-guanin}-5'-yl-O-(triphenylmethytoxy-tert-butyl-S-acyl-2-thioethyl) N-methylpiperazyl phosphoramidate

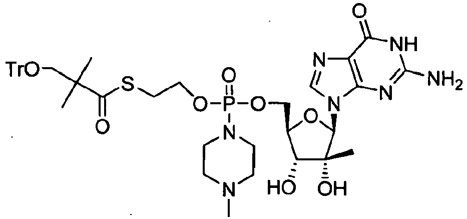

Compound **11-2d** was synthesized from compound **11-1** and N-methylpiperazine following the procedure as described for compound **11-2a. Beige solid. Molecular Formula: C₄₂H₅₂N₇O₉PS. Scan ES⁺** 862 (M+H)⁺

### Compound 11-3d

### 3-hydroxy-2,2-dimethyl-thiopropionic acid-S-{[9-[(2R)2-C-methyl-β-D-ribo-furanosyl]-guanin-5'-yl-[(4-methyl-piperazin-1-yl)-phosphinoyloxy]-ethyl}ester

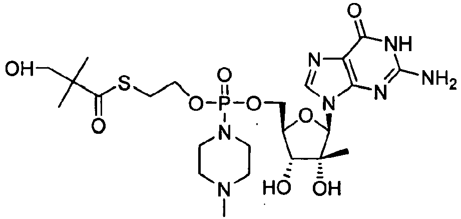

Compound **11-3d** was synthesized from compound **11-2d** following the procedure as described for compound **11-3a. White powder. Molecular Formula: C₂₃H₃₈N₇O₉PS. ¹H NMR** (DMSO-*d₆*, 400 MHz) δ (ppm) 0.83 (s, 3H), 1.11 (s, 6H), 2.50 (m, 3H), 2.52-2.65 (m, 4H), 2.90-3.02 (m, 2H), 3.05-3.12 (m, 4H), 3.42 (m, 2H), 3.95-4.02 (m, 4H), 4.21-4.24 (m, 2H), 4.64 (m, 1H), 5.22 (s, 1H), 5.45 (m, 1H), 6.62 (s, 2H), 7.74 (s, 1H), 10.73 (s, 1H). **³¹P NMR** (DMSO-*d₆*, 162 MHz) δ (ppm) 7.25-7.32 (2s, 1P). **Scan ES⁻** 618 (M+H)⁻

### Compound 11-2e

### {9-[(2R)2-C-methyl-β-D-ribo-furanosyl]-guanin}-5'-yl-O-(triphenylmethyloxy-tert-butyl-S-acyl-2-thioethyl)-acetic-acid-ethyl-ester phosphoramidate

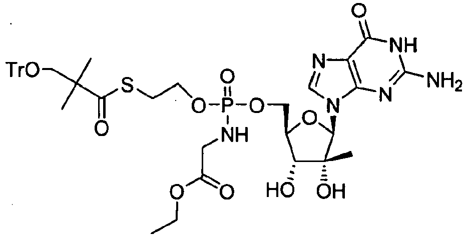

Compound **11-2e** was synthesized from compound **11-1** and glycine ethyl-ester following the procedure as described for compound **11-2a. Beige solid. Molecular Formula: C₄₁H₄₉N₆O₁₁PS. Scan ES⁺** 864 (M+H)⁺

### Compound 11-3e

### {9-[(2R)2-C-methyl-β-D-ribo-furanosyl]-guanin}-5'-yl-O-(hydroxy-tert-butyl-S-acyl-2-thioethyl)-N-acetic-acid-ethyl-ester phosphoramidate

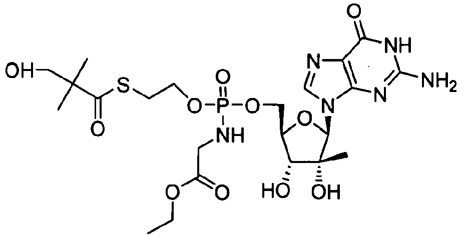

Compound **11-3e** was synthesized from compound **11-2e** following the procedure as described for compound **11-3a. White powder. Molecular Formula: C₂₂H₃₅N₆O₁₁PS. ¹H NMR** (DMSO-d₆, 400 MHz) δ (ppm) 0.81 (s, 3H), 1.10 (s, 6H), 1.12-1.17 (m, 3H), 3.05-3.07 (m, 2H), 3.29 (m, 1H), 3.42 (m, 2H), 3.53-3.57 (m, 2H), 3.85-3.96 (m, 4H), 4.07 (q, *J* = 7.00 Hz, 2H), 4.15 (m, 1H), 4.24 (m, 1H), 4.92 (td, *J* = 5.50 Hz and *J* = 1.30 Hz, 1H), 5.15 (s, 1H), 5.36-5.41 (m, 1H), 5.40-5.55 (m, 1H), 6.52 (s, 2H), 7.73 (s, 1H), 10.63 (brs, 1H). **³¹P NMR** (DMSO-*d₆* 162 MHz) δ (ppm) 9.07-9.19 (2s, 1P). **Scan ES⁺** 623 (M+H)⁺

### Compound 11-2f

### {9-[(2R)2-C-methyl-β-D-ribo-furanosyl]-guanin}-5'-yl-O-(triphenylmethyloxy-tert-butyl-S-acyl-2-thioethyl)-acetic-acid-tert-butyl ester phosphoramidate

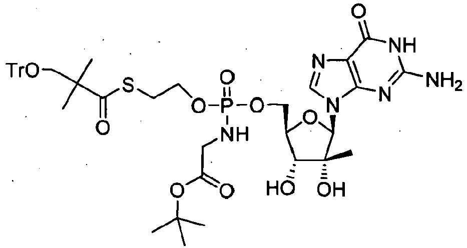

Compound **11-2f was** synthesized from compound **11-1** and glycine tert-butylester following the procedure as described for compound **11-2a. Beige solid. Molecular Formula: C₄₃H₅₃N₆O₁₁PS. Scan ES⁺** 893 (M+H)⁺

### Compound 11-3f

### {9-[(2R)2-C-methyl-β-D-ribo-furanosyl]-guanin}-5'-yl-O-(hydroxy-tert-butyl-S-acyl-2-thioethyl)-N-acetic-acid-tert-butyl-ester phosphoramidate

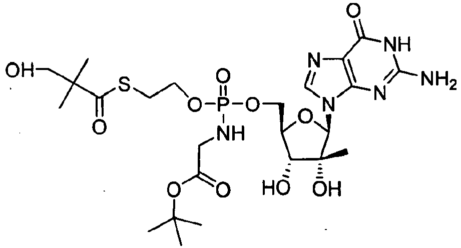

Compound **11-3f** was synthesized from compound **11-2f** following the procedure as described for compound **11-3a. White powder. Molecular Formula: C₂₄H₃₉N₆O₁₁PS. ¹H NMR** (DMSO-*d₆*, 400 MHz) δ (ppm) 0.81 (s, 3H), 1.10 (s, 6H), 1.37 (s, 9H), 3.05-3.07 (m, 2H), 3.29 (s, 1H), 3.40-3.44 (m, 4H), 3.93-3.97 (m, 4H), 4.13-4.15 (m, 1H), 4.22-4.25 (m, 1H), 4.92 *(td, J=* 5.50 Hz and *J*= 1.47 Hz, 1H), 5.15 (s, 1H), 5.36-5.44 (m, 2H), 6.51 (s, 2H), 7.74 (s, 1H), 10.63 (s, 1H). **³¹P NMR** (DMSO-*d₆*, 162 MHz) δ (ppm) 9.08-9.26 (2s, 1P). **Scan ES⁺** 651 (M+H)⁺

### Compound 11-2g

### {9-[(2R)2-C-methyl-β-D-ribo-furanosyl]-guanin}-5'-yl-O-(triphenylmethyloxy-tert-butyl-S-acyl-2-thioethyl)-dimethylamino-ethyl phosphoramidate

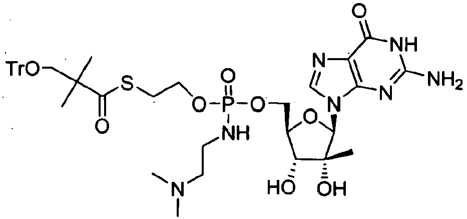

Compound **11-2g** was synthesized from compound **11-1** and dimethyl-amino-ethylamine following the procedure as described for compound **11-2a. Molecular Formula: C₄₁H₅₇N₇O₉PS. Scan ES⁺** 850 (M+H)⁺

### Compound 11-3g

### {9-[(2R)2-C-methyl-β-D-ribo-furanosyl]-guanin}-5'-yl-O-(hydroxy-tert-butyl-S-acyl-2-thioethyl)-N-dimethyl-amino-ethyl phosphoramidate

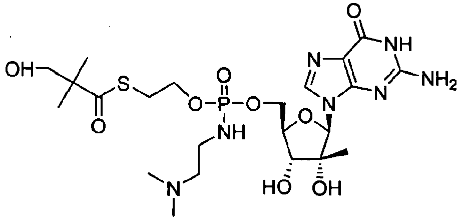

Compound **11-3g** was synthesized from compound **11-2g** following the procedure as described for compound **11-3a. White powder. Molecular Formula: C₂₂H₃₈N₇O₉PS. ¹H NMR** (DMSO-*d₆*, 400 MHz) δ (ppm) 0.83 (s, 3H), 1.11 (s, 6H), 2.67 (s, 6H), 3.08-3.10 (m, 2H), 3.10-3.12 (m, 4H), 3.42-3.45 (m, 2H), 3.94-4.01 (m, 4H), 4.15-4.26 (m, 2H), 4.95 (m, 1H), 5.22 (m, 1H), 5.41-5.47 (m, 2H), 6.63 (s, 2H), 7.30 (m, 1H), 7.77 (s, 1H), 10.76 (s, 1H). **³¹P NMR** (DMSO-*d₆*, 162 MHz) δ (ppm) 9.31-9.40 (2s, 1P). **Scan Es⁺** 608 (M+H)⁺

### Compound 11-3h

### {9-[(2R)2-C-methyl-β-D-ribo-furanosyl]-guanin}-5'-yl-O-(hydroxy-tert-butyl-S-acyl-2-thioethyl)-N-acetic-acid phosphoramidate

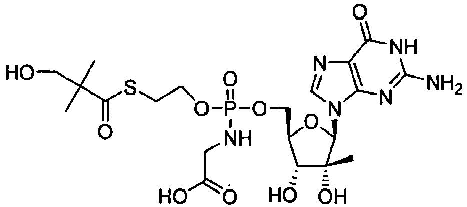

To a stirred solution of compound **11-3f** (0.16 mmol) in dichloromethane (2 ml) was added TFA (35 eq). The reaction mixture was stirred for 30 minutes at 50°C and at room temperature for 16 hours. The mixture was then evaporated and purified by silica gel chromatography (DCM/MeOH) to yield the title compound. **White powder. Molecular Formula: C₂₀H₃₁N₆O₁₁PS. ¹H NMR** (DMSO-*d₆*, 400 MHz) δ (ppm) 0.80 (s, 3H), 1.09 (s, 6H), 2.95 (t, *J* = 6.50 Hz, 2H), 3.28 (m, 2H), 3.40 (m, 2H), 3.67-3.69 (m, 2H), 3.84-3.86 (m, 2H), 4.19-4.20 (m, 1H), 4.27 (m, 1H), 4.89 (s, 1H), 5.08 (t, *J* = 5.70 Hz, 1H), 5.64 (s, 1H), 6.31 (s, 1H), 6.71 (s, 2H), 7.79 (s, 1H), 10.55 (s, 1H).

### EXAMPLE 13

### Biological activity of Compound 1, diastereomer 1 and diastereomer 2

The biological activity of Compound **1,** diastereomer 1 and diastereomer 2 was measured by monitoring expression of the NS4A protein by enzyme-linked immunosorbent assay (ELISA) and by luciferase assay as described below:

### Luciferase replicon assay

The HCV luciferase replicon assay measures the ability of a test compound to inhibit HCV replication in cell culture after 3 days of treatment in a human hepatoma cell line (Huh-7) bearing the HCV genotype 1b replicon and a luciferase-neomycin phosphotransferase fusion gene. The inhibition of HCV replication was measured by quantification of luciferase protein expression. The HCV luciferase replicon was constructed by fusing a luciferase gene to the amino terminus of the neomycin phosphotransferase gene of the ZS11 HCV replicon. The ZS11 HCV replicon (Zhu Q, Guo J-T, and Seeger C, 2003) is derived from the SP1 replicon, but contains three cell culture-adapted mutations: E1202G (NS3), S2204I (NS5A), and D2254E (NS5A). These mutations allow for efficient *in vitro* replication. The luciferase reporter gene was amplified from the pGL4.13[luc2/SV40] vector (Promega Corp.). The replicon resulting from the luciferase-neomycin fusion (ZS11-luc) was stably-transfected into Huh-7 cells, yielding the Zluc cell line.

The luciferase replicon assay is summarized as follows: Solid white 96-well tissue culture plates (BD Falcon) were seeded with Zluc cells. Antiviral compound solutions were made up freshly in medium as 2X-concentrated stocks. Eight additional 3-fold drug dilutions were prepared from these stocks in medium for a total of 9 dilutions. At least four hours after Zluc cells were seeded, drug treatment was initiated by adding one volume of each 2X-concentrated drug dilution to one volume of cells/medium in the tissue culture plates. Cells were then incubated for 3 days at 37°C/5% CO₂.

The media/compound was then removed from the plates and the ONE-glo Luciferase assay reagent (Promega) was added to each well. The assay plates were shaken for 3 minutes at room temperature and luciferase activity for each well was measured with a 1 sec read time on the Victor³V multilabel counter using a 700 nm cut-off filter (Perkin Elmer). EC₅₀ values were calculated from dose response curves from the resulting best-fit equations determined by Microsoft Excel and XLfit 4.1 software.

### HCV replicon assay (NS4A ELISA)

The HCV replicon assay measures the ability of a test compound to inhibit HCV replication in cell culture after 3 days of treatment in a human hepatoma cell line (Huh-7) bearing the HCV genotype 1b replicon (GS4.1 cells). The inhibition of HCV replication was measured by quantification of viral NS4A protein using an enzyme-linked immunosorbent assay (ELISA). The GS4.1 cell line (Zhu, Guo and Seeger 2003) was derived from the Huh-7 human liver cell line and stably possesses the bicistronic SP1 ΔS HCV replicon that contains nonstructural proteins from NS3 to NS5B from the HCV strain con1, genotype 1b, under control of the EMCV IRES promoter. Additionally, the replicon contains the neomycin phosphotransferase gene under the control of the HCV promoter (Source: Dr. Christoph Seeger, Fox Chase Cancer Center, Philadelphia, PA).

The NS4A ELISA assay is summarized as follows. Ninety-six-well tissue culture plates were seeded with GS4.1 cells. Antiviral compound solutions were made up freshly in medium as 2X-concentrated stocks. Eight additional 3-fold drug dilutions were prepared from these stocks in medium for a total of 9 dilutions. At least four hours after GS4.1 cells were seeded, drug treatment was initiated by adding one volume of each 2X concentrated drug dilution to one volume of cells/medium in the tissue culture plates. Cells were then incubated for 3 days at 37°C/5% CO₂.

The media/compound was then removed from the plates and cells were fixed with acetone:methanol, washed three times with a wash solution, and then blocked for 1 hour with 10% fetal bovine serum in a balanced salt solution. Cells were washed three times and incubated with an anti-hepatitis C NS4A monoclonal antibody (Virogen Corp.) for 2 hours at 37°C. Cells were washed three times and incubated with a horseradish peroxidase conjugated goat anti-mouse antibody (Zymed) for 1 hour at 37°C. Afterwards, cells were washed three times and exposed to a solution containing ortho-phenylenediamine (Zymed) and hydrogen peroxide (EMD Biosciences) for 30 minutes in the dark. The reaction was stopped with diluted sulfuric acid (Mallinckrodt Chemicals) and absorbance measured on a Victor³V 1420 multilabel counter (Perkin Elmer). EC₅₀ values were calculated from dose response curves from the resulting best-fit equations determined by Microsoft Excel and XLfit 4.1 software.

### Cytotoxicity assay

The cytotoxicity assay measures the viability of cells after treatment with a test compound for 3 days in either GS4.1 cells or Zluc cells. The assay readout is the bioreduction of the yellow MTS tetrazolium compound to a purple formazan product. This conversion is mediated by NADPH or NADH and is directly proportional to the number of live cells in a culture. The cytotoxicity assay is summarized as follows. Ninety-six-well tissue culture plates were seeded with GS4.1 or Zluc cells. Antiviral compound solutions were made up freshly in medium as 2X-concentrated stocks. Eight additional 3-fold drug dilutions were prepared from these stocks in medium for a total of 9 dilutions. At least four hours after the cells were seeded, drug treatment was initiated by adding one volume of each 2X-concentrated drug dilution to one volume of cells/medium in the tissue culture plates. Cells were then incubated for 3 days at 37°C/5% CO₂. After 3 days of treatment, the CellTiter 96^{®} Aqueous One Solution cell proliferation assay (Promega) was performed by adding the MTS solution to each well. The plates were then incubated at 37°C/5% CO₂ for 3.5 hours. Plates were then read in a Victor³V 1420 multilabel counter (Perkin Elmer) and CC₅₀ concentrations were determined using Microsoft Excel and XLfit 4.1 software.

| Compound | NS4A - ELISA Assay | | | Luciferase Assay | | |
|---|---|---|---|---|---|---|
| | EC₅₀ (nM) | CC₅₀ (µM) | n | EC₅₀ (nM) | CC₅₀ (µM) | N |
| Compound **1** | 379 ± 83 | >100 | 2 | 68.0 ± 0.4 | >100 | 5 |
| Diastereomer 2 | 244 ± 98 | >100 | 2 | 56.8 ± 0.6 | >100 | 4 |
| Diastereomer 1 | 704 ± 372 | >100 | 2 | 165 ± 23 | >100 | 4 |

### EXAMPLE 14

### In vitro metabolic disposition of compound 1 in rat, monkey and human liver microsomal fractions

The test compounds used in this study were: Compound **1**, Purity >95%, Compound **4**, Purity >95%; Compound **3**, Purity 90% and Compound **2**, Purity 80%.

Storage conditions - The test compounds were stored at 4 to 8°C protected from light, DMSO stock solutions were stored at -20°C.

The following liver subcellular fractions were used in the study.

**Table 1 Liver subcellular fraction**

| **Species** | **Sex** | **Cellular Fraction** | **Source** |
|---|---|---|---|
| Rat | Male | Microsomes, Liver | XenoTech, Lenexa, KS |
| Rat | Male | Cytosol, Liver | XenoTech, Lenexa, KS |
| Monkey | Male | Microsomes, Liver | XenoTech, Lenexa, KS |
| Monkey | Male | Cytosol, Liver | XenoTech, Lenexa, KS |
| Human | Male | Microsomes, Liver | XenoTech, Lenexa, KS |
| Human | Male | Cytosol, Liver | XenoTech, Lenexa, KS |

**Table 2 CYP450 supersomes**

| CYP450 | Source |
|---|---|
| 1A2 | BD Gentest, Woburn, MA |
| 2A6 | BD Gentest, Woburn, MA |
| 3A4 | BD Gentest, Woburn, MA |
| 2B6 | BD Gentest, Woburn, MA |
| 2D6 | BD Gentest, Woburn, MA |
| 2C8 | BD Gentest, Woburn, MA |
| 2C9 | BD Gentest, Woburn, MA |
| 2C19 | BD Gentest, Woburn, MA |
| 2E1 | BD Gentest, Woburn, MA |

The following reagents were used in the study:
NADPH regenerating system Solution A: NADP⁺ + glucose-6-phosphate and Solution B: glucose-6-phosphate dehydrogenase from BD Gentest, Woburn MA; ketoconazole from BD Gentest, Woburn MA; dimethyl sulfoxide (DMSO), pig liver esterase and alkaline phosphatase from Sigma-Aldrich, St Louis, MO; potassium phosphate, monobasic (K₂HPO₄) from Fisher Scientific, Pittsburg, PA; methanol and acetonitrile from Burdick and Jackson; Muskegon, MI; 0.2 M potassium phosphate buffer, pH 7.4 prepared as follows: For 100 mL of buffer, 19 mL solution A (13.6 g KH₂PO₄/0.5 L) mixed with 81 mL solution B (17.4 g K₂HPO₄/0.5 L). No pH adjustment required.

Disappearance of parent Compound **1** was used to evaluate the *in vitro* metabolism thereof. Relative levels of unchanged Compound **1** were measured by HPLC-UV method 1. Residual Compound **1** is reported as percent difference from time zero or control. The HPLC-UV system was as follows:
Method 1

| | |
|---|---|
| HPLC: Agilent 1100 | |
| Column: | Phenomenex Luna C18(2), 250 x 4.6 mm |
| Precolumn: | Phenomenex SecurityGuard C 18 cartridge 4 x 2 mm |
| Mobile phases (MP): | (MPA) 10 mM K₂HPO₄ pH5; (MPB) ACN |
| Gradient elution: | 5 to 60% MPB run from 0 to 15 min |
| Runtime: | 15 min |
| Post time: | 6 min |
| Flow rate: | 1 mL/min |
| Injection volume: | 20 µL |
| UV: 252 | nm |

Preliminary identification of putative metabolites was conducted by comparing the retention time of observed metabolites with retention times obtained with chemically synthesized standards using the following HPLC-UV method:
Method 2

| Column: | Phenomenex Luna C18(2), 250 x 4.6 mm | |
|---|---|---|
| Precolumn: | Phenomenex SecurityGuard C18 cartridge 4 x 2 mm | |
| Mobile phases (MP): | (MPA) 10 mM K₂HPO₄ pH5; (MPB) MeOH | |
| Gradient elution: | Time (min) | %MPB |
| | 0 | 5 |
| | 15 | 30 |
| | 20 | 30 |
| | 30 | 45 |
| Runtime: | 60 min | |
| Post time: | 6 min | |
| Flow rate: | 1 mL/min | |
| Injection volume: | 20 µL | |
| UV: | 252 nm | |

Representative HPLC chromatogram of Compound **1** (diastereomers 1 and 2) and metabolite standards obtained by method 2 is provided in Figure 2.

### Compound 1 stability in liver microsomes and cytosol

Incubations with liver microsomes or cytosol were conducted in a final volume of 0.1 mL per incubation time point. Ten µM Compound 1 from a stock solution in DMSO (final DMSO concentration was 0.1%) was incubated at 37°C from 0 - 60 min with pooled microsomal protein (1.0 mg/mL), suspended in incubation buffer (0.1 M potassium phosphate, pH 7.4, 5 mM MgCl₂, and 0.1 mM EDTA). The microsomal reaction was initiated by the addition of NADPH (3 mM final concentration). Incubations with (a) no protein or (b) no NADPH served as controls. Reactions were terminated by the addition of 0.2 mL of stop solution (acetonitrile). The samples were vortex-mixed for 30 sec and then centrifuged at 10,000xg for 10 min. The supernatant was dried using a Labconco CentriVap concentrator and the dry residue reconstituted in water, transferred to an HPLC glass vial and analyzed by HPLC-UV method 1 (described above).

Results: The extent of Compound **1** metabolism in liver microsomes was determined by assessing its depletion over time and is reported as percent (%) residual Compound **1** (Table 3). Significant NADPH-dependent metabolism was observed; 100% in monkey in 30 min, and 64 and 66% in rat and human in 60 min, respectively. Substantial NADPH-independent metabolism was also observed in monkey with approximately 60% of unchanged Compound 1 remaining after 60 min.

**Table 3 Residual Compound 1 following incubation with liver microsomes**

| | Time (min) | % Residual Compound 1 | |
|---|---|---|---|
| Species | | No Cofactor | NADPH |
| Rat | 0 | 100 | 100 |
| | 30 | 103 | 53 |
| | 60 | 103 | 36 |
| Monkey | 0 | 100 | 100 |
| | 30 | 81 | 0 |
| | 60 | 64 | 0 |
| Human | 0 | 100 | 100 |
| | 30 | 100 | 52 |
| | 60 | 94 | 34 |

| | | | |
|---|---|---|---|
| Microsome (1 mg/mL) incubations were conducted as described under experimental procedures. Test article concentration was 10 µM. Values are expressed in percent. | | | |

### CYP3A4 inhibition

Incubations were conducted in a final volume of 0.1 mL per incubation time point. Pooled rat (1 mg/mL), monkey (0.5 mg/mL) or human (1.0 mg/mL) liver microsomes, suspended in incubation buffer (0.1 M potassium phosphate, pH 7.4) were incubated with 10 µM Compound 1 in the presence of ketoconazole or ritonavir (0.1 and 1 µM). Final solvent (DMSO or methanol) concentration was maintained at 0.2%. Reactions were initiated by the addition of an NADPH regenerating system (1.3 mM NADP⁺, 3.3 mM glucose-6-phosphate, 0.4 U/ml glucose-6-phosphate dehydrogenase, and 3.3 mM MgCl₂, final concentrations). Incubations with no inhibitor served as control. Reactions were terminated at 30 min (monkey) or 60 min (rat and human) by the addition of 0.2 mL of stop solution (acetonitrile). The samples were vortex-mixed for 30 sec and then centrifuged at 10,000xg for 10 min. The supernatant was dried using a Labconco CentriVap concentrator and the dry residue reconstituted in water, transferred to an HPLC glass vial and analyzed by HPLC-UV method 1 (described above).

Additional control incubations were conducted with pooled human (0.25 mg/mL) liver microsomes suspended in incubation buffer (0.1 M potassium phosphate, pH 7.4, 5 mM MgCl₂, and 0.1 mM EDTA) and incubated with 50 µM testosterone (CYP3A4 marker substrate) in the presence of ketoconazole or ritonavir (0.1 and 1 µM). Final solvent (DMSO or methanol) concentration was maintained at 0.2%. Reactions were initiated by the addition of NADPH (3 mM final concentration). Incubations with no inhibitor served as control. Reactions were terminated after 15 min by the addition of 0.2 mL of stop solution (acetonitrile). The samples were vortex-mixed for 30 sec and then centrifuged at 10,000xg for 10 min. The supernatant was transferred to an HPLC glass vial and analyzed by HPLC-UV.

Results: The potential role of CYP3A4 in the NADPH-dependent metabolism of Compound **1** was evaluated using ketoconazole and ritonavir, two CYP3A4 inhibitors, and the results are summarized in Table 4.

**Table 4 Effect of ritonavir and ketoconazole on the metabolic stability of Compound 1 in microsomes**

| Species | Ketoconazole (µM) | % Residual Compound **1** | Ritonavir (µM) | % Residual Compound **1** |
|---|---|---|---|---|
| Rat | Control^{a} | 100 | Control | 100 |
| | 0 | 86 | 0 | 86 |
| | 0.1 | 83 | 0.1 | 101 |
| | 1 | 120 | 1 | 123 |

| Monkey | Control | 100 | Control | 100 |
|---|---|---|---|---|
| | 0 | 25 | 0 | 25 |
| | 0.1 | 68 | 0.1 | 41 |
| | 1 | 125 | 1 | 122 |

| Human | Control | 100 | Control | 100 |
|---|---|---|---|---|
| | 0 | 65 | 0 | 65 |
| | 0.1 | 103 | 0.1 | 92 |
| | 1 | 117 | 1 | 117 |

| | | | | |
|---|---|---|---|---|
| ^{a} No protein Microsomal incubations were conducted in the presence or absence of CYP3A4 inhibitors as described above. Test compound concentration was 10 µM. Values are expressed in percent. Formation of 6-β-hydroxytesosterone in human liver microsomes was inhibited 90% at 1 µM ketoconazole and ritonavir. | | | | |

Because Compound **1** is rapidly metabolized in monkey liver microsomes, incubation conditions were adjusted by reducing protein content to 0.5 mg/mL and incubation time to 30 min. Incubation conditions for rat and human were unchanged; protein was 1 mg/mL and incubation time was 60 min. Complete inhibition of Compound 1 was observed at 1 µM ketoconazole or ritonavir in all three species, suggesting CYP3A is associated with NADPH-dependent metabolism of Compound **1** in liver microsomes. 6-β-hydroxylation of testosterone in human liver microsomes was inhibited 90% with 1 µM ketoconazole or ritonavir, demonstrating suitable reaction conditions for the inhibitors.

### Identification of CYP isoenzyme associated with Compound 1 metabolism

Supersomes- Incubations were conducted in a final volume of 0.1 mL reaction buffer (50-100 mM potassium phosphate, pH 7.4 or 100 mM Tris-HCL, pH 7.4) according to protocol provided with each CYP450 supersome. Each reaction contained 4 pmole of CYP450 supersome, 1.3 mM NADP⁺, 3.3 mM glucose-6-phosphate, 0.4 U/mL glucose-6-phosphate dehydrogenase, 3.3 mM MgCl₂ and 10µM Compound **1**. Following incubation for 60 min at 37°C, 100 µL of stop solution (acetonitrile:methanol;1:1) was added and samples centrifuged for 10 min at 10,000xg. The supernatant was then dried and reconstituted in 10 mM potassium phosphate, pH 5, further transferred to an HPLC glass vial and analyzed by HPLC-UV (described above).

Results: Using human recombinant CYP enzymes, CYP3A4 was demonstrated to be the only CYP catalyzing Compound **1** metabolism (Table 5), further confirming CYP3A4 involvement.

**Table 5 Percent residual Compound 1 after incubation with human recombinant CYP450 enzymes (supersomes)**

| CYP450 | % Residual Compound 1 |
|---|---|
| 1A2 | 104 |
| 2A6 | 105 |
| 2B6 | 108 |
| 2D6 | 102 |
| 2C8 | 105 |
| 2C9 | 109 |
| 2C19 | 111 |
| 2E1 | 100 |
| 3A4 | 62 |

| | |
|---|---|
| Supersome incubations were carried out as described under experimental procedures above. Values are expressed in percent and represent the average of two independent experiments. | |

### Metabolite profiling

Incubations with liver microsomes, liver cytosol or pig liver esterase were conducted in a final volume of 0.1 mL per incubation time point. Pooled microsomal protein (1.0 mg/mL) suspended in incubation buffer (0.1 M potassium phosphate, pH 7.4) was incubated for 2 hours at 37°C with 50 µM Compound **1** from a stock solution in DMSO (final DMSO concentration was 0.1%); the microsomal reaction was initiated by the addition of an NADPH regenerating system (1.3 mM NADP⁺, 3.3 mM glucose-6-phosphate, 0.4 U/ml glucose-6-phosphate dehydrogenase, and 3.3 mM MgCl₂, final concentrations). Incubations with no NADPH or no protein served as controls. Reactions were terminated by the addition of 0.1 mL of stop solution (acetonitrile). The samples were vortex-mixed for 30 sec and then centrifuged at 10,000xg for 10 min. The supernatant was dried using a Labconco CentriVap concentrator and the dry residue reconstituted in water, transferred to an HPLC glass vial and analyzed by HPLC-UV method 2 (described above).

Pooled liver cytosol (1.0 mg protein/mL) suspended in incubation buffer (0.1 M potassium phosphate, pH 7.4) was incubated for 2 hours at 37°C with 10 µM Compound 1 from a stock solution in DMSO (final DMSO concentration was 0.1%); reaction was initiated with the addition of test article. Incubations with no protein served as control. Reactions were terminated by the addition of 0.1 mL of stop solution (acetonitrile). The samples were vortex-mixed for 30 sec and then centrifuged at 10,000xg for 10 min. The supernatant was dried using a Labconco CentriVap concentrator and the dry residue reconstituted in water, transferred to an HPLC glass vial and analyzed by HPLC-UV method 2 (described above).

Pig liver esterase (14 U) suspended in incubation buffer (0.1 M potassium phosphate, pH 7.4) was incubated for 60 min at 37°C with 10 µM Compound 1 from a stock solution in DMSO (final DMSO concentration was 0.1%); reaction was initiated with the addition of test article. Incubations with no protein served as controls. Reactions were terminated by the addition of 0.1 mL of stop solution (acetonitrile). The samples were vortex-mixed for 30 sec and then centrifuged at 10,000xg for 10 min. The supernatant was dried using a Labconco CentriVap concentrator and the dry residue reconstituted in water, transferred to an HPLC glass vial and analyzed by HPLC-UV method 2 (described above).

Metabolic stability of the proposed metabolites was conducted by incubating the chemically synthesized standards Compound **2**, Compound **4** and Compound **3** as described above for Compound **1**. When possible, formation of Compound **5** was verified by incubating samples with alkaline phosphatase (10 U) for 60 min at 37°C.

Results: Preliminary identification was accomplished by comparing LC elution of metabolites with that of available synthetic standards of putative metabolites (Figure 2). Metabolite assignments and preliminary identification are summarized in Table 6. Metabolites U1, U2 and U3 were characterized based on the retention times.

**Table 6: In vitro metabolite profile in microsomes following incubation with Compound 1**

| Metabolite assignment^{a} | Retention time (min) | Rat | Monkey | Human |
|---|---|---|---|---|
| Compound 5 | 5.430 | ND | + | + |
| 2'-C-methyl guanosine | 9.058 | + | + | + |
| Compound 2 | 22.861 | ND | ND | ND |
| Compound 3 (diastereomer 1) | 23.499 | + | + | + |
| Compound 3 (diastereomer 2) | 26.700 | ? | + | ? |
| Compound U1 | 35.597 | ND | + | ND |
| (structure unknown) | | | | |
| Compound U2 | 36.459 | + | ND | ND |
| (structure unknown) | | | | |
| Compound U3 | 37.758 | ND | + | ND |
| (structure unknown) | | | | |
| Compound 1 | 48.340 and 53.508 | + | + | + |
| ^{a} Assignment based on co-elution with synthetic standards. | | | | |
| ^{b} Due to matrix interference, formation of Compound 5 in the presence of NADPH could not be | | | | |
| assessed. Metabolite was observed in human and rat microsomes without NADPH. | | | | |
| +: peak detected | | | | |
| ?: peak observed | | | | |
| ND: not detected | | | | |

Seven potential metabolites were observed, four of which have been tentatively identified based on co-elution with synthetic standards as Compound 5, 2'-C-methyl guanoine, and diastereomers 1 and 2 for Compound **3**. Three unknown metabolites Compounds U1, U2 and U3. Diastereomers 1 and 2 for Compound **3**, and Compounds U1, U2 and U3 were observed in incubations conducted with NADPH, suggesting CYP450 involvement in their formation. Diastereomers 1 and 2 for Compound **3** were observed in all three species. Compound U2 was only observed in rat and Compounds U1 and U3 were only observed in monkey. Formation of Compound **5** was observed in monkey and human microsomes without NADPH. Its identification as the 5'-monophosphate of 2'-C-methyl guanosine, was confirmed by treating samples with phosphatase which cleaves the phosphate group, releasing the nucleoside 2'-C-methyl guanosine. Formation of Compound **5** in samples incubated with NADPH could not be determined due to matrix interference. However, it should be noted that Compound **5** could degrade to 2'-C-methyl guanosine, which was detected in NADPH samples for all three species, suggesting that Compound **5** was indeed formed.

Metabolism of Compound **1** was not observed in liver cytosol. In 60-min incubations with pig liver esterase, 25% of Compound **1** was converted to Compound **2** (Table 7), a metabolite that was not observed in microsomal incubations.

Compound **2** undergoes rapid metabolism to Compound **5** in microsomes (Table 8), in cytosol (Table 9) and with pig liver esterase (Table 7). Minor (10% in rat) to moderate (20-30% in human and monkey) NADPH-dependent metabolism of Compound 3 (diastereomer 1 and diastereomer 2) (Table 10) was noted. Minor NADPH-independent metabolism was also noted but only in monkey microsomes (Table 8).

**Table 7: Residual Compound 1, Compound 4, Compound 3 and Compound 2 following incubation with pig liver esterase**

| | Compound **1** | Compound **3** | Compound **4** | Compound **2** |
|---|---|---|---|---|
| Control^{a} | 100 | 100 | 100 | 100 |
| Esterase | 75 | 97 | 100 | 0 |
| ^{a}No protein | | | | |
| Esterase (14 U) incubations were conducted for 60 min as described under experimental conditions; test article concentration was 10 µM. Values are expressed as percent. | | | | |
| Compounds **2** and **3** have been identified as *in vitro* metabolites of Compound 1. Compound 4 has not been observed *in vitro.* | | | | |

**Table 8: Residual Compound 2 following incubation with liver microsomes**

| | % Residual Compound **2** | |
|---|---|---|
| Species | No Cofactor | NADPH |
| Control^{a} | 100 | 100 |
| Rat | 0 | 0 |
| Monkey | 0 | 19 |
| Human | 0 | 0 |
| ^{a}No protein | | |
| Microsome (1 mg/mL) incubations were carried out at 37°C for 15 min as described for | | |
| Compound **1** under experimental conditions; test article concentration was 10 µM. Values are expressed in percent. | | |
| Compound **2** has been identified as an *in vitro* metabolite of Compound **1**. | | |

**Table 9: Residual Compounds 1, 4, 3 and 2 following incubation with liver cytosol**

| Species | Compound **1** | Compound **3** | Compound **4** | Compound **2** |
|---|---|---|---|---|
| Control^{a} | 100 | 100 | 100 | 100 |
| Rat | 100 | 96 | 101 | 0 |
| Monkey | 100 | 99 | 103 | 0 |
| Human | 100 | 100 | 100 | 0 |
| ^{a}No protein | | | | |
| Cytosol (1 mg/mL) incubations were conducted for 2 hours as described under experimental conditions; test article concentration was 10 µM. Values are expressed in percent. | | | | |
| Compound **3** and Compound **2** have been identified as *in vitro* metabolites of Compound **1**. | | | | |
| Compound **4** has not been observed as a metabolite *in vitro.* | | | | |

**Table 10: Residual Compound 4 and Compound 3 following incubation with liver microsomes**

| | % Residual Compound **4** | | % Residual Compound **3** | |
|---|---|---|---|---|
| Species | No Cofactor | NADPH | No Cofactor | NADPH |
| Control^{a} | 100 | 100 | 100 | 100 |
| Rat | 100 | 89 | 101 | 90 |
| Monkey | 100 | 83 | 89 | 76 |
| Human | 100 | 93 | 103 | 82 |
| ^{a}No protein | | | | |
| Microsome (1 mg/mL) incubations were conducted for 60 min essentially as described for | | | | |
| Compound **1** under experimental conditions; test article concentration was 50 µM. Values are expressed in percent. | | | | |
| Compound **3** has been identified as an *in vitro* metabolite of Compound **1**. | | | | |
| Compound **4** has not been observed as a metabolite *in vitro.* | | | | |

Compound **4** was not observed in any sample following incubation with Compound **1**. The metabolic stability of this compound was evaluated to see if it could be converted to Compound **5** as was observed with Compound **2**. Only minor metabolism (10-20%) based on residual Compound **4** was observed in microsomes in the presence of NADPH. Formation of Compound **5** could not be determined in these samples due to matrix interference. NADPH-independent metabolism was not observed. No metabolism of Compound **4** was observed with either liver cytosol (Table 9) or pig liver esterase (Table 7). These observations suggest that the lack of Compound **4** in samples incubated with Compound **1** is not due to further metabolism as is the case for Compound **2**, thus suggesting that Compound **4** is not an *in vitro* metabolite of Compound **1**.

Minor (10% in rat) to moderate (20-30% in human and monkey) NADPH-dependent metabolism of Compound **3** (Table 10) was noted. Minor NADPH-independent metabolism was also noted but only in monkey microsomes (Table 10). Treatment of this sample with alkaline phosphatase resulted in the formation of 2'-C-methyl guanosine, suggesting Compound **5** was formed in monkey microsomes following incubation with Compound **3**. No metabolism of Compound **3** was observed with liver cytosol (Table 9) or pig liver esterase (Table 8).

### Conclusion

Compound **1** exhibited significant metabolism in monkey liver microsomes; metabolism in human and rat was also substantial. Compound **1** metabolism appears to be mediated by both CYP450 and non-CYP450 pathways. Using human recombinant CYP enzymes, 3A4 was identified as the only CYP involved in the NADPH-dependent metabolism of Compound **1**. Eight potential *in vitro* metabolites were observed, five identified as 2'-C-methyl guanosine, Compounds **2, 5** and two diastereomers of compound 3 (compounds **3a** and **3b**). Metabolites U1, U2 and U3 are unknown and appear to be species dependent; U2 was observed only in rat microsomes while U1 and U2 observed only in monkey microsomes. Formation of Compound **3** occurred in all three species. Formation of Compound **5** appears to be mediated by both CYP450 and non-CYP450 pathways through the formation of Compound **2**, although some formation of Compound **5** was also associated with metabolism to Compounds 3a and 3b. Although Compound **5** could not be observed in incubations with NADPH due to interference by the matrix, the presence of 2'-C-methyl guanosine in these samples suggests that Compound **5** was indeed formed, since the monophosphate can decompose to 2'-C-methyl guanosine. The non-CYP450 mediated Compound **5** formation appeared to be more significant in monkey than in rat and human, possibly accounting for the higher levels of Compound **5** and 2'-C-methyl guanosine observed in monkey microsomes.

### EXAMPLE 15

### In vitro metabolic disposition of Compound 1 and 2'-C-methyl guanosine in primary hepatocytes and in Huh-7 cells

The following cell lines were used in this study.

| Species | Sex | Cell type | Source |
|---|---|---|---|
| Rat, Sprague Dawley | Male | Primary hepatocytes | CellzDirect, Pittsboro, NC |
| Monkey, Cynomolgus | Male | Primary hepatocytes | CellzDirect, Pittsboro, NC |
| Human | Male/ Female | Primary hepatocytes | CellzDirect, Pittsboro, NC |
| Human | | Huh-7 | HCV group, Idenix Pharmaceuticals, Cambridge, MA |

Reagents: The following reagents were used in the study:
Acetonitrile, methanol and HPLC grade water from Burdick and Jackson, Muskegon, MI; alkaline phosphatase, dimethyl sulfoxide (DMSO), dexamethasone, insulin and tetrabutyl ammonium dihydrogenphosphate (TBAP) from Sigma-Aldrich, St Louis, MO; DMEM with pyruvate, L-glutamine, HEPES, non essential amino acids, penicillin-streptomycin, phosphate buffered saline (PBS) and trypsin-EDTA from Cellgro, Mediatech, Herndon, VA; Eco Lite liquid scintilation; fetal bovine serum (FBS), insulin-transferrin-selenium-A and William's E culture medium from Gibco, Invitrogen Corporation; potassium phosphate, monobasic (K₂HPO₄) from Fisher Scientific, Pittsburg, PA; and Ultima Flow-AP liquid scintilation from Perkin Elmer Life Sciences.

### Preparation of cell cultures

Primary hepatocytes: Freshly isolated cells from animal and human liver were obtained in suspension on ice. Cells were pelleted by centrifugation at 70-75 xg (rat) or 95-90 xg (monkey and human) and resuspended at 0.8 million cells per mL of platting medium (William's E supplemented with 5% FBS, 0.5% penicillin-streptomycin, 1% L-glutamine, 4 µg/mL insulin and 1 µM dexamethasone). Multi-well collagen I-coated plates (rat tail collagen type **I;** 12- or 6-well) were then seeded by addition of 1 mL (12-well; 0.8 million cells/well) or 2 mL (6-well; 1.6 million cells/well) of cell suspension. The plates were gently shaken to evenly distribute the cells and placed in an incubator at 37°C for approximately 4 to 6 hours to allow cells to attach. Once cells were attached, the platting medium was removed and replaced with hepatocyte culture medium (William's E supplemented with penicillin-streptomycin, 1% L-glutamine, 1% insulin-transferrin-selenium and 0.1 µM dexamethasone). Cells were left overnight in an incubator at 37°C to acclimatize to culture and the medium.

Huh-7 cells: Confluent monolayers of Huh-7 cells were dispersed using trypsin-EDTA, rinsed and pelleted by centrifugation at 1,000 rpm (Beckman, Model Allegra 6R). Cells were resuspended at 0.2, 0.3 and 0.4 x 10⁶ cells/mL of medium (DMEM supplemented with 1% pyruvate, 10% FBS, 0.5% penicillin-streptomycin, 1% L-glutamine, 1% nonessential amino acids and 22 mM HEPES). Multi-well collagen I-coated plates (rat tail collagen type I; 6-well) were then seeded by addition of 2.0 mL of cell suspension to achieve final cell density of 0.4, 0.6 and 0.8 x 10⁶ cells/well and placed in an incubator at 37°C overnight to allow cells to attach and acclimatize to culture and the medium. Duplicate plates were prepared with the same cell density for cell number determination at each time point.

### Incubations with [¹⁴C]-Compound 1 and [¹⁴C]-2'-C-methyl guanosine

Hepatocyte incubations with radiolabeled Compound 1 and 2'-C-methyl guanosine, both labeled in the C-8 position of the guanine moiety, were conducted at a final volume of 2.0 mL hepatocyte culture medium/well (1.6 million cells/mL). Culture medium from overnight incubation of cells was removed and replaced with fresh medium, pre-warmed to 37°C, containing 1 or 10 µM [¹⁴C]-Compound 1 (122 DPM/pmol) or [¹⁴C]-2'-C-methyl guanosine (127 DPM/pmol) from a stock solution in DMSO (final DMSO concentration was 0.1%) or 50% ethanol (final ethanol concentration was 0.2%). After 1, 4, 8 and 24 hours, 0.5-1.0 mL of incubation medium was removed and stored at -20°C until analysis (described below). The remaining medium was discarded and cell monolayers were carefully washed two times with ice-cold PBS. Following the last wash, all PBS was carefully removed and 1.0 mL of extraction solution (ice-cold 70% methanol) added. Cells were scrapped off and suspended in the extraction solution, transferred to 2 mL polypropylene microfuge tubes and intracellular contents extracted overnight at -20°C.

Huh-7 incubations were conducted in a final volume of 2.0 mL Huh7 culture medium/well (0.4, 0.6 and 0.8 million cells/mL). Culture medium from overnight incubation of cells was removed and replaced with fresh culture medium, pre-warmed to 37°C containing [¹⁴C]-Compound **1** or [¹⁴C]-2'-C-methyl guanosine as described above for primary hepatocytes. After 24, 48 and 72 hours, 0.5-1.0 mL of incubation medium was removed and stored at -20°C until analysis (described below). The remaining medium was discarded and cell monolayers processed and extracted as described above.

### Determination of the in vitro intracellular half-life (t_{1/2}) of 2'-C-methyl guanosine -5'-triphosphate in primary hepatocytes

Primary hepatocytes were incubated with 10 µM [¹⁴C]-Compound 1 (122 DPM/pmol) or [¹⁴C]-2'-C-methyl guanosine (127 DPM/pmol) for 24 hours at which time the incubation medium was removed and the cell monolayer carefully washed two times with warm (37°C) PBS and placed back in culture with drug-free medium. At selected times up to 24 hours, the medium was removed and the cell monolayers carefully washed two times with ice-cold PBS. Following the last wash, all PBS was carefully removed and the cell monolayers processed and extracted as previously described.

### Effect of ribavirin and ritonavir on formation of Compounds 5, 6 and 7 in primary hepatocytes and Huh7 cells following incubation with [¹⁴C]-Compound 1 and [¹⁴C]-2'-C-methyl guanosine

Primary hepatocyte (1.6 million cells/well) and Huh7 cell (0.4 million cells/well) - incubations were conducted essentially as described above with the following exceptions. Cells were incubated with [¹⁴C]-Compound 1 (122 DPM/pmol) and [¹⁴C]-2'-C-methyl guanosine (127 DPM/pmol) at 10 µM in the presence or absence of ritonavir (1 µM) or ribavirin (5 µM) for 24 hours (hepatocytes) or 72 hours (Huh7, ribavirin only). Incubation medium (0.5-1.0 mL) was removed and stored at -20°C for analysis (described below). The remaining medium was discarded and the cell monolayers washed and processed as previously described.

### Stability of 2'-C-methyl guanosine nucleotides (Compounds 5, 6 and 7)

The stability of 2'-C-methyl guanosine nucleotides was evaluated by preparing a 100 µM standard solution of Compound **7** (2'-C-methyl guanosine triphosphate) which also contained Compound **5** (2'-C-methyl guanosine monophosphate) (10%) and Compound **6** (2'-C-methyl guanosine diphosphate) (30%) in HPLC-grade water and spiking 100 µL of this standard solution into (a) 900 µL extraction solution (70% methanol) and (b) 900 µL blank hepatocyte cell extract in 70% methanol. Spiked samples were placed at -20°C for up to three days after which time they were processed as described for experimental samples.

### Preliminary identification of putative metabolites

Preliminary identification of putative metabolites was conducted by comparing the retention time of observed metabolites in incubation medium and cellular extracts with retention times obtained with chemically synthesized standards using the chromatography conditions described below. Further identification of 2'-C-methyl guanosine nucleotides (mono-, di- and triphosphate) was carried out by incubating cellular extract samples with alkaline phosphatase (10 units for 60 min at 37°C) and comparing the HPLC retention times of the resulting nucleosides with that of authentic 2'-C-methyl guanosine.

### Sample preparation and analysis

### Cellular extracts

Cellular extracts were prepared by centrifugation at 16,000 RCF using an Eppendorf microcentrifuge (Eppendorf, Model 5415D) for 10 min to remove cellular debris. An aliquot of 5-10 µL of the supernatant was added to 5 mL of EcoLite™ (MP, Irvine, CA) liquid scintillation cocktail and radioactivity measured by liquid scintillation counting (LSC) using a Beckman Coulter LS6500 multipurpose liquid scintillation counter (Beckman Instruments, Fullerton, CA). The remaining sample was then dried using a Labconco refrigerated centrivap concentrator (Labconco, Kansas City, MO). Dry extracts were reconstituted in 120 µL of HPLC-grade water and centrifuged at 16,000 RCF using an Eppendorf microcentrifuge for 10 min. Prior to HPLC analysis (described below), radioactivity of the concentrated extracts was measured by LSC using 5 µL aliquots. Extraction recovery was then determined by comparing total radioactivity in extracts before and after drying. The remaining extract was then analyzed by HPLC with online radioactivity detection using a Flow Scintillation Analyzer Radiomatic™ 515TR. Radioactivity in the separated components was quantified by integrating the peaks using Flo-One™ Software (Perkin Elmer Life Sciences). Samples with poor recovery (<85%) were re-analyzed when possible.

Nucleotide stability samples were prepared and processed as described above for experimental samples with the exception that no LSC was required as the samples were not radioactive. Dry extracts were reconstituted in 100 µL of HPLC-grade water, centrifuged at 16,000 RCF using an Eppendorf microcentrifuge for 10 min and analyzed by LC-UV (method 2; described below). The UV peak areas for Compound **5**, Compound **6** and Compound 7 in these samples were compared to peak areas obtained for the standard solution.

### Incubation medium

Incubation medium samples were thawed at room temperature, vortexed and centrifuged at 16,000 RCF using an Eppendorf microcentrifuge. An aliquot of 10 µL was added to 5 mL of EcoLite™ (MP, Irvine, CA) liquid scintillation cocktail and radioactivity measured by LSC using a Beckman Coulter LS6500 multipurpose liquid scintillation counter (Beckman Instruments, Fullerton, CA). Samples (50-100 µL) were then analyzed by HPLC (described below) with online radioactivity detection using a Flow Scintillation Analyzer Radiomatic™ 515TR. Radioactivity in the separated components was quantified by integrating the peaks using Flo-One™ Software (Perkin Elmer Life Sciences). Samples with poor recovery (<85%) were re-analyzed when possible.

### Analytical methods

All samples were analyzed using the following HPLC methods:

### Instrumentation:

Agilent 1100 with ChemStation LC3D Version A.10.02 (1757) (Agilent Technologies, Inc) Autosampler with thermostat
Diode Array Detector
Thermostatted Column Compartment
Quaternary Pump
Vacuum Degasser

### Chromatographic conditions:

### Method 1 - Analysis of Compound 1 incubation medium and cell extracts:

• Column: Phenomenex® Columbus 5 µ C18, 4.6 x 250 mm
• (Phenomenex USA, Torrance, CA)
• Guard Column: Security Guard Cartridge, 4 x 2 mm
• (Phenomenex USA, Torrance, CA)
• Column Temperature: ambient
• UV Detection: 252 nm
• Radioactivity Detection: 500 µL liquid cell, Scintillant Flow Cocktail (Ultima Flow-AP,
• Perkin Elmer Life Sciences, CT) at 3 mL/min.

| HPLC Gradient Conditions-Method 1 | | | |
|---|---|---|---|
| Time (min) | %MPA | %MPB | Flow (mL/min) |
| 0 | 95 | 5 | 1.0 |
| 15 | 70 | 30 | 1.0 |
| 20 | 70 | 30 | 1.0 |
| 30 | 50 | 50 | 1.0 |
| 50 | 50 | 50 | 1.0 |
| MPA = 225 mM potassium phosphate + 5mM TBAP pH 6.3 MPB = Methanol | | | |

| | | | |
|---|---|---|---|
| MPA = 25 mM potassium phosphate + 5mM TBAP pH 6.3 MPB = Methanol | | | |

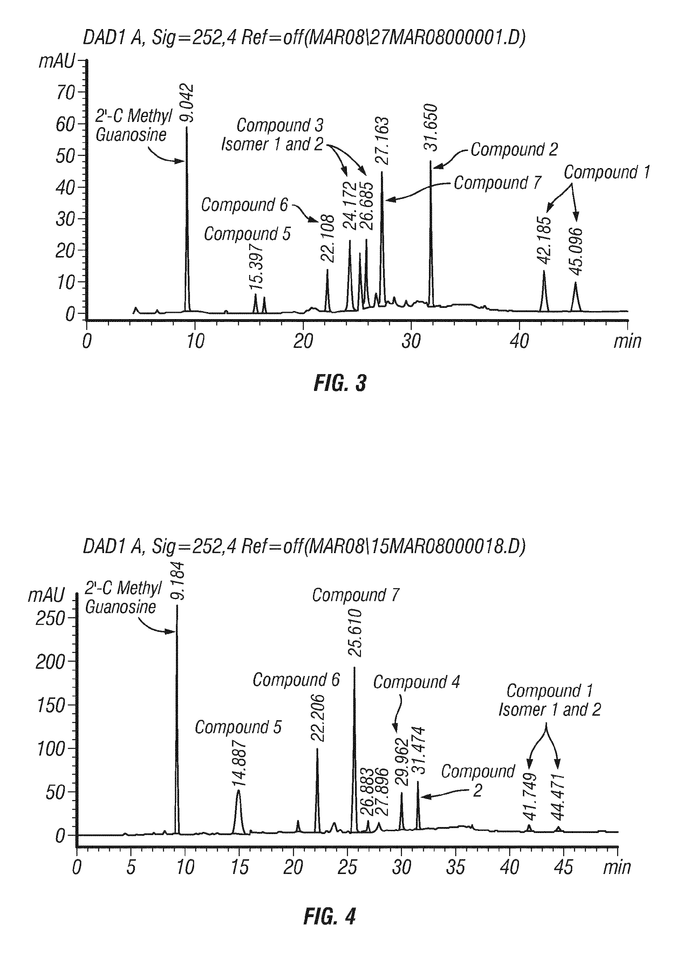

Representative HPLC chromatogram of Compound **1** (diastereomers 1 and 2) and metabolite standards obtained by method 1 is provided in Figure 3.

### Method 2 - Analysis of 2'-C-methyl guanosine cell extracts:

• Column: Phenomenex® Columbus 5 µ C18, 4.6 x 250 mm
• (Phenomenex USA, Torrance, CA)
• Guard Column: Security Guard Cartridge, 4 x 2 mm
• (Phenomenex USA, Torrance, CA)
• Column Temperature: ambient
UV Detection: 252 nm
• Radioactivity Detection: 500 µL liquid cell, Scintillant Flow Cocktail (Ultima Flow-AP, Perkin Elmer Life Sciences, CT) at 3 mL/min.

| HPLC Gradient Conditions-Method 2 | | | |
|---|---|---|---|
| Time (min) | %MPA | %MPB | Flow (mL/min) |
| 0 | 95 | 5 | 1.0 |
| 15 | 70 | 30 | 1.0 |
| 20 | 70 | 30 | 1.0 |
| 30 | 50 | 50 | 1.0 |

| | | | |
|---|---|---|---|
| MPA = 25 mM potassium phosphate + 5mM TBAP pH 6.3 MPB = Methanol | | | |

### Method 3 - Analysis of 2'-C-methyl guanosine incubation medium samples:

• Column: Phenomenex® Columbus 5 µ C18, 4.6 x 250 mm
• (Phenomenex USA, Torrance, CA)
• Guard Column: Security Guard Cartridge, 4 x 2 mm
• (Phenomenex USA, Torrance, CA)
• Column Temperature: ambient
UV Detection: 252 nm
• Radioactivity Detection: 500 µL liquid cell, Scintillant Flow Cocktail (Ultima Flow-AP, Perkin Elmer Life Sciences, CT) at 3 mL/min.

| HPLC Gradient Conditions-Method 3 | | | |
|---|---|---|---|
| Time (min) | %MPA | %MPB | Flow (mL/min) |
| 0 | 95 | 5 | 1.0 |
| 15 | 70 | 30 | 1.0 |
| 20 | 70 | 30 | 1.0 |

| | | | |
|---|---|---|---|
| MPA = 25 mM potassium phosphate + 5mM TBAP pH 6.3 MPB = Methanol | | | |

### Method 4 - Analysis of Compound 1 incubation medium samples:

This method was used for comparison of hepatocyte metabolite profiles with metabolite profiles observed with microsomal incubations, Example 14.
- Column: Phenomenex® Columbus 5µ C18, 4.6 x 250 mm
- (Phenomenex USA, Torrance, CA)
- Guard Column: Security Guard Cartridge, 4 x 2 mm
- (Phenomenex USA, Torrance, CA)
- Column Temperature: ambient
   UV Detection: 252 nm
- Radioactivity Detection: 500 µL liquid cell, Scintillant Flow Cocktail (Ultima Flow-AP, Perkin Elmer Life Sciences, CT) at 3 mL/min.

| HPLC Gradient Conditions-Method 4 | | | |
|---|---|---|---|
| Time (min) | %MPA | %MPB | Flow (mL/min) |
| 0 | 95 | 5 | 1.0 |
| 15 | 70 | 30 | 1.0 |
| 20 | 70 | 30 | 1.0 |
| 30 | 55 | 45 | 1.0 |
| 50 | 55 | 45 | 1.0 |

| | | | |
|---|---|---|---|
| MPA = 10 mM potassium phosphate pH 5 MPB = Methanol | | | |

### Calculations

The concentration-time data for Compound **1** and Compound **7** (2'-C-methyl guanosine triphosphate) were analyzed using the Microsoft^{®} Office Excel 2003 to obtain the area under the concentration-time curve (AUC) values. AUC₀₋₂₄ was the area under the concentration-time curve calculated using the linear trapezoidal rule and sampling times up to 24 hours. Linear regression analysis (Microsoft Excel) was used to estimate an *in vitro* intracellular t_{1/2} value for Compound 7 from the function t_{1/2} = 0.693/k where k is the slope of the linear regression obtained by plotting the natural log of Compound 7 intracellular concentration in pmol/million cells versus incubation time.

### Results

The metabolic disposition and intracellular activation of Compound **1** was evaluated in primary hepatocytes and in the human hepatoma cell line Huh7 using [¹⁴C]-labled Compound **1**. Activation of the parent nucleoside, 2'-C-methyl guanosine, was also evaluated in parallel using [¹⁴C]-labeled 2'-C-methyl guanosine to asses if delivery of Compound **5** via Compound **1** leads to higher levels of the active triphosphate, Compound **7**.

The radiopurity of [¹⁴C]-Compound **1** and [¹⁴C]-2'-C-methyl guanosine was assessed using HPLC. A 1:1000 dilution of the stock material was prepared in hepatocyte medium and analyzed by HPLC. Both compounds were > 98% pure.

### Metabolic profile: incubation medium

### Primary hepatocytes

### Incubation with 10 micromolar [¹⁴C]-Compound 1 or [¹⁴C]-2'-C-methyl guanosine

An extensive metabolism of [¹⁴C]-Compound **1** was observed in rat hepatocytes, with an approximate 50% decrease in Compound **1** medium levels at 4 hours and no detectable Compound **1** after 24 hours (Table 11). Metabolism in monkey hepatocytes was moderate; medium levels decreased about 40% by 8 hours and 77% after 24 hours (Table 11). In contrast to both rat and monkey hepatocytes, Compound **1** metabolism in human hepatocytes (Table 11) appeared to be low with only a 38% decrease in Compound **1** medium levels observed by 24 hours.

**Table 11: Percent residual of [¹⁴C]-Compound 1 in the incubation medium of primary hepatocytes and Huh7 cells following incubation with [¹⁴C]-Compound 1**

| Time (hour) | % Residual [¹⁴C]-Compound **1** (10 µM) | | | | | |
|---|---|---|---|---|---|---|
| | primary hepatocytes | | | | | |
| | Human (n=2) | | Monkey (n=2) | | Rat (n=2) | |
| 0 | 97 | 98 | 97 | 91 | 95 | 97 |
| 1 | 94 | 97 | 95 | 67 | 91 | 93 |
| 4^{a} | 91 | 91 | - | 66 | 48 | 55 |
| 8 | 84 | 79 | 63 | 61 | 18 | 32 |
| 24 | 63 | 60 | 16 | 31 | 0 | 0 |

| Time (hour) | % Residual [¹⁴C]-Compound **1** (1 µM) | | | | | |
|---|---|---|---|---|---|---|
| | primary hepatocytes | | | | | |
| | Human (n=1) | | | Rat (n=1) | | |
| 0 | 97 | | | 99 | | |
| 1 | 100 | | | 89 | | |
| 8 | 78 | | | 21 | | |
| 24 | 51 | | | 0 | | |

| Time (hour) | % Residual [¹⁴C]-Compound **1** (10 µM) | | | | | |
|---|---|---|---|---|---|---|
| | Huh7 cells (n=2) | | | | | |
| 0 | 98 | | | 96 | | |
| 24 | 85 | | | 86 | | |
| 48 | 76 | | | 77 | | |
| 72 | 65 | | | 68 | | |

| | | | | | | |
|---|---|---|---|---|---|---|
| Primary hepatocytes and Huh7 cells were incubated with [¹⁴C]-Compound **1** at 1 or 10 µM as described under experimental procedures. Values represent unchanged [¹⁴C]-Compound **1** levels in incubation medium and are expressed in percent. ^{a} Due to limited cell availability, sampling at 4 hours was not conducted for monkey 1 (Cy230). | | | | | | |

A total of ten metabolites were observed in the incubation medium of rat hepatocytes (Table 12); nine in monkey hepatocytes (Table 13) and seven in human hepatocytes (Table 14).

**Table 12: Compound 1 metabolites observed in the incubation medium of rat hepatocytes following incubation with [¹⁴C]-Compound 1**

| Lot no. | Major metabolites observed in medium after 10 µM Compound 1 (% of total radioactivity) | | | | | | | | | |
|---|---|---|---|---|---|---|---|---|---|---|
| Rs448 | | | | | | | | | | |
| Time (hour) | U8 | 2'-C- methyl guanosine | Compound **5** | Compound **3** | U5 | U7 | Compound **2^{a}** | U4 | U1 | U3 |
| 0 | 1.3 | BLD^{b} | BLD | BLD | BLD | BLD | 2.2 | 0.8 | BLD | BLD |
| 1 | 1.3 | 0.8 | 1.5 | BLD | BLD | BLD | 1.9 | 1.2 | 1.0 | 2.1 |
| 4 | 2.8 | 17.5 | 5.3 | 0.9 | 2.6 | BLD | 5.3 | BLD | 6.4 | 9.8 |
| 8 | 2.3 | 41.5 | 6.1 | 1.4 | 6.1 | 1.3 | 5.7 | BLD | 7.4 | 9.4 |
| 24 | 1.3 | 68.5 | 2.9 | 1.4 | 6.9 | BLD | 3.7 | BLD | 6.7 | 6.6 |
| | | | | | | | | | | |

| Lot no. | Major metabolites observed in medium after 10 µM Compound **1** (% of total radioactivity) | | | | | | | | | |
|---|---|---|---|---|---|---|---|---|---|---|
| Rs458 | | | | | | | | | | |
| Time (hour) | U8 | 2'-C- methyl guanosine | Compound **5** | Compound **3** | U5 | U7 | Compound **2^{a}** | U4 | U1 | U3 |
| 0 | 1.2 | BLD | BLD | BLD | BLD | BLD | 1.5 | 0.4 | BLD | BLD |
| 1 | 1.4 | 0.5 | BLD | BLD | BLD | BLD | 1.5 | BLD | 1.4 | 2.6 |
| 4 | 2.2 | 10.1 | 3.6 | 0.9 | 2.6 | 0.4 | 5.1 | BLD | 7.1 | 11.9 |
| 8 | 2.3 | 23.4 | 3.3 | 2.1 | 4.7 | BLD | 6.8 | BLD | 10 | 14.8 |
| 24 | 2.6 | 65.3 | 2.6 | 1.7 | 6.2 | BLD | 4.6 | BLD | 7.2 | 10.0 |

| | | | | | | | | | | |
|---|---|---|---|---|---|---|---|---|---|---|
| Rat hepatocytes were incubated for 1, 4, 8 and 24 hours with [¹⁴C]-Compound 1 at 10 µM as described under experimental procedures. Values represent metabolite levels in the incubation medium and are expressed as percent (%) of total radioactivity measured in the sample. ^{a} Possible co elution of an unknown metabolite with Compound 2. ^{b}BLD = below limit of detection (30 DPM) | | | | | | | | | | |

**Table 13: Compound 1 metabolites observed in the incubation medium of monkey hepatocytes following incubation with [¹⁴C]-Compound 1**

| Lot no. | Major metabolites observed in medium after 10 µM Compound **1** (% of total radioactivity) | | | | | | | | |
|---|---|---|---|---|---|---|---|---|---|
| Cy230^{a} | | | | | | | | | |
| Time (hour) | U8 | 2'-C- methyl guanosine | Compound **5** | Compound **3** | U5 | Compound **2^{b}** | U4 | U1 | U3 |
| 0 | 1.5 | BLD^{c} | BLD | BLD | BLD | 1.0 | BLD | BLD | BLD |
| 1 | 1.0 | BLD | 0.9 | BLD | BLD | 1.9 | 0.6 | 0.7 | 0 |
| 8 | 1.3 | 10.4 | 5.7 | 0.5 | BLD | 8.5 | 0.6 | 6.8 | 2.8 |
| 24 | BLD | 48.8 | 8.4 | 3.1 | BLD | 7.0 | 1.0 | 10.6 | 4.9 |
| | | | | | | | | | |

| Lot no. | Major metabolites observed in medium after 10 µM Compound **1** (% of total radioactivity) | | | | | | | | |
|---|---|---|---|---|---|---|---|---|---|
| Cy234 | | | | | | | | | |
| Time (hour) | U8 | 2'-C- methyl guanosine | Compound **5** | Compound **3** | U5 | Compound **2^{b}** | U4 | U1 | U3 |
| 0 | 1.1 | BLD | BLD | BLD | BLD | 6.2 | 2.1 | BLD | BLD |
| 1 | 0 | 2.1 | 28.5 | BLD | BLD | 0.6 | 1.8 | BLD | BLD |
| 4 | 1.4 | 6.8 | 19.0 | BLD | BLD | 1.1 | 2.8 | 1.8 | 0.5 |
| 8 | 0.9 | 14.8 | 16.6 | BLD | BLD | 1.8 | 1.6 | 3.1 | 0.8 |
| 24 | BLD | 35.6 | 7.5 | 0.9 | 2.2 | 8.2 | 1.7 | 8.6 | 4.3 |
| Monkey hepatocytes were incubated for 1, 4, 8 and 24 hours with [¹⁴C]-Compound 1 at 10 µM as described | | | | | | | | | |
| under experimental procedures. Values represent metabolite levels in the incubation medium and are expressed as percent (%) of total radioactivity measured in the sample. | | | | | | | | | |

| | | | | | | | | | |
|---|---|---|---|---|---|---|---|---|---|
| ^{a} Due to limited cell availability, sampling at 4 hours was not conducted for Monkey 1 (Cy230). ^{b} Possible co elution of an unknown metabolite with Compound **2**. ^{c} BLD = below limit of detection (30 DPM) | | | | | | | | | |

**Table 14: Compound 1 metabolites observed in the incubation medium of human hepatocytes following incubation with [¹⁴C]-Compound 1**

| Lot no. | Major metabolites observed in medium after 10 µM Compound **1** (% of total radioactivity) | | | | | | |
|---|---|---|---|---|---|---|---|
| Hu759 | | | | | | | |
| Time (hour) | U8 | 2'-C-methyl guanosine | Compound **5** | Compound **2^{a}** | U4 | U1 | U3 |
| 0 | 0.8 | BLD^{b} | BLD | 2.7 | BLD | BLD | BLD |
| 1 | 0.8 | BLD | 4.4 | 0 | 0.6 | 0 | 0 |
| 4 | 1.1 | 1.7 | 3.9 | 1.8 | 0.6 | 0 | 0.5 |
| 8 | 0.6 | 4.6 | 5.1 | 2.6 | 0.8 | 0.9 | 1.3 |
| 24 | BLD | 22.1 | 4.7 | 2.7 | 1.9 | 2.5 | 2.6 |
| | | | | | | | |

| Lot no. | Major metabolites observed in medium after 10 µM Compound **1** (% of total radioactivity) | | | | | | |
|---|---|---|---|---|---|---|---|
| Hu775 | | | | | | | |
| Time (hour) | U8 | 2'-C-methyl guanosine | Compound **5** | Compound **2^{a}** | U4 | U1 | U3 |
| 0 | 1.0 | BLD | BLD | 1.1 | 0.5 | BLD | BLD |
| 1 | 1.3 | BLD | 0.5 | BLD | BLD | BLD | BLD |
| 4 | 1.0 | 1.6 | 4.5 | 0.8 | 0.8 | BLD | BLD |
| 8 | 1.2 | 7 | 7.2 | 0.6 | 0.9 | 0.5 | 0.9 |
| 24 | 0.8 | 29 | 7.1 | 0.7 | 1.2 | 0.6 | 0.7 |

| | | | | | | | |
|---|---|---|---|---|---|---|---|
| Human hepatocytes were incubated for 1, 4, 8 and 24 hours with [¹⁴C]-Compound 1 at 10 µM as described under experimental procedures. Values represent metabolite levels in the incubation medium and are expressed as percent (%) of total radioactivity measured in the sample. ^{a} Possible co elution of an unknown metabolite with Compound **2**. ^{b} BLD = below limit of detection (30 DPM) | | | | | | | |

Metabolite identification was conducted by retention time comparison with reference standards, when possible. Both hepatocyte and microsome samples were analyzed using HPLC Methods 1 and 4 to further verify and confirm hepatocyte metabolite identifications and assignments as related to those reported in Example 14. Table 15 summarizes the metabolite assignments for Compound **1** metabolites observed in primary hepatocytes and Huh7 cells.

**Table 15: Metabolic assignments of Compound 1 metabolites observed in primary hepatocytes and Huh7 cells**

| Metabolite assignment | Retention time (min) LC Method 1 | Location |
|---|---|---|
| Compound **5** | 15 | Primary hepatocytes (H,M,R)^{a} Huh7 cells |
| 2'-C-methyl guanosine | 9.4 | Primary hepatocytes (H,M,R) Huh7 cells |
| Compound **2** | 32 | Primary hepatocytes (H,M,R) Huh7 cells |
| Compound 3, diastereomer 1 | 24.7 | Primary hepatocytes (R,M) Huh7 cells |
| Compound 3, diastereomer 2 | 27.5 | |
| U1 | 38.4 | Primary hepatocytes (H,M,R) |
| U3 | 39.8 | Primary hepatocytes (H,M,R) |
| U4 | 36.8 | Primary hepatocytes (H,M,R) |
| U5 | 26.8 | Primary hepatocytes (R) |
| U6 | 33.8 | Huh7 cells |
| U7 | 30.9 | Primary hepatocytes (R) |
| Compound **6** | 22.3 | Primary hepatocytes (H,M,R) Huh7 cells |
| Compound **7** | 25.9 | Primary hepatocytes (H,M,R) Huh7 cells |
| U8 | 4.6 | Primary hepatocytes (H,M,R) Huh7 cells |
| U9 | 28.1 | Huh7 cells |

2'-C-methyl guanosine was the predominate metabolite in all species, representing 26% (human), 42% (monkey) and 67% (rat) of the total radioactivity measured in the incubation medium after 24 hours.

**Table 16: 2'-C-methyl guanosine levels in the incubation medium of primary hepatocytes and Huh7 cells following incubation with [¹⁴C] -Compound 1**

| Time (hour) | 2'-C-methyl guanosine (% of total radioactivity) primary hepatocytes with [¹⁴C]-Compound **1** (10 µM) | | | | | | |
|---|---|---|---|---|---|---|---|
| | Human (n=2) | | Monkey (n=2) | | Rat (n=2) | | |
| 1 | BLD^{a} | BLD | BLD | 2.10 | 0.80 | 0.50 | |
| 4^{b} | 1.70 | 1.60 | - | 6.80 | | 17.0 | 10.0 |
| 8 | 4.60 | 7.00 | 10.4 | 14.8 | | 41.0 | 23.0 |
| 24 | 22.1 | 29.0 | 48.8 | 35.6 | | 69.0 | 65.0 |

| Time (hour) | 2'-C-methyl guanosine (% of total radioactivity) primary hepatocytes with [¹⁴C]-Compound **1** (1 µM) | | | | | | |
|---|---|---|---|---|---|---|---|
| | Human (n=1) | | | | Rat (n=1) | | |
| 1 | BLD | | | | BLD | | |
| 8 | 9.20 | | | | 29.4 | | |
| 24 | 38.7 | | | | 58.3 | | |

| Time (hour) | 2'-C-methyl guanosine (% of total radioactivity) Huh7 cells (n=2) with [¹⁴C]-Compound 1 (10 µM) | | | | | | |
|---|---|---|---|---|---|---|---|
| 24 | 2.20 | | | | 1.10 | | |
| 48 | 2.70 | | | | 0.50 | | |
| 72 | 2.30 | | | | 1.50 | | |

| | | | | | | | |
|---|---|---|---|---|---|---|---|
| Primary hepatocytes and Huh7 cells were incubated with [¹⁴C]-Compound 1 at 1 or 10 µM as described under experimental procedures. Values represent 2'-C-methyl guanosine levels in incubation medium and are expressed in percent. ^{a}BLD = below limit of detection (30 DPM) ^{b}Due to limited cell availability, sampling at 4 hours was not conducted for monkey 1 (Cy230). | | | | | | | |

Compound **5** and Compound **2** were highest in monkey, representing 8% of total radioactivity after 24 hours whereas in human and rat, they represented 3 to 5% after 24 hours, respectively. The presence of Compound **5** in the incubation medium was confirmed by alkaline phosphatase treatment. Another unidentified metabolite(s) co elutes with Compound **2** under the chromatographic conditions of HPLC Method 1 or co elutes with Compound **3** under the chromatographic conditions of HPLC method 4. The unknown metabolites, U1 and U3 accounted for 7 to 8% of the total radioactivity at 24 hours in rats, 10 and 5% in monkey and approximately 2% in human hepatocytes. Another unknown metabolite, U5, was only observed in rat hepatocytes and accounted for about 7% of the total radioactivity after 24 hours. Compound **3** (mixture of compounds **3a** and **3b**), identified as an important NADPH-dependent metabolite in liver microsomes, appeared to be a minor metabolite in hepatocytes, representing 3% or less of total radioactivity in monkey and rat hepatocytes and undetectable in human hepatocytes (Tables 12-13).

No metabolites were observed in the incubation medium of primary hepatocytes following incubation with [¹⁴C]-2'-C-methyl guanosine.

### Incubation with 1 micromolar [¹⁴C]-Compound 1 or [¹⁴C]-2'-C-methyl guanosine

Metabolism of [¹¹C]-Compound **1** at 1 µM was evaluated in human and rat hepatocytes and, as observed at 10 µM, metabolism in rat was extensive with approximately 79% reduction in Compound **1** medium levels at 8 hours and no detectable Compound **1** after 24 hours (Table 11). Metabolism in human hepatocytes (Table 11), was lower, with 51% Compound **1** remaining in the medium by 24 hours. A total of eight metabolites were observed in the incubation medium of rat hepatocytes (Table 17); only 2'-C-methyl guanosine, Compound **5** and Compound **2** were detectable in human hepatocytes medium (Table 17).

**Table 17: Compound 1 metabolites observed in the incubation medium of human and rat hepatocytes following incubation with [¹⁴C]-Compound 1**

| Hu775 | Major metabolites observed in medium after 1 µM Compound **1** (% of total radioactivity) | | | | | | | |
|---|---|---|---|---|---|---|---|---|
| Time (hour) | 2'-C-methyl guanosine | | Compound **5** | | | Compound **2^{b}** | | |
| 0 | BLD^{a} | | BLD | | | 2.22 | | |
| 1 | BLD | | BLD | | | BLD | | |
| 8 | 9.22 | | 10.4 | | | 2.27 | | |
| 24 | 38.7 | | 8.29 | | | 1.72 | | |
| | | | | | | | | |

| Rs458 | Major metabolites observed in medium after 1 µM Compound **1** (% of total radioactivity) | | | | | | | |
|---|---|---|---|---|---|---|---|---|
| Time (hour) | 2'-C- methyl guanosine | Compound **5** | Compound **3** | M10 | U7 | Compound **2^{b}** | U1 | U3 |
| 0 | BLD | BLD | BLD | BLD | BLD | 1.05 | BLD | BLD |
| 1 | BLD | BLD | BLD | BLD | BLD | 3.02 | 2.85 | 1.76 |
| 8 | 29.4 | 6.41 | BLD | 5.04 | BLD | 9.52 | 12.7 | 16.4 |
| 24 | 58.3 | BLD | 1.71 | 3.04 | 1.98 | 7.43 | 11.2 | 14.7 |

| | | | | | | | | |
|---|---|---|---|---|---|---|---|---|
| Human and rat hepatocytes were incubated for 1, 8 and 24 hours with [¹⁴C]-Compound 1 at 1 µM as described under experimental procedures. Values represent metabolite levels in the incubation medium and are expressed as percent (%) of total radioactivity measured in the sample. ^{a}BLD = below limit of detection (30 DPM) ^{b}Possible co elution of an unknown metabolite with Compound **2**. | | | | | | | | |

The major metabolite at 24 hours in human and rat hepatocytes was 2'-C-methyl guanosine, accounting for 39 and 58%, respectively (Table 16). In human hepatocytes Compound **5** was 8% and Compound **2** was 1.7% at 24 hours. In rat hepatocytes no Compound **5** was detected at 24 hours and Compound **2** was 7% of the total radioactivity. Unknown metabolites in rat hepatocytes accounted for 31% of the total radioactivity. Compound **3** (3a and 3b; 1.7%) was also detected in rat hepatocytes at 24 hours (Table 17).

No metabolites were observed in the incubation medium of primary hepatocytes following incubation with 1 µM [¹⁴C]-2'-C-methyl guanosine.

### Huh 7 cells

In contrast to primary hepatocytes, Compound **1** metabolism in the human hepatoma cell line, Huh7, was low. After 24 hours, Compound **1** accounted for 86% of the radioactivity measured in the incubation medium and by 72, it accounted for 67%. A total of six metabolites (Table 18) were observed in the incubation medium of Huh7 cells.

**Table 18: Compound 1 metabolites observed in the incubation medium of Huh7 cells following incubation with [¹⁴C]-Compound 1**

| Huh7 | Major metabolites observed in medium after 10 µM Compound **1** (% of total radioactivity) | | | | | |
|---|---|---|---|---|---|---|
| Time (hour) | U8 | 2'-C-methyl guanosine | Compound **5** | Compound **2** | U6 | U4 |
| 0 | 0.8 | BLD^{a} | BLD | 2.9 | BLD | 0.4 |
| 24 | 1.0 | 1.2 | 2.0 | 8.2 | BLD | 1.5 |
| 48 | 0.5 | 0.5 | 0.8 | 18.3 | 0.5 | 2.4 |
| 72 | 1.0 | 1.5 | 0.7 | 25.3 | 0.7 | 3.5 |
| | | | | | | |

| Huh7 | Major metabolites observed in medium after 10 µM Compound **1** (% of total radioactivity) | | | | | |
|---|---|---|---|---|---|---|
| Time (hour) | U8 | 2'-C-methyl guanosine | Compound **5** | Compound **2** | U6 | U4 |
| 0 | 1.0 | BLD | BLD | 1.7 | BLD | BLD |
| 24 | 0.7 | 2.2 | 3.2 | 8.0 | BLD | 1.3 |
| 48 | 0.6 | 2.7 | 1.5 | 17.2 | 0.3 | 2.4 |
| 72 | 1.2 | 2.3 | 1.0 | 27.2 | 0.4 | 3.2 |

| | | | | | | |
|---|---|---|---|---|---|---|
| Cells were incubated with [¹⁴C]-Compound **1** at 10 µM as described under experimental procedures. Values represent metabolite levels in the incubation medium and are expressed as percent (%) of total radioactivity measured in the sample. ^{a}BLD = below limit of detection (30 DPM) | | | | | | |

A minor metabolite (<1 %), U6 appeared to be unique to Huh7. Compound **2** appeared to be the major metabolite in Huh7 cells, accounting for 26% of total radioactivity by 72 hours. 2'-C-methyl guanosine and Compound **5** accounted for 2 and 0.9%, respectively, of total radioactivity at 72 hours. U4 accounted for 3.4% of the radioactivity at 72 hours.

No metabolites were observed in the incubation medium of Huh7 cells following incubation with [¹⁴C]-2'-C-methyl guanosine.

### Metabolic profile: cellular extracts

Stability of 2'-C-methyl guanosine nucleotides through the sample preparation and extraction process was evaluated using a reference standard of Compound **7** which also contained Compound **5** (10%) and Compound **6** (30%). No breakdown of 2'-C-methyl guanosine nucleotides was observed.

### Primary hepatocytes

### [¹⁴C]-Compound 1 and [¹⁴C]-2'-C-methyl guanosine at 10 micromolar

Extraction recovery of intracellular radioactivity was determined by LSC as described under experimental procedures described above. In general, the extraction recovery ranged from 66% to 79%. Radioactivity remaining with the cellular pellets was not determined. The metabolic profile of [¹⁴C]-Compound **1** in primary hepatocytes is summarized in Table 19 through Table 22 and Table 26 through Table 29 and metabolic profile of [¹⁴C]-2'-C-methyl guanosine in human, monkey and rat hepatocytes is summarized in Table 23A through Table 25 and Table 27.

**Table 19: Compound 1 metabolites observed in human hepatocyte extracts following incubation with [¹⁴C]-Compound 1**

| Hu759 | Major metabolites observed in cellular extracts after 10 µM Compound **1** (% of total radioactivity) | | | | | | | | |
|---|---|---|---|---|---|---|---|---|---|
| Time (hour) | 2'-C- methyl guanosine | Compound **5** | Compound **6** | Compound **7** | Compound **2^{b}** | U4 | U1 | U3 | Compound **1** |
| 1 | 3.69 | 6.44 | 4.93 | 34.2 | 6.50 | 7.06 | 6.05 | BLD^{a} | 31.1 |
| 4 | 4.20 | 5.70 | 8.01 | 63.1 | 1.81 | 4.24 | 3.44 | BLD | 8.48 |
| 8 | 7.52 | 7.99 | 13.99 | 70.5 | BLD | BLD | BLD | BLD | BLD |
| 24 | 10.3 | 5.38 | 13.4 | 69.5 | BLD | BLD | BLD | BLD | 1.38 |
| | | | | | | | | | |

| Hu775 | Major metabolites observed in cellular extracts after 10 µM Compound **1** (% of total radioactivity) | | | | | | | | |
|---|---|---|---|---|---|---|---|---|---|
| Time (hour) | 2'-C- methyl guanosine | Compound **5** | Compound **6** | Compound **7** | Compound **2^{b}** | U4 | U1 | U3 | Compound **1** |
| 1 | 3.75 | 8.77 | 6.09 | 46.4 | 2.30 | 3.85 | 3.52 | 1.42 | 23.9 |
| 4 | 3.10 | 5.54 | 12.4 | 69.7 | BLD | 1.07 | 1.20 | BLD | 6.96 |
| 8 | 5.89 | 4.44 | 11.1 | 74.1 | BLD | 0.57 | BLD | BLD | 3.90 |
| 24 | 9.06 | 12.8 | 17.05 | 59.6 | BLD | BLD | BLD | BLD | 1.59 |

| | | | | | | | | | |
|---|---|---|---|---|---|---|---|---|---|
| Human hepatocytes were incubated for 1, 4, 8 and 24 hours with [¹⁴C]-Compound 1 at 10 µM as described under experimental procedures. Values represent metabolite levels in the cellular extract and are expressed as percent (%) of total radioactivity measured in the sample. ^{a}BLD = below limit of detection (30 DPM) ^{b}Possible co elution of an unknown metabolite with Compound **2**. | | | | | | | | | |

**Table 20: Compound 1 metabolites observed in monkey hepatocyte extracts following incubation with [¹⁴C]-Compound 1**

| Cy230^{a} | Major metabolites observed in cellular extracts after 10 µM Compound **1** (% of total radioactivity) | | | | | | | |
|---|---|---|---|---|---|---|---|---|
| Time (hour) | 2'-C- methyl guanosine | Compound **5** | Compound **6** | Compound **7** | Compound **2^{b}** | U1 | U3 | Compound **1** |
| 1 | 1.68 | 8.41 | 9.25 | 63.4 | 3.20 | 6.19 | 2.72 | 4.03 |
| 8 | 0.95 | 3.84 | 13.8 | 80.9 | BLD^{c} | 0.55 | BLD | BLD |
| 24 | 2.31 | 6.96 | 18.5 | 72.2 | BLD | BLD | BLD | BLD |
| | | | | | | | | |

| Cy234 | Major metabolites observed in cellular extracts after 10 µM Compound **1** (% of total radioactivity) | | | | | | | |
|---|---|---|---|---|---|---|---|---|
| Time (hour) | 2'-C- methyl guanosine | Compound **5** | Compound **6** | Compound **7** | Compound **2^{b}** | U1 | U3 | Compound **1** |
| 1 | 3.04 | 18.5 | 9.65 | 34.9 | 7.96 | 6.46 | 2.20 | 17.3 |
| 4 | 1.35 | 5.47 | 17.1 | 70.3 | 1.25 | 1.49 | BLD | 3.03 |
| 8 | 2.06 | 5.38 | 14.9 | 77.6 | BLD | BLD | BLD | BLD |
| 24 | 1.25 | 3.34 | 16.0 | 79.4 | BLD | BLD | BLD | BLD |

| | | | | | | | | |
|---|---|---|---|---|---|---|---|---|
| Monkey hepatocytes were incubated for 1, 4, 8 and 24 hours with [¹⁴C]-Compound **1** at 10 µM as described under experimental procedures. Values represent metabolite levels in the cellular extract and are expressed as percent (%) of total radioactivity measured in the sample. ^{a} Due to limited cell availability, sampling at 4 hours was not conducted for Monkey 1 (Cy230). possible co elution of an unknown metabolite with Compound **2**. ^{c} BLD = below limit of detection (30 DPM) | | | | | | | | |

**Table 21: Compound 1 metabolites observed in rat hepatocyte extracts following incubation with [¹⁴C]-Compound 1**

| Rs448 | Major metabolites observed in cellular extracts after 10 µM Compound 1 (% of total radioactivity) | | | | | | | | | |
|---|---|---|---|---|---|---|---|---|---|---|
| Time (hour) | 2'-C- methyl guanosine | Compd **5** | Compd **6** | Compd **7** | Compd **3** | Compd **2^{b}** | U4 | U1 | U3 | Compd **1** |
| 1 | 1.84 | 4.37 | 11.4 | 59.0 | 1.88 | 1.92 | 1.21 | 1.90 | 0.91 | 15.0 |
| 4 | 5.04 | 4.02 | 13.4 | 70.9 | 0.74 | 0.78 | 0.44 | 0.86 | BLD^{a} | 3.86 |
| 8 | 8.77 | 3.23 | 12.7 | 73.0 | BLD | BLD | 0.26 | 0.89 | BLD | 1.20 |
| 24 | 14.7 | 2.72 | 15.2 | 66.2 | BLD | BLD | BLD | 1.22 | BLD | BLD |

| Rs458 | Major metabolites observed in cellular extracts after 10 µM Compound **1** (% of total radioactivity) | | | | | | | | | |
|---|---|---|---|---|---|---|---|---|---|---|
| Time (hour) | 2'-C- methyl guariosine | Compd **5** | Compd **6** | Compd **7** | Compd **3** | Compd **2^{b}** | U4 | U1 | U3 | Comp **1** |
| 1 | 1.75 | 2.65 | 12.3 | 50.8 | BLD | 3.48 | 1.44 | 5.55 | BLD | 19.7 |
| 4 | 3.78 | 4.53 | 16.7 | 69.1 | BLD | BLD | 0.32 | 1.54 | BLD | 4.02 |
| 8 | 4.95 | 3.84 | 16.8 | 72.5 | BLD | BLD | BLD | 0.92 | BLD | 0.95 |
| 24 | 16.9 | 12.8 | 14.6 | 54.5 | BLD | BLD | BLD | 1.2 | BLD | BLD |

| | | | | | | | | | | |
|---|---|---|---|---|---|---|---|---|---|---|
| Rat hepatocytes were incubated for 1, 4, 8 and 24 hours with [¹⁴C]-Compound 1 at 10 µM as described under experimental procedures. Values represent metabolite levels in the cellular extract and are expressed as percent (%) of total radioactivity measured in the sample. ^{a}BLD = below limit of detection (30 DPM) ^{b}Possible co elution of an unknown metabolite with Compound **2**. | | | | | | | | | | |

**Table 22: Compound 1 metabolites observed in human and rat hepatocyte extracts following incubation with [¹⁴C]-Compound 1**

| Hu775 | Major metabolites observed in cellular extracts after 1 µM Compound **1** (% of total radioactivity) | | | | | | | |
|---|---|---|---|---|---|---|---|---|
| Time (hour) | 2'-C- methyl guanosine | Compound **5** | Compound **6** | Compound **7** | Compound **2^{b}** | | U1 | Compound **1** |
| 1 | BLD^{a} | 5.45 | 7.94 | 48.5 | 4.94 | 5.38 | | 19.9 |
| 8 | 4.34 | 3.60 | 13.0 | 75.0 | BLD | BLD | | 4.07 |
| 24 | 9.05 | 6.41 | 12.4 | 67.0 | BLD | BLD | | 2.78 |
| | | | | | | | | |

| Rs458 | Major metabolites observed in cellular extracts after 1 µM Compound **1** (% of total radioactivity) | | | | | | | |
|---|---|---|---|---|---|---|---|---|
| Time (hour) | 2'-C- methyl guanosine | Compound **5** | Compound **6** | Compound **7** | Compound **2^{b}** | U1 | U3 | Compd **1** |
| 1 | 1.01 | 3.74 | 13.0 | 50.5 | 5.83 | 1.93 | 8.43 | 12.3 |
| 8 | 5.58 | 3.51 | 13.27 | 77.6 | BLD | BLD | BLD | BLD |
| 24 | 13.1 | 6.68 | 18.1 | 62.1 | BLD | BLD | BLD | BLD |

| | | | | | | | | |
|---|---|---|---|---|---|---|---|---|
| Human and rat hepatocytes were incubated for 1, 8 and 24 hours with [¹⁴C]-Compound 1 at 1 µM as described under experimental procedures. Values represent metabolite levels in the cellular extract and are expressed as percent (%) of total radioactivity measured in the sample. ^{a} BLD = below limit of detection (30 DPM) ^{b}Possible co elution of an unknown metabolite with Compound **2**. | | | | | | | | |

**Table 23A: 2'-C-methyl guanosine metabolites observed in human hepatocyte extracts following incubation with [¹⁴C]-2'-C-methyl guanosine**

| Hu759 | Major metabolites observed in cellular extracts after 10 µM 2'-C-methyl guanosine (% of total radioactivity) | | | |
|---|---|---|---|---|
| Time (hour) | 2'-C-methyl guanosine | Compound **5** | Compound **6** | Compound **7** |
| 1 | 97.1 | BLD^{a} | 0.51 | 2.40 |
| 4 | 87.3 | BLD | 1.64 | 11.1 |
| 8 | 73.9 | 1.89 | 4.54 | 19.6 |
| 24 | 52.4 | 3.82 | 7.71 | 36.1 |
| | | | | |
| Hu775 | Major metabolites observed in cellular extracts after 10 µM 2'-C-methyl guanosine (% of total radioactivity) | | | |
| Time (hour) | 2'-C-methyl guanosine | Compound **5** | Compound **6** | Compound **7** |
| 1 | 94.9 | 0.59 | 0.63 | 3.93 |
| 4 | 77.3 | 0.89 | 2.36 | 19.4 |
| 8 | 61.4 | 1.11 | 3.46 | 34.1 |
| 24 | 40.2 | 3.41 | 10.6 | 45.8 |

| | | | | |
|---|---|---|---|---|
| Human and rat hepatocytes were incubated for 1, 4, 8 and 24 hours with [¹⁴C]-2'-C-methyl guanosine at 10 µM as described under experimental procedures. Values represent metabolite levels in the cellular extract and are expressed as percent (%) of total radioactivity measured in the sample. ^{a} BLD=below limit of detection (30 DPM) | | | | |

**Table 23B: 2'-C-methyl guanosine metabolites observed in monkey hepatocyte extracts following incubation with [¹⁴C]-2'-C-methyl guanosine**

| Cy230^{a} | Major metabolites observed in cellular extracts after 10 µM 2'-C-methyl guanosine (% of total radioactivity) | | | |
|---|---|---|---|---|
| Time (hour) | 2'-C-methyl guanosine | Compound **5** | Compound **6** | Compound **7** |
| 1 | 96.4 | BLD^{b} | BLD | 3.63 |
| 8 | 77.3 | BLD | 2.82 | 19.9 |
| 24 | 65.0 | BLD | 8.41 | 26.6 |
| | | | | |

| Cy234 | Major metabolites observed in cellular extracts after 10 µM 2'-C-methyl guanosine (% of total radioactivity) | | | |
|---|---|---|---|---|
| Time (hour) | 2'-C-methyl guanosine | Compound **5** | Compound **6** | Compound **7** |
| 1 | 86.9 | 2.19 | 2.19 | 8.70 |
| 4 | 57.9 | BLD | 6.51 | 35.6 |
| 8 | 45.8 | 3.32 | 7.90 | 43.0 |
| 24 | 29.0 | 5.03 | 14.1 | 51.8 |

| | | | | |
|---|---|---|---|---|
| Monkey hepatocytes were incubated for 1, 4, 8 and 24 hours with [¹⁴C]-2'-C-methyl guanosine at 10 µM as described under experimental procedures. Values represent metabolite levels in the cellular extract and are expressed as percent (%) of total radioactivity measured in the sample. ^{a}Due to limited cell availability, sampling at 4 hours was not conducted for Monkey 1 (Cy230). ^{b}BLD = below limit of detection (30 DPM) | | | | |

**Table 24: 2'-C-methyl guanosine metabolites observed in rat hepatocyte extracts following incubation with [¹⁴C]-2'-C-methyl guanosine**

| Rs448 | Major metabolites observed in cellular extracts after 10 µM 2'-C-methyl guanosine (% of total radioactivity) | | | |
|---|---|---|---|---|
| Time (hour) | 2'-C-methyl guanosine | Compound **5** | Compound **6** | Compound **7** |
| 1 | 93.6 | BLD^{a} | 1.24 | 5.21 |
| 4 | 78.6 | BLD | 3.54 | 17.9 |
| 8 | 69.2 | BLD | 4.76 | 24.1 |
| 24 | 51.5 | 2.56 | 10.4 | 35.6 |
| | | | | |

| Rs458 | Major metabolites observed in cellular extracts after 10 µM 2'-C-methyl guanosine (% of total radioactivity) | | | |
|---|---|---|---|---|
| Time (hour) | 2'-C-methyl guanosine | Compound **5** | Compound **6** | Compound **7** |
| 1 | 94.5 | BLD^{a} | 1.08 | 4.42 |
| 4 | 79.8 | BLD | 4.01 | 16.2 |
| 8 | 67.2 | BLD | 5.42 | 27.0 |
| 24 | 47.1 | 9.39 | 9.65 | 33.9 |

| | | | | |
|---|---|---|---|---|
| Rat hepatocytes were incubated for 1, 4, 8 and 24 hours with [¹⁴C]-2'-C-methyl guanosine at 10 µM as described under experimental procedures. Values represent metabolite levels in the cellular extract and are expressed as percent (%) of total radioactivity measured in the sample. ^{a}BLD = below limit of detection (30 DPM) | | | | |

**Table 25: 2'-C-methyl guanosine metabolites observed in human and rat hepatocyte extracts following incubation with [¹⁴C]-2'-C-methyl guanosine**

| Hu775 | Major metabolites observed in cellular extracts after 1 µM 2'-C-methyl guanosine (% of total radioactivity) | | | |
|---|---|---|---|---|
| Time (hour) | 2'-C-methyl guanosine | Compound **5** | Compound **6** | Compound **7** |
| 1 | 94.6 | BLD^{a} | BLD | 5.36 |
| 8 | 65.8 | BLD | 6.83 | 27.3 |
| 24 | 41.3 | 3.39 | 9.04 | 46.3 |
| | | | | |

| Rs458 | Major metabolites observed in cellular extracts after 1 µM 2'-C-methyl guanosine (% of total radioactivity) | | | |
|---|---|---|---|---|
| Time (hour) | 2'-C-methyl guanosine | Compound **5** | Compound **6** | Compound **7** |
| 1 | 93.4 | BLD^{a} | 1.50 | 4.56 |
| 8 | 68.7 | BLD | 5.14 | 26.1 |
| 24 | 49.2 | 6.83 | 9.88 | 34.1 |

| | | | | |
|---|---|---|---|---|
| Human and rat hepatocytes were incubated for 1, 4, 8 and 24 hours with [¹⁴C]-2'-C-methyl guanosine at 1 µM as described under experimental procedures. Values represent metabolite levels in the cellular extract and are expressed as percent (%) of total radioactivity measured in the sample. ^{a}BLD = below limit of detection (30 DPM) | | | | |

**Table 26: Compound 1 metabolites observed in Huh7 cellular extracts following incubation with [¹⁴C]-Compound 1**

| Huh7 | Major metabolites observed in cellular extracts after 10 µM Compound **1** (% of total radioactivity) | | | | | |
|---|---|---|---|---|---|---|
| Time (hour) | 2'-C- methyl guanosine | Compound **5** | Compound **6** | Compound **7** | U9 | Compound **1** |
| 24 | BLD^{a} | BLD | BLD | 59.7 | BLD | 40.3 |
| 48 | BLD | 6.57 | 6.14 | 71.5 | BLD | 15.8 |
| 72 | BLD | 6.93 | 6.86 | 72.1 | BLD | 14.1 |
| | | | | | | |

| Huh7 | Major metabolites observed in cellular extracts after 10 µM Compound **1** (% of total radioactivity) | | | | | |
|---|---|---|---|---|---|---|
| Time (hour) | 2'-C- methyl guanosine | Compound **5** | Compound **6** | Compound **7** | U9 | Compound **1** |
| 24 | BLD^{a} | 4.81 | 7.55 | 71.8 | 5.40 | 10.4 |
| 48 | 2.65 | 5.82 | 6.42 | 59.3 | 4.61 | 21.2 |
| 72 | BLD | 5.74 | 8.44 | 69.6 | BLD | 16.2 |

| | | | | | | |
|---|---|---|---|---|---|---|
| Cells were incubated with [¹⁴C]-Compound **1** at 10 µM as described under experimental procedures. Values represent metabolite levels in the cellular extract and are expressed as percent (%) of total radioactivity measured in the sample. ^{a}BLD = below limit of detection (30 DPM) | | | | | | |

**Table 27: 2'-C-methyl guanosine metabolites observed in Huh7 cellular extracts following incubation with [¹⁴C]-2'-C-methyl guanosine**

| Huh7 | Major metabolites observed in cellular extracts after 10 µM 2'-C-methyl guanosine (% of total radioactivity) | | | |
|---|---|---|---|---|
| Time (hour) | 2'-C-methyl guanosine | Compound **5** | Compound **6** | Compound **7** |
| 24 | 89.5 | BLD^{a} | BLD | 10.5 |
| 48 | 89.3 | BLD | 3.56 | 7.18 |
| 72 | 89.6 | BLD | BLD | 10.4 |

| | | | | |
|---|---|---|---|---|
| Cells were incubated with [¹⁴C]-2'-C-methyl guanosine at 10 µM as described under experimental procedures. Values represent metabolite levels in the cellular extract and are expressed as percent (%) of total radioactivity measured in the sample. ^{a}BLD = below limit of detection (30 DPM) | | | | |

**Table 28: Levels of 2'-C-methyl guanosine-5'-triphosphate in primary hepatocytes following incubation with [¹⁴C]-Compound 1 or [¹⁴C]-2'-C-methyl guanosine**

| Compound 7 levels (pmol/million cells) Human hepatocytes | | | | |
|---|---|---|---|---|
| | [¹⁴C]-2'-C-methyl guanosine (10 µM) | | [¹⁴C]-Compound 1 (10 µM) | |
| Time (hour) | Human 1 (Hu759) | Human 2 (Hu775) | Human 1 (Hu759) | Human 2 (Hu775) |
| 0 | 0 | 0 | 0 | 0 |
| 1 | 9.70 | 18.5 | 24.0 | 54 |
| 4 | 51.0 | 86 | 166 | 216 |
| 8 | 96.0 | 149 | 225 | 366 |
| 24 | 214 | 215 | 360 | 564 |
| AUC^{a} | 2871 | 3548 | 5759 | 9036 |

| | Compound 7 levels (pmol/million cells) Monkey hepatocytes^{b} | | | |
|---|---|---|---|---|
| | [¹⁴C]-2'-C-methyl guanosine (10 µM) | | [¹⁴C]-Compound 1 (10 µM) | |
| Time (hour) | Monkey 1 (Cy230)^{b} | Monkey 2 (Cy234) | Monkey 1 (Cy230)^{b} | Monkey 2 (Cy234) |
| 0 | 0 | 0 | 0 | 0 |
| 1 | 2.87 | 4.00 | 180 | 22.0 |
| 4 | - | 29.0 | - | 183 |
| 8 | 14.58 | 39.0 | 895 | 476 |
| 24 | 6.85 | 57.0 | 344 | 552 |
| AUC | 241 | 956 | 13765 | 9861 |

| | Compound 7 levels (pmol/million cells) Rat hepatocytes (n=2) | | | |
|---|---|---|---|---|
| | [¹⁴C]-2'-C-methyl guanosine (10 µM) | | [¹⁴C]-Compound 1 (10 µM) | |
| Time (hour) | Rat 1 (Rs448) | Rat 2 (Rs458) | Rat 1 (Rs448) | Rat 2 (Rs458) |
| 0 | 0 | 0 | 0 | 0 |
| 1 | 28.0 | 23.0 | 580 | 534 |
| 4 | 141 | 113 | 1429 | 1347 |
| 8 | 258 | 239 | 1791 | 1618 |
| 24 | 347 | 319 | 937 | 770 |
| AUC | 5906 | 5384 | 31568 | 28123 |

| | | | | |
|---|---|---|---|---|
| Primary hepatocytes were incubated for 1, 4, 8 and 24 hours with [¹⁴C]-Compound 1 or [¹⁴C]-2'-C-methyl guanosine at 10 µM as described under experimental procedures. Values represent Compound **7** levels in cellular extracts from human (n=2), monkey (n=2) and rat (n=2) hepatocytes and are expressed in pmol/million cells. ^{a}AUC = area under the concentration-time curve expressed as pmol*hr/ million cells ^{b}Due to limited cell availability, sampling at 4 hours was not conducted for monkey #1 (Cy230). | | | | |

**Table 29: Levels of 2'-C-methyl guanosine-5'-triphosphate in primary hepatocytes following incubation with [¹⁴C]-Compound 1 or [¹⁴C]-2'-C-methyl guanosine**

| | Compound **7** levels (pmol/million cells) | | | |
|---|---|---|---|---|
| | Human hepatocytes | | | |
| | [¹⁴C]-2'-C-methyl guanosine (1 µM) | | [¹⁴C]-Compound 1 (1 µM) | |
| Time (hour) | Human 2 (Hu775) | Rat 2 (Rs458) | Human 2 (Hu775) | Rat 2 (Rs458) |
| 0 | 0 | 0 | 0 | 0 |
| 1 | 1.90 | 2.60 | 8.80 | 80.0 |
| 8 | 11.0 | 20.1 | 44.2 | 238 |
| 24 | 21.0 | 27.0 | 60.4 | 103 |
| | | | | |
| AUC^{a} | 302 | 458 | 1027 | 3881 |

| | | | | |
|---|---|---|---|---|
| Primary hepatocytes were incubated for 1, 8 and 24 hours with [¹⁴C]-Compound **1** or [¹⁴C]-2'-C-methyl guanosine at 1 µM as described under experimental procedures. Values represent Compound **7** levels in cellular extracts from human (n=1) and rat (n=1) hepatocytes and are expressed in pmol/million cells. ^{a}AUC = area under the concentration-time curve expressed as pmol*hr/million cells | | | | |

Confirmation of 2'-C-methyl guanosine nucleotides was carried out with alkaline phosphatase digestion. 2'-C-methyl guanosine nucleotide levels, after incubation with [¹⁴C]-Compound 1 and [¹⁴C]-2'-C-methyl guanosine, are summarized in Table 28 through Table 34.

**Table 30: Total 2'-C-methyl guanosine nucleotides in primary hepatocytes following incubation with [¹⁴C]-Compound 1 or [¹⁴C]-2'-C-methyl guanosine**

| Time (hour) | [¹⁴C]-Compound 1 (10 µM) | | | | | | | |
|---|---|---|---|---|---|---|---|---|
| | Total intracellular nucleotides (pmol/million cells) | | | | | | | |
| | Human (n=2) | | | Monkey^{a} (n=2) | | Rat (n=2) | | |
| 0 | 0 | 0 | | 0 | 0 | 0 | | 0 |
| 1 | 31.0 | 71.4 | | 231 | 39.8 | 735 | | 691 |
| 4 | 202 | 272 | | - | 242 | 1779 | | 1761 |
| 8 | 295 | 443 | | 1091 | 600 | 2181 | | 2079 |
| 24 | 457 | 846 | | 466 | 686 | 1191 | | 1158 |

| Time (how) | [¹⁴C]-2'-C-methyl guanosine (10 µM) | | | | | | | |
|---|---|---|---|---|---|---|---|---|
| | Total intracellular nucleotides (pmol/million cells) | | | | | | | |
| | Human (n=2) | | | Monkey (n=2) | | Rat (n=2) | | |
| 0 | 0 | 0 | | 0 | 0 | 0 | | 0 |
| 1 | 11.8 | 24.2 | | 2.87 | 5.90 | 35.0 | | 29.0 |
| 4 | 58.9 | 101 | | - | 34.3 | 169 | | 141 |
| 8 | 127 | 169 | | 16.7 | 49.0 | 305 | | 287 |
| 24 | 283 | 281 | | 9.01 | 77.5 | 473 | | 498 |

| Time (hour) | Total intracellular nucleotides (pmol/million cells) | | | | | | | |
|---|---|---|---|---|---|---|---|---|
| | [¹⁴C]-Compound 1 (1 µM) | | | | [¹⁴C]-2'-C-methyl guanosine (1 µM) | | | |
| | Human (n=1) | | Rat (n=1) | | Human (n=1) | | Rat (n=1) | |
| 0 | 0 | | 0 | | 0 | | 0 | |
| 1 | 12.6 | | 106 | | 1.90 | | 3.45 | |
| 8 | 54.0 | | 290 | | 13.7 | | 24.0 | |
| 24 | 77.4 | | 144 | | 26.5 | | 40.4 | |

| | | | | | | | | |
|---|---|---|---|---|---|---|---|---|
| Primary hepatocytes were incubated for 1, 4, 8 and 24 hours with [¹⁴C]-Compound **1** or [¹⁴C]-2'-C-methyl guanosine at 1 or 10 µM as described under experimental procedures. Values represent total 2'-C-methyl guanosine nucleotide levels (sum of MP, DP and TP) in cellular extracts from human, monkey and rat hepatocytes and are expressed in pmol/million cells. ^{a}Due to limited cell availability, sampling at 4 hours was not conducted for monkey 1 (Cy230). | | | | | | | | |

**Table 31: 2'-C-methyl guanosine nucleotide levels in human hepatocytes following incubation with [¹⁴C]-Compound 1 and [¹⁴C]-2'-C-methyl guanosine**

| [¹⁴C]-Compound **1** (10 µM) | Compound **5** | | Compound **6** | | Compound **7** | |
|---|---|---|---|---|---|---|
| Time (hour) | pmol/million cells | | | | | |
| 0 | 0 | 0 | 0 | 0 | 0 | 0 |
| 1 | 4.00 | 10.3 | 3.00 | 7.10 | 24.0 | 54.0 |
| 4 | 15.0 | 17.1 | 21.0 | 38.4 | 166 | 216 |
| 8 | 25.0 | 21.9 | 45.0 | 55 | 225 | 366 |
| 24 | 28.0 | 121 | 69.0 | 161 | 360 | 564 |

| [¹⁴C]-2'-C-methyl guanosine (10 µM) | Compound **5** | | Compound **6** | | Compound **7** | |
|---|---|---|---|---|---|---|
| Time (hour) | pmol/million cells | | | | | |
| 0 | 0 | 0 | 0 | 0 | 0 | 0 |
| 1 | BLD^{a} | 2.80 | 2.10 | 2.90 | 9.70 | 18.5 |
| 4 | BLD | 4.00 | 7.50 | 10.5 | 51.0 | 86.0 |
| 8 | 9.00 | 4.90 | 22.1 | 15.0 | 96.0 | 149 |
| 24 | 23.0 | 16.0 | 46.0 | 50.0 | 214 | 215 |

| | | | | | | |
|---|---|---|---|---|---|---|
| Human hepatocytes were incubated for 1, 4, 8 and 24 hours with [¹⁴C]-Compound **1** or [¹⁴C]-2'-C-methyl guanosine at 10 µM as described under experimental procedures. Values represent nucleotide levels in cellular extracts from two donors (Hu759 and Hu775) and are expressed in pmol/million cells. ^{a} BLD = below limit of detection (0.02 pmol/million cells) | | | | | | |

**Table 32: 2'-C-methyl guanosine nucleotide levels in monkey hepatocytes following incubation with [¹⁴C]-Compound 1 and [¹⁴C]-2'-C-methyl guanosine**

| [¹⁴C]-Compound 1 (10 µM) | Compound **5** | | Compound **6** | | Compound **7** | |
|---|---|---|---|---|---|---|
| Time (hour) | pmol/million cells | | | | | |
| | Monkey 1^{a} (Cy230) | Monkey 2 (Cy234) | Monkey 1 (Cy230) | Monkey 2 (Cy234) | Monkey 1 (Cy230) | Monkey 2 (Cy234) |
| 0 | 0 | 0 | 0 | 0 | 0 | 0 |
| 1 | 23.9 | 11.7 | 26.3 | 6.10 | 180 | 22.0 |
| 4 | - | 14.3 | - | 44.5 | - | 183 |
| 8 | 42.5 | 33.0 | 153 | 91.0 | 895 | 476 |
| 24 | 33.2 | 23.2 | 88.4 | 111 | 344 | 552 |

| [¹⁴C]-2'-C-methyl guanosine (10 µM) | Compound **5** | | Compound **6** | | Compound **7** | |
|---|---|---|---|---|---|---|
| Time (hour) | pmol/million cells | | | | | |
| | Monkey 1^{a} | Monkey 2 | Monkey 1 | Monkey 2 | Monkey 1 | Monkey 2 |
| | (Cy230) | (Cy234) | (Cy230) | (Cy234) | (Cy230) | (Cy234) |
| 0 | 0 | 0 | 0 | 0 | 0 | 0 |
| 1 | BLD^{b} | 0.95 | BLD | 0.95 | 2.87 | 4.00 |
| 4 | - | BLD | - | 5.30 | - | 29.0 |
| 8 | BLD | 3.00 | 2.07 | 7.00 | 14.6 | 39.0 |
| 24 | BLD | 5.50 | 2.16 | 15.0 | 6.85 | 57.0 |

| | | | | | | |
|---|---|---|---|---|---|---|
| Monkey hepatocytes were incubated for 1, 4, 8 and 24 hours with [¹⁴C]-Compound **1** or [¹⁴C]-2'-C-methyl guanosine at 10 µM as described under experimental procedures. Values represent nucleotide levels in cellular extracts from two donors (Cy230 and Cy234) and are expressed in pmol/million cells. ^{a}Due to limited cell availability, sampling at 4 hours was not conducted for monkey 1 (Cy230). ^{b} BLD = below limit of detection (0.02 pmol/million cells) | | | | | | |

**Table 33: 2'-C-methyl guanosine nucleotide levels in rat hepatocytes following incubation with [¹⁴C]-Compound 1 and [¹⁴C]-2'-C-methyl guanosine**

| [¹⁴C]-Compound **1** (10 µM) | Compound **5** | | Compound **6** | | Compound **7** | |
|---|---|---|---|---|---|---|
| Time (hour) | pmol/million cells | | | | | |
| | Rat 1 (Rs448) | Rat 2 (Rs458) | Rat 1 (Rs448) | Rat 2 (Rs458) | Rat 1 (Rs448) | Rat 2 (Rs458) |
| 0 | 0 | 0 | 0 | 0 | 0 | 0 |
| 1 | 43.0 | 28.0 | 112 | 129 | 580 | 534 |
| 4 | 81.0 | 88.0 | 269 | 326 | 1429 | 1347 |
| 8 | 79.0 | 86.0 | 311 | 375 | 1791 | 1618 |
| 24 | 39.0 | 181 | 215 | 207. | 937 | 770 |

| [¹⁴C]-2'-C-methyl guanosine (10 µM) | Compound **5** | | Compound **6** | | Compound **7** | |
|---|---|---|---|---|---|---|
| Time (hour) | pmol/million cells | | | | | |
| | Rat 1 (Rs448) | Rat 2 (Rs458) | Rat 1 (Rs448) | Rat 2 (Rs458) | Rat 1 (Rs448) | Rat 2 (Rs458) |
| | 0 | 0 | 0 | 0 | 0 | 0 |
| 1 | BLD^{a} | BLD | 7.00 | 5.60 | 28.0 | 23.0 |
| 4 | BLD | BLD | 28.0 | 28.0 | 141 | 113 |
| 8 | BLD | BLD | 47.0 | 48.0 | 287 | 239 |
| 24 | 25.0 | 88.0 | 101 | 91.0 | 347 | 319 |

| | | | | | | |
|---|---|---|---|---|---|---|
| Rat hepatocytes were incubated for 1, 4, 8 and 24 hours with [¹⁴C]-Compound **1** or [¹⁴C]-2'-C-methyl guanosine at 10 µM as described under experimental procedures. Values represent nucleotide levels in cellular extracts from two donors (Rs448 and Rs458) and are expressed in pmol/million cells. ^{a}BLD = below limit of detection (0.02 pmol/million cells) | | | | | | |

**Table 34: 2'-C-methyl guanosine nucleotide levels in human and rat hepatocytes following incubation with [¹⁴C]-Compound 1 and [¹⁴C]-2'-C-methyl guanosine**

| [¹⁴C]-Compound **1** (1 µM) | Compound **5** | | Compound **6** | | Compound **7** | |
|---|---|---|---|---|---|---|
| Time (hour) | pmol/million cells | | | | | |
| | Human 2 (Hu775) | Rat 2 (Rs458) | Human 2 (Hu775) | Rat 2 (Rs458) | Human 2 (Hu775) | Rat 2 (Rs458) |
| 0 | BLD^{a} | BLD | BLD | BLD | BLD | BLD |
| 1 | 2.40 | 6.00 | 1.40 | 20.0 | 8.80 | 80.0 |
| 8 | 2.10 | 11.0 | 7.70 | 41.2 | 44.2 | 238 |
| 24 | 5.80 | 11.0 | 11.2 | 30.0 | 60.4 | 103 |

| [¹⁴C]-2'-C-methyl guanosine(1 µM) | Compound **5** | | Compound **6** | | Compound **7** | |
|---|---|---|---|---|---|---|
| Time (hour) | pmol/million cells | | | | | |
| | Human 2 (Hu775) | Rat 2 (Rs458) | Human 2 (Hu775) | Rat 2 (Rs458) | Human 2 (Hu775) | Rat 2 (Rs458) |
| 0 | BLD | BLD | BLD | BLD | BLD | BLD |
| 1 | BLD | BLD | BLD | 0.85 | 1.90 | 2.60 |
| 8 | BLD | BLD | 2.70 | 4.00 | 11.0 | 20.1 |
| 24 | 1.50 | 5.40 | 4.00 | 8.00 | 21.0 | 27.0 |

| | | | | | | |
|---|---|---|---|---|---|---|
| Human and rat hepatocytes were incubated for 1, 8 and 24 hours with [¹⁴C]-Compound 1 or [¹⁴C]-2'-C-methyl guanosine at 1 µM as described under experimental procedures. Values represent nucleotide levels in cellular extracts from two donors (Rs448 and Rs458) and are expressed in pmol/million cells. ^{a}BLD = below limit of detection (0.02 pmol/million cells) | | | | | | |

After 24 hours incubation with [¹⁴C]-Compound **1** in human hepatocytes the major metabolite was Compound **7** (65%), followed by Compound **6** (15.3%) and 2'-C-methyl guanosine and Compound **5** with 9-10% of total radioactivity. Compound 1 (1.5%) was also detected at 24 hours. Maximum Compound **7** levels of 296 pmol/million cells (mean of two donors) were observed at 24 hours. Because sampling beyond 24 hours was not conducted, it is unclear if Compound **7** levels would continue to rise. The mean Compound **7** AUC₀₋₂₄ₕ in human hepatocytes (n=2) was 7,398 pmol*h/million cells.

Compound **7** was also the major intracellular metabolite in monkey (>70%) and rat (70-55%) hepatocytes from 4-24 hours. Compound **1** was undetected after 24 hours in both species. In monkey hepatocytes, Compound **6** was 17% whereas 2'-C-methyl guanosine and Compound **5** were 2-5% of total intracellular radioactivity at 24 hours. 2'-C-methyl guanosine, Compounds **5** and **6** accounted for 8-16% of total radioactivity in rat hepatocytes at 24 hours. Compound **7** reached maximum levels of 1,705 pmol/million cells at 8 hours in rat hepatocytes while in monkey, peak levels of 895 and 552 pmol/million cells were reached at 8 hours in one donor and at 24 hours in the second. For Compound **7,** AUC₀₋₂₄ₕ values were 11,813 and 29,846 pmol*h/million cells in monkey and rat hepatocytes, respectively.

[¹⁴C]-2'-C-methyl guanosine metabolism was also evaluated in primary hepatocytes. The metabolic profile of [¹⁴C]-2'-C-methyl guanosine in primary hepatocytes is summarized in Table 21 through Table 25 and Table 28 through Table 34. Percent of mono-, di- and triphosphate in human, monkey and rat hepatocytes were 7-12%, 17-20% and 69-76%, respectively. Levels of Compound **7** after incubation with [¹⁴C]-2'-C-methyl guanosine were 2.3 to 19.7 times lower than after incubation with [¹⁴C]-Compound **1.** Maximum Compound **7** levels of 215, 32 and 249 pmol/million cells were observed at 24 hours in human, monkey and rat hepatocytes, respectively.

### [¹⁴C]-Compound 1 and [¹⁴C]-2'-C-methyl guanosine at 1 micromolar

A similar metabolic trend and profile was observed at 1 µM [¹⁴C]-Compound 1 in rat and human hepatocytes (Table 21, Table 29, Table 30 and Table 34); 1 µM [¹⁴C]-Compound **1** was not evaluated in monkey hepatocytes. Compound **7** was the major metabolite in both species, accounting for 70-78% of total radioactivity at 24 hours with maximal levels of 60.4 and 103 pmol/million cells in human and rat hepatocytes, respectively. The AUC₀₋₂₄ₕ for Compound **7** was 3,881 and 1,027 pmol*h/million cells for rat (n=1) and human (n=1) hepatocytes, respectively. These values are approximately 8.8-and 7.2-fold lower than values observed for 10 µM [¹⁴C]-Compound **1** in human and rat hepatocytes, respectively. [¹⁴C]-2'-C-methyl guanosine at 1 µM exhibited a similar profile as observed at 10 µM. The AUC₀₋₂₄ₕ for Compound **7** following incubation with 1 µM [¹⁴C]-2'-C-methyl guanosine was 302 and 458 pmol*h/million cells for human (n=1) and rat (n=1) hepatocytes, respectively, 11.7-fold lower than the AUC observed with the 10 µM 2'-C-methyl guanosine. These observations suggest that formation of Compound **7** is dose dependent for both Compound **1** and 2'-C-methyl guanosine in this concentration range.

### [¹⁴C]-Compound 7 half-life in human and rat hepatocytes

Table 35 and Table 36 summarize Compound **7** decay in human and rat hepatocytes. Compound **7** levels at time zero were 692 and 932 pmol/million cells in human and rat hepatocytes, respectively. After 24 hours without [¹⁴C]-Compound **1,** Compound **7** levels decreased to 273 (61% reduction) and 83 (91% reduction) pmol/million cells in human and rat hepatocytes, respectively. The *in vitro* half-life for Compound **7** in human hepatocytes was 17.4 hours, whereas in rat hepatocytes it was 6.61 hours.

**Table 35: Decay of Compound 7 in human hepatocytes following incubation with [¹⁴C]-Compound 1 and [¹⁴C]-2'-C-methyl guanosine.**

| Time (hour) | Compound **7** (pmol/million cells) | |
|---|---|---|
| | [¹⁴C]-Compound **1** (10 µM) | [¹⁴C]-2'-C-methyl guanosine (10 µM) |
| 0 | 692 | 266 |
| 1 | 702 | 259 |
| 4 | 597 | 217 |
| 8 | 497 | 190 |
| 24 | 273 | 58.0 |
| t_{1/2} (hour) | 17.4 | 10.8 |

| | | |
|---|---|---|
| Compound **7** decay was measured after 24 hours incubation with [¹⁴C]-Compound **1** or [¹⁴C]-2'-C-methyl guanosine at 10µM as described under experimental procedures. Values represent Compound **7** levels in cellular extracts from 1 donor (Hu775) and are expressed in pmol/million cells. Linear regression analysis (Microsoft Excel) was used to estimate the *in vitro* intracellular t_{½} value for Compound **7.** | | |

**Table 36: Decay of Compound 7 in rat hepatocytes following incubation with [¹⁴C]-Compound 1**

| Time (hour) | [¹⁴C]-Compound **1** (10 µM) Compound **7** (pmol/million cells) |
|---|---|
| 0 | 932 |
| 1 | 954 |
| 4 | 811 |
| 8 | 545 |
| 24 | 83.0 |
| **t_{½} (hours)** | **6.61** |

| | |
|---|---|
| Compound **7** decay was measured after 24 hours incubation with [¹⁴C]-Compound **1** at 10µM as described under experimental procedures. Values represent Compound **7** levels in cellular extracts from 1 donor (Rs458) and are expressed in pmol/million cells. Linear regression analysis (Microsoft Excel) was used to estimate the *in vitro* intracellular t_{½} value for Compound **7**. | |

The *in vitro* Compound **7** half-life was also determined following [¹⁴C]-2'-C-methyl guanosine in human hepatocytes. Compound **7** levels at time zero following incubation with [¹⁴C]-2'-C-methyl guanosine for 24 hours was 266 pmol/million cells. After 24 hours without [¹⁴C]-2'-C-methyl guanosine, Compound **7** levels decreased to 58 pmol/million cells (78% reduction). The *in vitro* Compound **7** half-life after 2'-C-methyl guanosine in human hepatocytes was 10.8 hours, almost half of that observed following Compound **1.**

### Huh7 cells

The metabolic profile of [¹⁴C]-Compound **1** in Huh7 cells is summarized in Table 26, Table 37 and Table 38. 2'-C-methyl guanosine nucleotide levels after incubation with [¹⁴C]-Compound 1 and [¹⁴C]-2'-C-methyl guanosine are summarized in Table 37 and Table 38.

**Table 37: Triphosphate levels of 2'-C-methyl guanosine in Huh7 cells following incubation with [¹⁴C]-Compound 1 or [¹⁴C]-2'-C-methyl guanosine**

| Time (hour) | Compound **7** levels (pmol/million cells) | | |
|---|---|---|---|
| | [¹⁴C]-2'-C-methyl guanosine (10 µM) (n=1) | [¹⁴C]-Compound **1** (10 µM) (n=2) | |
| 0 | 0 | 0 | 0 |
| 24 | 3.10 | 21.3 | 8.60 |
| 48 | 1.40 | 15.2 | 16.1 |
| 72 | 1.60 | 13.3 | 11.1 |
| AUC^{a} | 120 | 1,020 | 726 |

| | | | |
|---|---|---|---|
| Cells were incubated with [¹⁴C]-Compound **1** or [¹⁴C]-2'-C-methyl guanosine at 10 µM as described under experimental procedures. Values represent Compound **7** levels in cellular extracts from Huh7 cells and are expressed in pmol/million cells. ^{a} AUC = area under the concentration-time curve expressed as pmol*hr/ million cells | | | |

**Table 38: 2'-C-methyl guanosine nucleotide levels in Huh7 cells following incubation with [¹⁴C]-Compound 1 and [¹⁴C]-2'-C-methyl guanosine**

| [¹⁴C]-Compound **1** (10 µM) | Compound **5** | | Compound **6** | | Compound **7** | |
|---|---|---|---|---|---|---|
| Time (hour) | pmol/million cells (n=2) | | | | | |
| | 0 | 0 | 0 | 0 | 0 | 0 |
| 24 | BLD^{a} | 1.40 | 2.20 | 0 | 8.60 | 21.3 |
| 48 | 1.50 | 1.50 | 1.60 | 1.40 | 16.1 | 15.2 |
| 72 | 1.10 | 1.10 | 1.60 | 1.10 | 11.1 | 13.3 |

| [¹⁴C]-2'-C- methyl guanosine (10 µM) | Compound **5** | | Compound **6** | | Compound **7** | |
|---|---|---|---|---|---|---|
| Time (hour) | pmol/million cells (n=1) | | | | | |
| 0 | 0 | | 0 | | 0 | |
| 24 | BLD^{a} | | BLD | | 3.10 | |
| 48 | BLD | | BLD | | 1.40 | |
| 72 | BLD | | BLD | | 1.60 | |

| | | | | | | |
|---|---|---|---|---|---|---|
| Cells were incubated for 24, 48 and 72 hours with [¹⁴C]-Compound **1** or [¹⁴C]-2'-C-methyl guanosine at 10 µM as described under experimental procedures. Values represent nucleotide levels in cellular extracts from and are expressed in pmol/million cells. ^{a}BLD = below limit of detection (0.02 pmol/million cells) | | | | | | |

Compound **7** was the major metabolite (83-93% of total radioactivity) in Huh7 cells up to 72 hours after [¹⁴C]-Compound **1** incubation. Levels of Compound **7** in Huh7 cells were 88% lower than in human hepatocytes. Maximum Compound **7** levels of 16 pmol/million cells occurred at 48 hours and the AUC₀₋₇₂ₕ was 873 pmol*h/million cells (n=2). After 72 hours incubation with [¹⁴C]-Compound **1**, unchanged Compound **1** represented 15% of the total radioactivity. One unknown metabolite (U9) was observed at 24 and 48 hours in Huh7 (n=1) cells and appears to be unique to Huh7 as it was not observed in primary hepatocytes.

Compound **7** levels were significantly lower following incubation with [¹⁴C]-2'-C-methyl guanosine (Table 18 through Table 38). Maximum concentration of 3 pmol/million cells was observed at 24 hours and the AUC₀₋₇₂ₕ was 120 pmol*h/million cells (n=1), 7.3-fold lower than with [¹⁴C]-Compound **1**. Compound **7** was the only intracellular metabolite observed.

### Effect of ribavirin and ritonavir on 2'-C-methyl guanosine nucleotide levels in primary hepatocytes

Ribavirin is a nucleoside analog currently in use for the treatment of HCV and its effects on 2'-C-methyl guanosine nucleotide levels in human (n=1) and rat (n=2) hepatocytes were evaluated after 24 hours. Table 39 and Table 40 summarizes 2'-C-methyl guanosine nucleotide levels achieved after 10 µM [¹⁴C]-Compound **1** or 10 µM [¹⁴C]-2'-C-methyl guanosine in the presence of 5 µM ribavirin.

**Table 39: 2'-C-methyl guanosine nucleotide levels in human hepatocytes following incubation with [¹⁴C]-Compound 1 or [¹⁴C]-2'-C-methyl guanosine in the presence or absence of ribavirin or ritonavir.**

| Treatment | Compound **5** | Compound **6** | Compound **7** | Total nucleotides |
|---|---|---|---|---|
| | pmol/million cells | | | |
| [¹⁴C]-Compound 1 (10 µM) | Hu775 | Hu775 | Hu775 | Hu775 |
| Control | 57.0 | 139 | 600 | 796 |
| Ribavirin 5µM | 36.0 | 148 | 548 | 732 |
| Ritonavir 1µM | 57.0 | 141 | 409 | 607 |

| | Compound **5** | Compound **6** | Compound **7** | Total nucleotides |
|---|---|---|---|---|
| | pmol/million cells | | | |
| [¹⁴C]-2'-C-methyl guanosine (10 µM) | Hu775 | Hu775 | Hu775 | Hu775 |
| Control | 24.0 | 46.0 | 243 | 313 |
| Ribavirin 5µM | 14.0 | 38.0 | 151 | 203 |
| Ritonavir 1µM | 23.0 | 71.0 | 217 | 311 |

| | | | | |
|---|---|---|---|---|
| Human hepatocytes were incubated for 24 hours with [¹⁴C]-Compound 1 or [¹⁴C]-2'-C-methyl guanosine at 10 µM in the presence or absence of ribavirin at 5 µM or ritonavir at 1µM as described under experimental procedures. Values represent nucleotide levels in cellular extracts from one donor (Hu775) and are expressed in pmol/million cells. | | | | |

**Table 40: 2'-C-methyl guanosine nucleotide levels in rat hepatocytes following incubation with [¹⁴C]-Compound 1 or [¹⁴C]-2'-C-methyl guanosine in the presence or absence of ribavirin or ritonavir.**

| Treatment | Compound **5** | | Compound **6** | | Compound **7** | | Total nucleotides | |
|---|---|---|---|---|---|---|---|---|
| | pmol/million cells | | | | | | | |
| [¹⁴C]-Compound **1** (10 µM) | Rat 1 (Rs448) | Rat 2 (Rs458) | Rat 1 (Rs448) | Rat 2 (Rs458) | Rat 1 (Rs448) | Rat 2 (Rs458) | Rat 1 (Rs448) | Rat 2 (Rs458) |
| Control | 75.0 | 91.0 | 382 | 314 | 1134 | 893 | 1591 | 1298 |
| Ribavirin 5µM | 76.0 | 83.0 | 335 | 300 | 1087 | 977 | 1498 | 1360 |
| Ritonavir 1µM | 96.0 | 116 | 364 | 411 | 1179 | 1235 | 1639 | 1762 |

| Treatment | Compound **5** | | Compound **6** | | Compound **7** | | Total nucleotides | |
|---|---|---|---|---|---|---|---|---|
| | pmol/million cells | | | | | | | |
| 14C-2'-C-methyl guanosine (10 µM) | Rat 1 (Rs448) | Rat 2 (Rs458) | Rat 1 (Rs448) | Rat 2 (Rs458) | Rat 1 (Rs448) | Rat 2 (Rs458) | Rat 1 (Rs448) | Rat 2 (Rs458) |
| Control | 24.0 | 33.0 | 164 | 105 | 419 | 299 | 607 | 437 |
| Ribavirin 5µM | 23.0 | 20.0 | 125 | 83.0 | 324 | 272 | 472 | 375 |
| Ritonavir 1µM | 37.0 | 27.0 | 136 | 106 | 378 | 279 | 551 | 412 |

| | | | | | | | | |
|---|---|---|---|---|---|---|---|---|
| Rat hepatocytes were incubated for 24 hours with [¹⁴C]-Compound 1 or [¹⁴C]-2'-C-methyl guanosine at 10 µM in the presence or absence of ribavirin at 5µM or ritonavir at 1 µM as described under experimental procedures. Values represent nucleotide levels in cellular extracts from two donors (Rs448 and Rs458) and are expressed in pmol/million cells. | | | | | | | | |

For Compound **1**, no major difference from control levels was observed in 2'-C-methyl guanosine nucleotide levels in the presence of ribavirin in human or rat hepatocytes. For 2'-C-methyl guanosine, a slight decrease (17%) in triphosphate levels was noted in rat hepatocytes while a 38% decrease was observed in human hepatocytes (n=1). However, further evaluation will be needed to determine if this effect is indeed related to inhibition of 2'-C-methyl guanosine phosphorylation by ribavirin or due to assay variability as only one donor was evaluated.

Studies in microsomes described above suggest that CYP3A may have a role in Compound **1** metabolism. In that study, ritonavir, a CYP3A inhibitor, blocked NADPH-dependent Compound **1** metabolism in liver microsomes. The effect of 1 µM ritonavir on the metabolism and subsequent formation of 2'-C-methyl guanosine nucleotides in human and rat hepatocytes following incubation with 10 µM [¹⁴C]-Compound **1** was evaluated after 24 hours and is summarized in Table 39 and Table 40. In human hepatocytes, ritonavir decreased Compound **7** levels by 30% as compared to the untreated control. However, further evaluation will be needed to determine if this effect is indeed related to inhibition of CYP450 catalyzed metabolism or due to assay variability. No decrease in 2'-C-methyl guanosine nucleotide levels was observed in rat hepatocytes (n=2).

### Conclusion

As demonstrated in this study, [¹⁴C]-Compound **1,** undergoes extensive *in vitro* metabolism in primary hepatocytes (rat>monkey>human), including formation of 2'-C-methyl guanosine nucleotides (Compounds **5, 6** and **7).**

In the incubation medium, 2'-C-methyl guanosine was the major metabolite observed in all three species. Studies in liver microsomes in Example 14 identified five major NADPH (CYP)-dependent metabolites, namely Compound **3 (3a** and **3b),** U1, U2 and U3. In the present study, U1 and U3 were detected in all three species. In contrast, U2 was not detected in any species and Compound **3 (3a** and **3b)** appeared to be a minor metabolite detectable only in rat and monkey hepatocytes. Compound **5** was observed in the incubation medium, suggesting that some metabolism/breakdown of Compound **1** occurs prior to cell penetration and may contribute towards 2'-C-methyl guanosine medium levels.

In extracts of primary hepatocytes, Compound 7 was the predominant metabolite, accounting for 60-81 % of the total intracellular radioactivity in all three species at 4-24 hours. At 24 hours, other than a small amount (1%) of metabolite U1 in rat hepatocytes and a small amount (1-2%) of unchanged Compound **1** in human hepatocytes, 2'-C-methyl guanosine and its nucleotides accounted for all of the intracellular radioactivity.

2'-C-methyl guanosine nucleotides are substantially higher following incubation of hepatocytes with [¹⁴C]-Compound **1** compared to incubation with [¹⁴C]-2'-C-methyl guanosine. This was particularly true in monkey hepatocytes where 2'-C-methyl guanosine phosphorylation was limited following incubation with 2'-C-methyl guanosine. In all three species, levels of the active triphosphate, Compound **7,** were 2.3- to 19.7-times higher from Compound **1** than from 2'-C-methyl guanosine. Triphosphate formation appeared to be dose dependent and exhibited an *in vitro* half-life of 17.4 and 6.61 hours in human and rat hepatocytes, respectively. Furthermore, Compound **5** was detectable in primary hepatocytes following incubation with Compound **1** and levels were significantly higher than observed with 2'-C-methyl guanosine, especially in monkey and rat hepatocytes where the monophosphate was undetectable or at the limit of detection following incubation with 2'-C-methyl guanosine.

In contrast to primary hepatocytes, Compound **1** metabolism including formation of Compound **7**, was significantly lower in Huh7 cells, suggesting that the enzymatic pathway(s) leading to Compound **5** releases with subsequent phosphorylation to the triphosphate may be more active in primary hepatocytes than in the human hepatoma derived cell line. Nevertheless, Compound **7** levels in Huh7 cells following incubation with [¹⁴C]-Compound **1** are substantially higher than following incubation with [¹⁴C]-2'-C-methyl guanosine.

### EXAMPLE 16

### Rat metabolism study with radiolabeled Compound 1

A preliminary rat metabolism study was conducted with radiolabeled Compound **1**. The compound was labeled with [¹⁴C] in the C-8 position of the guanine moiety. The objectives of the study were to determine, 1) the plasma pharmacokinetics of total radioactivity, 2) the time-course of total radioactivity and the amount of 2'-C-methyl guanosine-5'-TP in liver, 3) the major route of excretion, and 4) the metabolic profile in excreta, liver, and plasma.

### Study design

Isotopically diluted [¹⁴C]-Compound **1** was administered intravenously to male Sprague-Dawley rats at 50 mg/kg or by oral gavage at 100 mg/kg. The study design and sample collection are summarized in the following tables:

| Group Number | N | Dose Route | Target Dose Level (mg/kg) | Target Dose Volume (mL/kg) | Target Dose Conc. (mg/mL) | Target Radioactivity Level (µCi/kg) | Termination Time (Hours) |
|---|---|---|---|---|---|---|---|
| 1A (JVC/FVC) | 4 | IV | 50 | 1 | 50 | 340 | 2 |
| 1B (JVC/FVC) | 4 | IV | 50 | 1 | 50 | 340 | 6 |
| 1C (JVC/FVC) | 4 | IV | 50 | 1 | 50 | 340 | 24 |
| 2A (JVC) | 4 | PO | 100 | 1 | 100 | 340 | 2 |
| 2B (JVC) | 4 | PO | 100 | 1 | 100 | 340 | 6 |
| 2C (JVC) | 4 | PO | 100 | 1 | 100 | 340 | 24 |

| | | | | | | | |
|---|---|---|---|---|---|---|---|
| FVC: Femoral vein cannulated JVC: Jugular vein cannulated | | | | | | | |

### Sample Collection Summary

| Group | Urine | Feces | Blood / Plasma | Liver |
|---|---|---|---|---|
| 1A (IV) | Pre-dose, 0-1, 1-2 h | Pre-dose, 0-2 h | 15 min., 1, 2 h | 2 h |
| 1B (IV) | Pre-dose, 0-1, 1-2, 2-6 h | Pre-dose, 0-6 h | Pre-dose, 45 min., 4, 6 h | 6 h |
| 1C (IV) | Pre-dose, 0-1, 1-2, 2-6, 6-24 h | Pre-dose, 0-6, 6-24 h | 5, 30 min., 10, 24 h | 24 h |
| 2A (PO) | Pre-dose, 0-1, 1-2h | Pre-dose, 0-2 h | 15 min., 1, 2 h | 2 h |
| 2B (PO) | Pre-dose, 0-1, 1-2, 2-6 h | Pre-dose, 0-6 h | Pre-dose, 45 min., 4, 6 h | 6 h |
| 2C (PO) | Pre-dose, 0-1, 1-2, 2-6, 6-24 h | Pre-dose, 0-6, 6-24 h | 5, 30 min., 10, 24 h | 24 h |

Compound **1** [guanyl-8-¹⁴C] was obtained from Moravek Biochemicals, Inc, Brea, CA and had following specifications:

| Identity | Compound 1 [guanyl-8-¹⁴C]- |
|---|---|
| Radiochemical Purity | 98.4% (as isomers) |
| Specific Activity | 54.8 mCi/mmol |
| Batch/Lot Number | 448-108-0548 |
| Molecular weight | 628.4 g/mol |
| Supplier | Moravek Biochemicals, Inc, Brea, CA |

Compound 1 was prepared by following the procedures described previously or routine modifications thereof and had following specifications:

| Identity | Compound 1 |
|---|---|
| HPLC Purity | 98.9% by peak area at 254 nm |
| Batch/Lot Number | JW-210-112-01 |
| Molecular weight | 626.619 g/mol |
| Supplier | Idenix Pharmaceuticals |

Reference compounds 2'-C-methyl guanosine, Compound **2,** Compound **3,** Compound **4,** Compound **5** (2'-C-methyl guanosine-5'-MP), Compound **7** (2'-C-methyl guanosine-5'-TP), Compound **6** (2'-C-methyl guanosine-5'-DP, present in Compound **7)** were prepared following the procedures described herein or routine modifications thereof.

All chemicals used for the study were of reagent grade or better. Solvents were HPLC grade and were purchased from Burdick and Johnson.

### Analytical procedures

### Liquid scintillation counting (LSC)

Radioactivity in all samples was quantitated by liquid scintillation counting (LSC). A Beckman Coulter LS6500 multipurpose liquid scintillation counter (Beckman Instruments, Fullerton, CA) was used for LSC. Samples were aliquoted by volume and were analyzed in duplicate. EcoLite™ (MP, Irvine, CA) liquid scintillation cocktail was added to each liquid sample.

### High-performance liquid chromatography

For HPLC analyses, radioactivity in the eluate was detected using a radioactivity monitor that employed a liquid scintillant cell as indicated below. The percentages of radioactivity in the separated components were quantified by integrating the peaks using ProFSA software. HPLC analyses were conducted using the systems and conditions listed below.

### System 1:Agilent 1100 Series

- Well Plate Autosampler
- Thermostat for 1100 Sampler
- Diode Array Detector
- Thermostatted Column Compartment
- Quaternary Pump
- Vacuum Degasser
- Chemstation for LC3 D Version A.10.02 (1757)

### (Agilent Technologies, Inc., DE)

- Flow Scintillation Analyzer Radiomatic™ 625TR
- ProFSA Software

### (Perkin Elmer Life Sciences, MA)

This system was used for analysis of dose formulations and for metabolite profile in urine, feces and plasma.

### System 2: Agilent 1100 Series

- Well Plate Autosampler
- Thermostat for 1100 Sampler
- Diode Array Detector
- Thermostatted Column Compartment
- Quaternary Pump
- Vacuum Degasser
- Chemstation for LC3D Version B.01.01 (164) (Agilent Technologies, Inc., DE)
- Flow Scintillation Analyzer Radiomatic™ 515TR
- Flo-One™ Software (Perkin Elmer Life Sciences, MA)

This system was used for analysis of liver extracts.

### Chromatographic Conditions

### Method 1

• Column: Phenomenex® Luna 5 µ C18 (2), 4.6 x 250 mm (Phenomenex USA, Torrance, CA)
• Guard Column: Security Guard Cartridge, Polar RP 4 x 2 mm (Phenomenex USA, Torrance, CA)
• Column Temperature: 35°C
• UV Detection: 252 nm
• Radioactivity Detection: 500 µL liquid cell, Scintillant Flow Cocktail (Ultima Flow-AP, Perkin Elmer Life Sciences, CT) @ 4.5 mL/min.

| HPLC Gradient Conditions - Method 1 | | | |
|---|---|---|---|
| Time (min) | %A | %B | Flow (mL/min) |
| 0 | 100 | 0 | 1.5 |
| 4 | 100 | 0 | 1.5 |
| 10 | 85 | 15 | 1.5 |
| 12 | 75 | 25 | 1.5 |
| 17 | 70 | 30 | 1.5 |
| 28 | 25 | 75 | 1.5 |
| 33 | 10 | 90 | 1.5 |
| 34 | 10 | 90 | 1.5 |
| 35 | 100 | 0 | 1.5 |

| | | | |
|---|---|---|---|
| A = 20 mM ammonium formate in water B = 10 mM ammonium formate in methanol-water (90:10) | | | |

This method was used for analyzing predose and postdose formulation aliquots, and for HPLC profiling of urine, feces extracts, and plasma samples.

### Method 2

- Column: Phenomenex® Columbus 5 µ C-18, 4.6 x 250 mm (Phenomenex USA, Torrance, CA)
- Guard Column: Security Guard Cartridge, 4 x 2 mm (Phenomenex USA, Torrance, CA)
- Column Temperature: ambient
- UV Detection: 252 nm
- Radioactivity Detection: 500 µL liquid cell, Scintillant Flow Cocktail (Ultima Flow-AP, Perkin Elmer Life Sciences, CT) @ 3 mL/min.

| HPLC Gradient Conditions - Method 2 | | | |
|---|---|---|---|
| Time (min) | %A | %B | Flow (mL/min) |
| 0 | 95 | 5 | 1.0 |
| 15 | 70 | 30 | 1.0 |
| 20 | 70 | 30 | 1.0 |
| 30 | 50 | 50 | 1.0 |
| 50 | 50 | 50 | 1.0 |

| | | | |
|---|---|---|---|
| A = 25mM potassium phosphate + 5mM tetrabutylammonium dihydrogen phosphate pH 6.3, B = Methanol | | | |

This method was used to analyze the liver extracts.

Figure 4 provides a representative HPLC chromatogram of a mixture of reference standards and Compound 1 (diastereomers 1 and 2) obtained by method 2.

### Sample preparation and analysis

The liver and plasma samples were stored in a -80°C freezer and all other samples were stored in a -20°C freezer.

### Dose formulations for radiochemical purity

Two dose formulations (50 mg/mL for the IV dose Groups 1A-C and 100 mg/mL for the PO dose Groups 2A-C) were prepared in PEG 200 containing 5% DMSO. Subsamples of the dose formulations were removed before and after the dose administration. An aliquot (10 µL) of each subsample was diluted by adding methanol (490 µL) and 20 mM ammonium formate in water (1500 µL). The diluted dose formulations were analyzed for determination of radiochemical purity by HPLC method 1.

### Urine

Pooled urine samples were prepared for Groups 1C and 2C by mixing a fixed percent of the total volume for all four animals from the selected time points. For Group 1C, samples of the 1-, 2-, 6-, and 24-h collection intervals were pooled. For Group 2C, a pool of samples from all four animals was prepared for the 24 h collection intervals only. For 1-, 2-, and 6 h collection intervals, a urine sample of a single animal of Group 2A or 2B was used for analysis. All samples were brought to room temperature and vortexed briefly. The samples were diluted as necessary (2- to 20-fold) with appropriate aqueous mobile phase for the selected HPLC method. The pooled and diluted samples from the 24 h collection interval were centrifuged at 2040 x g for 10 minutes. All other samples were analyzed without centrifugation. HPLC method 1 was used for HPLC profiling. Selected samples were analyzed by HPLC method 2 for comparison with the metabolite profile generated by microsomal and hepatocyte incubations (Examples 14 and 15) and with the metabolite profile of liver extracts.

### Feces

Pooled samples were prepared for the 24 h time interval from Groups 1C and 2C by combining a fixed percentage of the total weight of each individual animal sample homogenate for each group. The aliquots were pooled in a 15-mL polypropylene centrifuge tube. To the pooled sample (approximately 3 g each), 4.5 mL of MeOH-water (70:30) was added. The sample was vortexed for one minute, sonicated for 10 minutes, and then centrifuged at 5700 x g for 10 minutes. The supernatant was transferred to a tube and the total volume of the supernatant was measured. The extraction procedure was repeated two additional times. The radioactivity in each extract was determined by LSC of duplicate aliquots (20 µL each) for extraction recovery. A portion (10% of total volume) of each extract was pooled for analysis by LSC and HPLC. The combined extracts were diluted (1:4) with aqueous mobile phase and analyzed using HPLC method 1 to obtain the metabolic profile. For comparing the *in vitro* with the *in vivo* metabolism data, these same fecal samples were analyzed by HPLC method 2.

### Plasma extraction for HPLC analysis

Pooled terminal rat plasma samples were prepared for Group 1A, Group 1C, and Groups 2A-C. The pools were prepared by combining 1 mL of plasma from each of the four animals. Subsamples (2 mL each) of the pooled samples were transferred to 15-mL conical centrifuge tubes for extraction. To each subsample, 6 mL of methanol were added and each mixture was vortexed vigorously and then centrifuged at 5700 x g for 15 minutes. The supernatant (extract 1) was transferred to a 15-mL tube. The pellets were further extracted with methanol (2 mL). Each sample was vortexed thoroughly to ensure mixing. The samples were centrifuged as described above. The supernatant (extract 2) was removed. The total volume of each extract was recorded. Proportionate subsamples of extracts 1 and 2 of each pooled plasma sample were combined, concentrated in a SpeedVac at ambient temperature, and reconstituted by adding methanol (50 µL) and the aqueous mobile phase of HPLC method 1 to a total volume of 500 µL for each sample. Pooled terminal plasma of Group 1 B was prepared and extracted similarly with the exception of the pooled sample volume (3.8 mL) and extraction volumes (11.4 mL and 4 mL for the first and second extraction, respectively). All extracts and reconstituted extracts were analyzed by LSC. Reconstituted extracts containing sufficient radioactivity were analyzed by HPLC.

### Liver extraction for HPLC analysis

Homogenates were prepared on dry ice in 70% methanol-30% water containing 20mM EDTA and 20mM EGTA (about 1x (w/v) weight of the liver) and were stored frozen. Pooled liver samples were prepared for all groups by combining a fixed percentage of the total weight of each individual animal sample homogenate for each group. Each pooled liver homogenate (3.77-5.02 g) was placed in a 50-mL polypropylene centrifuge tube and 16.0 mL of methanol:water (70:30) was added. The sample was vortexed for one minute, and then centrifuged at 11200 x g for 20 minutes. The supernatant was transferred to a tube and total volume was measured. The extraction procedure was repeated three additional times. Radioactivity of each supernatant was determined by LSC of duplicate aliquots (100 µL each) for extraction recovery. Subsamples of all four extracts from each sample were taken for proportionate pooling. For Group 1, the subsamples were pooled before concentration and, for Group 2, each subsample was concentrated separately and then pooled due to the larger volumes involved. Each extract was completely dried or reduced to approximately 100 µL. Reconstitution was accomplished with methanol (20 µL) and sufficient volume of the aqueous mobile phase of HPLC method 2 such that the total volume of each reconstituted sample was 500 µL. Duplicate aliquots of the reconstituted samples were analyzed by LSC. Each sample was analyzed by HPLC method 2.

### Calculations for µg equivalents/g and percent of the administered dose

Percent of the administered dose (% AD) for parent drug and each metabolite at each collection period = (% of the total radioactivity in the sample attributed to the metabolite) * (total radioactivity expressed as % of the administered dose, recovered during the corresponding collection period).
Correction for extraction recovery was not made for these calculations.

Microgram equivalents/g for parent drug or metabolite at each collection period = (% radioactivity for each metabolite) * (total µg equivalents/g during the corresponding collection period).

### Calculations for PK parameters

The plasma concentration-time data for total radioactivity was analyzed using Microsoft^{®} Office Excel 2003 to obtain the area under the concentration-time curve (AUC). AUC₀₋₂₄ (area under the concentration-time curve from 0 to 24 h after dosing) was calculated using the linear trapezoidal rule and nominal sampling times. For the IV dose group, concentration at time zero was extrapolated from the concentration data for the first two time points. The maximum (peak) plasma concentrations (Cₘₐₓ) and the time to peak concentrations (tₘₐₓ) were the observed values. Descriptive statistics were prepared in Excel.

### Significant figures and rounding of numbers

No general rule was applied for significant figures. Due to the general analytical methodology used in the study, values presented with more significant figures do not imply that these values are more precise. For entry into a spreadsheet program or calculator, instrumentally-obtained values were entered as obtained with the exception of LSC where whole rounded integers were used. Data in tables are shown as rounded numbers.

### Dose analysis and dose administration

### Analysis of dose formulations

Two dose formulations (50 mg/kg for the IV dose Groups 1A-C and 100 mg/kg for the PO dose Groups 2A-C) were prepared by the following procedure. Both formulations were prepared in PEG 200 containing 5% DMSO. The required amount of radioactive material was weighed and ground to a fine powder with a spatula. 30% of the final volume of PEG 200 and the weighed test article was added to a formulation vial while stirring under magnetic agitation to obtain a uniform formulation. The final volume was made up with PEG 200. The formulation was sonicated at room temperature for 15 minutes or longer to obtain homogenous suspension. The cycles of sonication/agitation were continued until the formulation was homogeneous. Triplicate aliquots were removed for determination of homogeneity (CV). The radioactivity concentration of the formulation was determined and specific activity was calculated.

The dose aliquots were analyzed by HPLC method 1 after diluting 10 µL of each formulation with 490 µL of methanol and 1.5 mL of the aqueous mobile phase.

Radiochemical purity of the test article in the dose formulations before and after dosing is shown in the following table:

**Table 41 Radiochemical purity of the test article in dose formulations**

| Dose Group | Radiochemical Purity¹ | |
|---|---|---|
| | Predose | Postdose |
| 1A-C | 99.0% | 99.1% |
| 2A-C | 98.6% | 98.9% |

| | | |
|---|---|---|
| ¹Determined as the total of two isomers. | | |

The data indicated that the dose formulations were stable during the time of dose administration.

### Dose administration

Target dose levels for Groups 1 and 2 were 50 mg/kg (IV) and 100 mg/kg (PO), respectively. Average dose data is summarized in the table:

**Table 42 Average dose administration data**

| Group | µCi/Kg | mg/kg |
|---|---|---|
| 1A | 270 (±4) | 50.3 (±0.7) |
| 1B | 267 (±2) | 49.7 (±0.4) |
| 1C | 269 (±3) | 50.1 (±0.6) |
| 2A | 254 (±4) | 100 (±2) |
| 2B | 254 (±3) | 99.7 (±1.2) |
| 2C | 253 (±2) | 99.5 (±0.6) |

### Pharmacokinetics

### Pharmacokinetics of total radioactivity

Radioanalyses of plasma samples were conducted. The plasma concentration data for total radioactivity expressed as µg equivalents/mL are summarized below:

### Plasma Total Radioactivity Concentrations Following IV Administration of [¹⁴C] Compound 1 to Group 1 Rats at a Target Dose of 50 mg/kg

| Group 1A | Time | | | µg Equivalents/mL | | | |
|---|---|---|---|---|---|---|---|
| | hours | Rat 1 | Rat 2 | Rat 3 | Rat 4 | Mean | SD |
| | 0.25 | 10.883 | 9.006 | 7.913 | 8.978 | 9.195 | 1.235 |
| | 1 | 1.371 | 1.358 | 1.600 | 1.267 | 1.399 | 0.142 |
| | 2 | 2.202 | 1.861 | 2.545 | 2.092 | 2.175 | 0.285 |

| Group 1B | Time | µg Equivalents/mL | | | | | |
|---|---|---|---|---|---|---|---|
| | hours | Rat 5 | Rat 6 | Rat 7 | Rat 8 | Mean | SD |
| | 0 | 0.000 | 0.000 | 0.000 | 0.000 | 0.000 | 0.000 |
| | 0.75 | 1.576 | 1.600 | 1.710 | 2.454 | 1.835 | 0.417 |
| | 4 | 0.755 | 0.713 | 0.938 | 0.790 | 0.799 | 0.098 |
| | 6 | 2.376 | 2.233 | 2.160 | 3.837 | 2.652 | 0.795 |

| Group 1C | Time | µg Equivalents/mL | | | | | |
|---|---|---|---|---|---|---|---|
| | hours | Rat 9 | Rat 10 | Rat 11 | Rat 12 | Mean | SD |
| | 0.083 | 50.653 | 45.178 | 35.246 | 37.600 | 43.692 | 7.067 |
| | 0.5 | 4.824 | 4.036 | 4.072 | 3.857 | 4.311 | 0.428 |
| | 10 | 0.566 | 0.675 | 0.573 | 0.562 | 0.605 | 0.054 |
| | 24 | 0.515 | 0.759 | 0.553 | 0.453 | 0.570 | 0.133 |

### Plasma Total Radioactivity Concentrations Following PO Administration of [¹⁴C] Compound 1 to Group 2 Rats at a Target Dose of 100 mg/kg

| Group 2A | Time | µg Equivalents/mL | | | | | |
|---|---|---|---|---|---|---|---|
| | hours | Rat 13 | Rat 14 | Rat 15 | Rat 16 | Mean | SD |
| | 0.25 | 0.216 | 0.433 | 0.474 | 0.260 | 0.346 | 0.127 |
| | 1 | 0.305 | 0.439 | 0.470 | 0.401 | 0.404 | 0.072 |
| | 2 | 0.634 | 0.877 | 1.086 | 0.879 | 0.869 | 0.185 |

| Group 2B | Time | µg Equivalents/mL. | | | | | |
|---|---|---|---|---|---|---|---|
| | hours | Rat 17 | Rat 18 | Rat 19 | Rat 20 | Mean | SD |
| | 0 | 0.000 | 0.000 | 0.000 | 0.000 | 0.000 | 0.000 |
| | 0.75 | 0.346 | 0.327 | 0.331 | 0.256 | 0.315 | 0.040 |
| | 4 | 0.578 | 0.618 | 0.675 | 0.630 | 0.625 | 0.040 |
| | 6 | 1.157 | 1.163 | 1.522 | 1.480 | 1.331 | 0.198 |

| Group 2C | Time | µg Equivalents/mL | | | | | |
|---|---|---|---|---|---|---|---|
| | hours | Rat 21 | Rat 22 | Rat 23 | Rat 24 | Mean | SD |
| | 0.083 | 0.228 | 0.394 | 0.228 | 0.596 | 0.283 | 0.175 |
| | 0.5 | 0.236 | 0.335 | 0.378 | 0.408 | 0.316 | 0.075 |
| | 10 | 0.806 | 0.555 | 0.506 | 0.511 | 0.622 | 0.143 |
| | 24 | 0.450 | 0.303 | 0.458 | 0.384 | 0.399 | 0.072 |

### Mean (SD) Plasma Total Radioactivity Concentrations Following IV Administration of [¹⁴C] Compound 1 to Group 1 Rats at a Target Dose of 50 mg/kg and PO Dosing in Group 2 Rats at a Target Dose of 100 mg/kg

| Plasma Concentration | | | | |
|---|---|---|---|---|
| (µg Equivalents/mL) | | | | |
| Time | IV | | PO | |
| hours | Mean | SD | Mean | SD |
| 0 | 0.000 | 0.000 | 0.000 | 0.000 |
| 0.0833 | 43.692 | 7.067 | 0.283 | 0.175 |
| 0.25 | 9.195 | 1.235 | 0.346 | 0.127 |
| 0.5 | 4.311 | 0.428 | 0.316 | 0.075 |
| 0.75 | 1.835 | 0.417 | 0.315 | 0.040 |
| 1 | 1.399 | 0.142 | 0.404 | 0.072 |
| 2 | 2.175 | 0.285 | 0.869 | 0.185 |
| 4 | 0.799 | 0.098 | 0.625 | 0.040 |
| 6 | 2.652 | 0.795 | 1.331 | 0.198 |
| 10 | 0.605 | 0.054 | 0.622 | 0.143 |
| 24 | 0.570 | 0.133 | 0.399 | 0.072 |
| AUC₀₋₂₄ | | | | |
| (µg equivalents·h/mL) | 36.000 | | 15.445 | |

For the IV dose group, the concentration of total radioactivity declined rapidly up to 1 h and then increased again at 2 h. After declining at 4 h, a secondary increase in concentration was observed at 6 h. Similarly, elevated concentrations were observed at 2 and 6 h for the PO dose groups. This pattern is indicative of enterohepatic recirculation and reabsorption of radioactivity.

AUC values were calculated from the average concentration data for each dose group. The concentration at time zero (C₀) was calculated from the concentrations at the 0.08- and 0.25-h time points by extrapolation. The AUC₀₋₂₄ values were 36.0 and 15.4 hr·µg equivalent/mL for the IV and PO dose groups, respectively. Dose normalized absorption of total radioactivity was approximately 22%.

The plasma concentrations of Compound **1** and 2'-C-methyl guanosine is determined by LC-MS.

### Total radioactivity in liver

Liver samples were collected at 2, 6 and 24 h postdose from the animals of both the IV and the PO dose groups. The samples were flash frozen after excision and kept on dry ice during homogenization. The homogenization was conducted on dry ice in 70% methanol-30% water containing 20mM EDTA and 20mM EGTA (about 1x (w/v) weight of the liver). Triplicate weighed aliquots (about 500 mg) were combusted in the Packard Sample Oxidizer and followed by LSC analysis to determine radioactivity 9100 µL Spec-Chec®). The average data for levels of total radioactivity in liver are summarized below.

**Table 43**

| Group | Total radioactivity as % AD¹ | Concentration of total radioactivity as µg equivalents/gram |
|---|---|---|
| 1A | 15.5 ± 0.7 | 206 ± 24 |
| 1B | 10.1 ± 1.0 | 137 ± 12 |
| 1C | 1.76 ± 0.11 | 23.2 ± 3.1 |
| 2A | 0.20 ± 0.04 | 5.09 ± 0.78 |
| 2B | 0.37 ± 0.05 | 10.3 ± 1.2 |
| 2C | 0.17 ± 0.03 | 4.08 ± 1.10 |
| ¹AD = Administered Dose | | |

The data for the IV dose group suggests that hepatic extraction of the drug occurred very early (within 2 h of dose) and was very efficient.

### Excretion of administered dose

The urine and feces of each animal were analyzed for radiolabel content. The data as a sum of urinary and fecal excretion are summarized below:

**Table 44**

| Dose Level (mg/kg) | Termination Time (h) | Urinary + Fecal Excretion (%.AD)¹ |
|---|---|---|
| 1A | 2 | 26.0 ± 2.3 |
| 1B | 6 | 27.8 ± 4.9 |
| 1C | 24 | 86.0 ± 4.8 |
| 2A | 2 | 0.05 ± 0.06 |
| 2B | 6 | 0.36 ± 0.18 |
| 2C | 24 | 94.1±8.9 |

| | | |
|---|---|---|
| ¹ Average of data from four animals and a sum of all collection time points | | |

The data of urinary excretion for all dose groups are summarized in the following table:

**Table 45**

| Dose Level (mg/kg) | Termination Time (h) | Urinary Excretion (%AD)¹ |
|---|---|---|
| 1A | 2 | 25.9 ± 2.4 |
| 1B | 6 | 27.7 ± 4.9 |
| 1C | 24 | 46.9 ± 4.1 |
| 2A | 2 | 0.05 ± 0.06 |
| 2B | 6 | 0.36 ± 0.18 |
| 2C | 24 | 2.84 ± 0.82 |

| | | |
|---|---|---|
| ¹Average of data from four animals and a sum of all collection time points | | |

Low urinary excretion of radioactivity in the oral dose group indicated low absorption and this observation is consistent with the pharmacokinetic data.

The data of fecal excretion are summarized below:

**Table 46**

| Dose Level (mg/kg) | Termination Time (h) | Fecal Excretion (%AD) ¹ |
|---|---|---|
| 1A | 2 | 0.05 ± 0.08 |
| 1B | 6 | 0.13 ± 012 |
| 1C | 24 | 39.0 ± 4.0 |
| 2A | 2 | 0.001 ± 0.001 |
| 2B | 6 | 0.006 ± 0.005 |
| 2C | 24 | 91.3 ± 8.7 |

| | | |
|---|---|---|
| ¹ Average of data from four animals and a sum of all collection time points | | |

The data for Group 1C suggested extensive biliary secretion, as 39% of the IV dose was recovered in feces. For the oral dose group, fecal excretion was the primary route of elimination. Approximately 91 % of the total administered radioactivity was eliminated via feces. The amount of the orally administered dose recovered in the urine and feces cannot be used to estimate the extent of absorption since biliary secretion represents a significant route of elimination.

### Quantitation and distribution of metabolites

Urine, feces, plasma and liver extracts were prepared for HPLC analysis with radiochemical detection as described above. Solvent extraction recoveries for the feces, liver and plasma extracts are presented in Table 47. Retention times of reference standards analyzed by HPLC method 1 and method 2 are given in Table 48.

**Table 47**

| Sample | Extraction Recovery |
|---|---|
| Feces (Group 1 C, 24 h) | 109.3% |
| Feces (Group 2C, 24 h) | 96.7% |
| Liver (Group 1A, 2 h) | 96.5% |
| Liver (Group 1B, 6 h) | 103.7% |
| Liver (Group 1C, 24 h) | 82.1% |
| Liver (Group 2A, 2 h) | 83.3% |
| Liver (Group 2B, 6 h) | 93.1% |
| Liver (Group 2C, 24 h) | 71.4% |
| Terminal plasma (Group 1A, 2 h) | 86.6% |
| Terminal plasma (Group 1B, 6 h) | 77.8% |
| Terminal plasma (Group 1C, 24 h) | 87.5% |
| Terminal plasma (Group 2A, 2 h) | 52.5% |
| Terminal plasma (Group 2B, 6 h) | 59.1% |
| Terminal plasma (Group 2C, 24 h) | 34.9% |

**Table 48: Typical HPLC retention times (minutes) of reference standards**

| Reference Standards | HPLC Method 1 | HPLC Method 2 |
|---|---|---|
| Compound **1** | 28.6 and 28.3 | 45.2 and 42.3 |
| Compound **3** (diastereomer 1 and diastereomer 2) | 22.7 and 21.6 | 24.3 and 27.2 |
| Compound **2** | 20.4 | 31.6 |
| Compound **4** | 19.5 | 30.1 |
| 2'-C-methyl guanosine | 11.3. | 9.4 |
| Compound **7** | na¹ | 25.9 |
| Compound **6** | na | 22.6 |
| Compound **5** | na | 16.2 |

| | | |
|---|---|---|
| ¹na = not applicable | | |

### Urinary metabolites

HPLC analyses of pooled urine samples of all time points were conducted for Group 1C. The data, expressed as percentage of HPLC peak area distribution and as percentage of administered dose, are summarized in Table 49. Metabolites R1, R2, R3, R4, R5, R6, R7 and R8 were characterized based on retention time.

**Table 49: Metabolite profile: group 1C (IV Dose) urine**

| | | | | | | | | | | | |
|---|---|---|---|---|---|---|---|---|---|---|---|
| Collection Time | | 1 h¹ | | 2 h¹ | | 6 h¹ | | 24 h¹ | | Total | |
| **Percent AD (Mean)** | | **22.9** | | **5.11** | | **2.33** | | **16.6** | | **46.9** | |

| Compound | Retention Time (Minutes) | % HPLC ² | % AD | % HPLC | % AD | % HPLC | % AD | % HPLC | % AD | % AD | % of total urinary excretion |
|---|---|---|---|---|---|---|---|---|---|---|---|
| Compd 1 | 28.6 and 28.3 | 93.5 | 21.39 | 48.9 | 2.50 | 30.5 | 0.71 | 9.09 | 1.51 | **26.1** | **55.6** |
| U3³ | 26.5 | nd⁴ | nd | 0.24 | 0.01 | nd | nd | nd | nd | **0.01** | **0.03** |
| U1³ | 25.2 | nd | nd | 0.18 | 0.01 | nd | nd | nd | nd | **0.01** | **0.02** |
| Compd 3 Diastereo mer 1 | 22.7 | 0.24 | 0.05 | 1.01 | 0.05 | 0.37 | 0.01 | nd | nd | **0.12** | **0.25** |
| Compd 3 Diastereo mer 2 | 21.6 | 1.00 | 0.23 | 2.69 | 0.14 | 1.19 | 0.03 | nd | nd | **0.39** | **0.84** |
| Compd 2 | 20.4 | 2.29 | 0.52 | 2.57 | 0.13 | 1.40 | 0.03 | 0.45 | 0.07 | **0.76** | **1.62** |
| R8 | 17.1 | nd | nd | 0.95 | 0.05 | 0.52 | 0.01 | nd | nd | **0.06** | **0.13** |
| R7 | 13.1 | nd | nd | 0.59 | 0.03 | 0.68 | 0.02 | 0.63 | 0.10 | **0.15** | **0.32** |
| R6 | 12.4 | nd | nd | 1.01 | 0.05 | 0.90 | 0.02 | nd | nd | **0.07** | **0.15** |
| 2'-C-methyl guanosine | 11.3 | 2.12 | 0.48 | 35.1 | 1.79 | 57.7 | 1.35 | 86.7 | 14.4 | **18.0** | **38.4** |
| R5 | 10.9 | nd | nd | nd | nd | nd | nd | nd | nd | **nd** | **nd** |
| R4 | 9.4 | nd | nd | 1.03 | 0.05 | 0.91 | 0.02 | nd | nd | **0.07** | **0.16** |
| R3 | 8.0 | nd | nd | nd | nd | nd | nd | nd | nd | **nd** | **Nd** |
| R2 | 6.2 | nd | nd | 0.58 | 0.03 | 0.94 | 0.02 | nd | nd | **0.05** | **0.11** |
| R1 | 2.1 | 0.83 | 0.19 | 5.12 | 0.26 | 4.90 | 0.11 | 3.09 | 0.51 | **1.08** | **2.30** |
| **Total** | | **100** | **22.9** | **100** | **5.11** | **100** | **2.33** | **100** | **16.6** | **46.9** | **100** |

| | | | | | | | | | | | |
|---|---|---|---|---|---|---|---|---|---|---|---|
| ¹Pool of urine from all animals of Group1C was prepared by proportional mixing by volume. ²Percent HPLC = Abundance of each metabolite expressed as percent of HPLC peak area in the sample extract. ³*In vitro* metabolite observed in Example 15 ⁴nd = not detected | | | | | | | | | | | |

All urine samples were also analyzed by HPLC method 2. This method was used for *in vitro* metabolism experiments decribed above and for the analysis of liver extracts. This method also provided additional evidence for identification of metabolites by retention time comparison with reference standards.

For the PO dose group, a pooled urine sample from the 24-h collection time was analyzed. For 1-, 2-, and 6-h collection times, the majority of the samples had been used for radioanalysis. The remaining samples were insufficient for proportional pooling. Therefore, single animal samples of 1A or 1B groups were used for HPLC profiling. The data, as percentage of HPLC distribution and as percentage of administered dose, are summarized in Table 50. Metabolites R1, R2, R3, R4, R5, R6, R7 and R8 were characterized based on retention time.

**Table 50: Metabolite profile: group 2C (PO Dose) urine**

| | | | | | | | | | |
|---|---|---|---|---|---|---|---|---|---|
| Collection Time | | 1 h¹ | | 2 h¹ | | 6 h¹ | | 24 h² | |
| **Percent AD** | | **0.08** | | **0.10** | | **0.28** | | **2.32** | |

| Compound | Typical Retention Time (Min) | % HPLC³ | % AD | % HPLC | % AD | % HPLC | % AD | % HpLC | % AD |
|---|---|---|---|---|---|---|---|---|---|
| Compound **1** | 28.6 and 28.3 | 74.8 | 0.06 | 6.57 | 0.01 | nd | nd | 0.30 | 0.01 |
| U3⁴ | 26.5 | nd⁵ | nd | 4.31 | 0.004 | nd | nd | nd | nd |
| U1⁴ | 25.2 | nd | nd | nd | nd | nd | nd | nd | nd |
| Compound **3** (diastereomer 1) | 22.7 | nd | nd | nd | nd | nd | nd | nd | nd |
| Compound **3** (diastereomer 2) | 21.6 | nd | nd | nd | nd | nd | nd | nd | nd |
| Compound **2** | 20.4 | nd | nd | 1.81 | 0.002 | nd | nd | nd | nd |
| R8 | 17.1 | nd | nd | nd | nd | nd | nd | nd | nd |
| R7 | 13.1 | nd | nd | 8.39 | 0.01 | 1.33 | 0.004 | 0.85 | 0.02 |
| R6 | 12 4 | 4.39 | 0.004 | nd | nd | nd | nd | nd | nd |
| 2'-C-methyl guanosine | 11 3 | 5.33 | 0.004 | 56.2 | 0.06 | 86.18 | 0.24 | 92.1 | 2.14 |
| R5 | 10.9 | nd | nd | nd | nd | 3.01 | 0.001 | 2.85 | 0.07 |
| R4 | 9.4 | nd | nd | nd | nd | nd | nd | nd | nd |
| R3 | 8.0 | nd | nd | 2.95 | 0.003 | 1.51 | 0.004 | 0.46 | 0.01 |
| R2 | 6.2 | nd | nd | nd | nd | nd | nd | nd | nd |
| R1 | 2.1 | 15.5 | 0.01 | 19.7 | 0.02 | 7.97 | 0.02 | 3.41 | 0.08 |
| **Total** | | **100** | **0.08** | **100** | **0.10** | **100** | **0.28** | **100** | **2.32** |

| | | | | | | | | | |
|---|---|---|---|---|---|---|---|---|---|
| ¹Sample of a single animal (#15, Group 2A for 1 h and #20 Group 2B for 2 h and 6 h). ²Pool of urine from all animals of Group2C was prepared by proportional mixing by volume. ³% HPLC = Abundance of each metabolite expressed as % of HPLC peak area in the sample extract. ⁴*In vitro* metabolite observed in Example 15 ⁵nd = not detected | | | | | | | | | |

Table 50 contains data from single animal samples and pooled samples. To estimate overall distribution of urinary metabolites, it was assumed that the profile from a single animal is reflective of the overall average metabolite profile. Therefore, HPLC distribution data from single animals was used to calculate the percentage of administered dose excreted as various metabolites during the 1-, 2-, and 6-h collection intervals. The estimated data is summarized in Table 51. Metabolites R1, R2, R3, R4, R5, R6, R7 and R8 were characterized based on retention time.

**Table 51: Estimated metabolite profile: group 2C (PO Dose) urine**

| | | | | | | | | | | | |
|---|---|---|---|---|---|---|---|---|---|---|---|
| Collection Time | | 1 h | | 2 h | | 6 h | | 24 h | | Total | |
| **% AD (Mean)** | | **0.02** | | **0.04** | | **0.46** | | **2.32** | | **2.84** | |

| Metabolite -on Code | Typical Retenti Time (Min) | % HPLC ¹ | % AD | % HPL C | % AD | % HPL C | % AD | % HPL C | % AD | | **% of total urinary excretio n** |
|---|---|---|---|---|---|---|---|---|---|---|---|
| Compound **1** | 28.6 and 28.3 | 74.8 | 0.01 | 6.57 | 0.003 | nd | nd | 030 | 0.01 | **0.02** | **0.73** |
| U3³ | 26.5 | Nd³ | nd | 4.31 | 0.002 | nd | nd | nd | nd | **0.002** | **0.06** |
| U1³ | 25.2 | nd | nd | nd | nd | nd | nd | nd | nd | nd | nd |
| Compound **3** (diastereomer 1) | 22.7 | nd | nd | nd | nd | nd | nd | nd | nd | nd | nd |
| Compound **3** (diastereomer 2) | 21.6 | nd | nd | nd | nd | nd | nd | nd | nd | nd | nd |
| Compound **2** | 20.4 | nd | nd | 1.81 | 0.001 | nd | nd | nd | nd | **0.001** | **0.03** |
| R8 | 17.1 | nd | nd | nd | nd | nd | nd | nd | nd | nd | nd |
| R7 | 13.1 | nd | nd | 8.39 | 0.003 | 1.33 | 0.01 | 0.85 | 0.02 | **0.02** | **0.81** |
| R6 | 12.4 | 4.39 | 0.001 | nd | nd | nd | nd | nd | nd | **0.001** | **0.02** |
| 2'-C-methyl guanosine | 11.3 | 5.33 | 0.001 | 56.2 | 0.02 | 86.18 | 0.40 | 92.1 | 2.14 | **2.56** | **90.4** |
| R5 | 10.9 | nd | nd | nd | nd | 3.01 | 0.01 | 285 | 0.07 | **0.08** | 2.83 |
| R4 | 9.4 | nd | nd | nd | nd | nd | nd | nd | nd | nd | nd |
| R3 | 8.0 | nd | nd | 2.95 | 0.001 | 1.51 | 0.01 | 0.46 | 0.01 | **0.02** | **0.67** |
| R2 | 6.2 | nd | nd | nd | nd | nd | nd | nd | nd | nd | nd |
| R1 | 2.1 | 15.5 | 0.002 | 19.7 | 0.01 | 7.97 | 0.04 | 3.41 | 0.08 | **0.13** | **4.46** |
| Total | | **100** | **0.02** | **100** | **0.04** | **100** | **0.460** | **100** | **2.32** | **2.84** | **100** |

| | | | | | | | | | | | |
|---|---|---|---|---|---|---|---|---|---|---|---|
| ¹% HPLC = Abundance of each metabolite expressed as % of HPLC peak area in the sample extract. The average metabolite profile data for 1 h, 2 h, and 6h was estimated using single animal % HPLC distribution from Table 9-4. In the estimation it is assumed that profile from single animal is reflective of the average metabolite profile. ³In *vitro* metabolite observed in example 15 ⁴nd = not detected | | | | | | | | | | | |

Two urine samples (6-h sample of animal number 19 and pooled urine from the 24-h collection) were analyzed by HPLC method 2.

Data from HPLC analyses using method 1 were used for calculating the distribution of metabolites. Compound **1**, was the major component in urine of the IV dose group. It accounted for 26.1% of the administered dose and 55.6% of the total urinary excretion. 2'-C-methyl guanosine was the major metabolite (18.0% AD, 38.4% of the total urinary excretion). One polar metabolite (designated as R1) represented 1.0% AD and 2.3% of the total urinary excretion. All other metabolites were present at less than 0.2% AD.

For the PO dose group, 2'-C-methyl guanosine was the major urinary component present at an estimated level of 2.6% AD and accounted for 90.4% of the total urinary excretion (Table 51). The polar metabolite, R1, was detected at 0.1% AD and accounted for 4.5% of the total urinary excretion. Compound **1** accounted for only 0.02% AD. It was the most abundant component only at the 1-h time point, accounting for 74.8% of the total radioactivity eliminated via urine within one hour. By 6 h, it accounted for only 6.6% of the total urinary excretion.

Compound 2 and Compound 3 were present at very low levels and were identified on the basis of comparable retention times with appropriate reference standards using both HPLC methods 1 and 2.

### Fecal metabolites

Pooled fecal samples were extracted and prepared for HPLC analysis as described above. Fecal samples of only the 24 h collections were analyzed. Due to low total radioactivity and sample size, 6 h collections were not analyzed.

The samples were analyzed by HPLC with radiochemical detection. The distribution of metabolites as % of the administered dose was derived from these analyses. Data for HPLC analysis of pooled fecal extracts of both IV and PO dose groups are given in Table 52. Both pooled fecal extracts were also analyzed by HPLC method 2. Metabolites R1, R2, R3, R4, R5, R6, R7 and R8 were characterized based on retention time.

**Table 52: Metabolite profile: groups 1C (IV) and 2C (PO Dose) fecal extracts**

| | | | | | |
|---|---|---|---|---|---|
| Dose Group | | 1C (IV) | | 2C (PO) | |
| Collection Time | | 24 h¹ | | 24 h¹ | |
| **% AD (Mean)** | | **39.0** | | **91.2** | |

| Metabolite Code | Typical Retention Time (Minutes) | % HPLC² | % AD | % HPLC | % AD |
|---|---|---|---|---|---|
| Compound 1 | 28.6 and 28.3 | 50.6 | 19.7 | 71.9 | 65.6 |
| U3³ | 26.5 | 4.97 | 1.90 | nd⁴ | nd |
| U1³ | 25.2 | 3.37 | 1.31 | nd | nd |
| Compound 3 (diastereomer 1) | 22.7 | nd | nd | nd | nd |
| Compound 3 (diastereomer 2) | 21.6 | nd | nd | nd | nd |
| Compound 2 | 20.4 | nd | nd | nd | nd |
| R8 | 17.1 | nd | nd | nd | nd |
| R7 | 13.1 | nd | nd | nd | nd |
| R6 | 12.4 | nd | nd | nd | nd |
| 2'-C-methyl guanosine | 11.3 | 41.0 | 16.0 | 28.1 | 25.6 |
| R5 | 10.9 | nd | nd | nd | nd |
| R4 | 9.4 | nd | nd | nd | nd |
| R3 | 8.0 | nd | nd | nd | nd |
| R2 | 6.2 | nd | nd | nd | nd |
| R1 | 2.1 | nd | nd | nd | nd |
| Total | | 100 | 39.0 | 100 | 91.2 |

| | | | | | |
|---|---|---|---|---|---|
| ¹ Pool of feces from all animals were prepared by proportional mixing by weight. ²% HPLC = Abundance of each metabolite expressed as % of HPLC peak area in the sample extract. *³In* vitro metabolites observed in example 14 ⁴nd = not detected | | | | | |

Data from HPLC analyses using method 1 were used for calculating distribution of metabolites. Compound **1** was the most abundant component of feces for both dose groups. It accounted for 19.7% and 65.6% AD (50.6% and 71.9% of the total fecal excretion) for the IV and PO dose groups, respectively (Table 52). 2'-C-methyl guanosine was the major metabolite for the IV dose group, accounting for 16.0% AD (41.0% of the total fecal excretion). For the PO dose group, 2'-C-methyl guanosine was the only metabolite (25.6% AD, 28.1% of the total fecal excretion). For the IV dose group, two minor metabolites, designated as R11 and R10, were detected at 1.9 and 1.3% AD (5.0% and 3.4% of the total fecal excretion), respectively. Metabolites R11 and R12 were chromatographically identical to metabolites U3 and U1, respectively generated by microsomal and hepatocyte incubations in Examples 14 and 15.

### Total excretion of Compound 1 and its major metabolite 2'-C-methyl guanosine

The total excretion of Compound **1** and its major metabolite 2'-C-methyl guanosine was calculated as a sum of the total urinary and total fecal excretion of each component. For Group C, the estimated data was used in calculating urinary recovery. The data are presented in Table 53. Overall excretion of the Compound 1 was 45.9% and 65.6% of the administered dose for the IV and PO dose groups, respectively. Excretion of 2'-C-methyl guanosine accounted for 34.0% and 28.2% of the respective doses.

**Table 53: Total excretion (% AD) of Compound 1 and 2'-C-methyl guanosine**

| Dose Group | Compound | Urine | Feces | Total |
|---|---|---|---|---|
| IV (1C) | Compound **1** | 26.1 | 19.7 | 45.9 |
| | 2'-C-methyl guanosine | 18.0 | 16.0 | 34.0 |
| PO (2C) | Compound **1** | 0.02 | 65.6 | 65.6 |
| | 2'-C-methyl guanosine | 2.56 | 25.6 | 28.2 |

| | | | | |
|---|---|---|---|---|
| Concentration data for Group 2C urine is estimated. | | | | |

### Plasma metabolites

Terminal plasma samples were pooled, extracted and prepared for HPLC analysis as described above. For Group 1, terminal plasma samples (2, 6, and 24 h) were pooled and analyzed by HPLC as described above. For Group 2, only the 6-h sample had sufficient radioactivity for analysis. The only detectable metabolite observed in all plasma samples was 2'-C-methyl guanosine. The 2-h plasma showed a polar peak eluting at 2.1 minutes which could be 2'-C-methyl guanosine. It was observed that 2'-C-methyl guanosine interacted with matrix components which caused partial elution of the metabolite at the solvent front (approximately 2 minutes). When the sample was reanalyzed after dilution with the aqueous mobile phase this component disappeared.

### Liver metabolic profile

Liver homogenates were pooled for each time point for both dose groups by proportional mixing according to the weight of the samples from individual animals. The pooled samples were extracted and prepared for HPLC analysis as described above. The abundance of metabolites was determined as a % of the administered dose and as µg equivalent/g. The data are presented in Table 54, Table 55, Table 56 and Table 57, respectively. Metabolites R1, R8 and R9 were characterized based on retention time.

**Table 54: Metabolite profile (expressed as % AD): group 1 (IV Dose) liver**

| | | | | | | | |
|---|---|---|---|---|---|---|---|
| Collection Time | | 2 h | | 6 h | | 24 h | |
| **% AD (Mean)** | | **15.5** | | **10.1** | | **1.76** | |

| Metabolite Code | Retention Time (Minutes) | % HPLC¹ | % AD | % HPLC | % AD | % HPLC | % AD |
|---|---|---|---|---|---|---|---|
| R1 | 4.1 | 0.65 | 0.10 | nd² | nd | nd | nd |
| 2'-C-methyl guanosine | 9.6 | 13_{.}8 | 2.14 | 14.6 | 1.48 | 23.9 | 0.42 |
| Compound **5** | 15.0 | 62.6 | 9.73 | 63.1 | 6.39 | 59.9 | 1.05 |
| R9 | 16.4 | 0.70 | 0.11 | 0.89 | 0.09 | nd | nd |
| R8 | 17.2 | 0.40 | 0.06 | 0.92 | 0.09 | nd | nd |
| Compound **6** | 22.5 | 21.6 | 3.35 | 20.1 | 2.04 | 16.2 | 0.29 |
| Compound **7** | 26.0 | 0.29 | 0.05 | 0.35 | 0.04 | nd | nd |
| **Total** | | **100** | **15.5** | **100** | **10.1** | **100** | **1.76** |

| | | | | | | | |
|---|---|---|---|---|---|---|---|
| ¹% HPLC = Abundance of each metabolite expressed as % of HPLC peak area in the sample extract. ²nd = not detected | | | | | | | |

**Table 55: Metabolite profile (expressed as % AD): group 2 (PO Dose) liver**

| | | | | | | | |
|---|---|---|---|---|---|---|---|
| Collection Time | | 2 h | | 6 h | | 24 h | |
| **% AD (Mean)** | | **0.20** | | **0.37** | | **0.17** | |

| Metabolite Code | Retention Time (Minutes) | % HPLC¹ | % AD | % HPLC | % AD | % HPLC | % AD |
|---|---|---|---|---|---|---|---|
| R1 | 4.1 | nd² | nd | nd | nd | nd | nd |
| 2'-C-methyl guanosine | 9.6 | 77.5 | 0.15 | 85.0 | 0.32 | 53.9 | 0.09 |
| Compound **5** | 15.0 | nd | nd | nd | nd | nd | nd |
| R9 | 16.4 | nd | nd | nd | nd | nd | nd |
| R8 | 17.2 | nd | nd | nd | nd | nd | nd |
| Compound **6** | 22.5 | 22.6 | 0.04 | 15.0 | 0.06 | 46.1 | 0.08 |
| Compound **7** | 26.0 | nd | nd | nd | nd | nd | nd |
| **Total** | | **100** | **0.20** | **100** | **0.37** | **100** | **0.17** |

| | | | | | | | |
|---|---|---|---|---|---|---|---|
| '% HPLC = Abundance of each metabolite expressed as % of HPLC peak area in the sample extract. ²nd = not detected | | | | | | | |

**Table 56: Metabolite profile: group 1 (IV Dose) liver**

| | | | | | | | |
|---|---|---|---|---|---|---|---|
| Collection Time | | 2 h | | 6 h | | 24 h | |
| Mean concentration (µg equivalents/g) | | **206** | | **137** | | **23.2** | |

| Metabolite Code | Retention time (Minutes) | % HPLC¹ | as µg equivalents/g | % HPLC | as µg equivalents/g | % HPLC | as µg equivalents/g |
|---|---|---|---|---|---|---|---|
| R1 | 4.1 | 0.65 | 1.34 | nd² | nd | nd | nd |
| 2'-C-methyl guanosine | 9.6 | 13.8 | 28.4 | 14.6 | 20.0 | 23.9 | 5.56 |
| Compound **5** | 15.0 | 62.6 | 129 | 63.1 | 86.2 | 59.9 | 13.9 |
| R9 | 16.4 | 0.70 | 1.44 | 0.89 | 1.22 | nd | nd |
| R8 | 17.2 | 0.40 | 0.82 | 0.92 | 1.26 | nd | nd |
| Compound **6** | 22.5 | 21.6 | 44.4 | 20.1 | 27.5 | 16.2 | 3.77 |
| Compound **7** | 26.0 | 0.29 | 0.60 | 0.35 | 0.48 | nd | nd |
| **Total** | | **100** | **206** | **100** | **137** | **100** | **23.2** |

| | | | | | | | |
|---|---|---|---|---|---|---|---|
| ¹% HPLC = Abundance of each metabolite expressed as % of HPLC peak area in the sample extract. ²nd = not detected | | | | | | | |

**Table 57: Metabolite profile: group 2 (PO Dose) liver**

| | | | | | | | |
|---|---|---|---|---|---|---|---|
| Collection Time | | 2 h | | 6 h | | 24 h | |
| Mean concentration (µg equivalents/g) | | **5.09** | | **10.3** | | **4.08** | |

| Metabolite Code | Retention Time (Minutes) | % HPLC¹ | as µg equivalents/g | % HPLC | as µg equivalents/g | % HPLC | as µg equivalents/ g |
|---|---|---|---|---|---|---|---|
| R1 | 4.1 | nd² | nd | nd | nd | nd | nd |
| 2'-C-methyl guanosine | 9.6 | 77.5 | 3.94 | 85.0 | 8.76 | 53.9 | 2.20 |
| Compound **5** | 15.0 | nd | nd | nd | nd | nd | nd |
| R9 | 16.4 | nd | nd | nd | nd | nd | nd |
| R8 | 17.2 | nd | nd | nd | nd | nd | nd |
| Compound **6** | 22.5 | 22.6 | 1.15 | 15.0 | 1.55 | 46.1 | 1.88 |
| Compound **7** | 26.0 | nd | nd | nd | nd | nd | nd |
| **Total** | | **100** | **5.09** | **100** | **10.3** | **100** | **4.08** |

| | | | | | | | |
|---|---|---|---|---|---|---|---|
| ¹% HPLC = Abundance of each metabolite expressed as % of HPLC peak area in the sample extract. ²nd = not detected | | | | | | | |

By the HPLC radiochemical detection method, 2'-C-methyl guanosine-5'-TP was observed at low levels only in two liver extracts of the IV dose groups (2-h sample from Group 1 A and 6-h sample from Group 1B). It accounted for 0.05% AD in the Group 1A liver and for 0.04% in the Group 1B liver (Table 54). The concentration of Compound 7 was 0.60 µg equivalent of Compound 1/g of liver for Group 1A and 0.48 µg equivalent of Compound 1/g of liver for Group 1B (Table 56).

The major metabolite in the liver extracts of the IV dose group was Compound 5 accounting for 9.7%, 6.4%, and 1.1% of dose at 2, 6, and 24 h. These values represented 60-63% of the total radioactivity in the samples (Table 54). Compound **5** was not detected by the HPLC method in any liver from the oral dose group. Compound **6** was present at 3.4%, 2.0%, and 0.29% of the dose in the IV group samples. These levels represented approximately 16-22% of the total radioactivity in liver (Table 54). For the samples of the oral dose group, Compound **6** was observed at 0.04%, 0.06%, and 0.08% of the administered dose at 2, 6, and 24 h, respectively, representing 15-46% of the total radioactivity in liver (Table 55).

2'-C-methyl guanosine accounted for approximately 14-24% of the total radioactivity in liver for the IV dose group and approximately 54-85% of the total radioactivity in liver for the PO dose group. Three minor unidentified metabolites were detected in the liver samples of the IV dose group.

Preliminary investigation of the extracts revealed the presence of all three phosphorylated compounds in the samples of the oral dose group. Due to their low concentrations and the low specific activity of the dosed [¹⁴C]Compound 1, the monophosphate and the triphosphate were not detected in these samples by radioHPLC analysis.

A comparative metabolite profile for urine, feces and liver after an exposure period of 24h is shown in Table 58. Metabolites R1, R2, R3, R4, R5, R6, R7 and R9 were characterized based on retention time.

**Table 58: Comparison of metabolite profile in rat urine, feces and liver at 24 h after administration of [¹⁴C]Compound 1**

| | Percent of Dose | | | | | | | |
|---|---|---|---|---|---|---|---|---|
| | Urine 0-24 h | | Feces 0-24 h | | Liver 24 h | | Total | |
| Compound Code | Oral | IV | Oral | IV | Oral | IV | Oral | IV |
| Compound **1** | 0.02 | 26.1 | 65.6 | 19.7 | nd² | nd | 65.6 | 45.9 |
| U3¹ | 0.002 | 0.01 | nd | 1.94 | nd | nd | 0.002 | 1.95 |
| U1¹ | nd | 0.01 | nd | 1.31 | nd | nd | nd | 1.32 |
| Compound **3** | nd | 0.12 | nd | nd | nd | nd | nd | 0.12 |
| (diastereomer 1) | | | | | | | | |
| Compound **3** (diastereomer 2) | nd | 0.39 | nd | nd | nd | nd | nd | 0.39 |
| Compound **2** | 0.001 | 0.76 | nd | nd | nd | nd | 0.001 | 0.76 |
| U8¹ | nd | 0.06 | nd | nd | nd | nd | nd | 0.06 |
| R9 | nd | nd | nd | nd | nd | nd | nd | nd |
| R7 | 0.02 | 0.15 | nd | nd | nd | nd | 0.02 | 0.15 |
| R6 | 0.001 | 0.07 | nd | nd | nd | nd | 0.001 | 0.07 |
| 2'-C-methyl guanosine | 2.56 | 18.0 | 25.6 | 16.0 | 0.09 | 0.42 | 28.3 | 34.5 |
| Compound **5** | nd | nd | nd | nd | nd | 1.05 | nd | 1.05 |
| Compound **6** | nd | nd | nd | nd | 0.08 | 0.29 | 0.08 | 0.29 |
| Compound **7** | nd | nd | nd | nd | nd | nd | nd | nd |
| R5 | 0.08 | nd | nd | nd | nd | nd | 0.08 | nd |
| R4 | nd | 0.07 | nd | nd | nd | nd | nd | 0.07 |
| R3 | 0.02 | nd | nd | nd | nd | nd | 0.02 | nd |
| R2 | nd | 0.05 | nd | nd | nd | nd | nd | 0.05 |
| R1 | 0.13 | 1.08 | nd | nd | nd | nd | 0.13 | 1.08 |
| Total | 2.84 | 46.9 | 91.2 | nd | 0.17 | 1.76 | 94.2 | 87.7 |

| | | | | | | | | |
|---|---|---|---|---|---|---|---|---|
| *¹ In vitro* metabolites obsereved in example 14 ²nd = not detected | | | | | | | | |

### Conclusion

In rats given single doses of [¹⁴C]Compound **1**, overall urinary and fecal recovery of radioactivity accounted for 86% of the IV dose and 94% of the PO dose within 24 h. The primary route of elimination for the orally dosed group was fecal excretion (91% of dose). For the IV group, almost equal amounts of radioactivity were recovered in urine (47% of dose) and feces (39% of dose).

After IV dosing, unchanged Compound **1** was the major radioactive component (26% of dose) and 2'-C-methyl guanosine was the major metabolite (18% of dose) observed in urine. A second, more polar metabolite accounted for 1.1% of the IV dose. All other metabolites were present at less than 0.2% of the administered dose. Over 91% of the radioactivity excreted in feces was recovered as unchanged Compound **1** and 2'-C-methyl guanosine. The fecal recovery of these two compounds accounted for 20 and 16% of the IV dose and 66 and 26% of the PO dose.

After oral administration, 2'-C-methyl guanosine was the major dose-related component in the urine (2.6% of dose). A polar metabolite, R1, accounted for 0.1% and unchanged Compound **1** accounted for only 0.02% of the oral dose. The putative metabolites, Compound **2** and Compound **3,** were detected at very low levels.

Compound **1** was the most abundant component of feces for both dose groups (20% of IV dose and 66% of oral dose). 2'-C-methyl guanosine was the major metabolite observed in the feces of the IV group (16.0% of dose) and the only metabolite detected in the feces of the PO dose group (26% of dose). For the IV dose group, two minor metabolites, designated R11 and R10, were detected at 1.9% and 1.3% of the administered dose, respectively.

In the rat, the dose normalized AUC₀₋₂₄ values suggest that the oral bioavailability of the radioactive dose was 22%. This data reflects a combined bioavailability of Compound **1**, its major metabolite 2'-C-methyl guanosine and various other minor metabolites. The extent of absorption of the oral dose is likely greater than 22%, however, since a significant amount of the IV dose (39%) is excreted in the feces, an indication of extensive biliary secretion.

After IV administration of [¹⁴C]Compound 1, levels of total radioactivity in liver represented 16% of the administered dose at 2 h and 10% at 6 h. Total radioactivity in the liver declined to 1.8% of dose by 24 h. These levels of radioactivity correspond to 206, 137 and 23 µg equivalents of Compound **1**/g of liver.

Liver levels were significantly lower after oral administration, accounting for 0.2%, 0.4% and 0.2% of the dose at 2, 6 and 24 h, respectively. Nevertheless, these levels correspond to 5, 10 and 4 µg equivalents of Compound **1**/g of liver.

The major metabolite in the liver extracts of the IV dose group was the monophosphate of 2'-C-methyl guanosine, which accounted for 9.7%, 6.4%, and 1.1% of the administered dose at 2, 6, and 24 h, respectively. These values represented 60-63% of the total radioactivity in liver. The levels of diphosphate accounted for 16-22% of the radioactivity in liver and represented 3.4, 2.0, and 0.3% of the IV dose at the three respective time points. Radiolabeled 2'-C-methyl guanosine-5'-TP was detected at low levels by HPLC analysis in two pooled liver extracts at 2 h and 6 h sampling points of the IV dose group and represented 0.04-0.05% of the administered dose. 2'-C-methyl guanosine accounted for approximately 14-24% of the total radioactivity in liver of the IV dose group.

After oral administration of [¹⁴C]Compound **1**, neither Compound **5** or **7** were detected by the radioHPLC method in any of the liver samples. The diphosphate represented 0.04, 0.06 and 0.08% of the radioactive dose at the 2-, 6- and 24-h time points, respectively. These levels corresponded to 15-46% of the total radioactivity in liver at these sampling times and equate to 1.1, 1.6 and 1.9 µg equivalents of Compound 1/g of liver, respectively. 2'-C-methyl guanosine accounted for 54-85% of the radioactivity in liver of the PO group.

This study shows that Compound **5** is the predominant dose-related component found in rat liver when a relatively high amount of Compound **1** is delivered by IV administration. After oral administration, significantly lower levels of dose-related material are found in the liver, with Compound **6** being the predominant molecular form.

### EXAMPLE 17

### In-vitro Combination study of Compound 1 and ribavirin (RBV) on HCV replicon

Antiviral activity of Compound **1** in combination with ribavirin (RBV) on HCV replicon in GS4.1 cells (Zhu et al., 2003, J Virol., 77:9204-9210) was measured as described below.

The GS4.1 cell line harbors an HCV genotype 1b replicon (Lohmann 1999, Science 285 (5424):110-3). Compound **1** and RBV were diluted to 4X stocks in assay medium. Four additional 1.25-fold serial dilutions were made from the initial 4X stocks in assay medium for a 5-point titration. Compound **1** drug dilutions were added horizontally and RBV dilutions were added vertically to 96-well plates in a 5x5 checkerboard design. This setup was run in duplicate in each plate. Final drug concentrations ranged from 1.5X to 0.6X of the Compound **1** and RBV EC₅₀ values which were empirically determined in single-drug titration experiments; the EC₅₀ of Compound **1** was 0.4 ± 0.09 µM and the EC₅₀ of RBV was 21.8 ± 3.6 µM. After three days of drug treatment, the inhibition of HCV replication was measured by quantification of viral NS4A protein using an enzyme-linked immunosorbent assay (ELISA). The development and validation of the HCV replicon ELISA has been described in detail in a study report [IDIX-08-106].

Since cytotoxicity of the test compound could skew the drug-drug interaction analysis in these assays, cytotoxicity assays were performed in parallel employing the procedures described below (Mosmann 1983 J. Immunol. Meth, 65, 55-63). The readout of the assay was measured by the bioreduction of the yellow MTS tetrazolium compound to a purple formazan product. This conversion is mediated by NADPH or NADH in metabolically active cells and is therefore directly proportional to the number of live cells in a culture. Plates were setup as described above.

*In vitro* combination data analysis was conducted using the MacSynergyTM II program (Bliss independence model), and the CombiTool software (Loewe additivity model). In addition, the CalcuSyn program was used to calculate the CI at the calculated EC₅₀.

### Controls

1. No drug control: HCV replicon (GS4.1) cells in assay medium.
2. Single drug control: HCV replicon (GS4.1) cells treated with Compound 1 alone.
3. Single drug control: HCV replicon (GS4.1) cells treated with RBV alone.
4. Positive NS4A ELISA control: HCV replicon (GS4.1) cells in assay medium.
5. Negative NS4A ELISA control: Huh-7 cells in assay medium.
6. Positive cytotoxicity control: assay medium.
7. Negative cytotoxicity control: HCV replicon (GS4.1) cells in assay medium.

### Cell lines

Each cell line was tested and found to be negative for mycoplasma as detected by DNA stain and Direct Culture PCR procedures at the ATCC Mycoplasma Testing Services.

The Huh-7 cell line was derived from human hepatoma cells (Nakabayashi, 1982, Cancer Res. 42:3858-3863). Huh-7 cells were maintained in 75 cm² Corning flasks in Huh-7 growth medium at 37°C with 5% CO₂, and split with trypsin-EDTA at approximately 80% confluence.

The GS4.1 cell line was derived from the Huh-7 human liver cell line and stably possesses a bicistronic HV replicon. Cells were maintained in 75 cm² Corning flasks in GS4.1 growth medium at 3°C with 5% CO₂ and split with trypsin-EDTA at approximately 80% confluence.

### Reagents

The reagents used in this study were purchased from the following sources:
Trypsin-EDTA, Source: Cellgro; Methanol, Source: Burdick & Jackson; Acetone, Source: J.T. Baker; Anti-hepatitis C NS4A mouse monoclonal antibody (mAb), Source: Virogen; Wash solution concentrate (20X), Source: KPL; HRP-goat anti-mouse IgG (H+L) conjugate antibody, Source: Zymed; Ortho-phenylenediamine (OPD tablet), Source: Zymed; Hydrogen peroxide 30% solution (H₂O₂), Source: EMD; Sulfuric acid (H₂SO₄), Source: Mallinckrodt Chemicals; CellTiter 96® AQueous One Solution cell proliferation assay (MTS-based), Source: Promega; Citric acid, trisodium salt, dihydrate, Source: Sigma-Aldrich; Sodium phosphate, dibasic (Na₂HPO₄), Source: Fisher Chemical; Trizma® hydrochloride solution (Tris-HCl), Source: Sigma-Aldrich;; Sodium chloride (NaCl) solution 5.0 M, Source: Applied Biosystem Ethylenediaminetetraacetic acid (EDTA) solution 0.5 M Source: Sigma-Aldrich; Dimethyl sulfoxide (DMSO), Source: Sigma-Aldrich; Geneticin® (G418), 100X, Source: Cellgro; Dulbecco's modification of Eagle's medium (DMEM), Source: Cellgro; Fetal bovine serum (FBS), heat inactivated, Source: Cellgro; Penicillin/streptomycin solution (10,000 IU per mL/10,000 µg per mL), Source: Cellgro; GlutaMAX, dipeptide L-glutamine (200 mM), Source: Gibco; and MEM nonessential amino acids, Source: Cellgro.

### Media and buffers

1. Complete growth media for Huh-7 cells: 1X DMEM (containing glucose, L-glutamine and sodium pyruvate), supplemented with 10% fetal bovine serum, 100 IU/mL penicillin, 100 µg/mL streptomycin, 2 mM GlutaMAX, and 1% MEM nonessential amino acids.

2. Complete growth/selection media for GS4.1 cells: 1X DMEM (containing glucose, L-glutamine and sodium pyruvate), supplemented with 10% fetal bovine serum, 100 IU/mL penicillin, 100 µg/mL streptomycin, 2 mM GlutaMAX, 1% MEM nonessential amino acids, and 0.5 mg/mL geneticin (G418)

3. Assay medium: Huh-7 complete growth media with 1% DMSO

4. ELISA wash solution: KPL wash solution diluted to 1X in dH₂O

5. 10% FBS-TNE: 50 mM Tris-HCl (pH 7.5; Sigma), 100 mM NaCl, 1 mM EDTA with 10% FBS

6. Citrate/phosphate buffer: 16 mM citric acid, 27 mM Na₂HPO₄

7. OPD solution: 1 OPD tablet + 12 mL citrate/phosphate buffer + 5 mL 30% H₂O₂ per plate.

### Drug treatment

96-well plates were seeded with GS4.1 (rows A-G) and Huh-7 (row H) cells, respectively, at a density of 7.5x10³ cells per well in 50 µL complete growth media.

Compound 1 and RBV were diluted in assay medium to 4X stocks. Four additional 1.25-fold serial dilutions of each compound were prepared from the 4X stock in assay medium.

Cells were incubated at 37°C/5% CO₂ for at least four hours, and then drug treatment was initiated by adding 25 µL of the serial Compound **1** drug dilutions horizontally and 25 µL of the serial RBV dilutions vertically in a 5x5 checkerboard design. This setup was run in duplicate in each 96-well plate. Final concentrations of both drugs ranged from 1.5X to 0.6X their respective EC₅₀ values.

Cells were incubated with inhibitors for three days at 37°C/5% CO₂.

### HCV replicon ELISA

After three days of drug treatment, media was removed from the plates via gentle tapping and the cells were fixed to the plates with 100 µL/well of 1:1 acetone:methanol.

After one minute of incubation at room temperature, plates were washed three times with 100 µL/well of ELISA wash solution and then blocked with 150 µL/well of 10% FBS-TNE.

After a one hour incubation at room temperature, plates were washed three times with 100 µL/well of ELISA wash solution, and then 100 µL/well of mouse anti-hepatitis C NS4A mAb (1 mg/mL stock diluted 1:4,000 in 10% FBS-TNE) was added.

Following 2 hours of incubation at 37°C, plates were washed three times with 100 µL/well of ELISA wash solution, and then 100 µL/well of HRP-goat anti-mouse antibody (diluted 1:3,500 in 10% FBS-TNE) was added.

After a one hour incubation period at 37°C, each plate was washed three times with 100 µL/well of ELISA wash solution. Color development was then initiated with 100 µL/well of OPD solution.

After a 30 minute incubation period in the dark at room temperature, reactions were stopped by adding 100 µL of 2N H₂SO₄ to each well.

The absorbance was measured at 490 nm on a Victor³ V 1420 multilabel counter (Perkin Elmer). The percent inhibition of HCV replicon replication was calculated for each drug and drug combination using an average of the duplicate wells. The 50% effective concentration (EC₅₀) value was calculated for Compound 1 alone and RBV alone from the resulting best-fit equations determined by XLfit 4.1 software. *In vitro* combination data analysis was conducted using the Bliss independence model (MacSynergy^{™} II) and Loewe additivity model (CombiTool). In addition, the CalcuSyn program was used to calculate the average CI at the calculated EC₅₀.

### Cytotoxicity determination of Compound 1 and RBV on GS4.1 cells using MTS assay

Cells were plated and treated with inhibitors using the drug matrix described above.

After three days of drug treatment, 20 µL of MTS was added to each well, and then plates were incubated at 37°C/5% CO₂.

After 3 hours of incubation, the absorbance was measured at 490 nm in a Victor³ V 1420 multilabel counter (Perkin Elmer). The 50% cytotoxic concentration (CC₅₀) value for each drug and drug combination was determined from the resulting best-fit equations determined by XLfit 4.1 software.

Under the experimental conditions used in this study, no evidence of in vitro cytotoxicity was observed in cells treated with Compound 1 and RBV alone or in combination.

### Analysis of drug combination data

The MacSynergy^{™} II program, version 1.0 (Prichard, Aseltine, Shipman; University of Michigan, Michigan, USA) was used to evaluate antiviral potency data according to the Bliss independence model. MacSynergy^{™} II calculates a theoretical, additive, dose-response surface based on the dose-response curves for each drug titrated individually. The calculated additive interaction surface is then subtracted from the experimentally derived interaction surface, revealing areas of non-additive interaction. Peaks above the plane at 0% inhibition indicate areas of greater than additive interaction, or synergism. Conversely, peaks below the plane at 0% inhibition indicate areas of less than additive interaction, or antagonism. Data sets are statistically evaluated with confidence intervals around the experimental dose-response surface. Peak volumes are calculated quantifying the degree of synergy or antagonism that lies outside of the confidence interval; synergy and antagonism volumes and log volumes (correcting for drug dilutions) are reported. In this study, data sets were assessed at the 99.9% confidence level; volumes of <25 µM²% (log volumes <2) were considered additive, those >25 but <50 µM²% (log volumes >2 and <5) were perceived as weak synergy or antagonism, values >50 but <100 µM²% (log volumes >5 and <9) were considered moderate synergy or antagonism, and values >100 µM²% (log volumes >9) were considered strong synergy or antagonism (Prichard, et al. 2004, Antimicrobial Agents and Chemotherapy; 37(3): 540-545).

The CombiTool program, version 2.001 (Dressler, Muller, and Suhnel; Institute of Molecular Biotechnology, Jena, DE) was used to calculate the differences between predicted effects (according to the Loewe additivity model) and observed effects (experimental data). When the differences from predicted effects are graphed, a visual representation of compound interaction is obtained. The line at zero represents the additive zero interaction plane. Points falling below or above the zero interaction plane represent areas of synergy or antagonism respectively. Deviations greater than 0.25 from the predicted effects were considered significant (Dressler et al. 1999, Computers and Biomedical Research; 32: 145-160).

The CalcuSyn program, version 2.1 (BIOSOFT, Cambridge, UK) was used to determine the nature of drug-drug interaction based on the Combination Index equation proposed by Chou and Talalay (1984) Adv. Enz. Regul. 22: 27-55. The Combination Index at the calculated EC₅₀ for the drug combination is reported; the description of the degree of drug interaction based on the CI value is as recommended in the CalcuSyn for Windows software manual (Table 59).

**Table 59: Description of the degree of interaction as analyzed by the Combination Index method**

| Range of Cl | Description |
|---|---|
| <0.1 | very strong synergism |
| 0.1-0.3 | strong synergism |
| 0.3-0.7 | synergism |
| 0.7-0.85 | moderate synergism |
| 0.85-0.90 | slight synergism |
| 0.90-1.10 | additive |
| 1.10-1.20 | slight antagonism |
| 1.20-1.45 | moderate antagonism |
| 1.45-3.3 | antagonism |
| 3.3-10 | strong antagonism |
| >10 | very strong antagonism |

### MacSynergy^{™} II analysis (Bliss independence)

Five individual experimental data sets were analyzed using the MacSynergy^{™} II software at the 99.9% confidence interval.

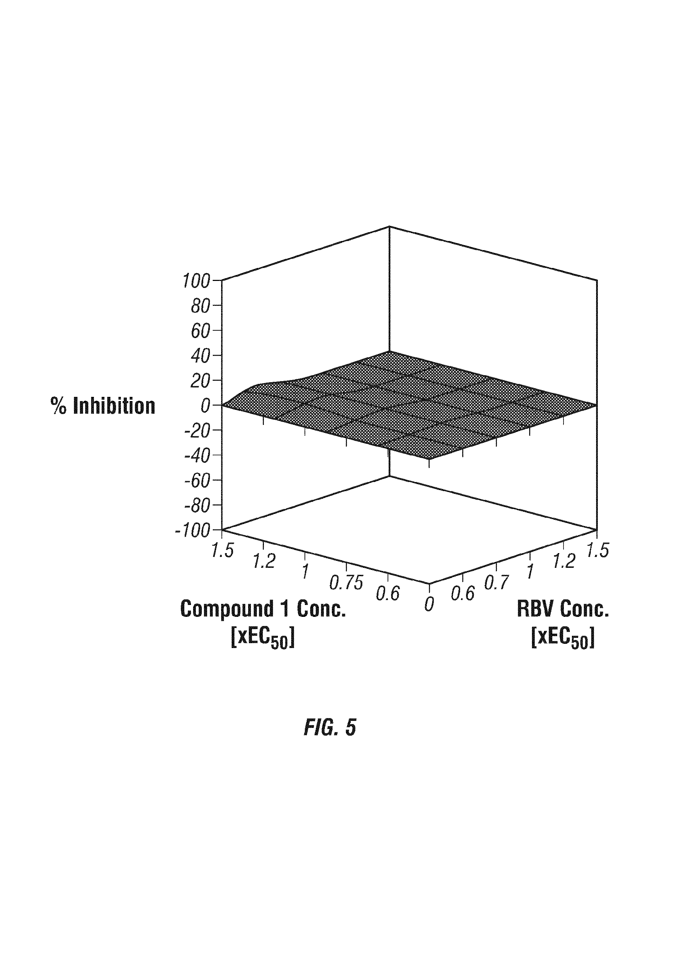

The result of this analysis is presented in Figure 5. A synergistic interaction is localized in the area of the lowest RBV concentration (0.6xEC₅₀) and Compound **1** concentrations at or above its EC₅₀ (1x to 1.5xEC₅₀₎, indicated by the small peak at these concentrations in Figure 5. All other data points presented in Figure 5 are clustered close to the additive zero-interaction plane suggesting that the two drugs inhibit HCV replicon *in vitro* in an additive fashion at these concentrations.

The calculated interaction volume of 56.7 µM²% suggests moderate synergy. When corrected for drug dilutions, a log interaction volume of 0.53 µM²% is produced, again suggesting an additive interaction of Compound **1** and RBV.

Overall, in one embodimnt, the combined effect of Compound **1** and RBV is weakly synergistic when ≥ 0.4 µM of Compound **1,** is combined with low concentrations (0.6xEC₅₀) of RBV. In another embodiment, a combination of Compound **1** and RBV is additive.

### CombiTool analysis (Loewe additivity)

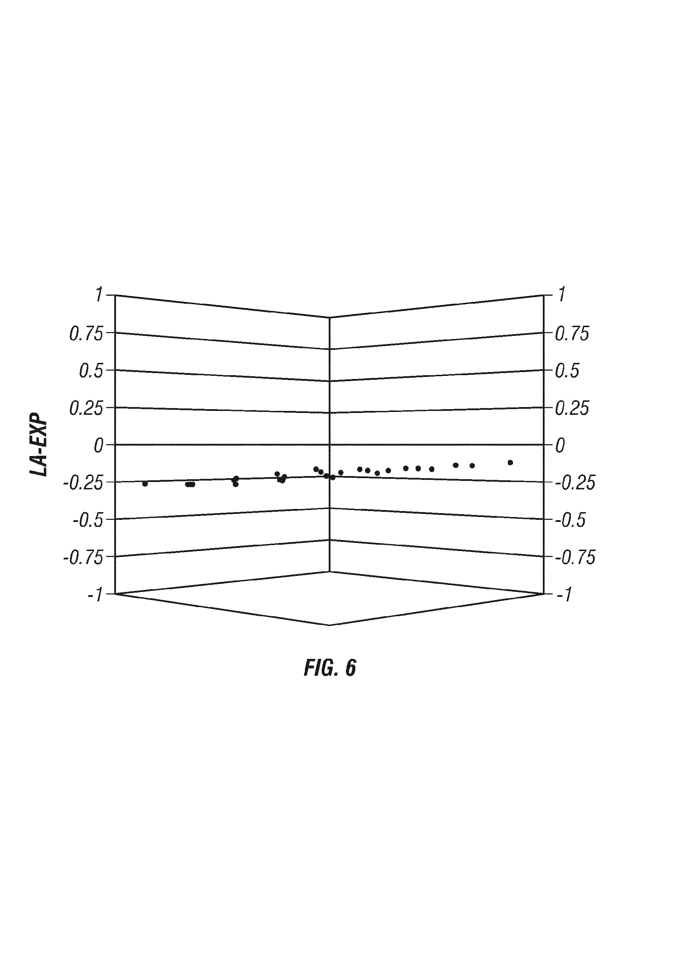

The differences between calculated additive effect and experimental data were quantified and plotted for each drug combination. Figure 6 depicts the differences between calculated additive and observed anti-HCV effects for all drug combinations from 5 independent experiments, obtained with the CombiTool software using the Loewe additivity model. In Figure 6, LA-EXP = differences between observed effects from 5 experiments and predicted effects as determined by the Loewe additivity model by CombiTool software. The line at zero represents the additive zero interaction plane. Points falling below or above the zero interaction plane represent areas of synergy or antagonism respectively. Deviations greater than 0.25 from the predicted effects were considered significant.

Similar to MacSynergy^{™} II analysis, CombiTool shows that drug combinations involving high concentrations of Compound **1** and low concentrations of RBV are weakly synergistic, as seen by data points clustering around the -0.25 plane in the CombiTool graph. All other drug combination data points fall above the -0.25 plane, suggesting that the two drugs inhibit HCV replicon in an additive fashion at these drug concentrations. According to the Loewe additivity model examined with the CombiTool program, in one embodiment, the interaction of Compound **1** and RBV appears to be weakly synergistic when high concentrations of Compound **1** are combined with low concentrations of RBV; all other combinations result in additive interactions.

### CalcuSyn analysis (combination index)

The CalcuSyn program determined that the average CI at the calculated EC₅₀ was 0.37 ± 0.12, consistent with an interpretation of synergism at this effect level.

No evidence of *in vitro* cytotoxicity was observed in cells treated with Compound 1 and RBV alone or in combination, suggesting that the combined activity relationship is due to inhibition of HCV replication rather than cell cytotoxicity.

In certain embodiments, the combination of ribavirin and Compound **1** has enhanced antiviral efficacy compared to either agent tested alone. In certain embodiments, the combination is synergistic in nature. This is contrary to the reports in the literature that there is antagonism between 2'-C-methyl nucleosides and ribavirin, *see*, Coelmont et al. Antimicrobial Agents and Chemotherapy, 2006, 50, 3444-3446.

Without being bound to any theory, it is believed that cells exposed to ribavirin generate a substantial amount of ribavirin triphosphate which causes depletion of the levels of cellular GTP - the natural substrate for HCV RNA synthesis - via a nucleotide synthesis feedback mechanism. The result is that 2'-C-methyl guanosine-5'-triphosphate has less of the natural GTP substrate to compete with and therefore is a more effective inhibitor.

It is also be noted that the feedback effect of ribavirin would be expected on the first phosphorylation step that converts guanosine to guanosine monophosphate. Since Compound 1 bypasses this step by delivering 2'-C-methyl guanosine -5'-monophosphate, ribavirin would be expected to have no negative impact on conversion of Compound 1 to 2'-C-methyl guanosine-5'-triphosphate.

### EXAMPLE 18

### In vitro susceptibility of S282T substituted HCV to ribavirin and Compound 1

In this experiment, the *in vitro* susceptibility of S282T substituted hepatitis C virus (HCV) to ribavirin (RBV) and compound 1 in two test systems: a standard biochemical assay and a genotype 1 b replicon test system was evaluated. The antiviral potency of ribavirin 5'-triphosphate) and compound 7 was first determined using a standard *in vitro* enzyme assay that measures NS5B-mediated incorporation of radiolabeled guanosine 5'-monophosphate (GMP) into an oligomeric ribonucleic acid (RNA) template in the presence or absence of drug. Wild-type and site-directed S282T mutant enzymes were assayed in the presence of ribavirin triphosphate (RBV-TP) or compound 7, which is the active metabolite of compound 1 in cells.

### Controls

1. No-drug (positive) control for biochemical HCV assay: polymerase in reaction buffer without drug.
2. Background (negative) control for biochemical HCV assays: same as positive control plus 100 mM EDTA.
3. No-drug (positive) control for HCV replicon assay: HCV replicon (GS4.1) cells in assay medium.
4. Positive NS5A ELISA control: untreated HCV replicon (GS4.1) cells in assay medium.
5. Negative NS5A ELISA control: Huh-7 cells in assay medium.
6. Positive cytotoxicity control: assay medium.
7. Negative cytotoxicity control: untreated HCV replicon (GS4.1) cells in assay medium.

### HCV polymerases

The polymerase enzymes employed in this study were expressed and purified as follows. The wild-type construct encodes the 65 kDa HCV NS5B protein of genotype 1b (strain Con-1) in a standard bacterial expression system used to generate recombinant polymerase enzyme. A mutant HCV polymerase plasmid carrying a single serine-to threonine substitution at residue 282 of the NS5B gene was generated by site-directed mutagenesis of the wild-type construct using a commercial mutagenesis kit as recommended by the manufacturer. The expression constructs utilized in this study were:
1. HCV genotype 1b wild-type polymerase (with a deletion of the 21 carboxyterminal amino acids) cloned into NheI/XhoI restriction sites of expression vector pET21a (Ampr). Clone number 9-15.
2. HCV 1b S282T polymerase contains a single serine-to-threonine substitution at residue 282 within the B domain of the NS5B gene in same expression vector.

### HCV replicon cell lines

Each cell line was tested and found to be negative for mycoplasma as detected by DNA stain and Direct Culture PCR procedures at the ATCC Mycoplasma Testing Services.
1. The Huh-7 cell line was derived from human hepatoma cells (Nakabayashi H., Growth of human hepatoma cell lines with differentiated functions in chemically defined medium, Cancer Res.; 1982, 42:3858-3863). Huh-7 cells were maintained in 75 cm² Corning flasks in Huh-7 growth medium at 37°C with 5% CO₂, and split with trypsin-EDTA at approximately 80% confluence.
   Source: Dr. Christoph Seeger, Fox Chase Cancer; Philadelphia, PA
2. The GS4.1 cell line (Zhu et al. Replication of hepatitis C virus subgenomes in nonhepatic epithelial and mouse hepatoma cells, J. Virol.; 2003, 77:9204-9210) was derived from the Huh-7 human liver cell line and stably possesses a bicistronic HCV replicon. Cells were maintained in 75 cm² Corning flasks in GS4.1 growth medium at 37°C with 5% CO₂ and split with trypsin-EDTA at approximately 80% confluence.
   Source: Dr. Christoph Seeger, Fox Chase Cancer; Philadelphia, PA
3. The 184R-A cell line was derived by culturing wild-type replicon cells in the presence of 1 to 5 µM of Compound 1 for > 100 days. Population sequencing indicated that the S282T variant had outgrown the wildtype by day 103.
4. The 184R-D2 cell line was also derived by culturing wild-type replicon cells in the presence of increasing concentrations of Compoud 1, beginning with 0.9 µM and ending with 3.6 µM. The S282T mutation emerged in the replicon after 69 days of selection, and outgrew wild-type S282 variant after 80 days of selection. Two additional mutations, S189P and Y586C were observed in the 184R-D replicon cell line. These mutations were not selected in any other Compound 1-resistant replicons and have not been implicated in resistance to HCV nucleos(t)ide analogs.

### Reagents for biochemical assay

RNase-free water, Source: Applied Biosystems (Catalog#: AM9932); Magnesium chloride, Source: Sigma-Aldrich (Catalog#: M1028); Manganese chloride, Source: Sigma-Aldrich (Catalog#: M1787); Dithiothreitol (DTT), Source: Sigma-Aldrich (Catalog#: 43815); α-[³³P]GTP, Source: Perkin Elmer Life Sciences (Catalog#: NEG 606H); Flexible 96-well lid, Source: BD Falcon (Catalog#: 353913); Flexible 96-well U-bottom plate, Source: BD Falcon (Catalog#: 353911); Microscint-O scintillation fluid, Source: Perkin Elmer Life Sciences (Catalog#: 6013611); UltraPure bovine serum albumin (BSA) 50 mg/mL, Source: Applied Biosystems (Catalog#: AM2616); Unifilter-96 GF/B plates, Source: Perkin Elmer Life Sciences (Catalog#: 6005177); Ethanol (EtOH), 200 proof, Source: Sigma-Aldrich (Catalog#: E7023-4L); Sodium pyrophosphate decahydrate, Source: EMD Chemicals (Catalog#: SX0740-1); Triton X-100, Source: Sigma-Aldrich (Catalog#: T8787); SUPERase-In RNase inhibitor, Source: Applied Biosystems (Catalog#: AM2696); Glutamic acid monosodium salt monohydrate, Source: Sigma-Aldrich (Catalog#: G2834); Trichloroacetic acid (TCA), Source: EMD Chemicals (Catalog#: TX1045-1), Ethylenediaminetetraacetic acid (EDTA) 0.5 M, pH 8.0, Source: Applied Biosystems (Catalog#: AM9260G); Adenosine triphosphate (ATP), Source: GE Healthcare Life Sciences (Catalog#: 27-2056-01); Cytidine triphosphate (CTP), Source: GE Healthcare Life Sciences (Catalog#: 27-2066-01); Guanosine triphosphate (GTP), Source: GE Healthcare Life Sciences (Catalog#: 27-2076-01); Uridine triphosphate (UTP), Source: GE Healthcare Life Sciences (Catalog#: 27-2086-01); and Synthetic RNA oligonucleotides, Source: Dharmacon (Catalog#: custom synthesis).

### Reagents for replicon assay and ELISA

75 cm² flasks, Source: Corning Costar (Catalog#: 430641); 96-well plates, Source: Corning Costar (Catalog#: 3595); Trypsin-EDTA, Source: Cellgro(Catalog#: 25-053-CI); Methanol, Source: Burdick & Jackson (Catalog#: AH230-4); Acetone, Source: J.T. Baker (Catalog#: 9006-03); Anti-hepatitis C NS5A mouse monoclonal antibody (mAb), Source: Virogen (Catalog#: 256-A); Wash solution concentrate (20X), Source: KPL (Catalog#: 50-63-00); HRP-goat anti-mouse IgG (H+L) conjugate antibody, Source: Zymed (Catalog#: 81-6520); Ortho-phenylenediamine (OPD tablet), Source: Zymed (Catalog#: 00-2003); Hydrogen peroxide 30% solution (H₂O₂), Source: EMD (Catalog#: HXO635-1); Sulfuric acid, Source: Mallinckrodt Chemicals (Catalog#: 2876-05); CellTiter 96® AQueous One Solution cell proliferation assay (MTS-based), Source: Promega (Catalog#: G3582); Citric acid, trisodium salt, dehydrate, Source: Sigma-Aldrich (Catalog#: C-8532); Sodium phosphate, dibasic, Source: Fisher Chemical (Catalog#: S373-3); Trizma® hydrochloride solution (Tris-HCl), Source: Sigma-Aldrich (Catalog#. T2663-1L); Sodium chloride, solution 5.0 M, Source: Applied Biosystem (Catalog#: AM9759); Ethylenediaminetetraacetic acid (EDTA) solution 0.5 M, Source: Sigma-Aldrich (Catalog#: E7889-100 mL); Dimethyl sulfoxide, Source: Sigma-Aldrich (Catalog#: D2650); Geneticin® (G418), 100X, Source: Cellgro (Catalog#: 30-324-CI); Dulbecco's modified Eagle's medium (DMEM), Source: Cellgro (Catalog#: 10-013-CV); Fetal bovine serum (FBS), heat inactivated, Source: Cellgro (Catalog#: 35-016-CV); Penicillin/streptomycin solution (10,000 IU per mL/10,000 µg per mL), Source: Cellgro (Catalog#: 30-001-CI); GlutaMAX, dipeptide L-glutamine (200 mM), Source: Gibco (Catalog#: 35050-061); and MEM nonessential amino acids, Source: Cellgro (Catalog#: 25-025-CI).

### Media and buffers

In this experiment, the following media and buffers were used.
1. Complete growth media for Huh-7 cells: 1X DMEM (containing glucose, L-glutamine and sodium pyruvate), supplemented with 10% fetal bovine serum, 100 IU/mL penicillin, 100 µg/mL streptomycin, 2 mM GlutaMAX, and 1% MEM nonessential amino acids
2. Complete growth/selection media for wild-type and 184Rreplicon cells: 1X DMEM (containing glucose, L-glutamine and sodium pyruvate), supplemented with 10% fetal bovine serum, 100 IU/mL penicillin, 100 µg/mL streptomycin, 2 mM GlutaMAX, 1% MEM nonessential amino acids, and 0.5 mg/mL geneticin (G418)
3. Assay medium: Huh-7 complete growth media with 1% DMSO
4. ELISA wash solution: KPL wash solution diluted to 1X in dH₂O
5. 10% FBS-TNE: 50 mM Tris-HCl (pH 7.5; Sigma), 100 mM NaCl, 1 mM EDTA with 10% FBS
6. Citrate/phosphate buffer: 16 mM citric acid, 27 mM Na₂HPO₄
7. OPD solution: 1 OPD tablet + 12 mL citrate/phosphate buffer + 5 mL 30% H₂O₂ per plate.

### I: in vitro HCV polymerase assays

In this experiment, evaluation inhibition of the *in vitro* polymerase activity of HCV wild-type and S282T mutant polymerases by RBV-TP and compound 7 was conducted.

### Generation of wild-type and S282T mutant HCV 1b polymerases

Carboxyterminally hexa-histidine tagged wild-type and S282T HCV polymerase enzymes were expressed and purified to 85-90% purity from *E*. *coli* BL21 (DE3) cells utilizing a single step nickel affinity chromatography procedure.

### Preparation of compound solutions

Compound 7 and ribavirin-TP stocks were prepared as 10 mM solutions in nuclease-free water and stored in small aliquots at -20°C.

Eight serial dilutions of RBV-TP and compound 7 were freshly prepared in an aqueous 5 µM ATP solution yielding final drug test concentrations of 1000 - 0.46 µM (RBV-TP) and 100 - 0.05 µM (compound 7).

### Biochemical HCV polymerase assay for IC₅₀ determinations

This assay was conducted to measure the inhibitory effect of drug on the incorporation of α-[³³P]GMP into trichloroacetic acid (TCA) precipitable material. Radiolabeled product was collected by filtration onto 96-well filter plates and quantitated by liquid scintillation counting. A synthetic RNA oligonucleotide was used as template for the synthesis of a complementary RNA strand.
• Ninety-six-well flexible plates containing the test articles were prepared as follows: 10 µL of the serially-diluted RBV-TP or compound 7 in 5 µM ATP was transferred to triplicate wells in adjacent columns. Plates were kept on ice prior to enzyme addition.
• Ten µL of 500 mM EDTA was added to column 1 of the assay plate to determine the background radioactivity.
• Ten µL of 5 µM ATP was added to columns 2 and 3 of the assay plate for the no-drug control.
• Reaction cocktail was prepared in bulk on ice; to start the reactions 40 µL of reaction cocktail was added to each well of the assay. Thus, the final reaction volume was 50 µL. Final concentrations of all reagents in reactions are provided below in Table 60:

**Table 60**

| Reagent | Concentration | |
|---|---|---|
| Sodium glutamate | 20 | mM |
| Magnesium chloride | 4 | mM |
| Manganese chloride | 1 | mM |
| Dithiothreitol | 10 | mM |
| Triton X-100 | 0.1 | % |
| RNase inhibitor | 5 | units/reaction |
| Bovine serum albumin | 50 | µg/mL |
| ATP | 1 | µM |
| CTP | 1 | µM |
| UTP | 1 | µM |
| GTP | 50 | nM |
| α-[³³P]GTP | 0.1 | µCi/reaction |
| RNA template | 30 | nM |
| HCV 1b polymerase | 60 | nM |
| Compound 7 or RBV-TP Nuclease-free water | | variable, as described to final volume of 50 µL |

• Reactions were incubated at 30°C for 2 h.
• Reactions were terminated by the addition of 50 µL precipitation solution (22.5% TCA, 25 mM sodium pyrophosphate, ice cold), followed by incubation on ice for at least 20 minutes.
• Plates were harvested on a Packard Unifilter Harvester (Perkin Elmer Life Sciences) using GF/B filter plates (Perkin Elmer Life Sciences) pre-rinsed with 0.1 M sodium pyrophosphate. Plates were washed extensively with deionized water followed by an ethanol wash to aid drying.
• Plates were allowed to air dry, followed by addition of MicroScint-O (35 µL per well; Perkin Elmer Life Sciences). Plates were counted on the Packard TopCount Liquid Scintillation counter (Perkin Elmer Life Sciences).
• IC₅₀ values were calculated from single-site dose response curves by best-fit equations determined with XLfit 4.1 software.

In vitro inhibition of wild-type HCV NS5B (genotype 1b) by Ribavirin-TP and Compound 7 is provided in Table 61 below (IC₅₀ values in µM)

**Table 61**

| **Compound ID** | **N** | **Wild-type** | **S282T** |
|---|---|---|---|
| **Ribavirin-TP (Moravek M1593)** | **3** | 883.4 ± 378 | 45.9 ± 16.2 |
| **Compound 7** | **3** | 0.27 ± 0.06 | 2.61 ± 0.72 |

As seen in Table 61, RBV-TP exhibited marginal inhibition of wild-type genotype 1b NS5B and was >3,000 fold less active than Compound 7 *in vitro.* The S282T mutant enzyme was 9.73 ± 1.79-fold less susceptible to inhibition by compound 7 as seen by the mean IC₅₀ value of 2.61 ± 0.72 µM. In contrast, RBV-TP exhibited enhanced *in vitro* activity against the S282T mutant polymerase as seen by the 19.2-fold lower IC₅₀ of 45.9 ± 16.2 µM.

These data suggest a different cross-resistance profile of RBV-TP and Compound 7 against the S282T mutant enzyme *in vitro* and imply that ribavirin may counteract emergence of the S282T resitance mutation when used in combination with Compound 1.

In certain embodiments, interferon may counteract emergence of the S282T resitance mutation when used in combination with Compound 1.

Table 62 provides fold change from wild-type to mutant S282T.

**Table 62**

| **Compound ID** | **N** | **Wild-type** | **S282T** |
|---|---|---|---|
| **Ribavirin-TP (Moravek M1593)** | **3** | 1 | 0.06 ± 0.02 |
| **Compound 7** | **3** | 1 | 9.73 ± 1.79 |

The S282T mutant enzyme was 9.73 ± 1.79-fold less susceptible to inhibition by compound 7 as seen by the mean IC₅₀ value of 2.61 ± 0.72 µM (Table 61, Table 62). In contrast, RBV-TP exhibited enhanced *in vitro* activity against the S282T mutant polymerase as seen by the 19.2-fold lower IC₅₀ of 45.9 ± 16.2 µM (Table 61).

### II: A. Antiviral activity of ribavirin on wild-type or S282T carrying stable HCV replicon cell lines

In this study the *in vitro* anti-HCV activity of ribavirin and compound 1 in stable cell lines carrying wild-type or Compound 1-resistant S282T replicons of genotype 1b was determined.

### HCV replicon assay with RBV and Compound 1

### Drug treatment

The following protocol was used:
1. Columns 2-12 of 96-well plates were seeded with either wild-type (GS4.1) or S282T (184R-A and 184R-D2) replicon cells at a density of 7.5x10³ cells per well in 50 µL complete growth media. Huh-7 cells were seeded into column 1 of each plate at a similar density.
2. Compound 1 and RBV were diluted in assay medium to 2X stocks. Eight additional 3-fold serial dilutions of each compound were prepared from the 2X stock in assay medium. Final drug concentrations ranged from 100 to 0.015 µM.
3. Cells were incubated at 37°C/5% CO₂ for at least four hours, and then drug treatment was initiated by adding 50 µL of the serial RBV and Compound 1 drug dilutions. This setup was run in duplicate in each 96-well plate.
4. Cells were incubated with inhibitors for three days at 37°C/5% CO₂.

### HCV replicon ELISA

The following protocol was used for HCV replicon assay:
1. After three days of drug treatment, media was removed from the plates via gentle tapping and the cells were fixed to the plates with 100 µL/well of 1:1 acetone:methanol.
2. After one minute of incubation at room temperature, plates were washed three times with 100 µL/well of ELISA wash solution and then blocked with 150 µL/well of 10% FBS-TNE.
3. After a one hour incubation at room temperature, plates were washed three times with 100 µL/well of ELISA wash solution, and then 100 µL/well of mouse anti-hepatitis C NS5A mAb (1 mg/mL stock diluted 1:1,500 in 10% FBS-TNE) was added.
4. Following 2 hours of incubation at 37°C, plates were washed three times with 100 µL/well of ELISA wash solution, and then 100 µL/well of HRP-goat anti-mouse antibody (diluted 1:4,000 in 10% FBS-TNE) was added.
4. After a one hour incubation period at 37°C, each plate was washed three times with 100 µL/well of ELISA wash solution. Color development was then initiated with 100 µL/well of OPD solution.
5. After a 30 minute incubation period in the dark at room temperature, reactions were stopped by adding 100 µL of 2N H₂SO₄ to each well.
6. The absorbance was measured at 490 nm on a Victor³ V 1420 multilabel counter (Perkin Elmer). The percent inhibition of HCV replicon replication was calculated for each drug using an average of the duplicate wells. The 50% effective concentration (EC₅₀) value was calculated for Compound 1 and RBV from the resulting best-fit equations determined by XLfit 4.1 software.

### Cytotoxicity determination of RBV and Compound 1 on GS4.1 cells using 3-(4,5-dimethylthiazol-2-yl)-5-(3-carboxymethoxyphenyl)-2-(4-sulfophenyl)-2H-tetrazolium, inner salt (MTS) assay

Cells were plated and treated with inhibitors as described above.

After three days of drug treatment, 20 µL of MTS was added to each well, and then plates were incubated at 37°C/5% CO₂ for 3 hours.

The absorbance was measured at 490 mn in a Victor³ V 1420 multilabel counter (Perkin Elmer). The 50% cytotoxic concentration (CC₅₀) value for each drug and drug combination was determined from the resulting best-fit equations determined by XLfit 4.1 software.

Under the experimental conditions used in this study, no evidence of *in vitro* cytotoxicity was observed in wild-type or S282T replicon cells treated with up to 100 µM of Compound 1 or RBV, *see,* Table 63. Compound 1 exhibited the expected antiviral activity in wild-type HCV replicon bearing GS4.1 cells and in S282T mutant replicon cells. The mean EC₅₀ values determined from 3 independent experimental data sets are provided in Table 63. Table 64 provides fold change from wild-type to mutant S282T.

**Table 63: Antiviral activity and cytotoxicity of ribavirin and Compound 1 in wild-type and S282T HCV replicon cell lines**

| **Compound** | **N^{a}** | **GS4.1^{d} (wild-type)** | | **184R-A^{e} (S282T)** | | **184R-D2^{e} (S282T)** | |
|---|---|---|---|---|---|---|---|
| | | EC₅₀^{b} (µM) | CC₅₀^{c} (µM) | EC₅₀ (µM) | CC₅₀ (µM) | IC₅₀ (µM) | IC₅₀ (µM) |
| Ribavirin | 3 | 38 ± 2.8 | >100 | 12.3 ± 1.9 | >100 | 17 ± 3.5 | >100 |
| Compound 1 | 3 | 0.3 ± 0.06 | >100 | 9 ± 0.9 | >100 | 39 ± 10 | >100 |
| ^{a}N= number of independent experiments | | | | | | | |

| | | | | | | | |
|---|---|---|---|---|---|---|---|
| ^{a}N= number of independent experiments ^{b}EC₅₀ = 50% effective concentration ± standard deviation ^{c}CC₅₀ = cytotoxic concentration that reduces cell viability by 50% in cell culture as determined by the MTS assay. ^{d}GS4.1 = Human hepatoma cell line bearing the wild-type HCV con1 replicon ^{e}184R-A & 184R-D2 = Compound 1 selected replicon cell lines containing the S282T signature mutation | | | | | | | |

**Table 64 Fold shift from wild-type**

| **Compound** | **GS4.1 (wild-type)** | **184R-A (S282T)** | **184R-D2 (S282T)** |
|---|---|---|---|
| Ribavirin | 1 | 0.32 | 0.45 |
| Compound 1 | 1 | 30 | 130 |

Fold shift was determined by dividing the EC₅₀ obtained in the compound 1 resistant cell lines by the EC₅₀ obtained in wild-type cells.

Ribavirin had a measurable anti-HCV activity with a mean EC₅₀ of 38 ± 2.8 µM in wild-type GS4.1 replicon cells and, thus, was approximately 126-fold less active than Compound 1. However, when tested in the two Compound 1-resistant replicon cell lines, 184R-A and 184R-D2, ribavirin showed a 2- to 3-fold increase in activity as indicated by the lower mean EC₅₀ values of 12.3 ± 1.9 µM (184R-A) and 17 ± 3.5 µM (184R-D2) (Table 63).

This data from the HCV replicon appear to confirm the fact that RBV is indeed more active against the S282T mutant HCV replicon than it is against the wild-type replicon where RBV activity is very difficult to measure as it overlaps with the cytotoxicity of the compound.

The improved activity of RBV against the S282T mutant polymerase appears to be consistent with RBV being a direct antiviral agent against the mutant HCV strain, although other mechanisms of action of RBV have not been ruled out. In summary, ribavirin and its active metabolite RBV-TP appeared to demonstrate enhanced antiviral *in vitro* activity against the S282T variant and showed no evidence of cross-resistance and/or antagonism to Compound 1 and Compound 7 with respect to HCV replicon bearing the Compound 1(2'-C-methyl nucleoside) signature HCV resistance mutation S282T.

All publications and patent, applications cited in this specification are herein incorporated by reference as if each individual publication or patent application were specifically and individually indicated to be incorporated by reference. While the claimed subject matter has been described in terms of various embodiments, the skilled artisan will appreciate that various modifications, substitutions, omissions, and changes may be made without departing from the spirit thereof. Accordingly, it is intended that the scope of the subject matter limited solely by the scope of the following claims, including equivalents thereof.

**The invention also relates to the following items:**
1. A diastereomerically pure compound having formula: 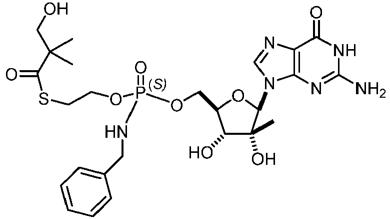 or a pharmaceutically acceptable salt, solvate or hydrate thereof.
2. A diastereomerically pure compound having formula: 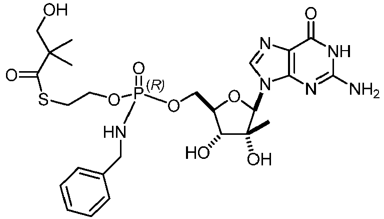 or a pharmaceutically acceptable salt, solvate or hydrate thereof.
3. A purified compound having formula: 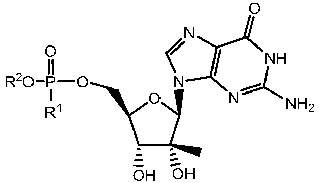 or a pharmaceutically acceptable salt, solvate, hydrate, a stereoisomeric or tautomeric form thereof, wherein R¹ is hydroxyl, amino or benzylamino; and R² is hydrogen, 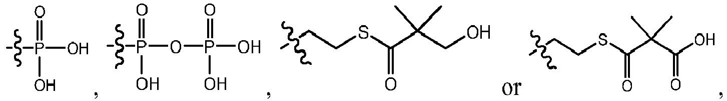 such that when R¹ is hydroxyl, R² is other than hydrogen, 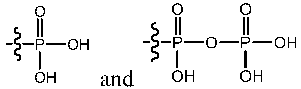 and
when R¹ is benzylamino, then R² is other than 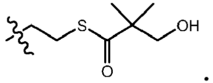 4. The purified compound of item 3 having formula II: 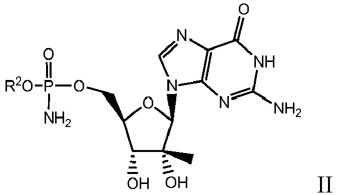 or a pharmaceutically acceptable salt, solvate, hydrate, a stereoisomeric or tautomeric form thereof, where R² is hydrogen, 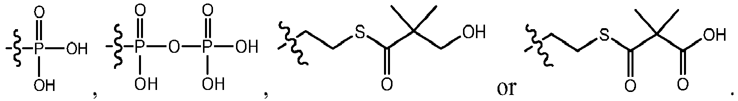 5. The purified compound of item 3 having formula III: 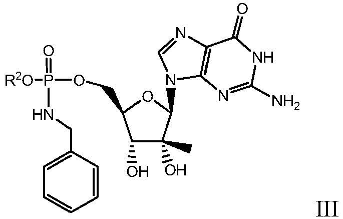 or a pharmaceutically acceptable salt, solvate, hydrate, a stereoisomeric or tautomeric form thereof, wherein R² is hydrogen, 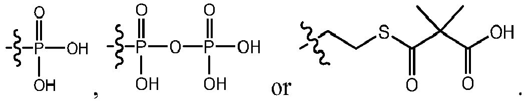 6. The purified compound of item 3 having formula IV: 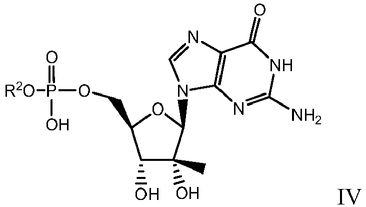 or a pharmaceutically acceptable salt, solvate, hydrate, a stereoisomeric or tautomeric form
thereof, wherein R² is 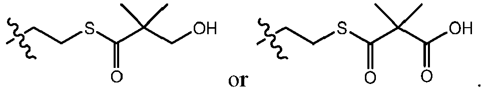 7. The purified compound of item 3 having formula V: 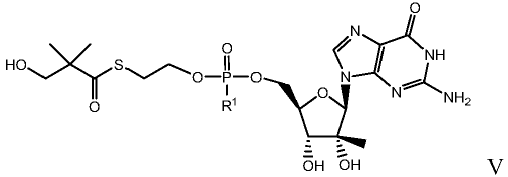 or a pharmaceutically acceptable salt, solvate, hydrate, a stereoisomeric or tautomeric form thereof, wherein R¹ is hydroxyl or amino.
8. The purified compound of item 3 selected from: 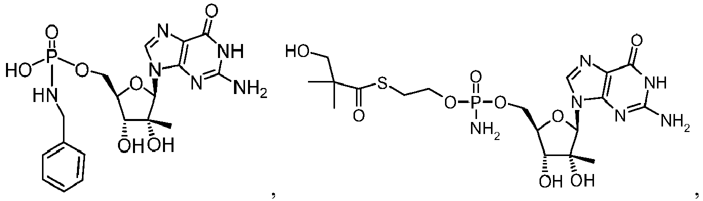 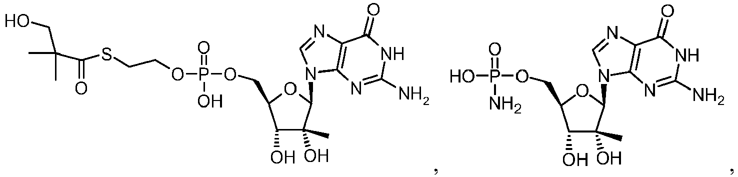 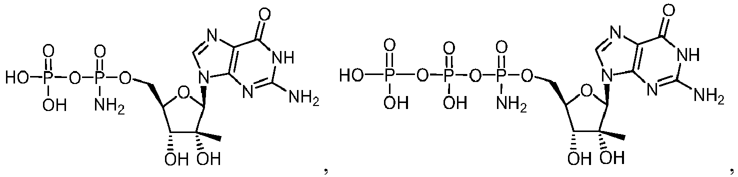 and 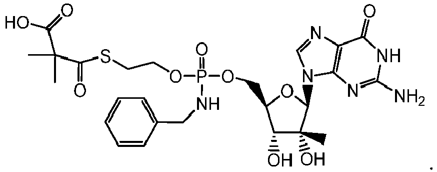 9. The purified compound of item 3 selected from: 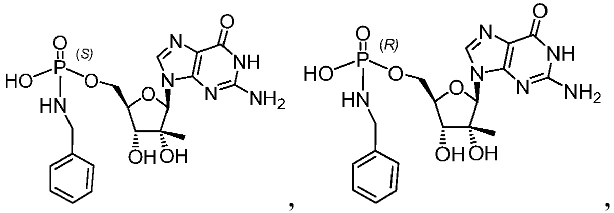 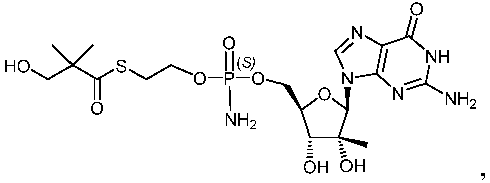 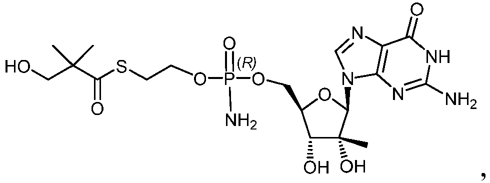 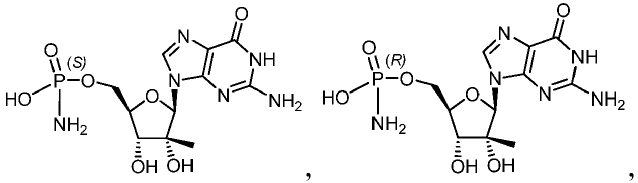 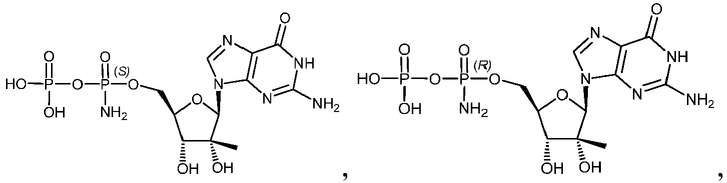 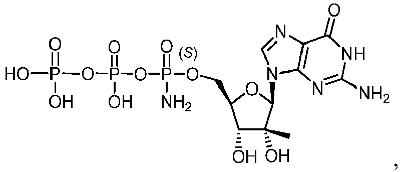 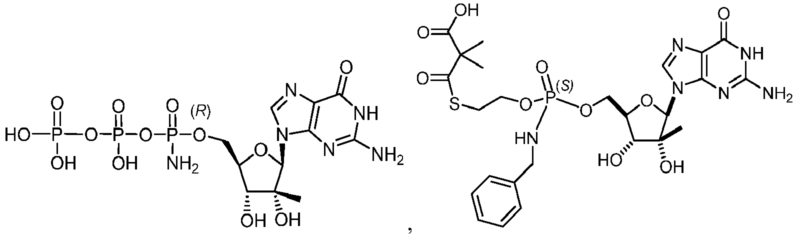 and 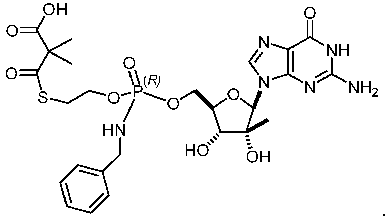 10. A purified metabolite of the compound having the formula 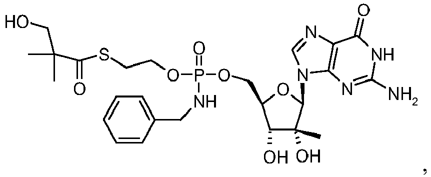 and wherein the metabolite is selected from the group consisting of:
(a) a compound which elutes off a C-18 (2) 4.6 x 250 mm 5 µm particle size column, at about 2.1 minutes;
(b) a compound which elutes off a C-18 (2) 4.6 x 250 mm 5 µm particle size column, at about 6.2 minutes,
(c) a compound which elutes off a C-18 (2) 4.6 x 250 mm 5 µm particle size column, at about 8.0 minutes,
(d) a compound which elutes off a C-18 (2) 4.6 x 250 mm 5 µm particle size column, at about 9.4 minutes,
(e) a compound which elutes off a C-18 (2) 4.6 x 250 mm 5 µm particle size column, at about 10.9 minutes,
(f) a compound which elutes off a C-18 (2) 4.6 x 250 mm 5 µm particle size column, at about 12.4 minutes,
(g) a compound which elutes off a C-18 (2) 4.6 x 250 mm 5 µm particle size column, at about 13.1 minutes,
(h) a compound which elutes off a C-18 (2) 4.6 x 250 mm 5 µm particle size column, at about 17.1 minutes,
(i) a compound which elutes off a C-18 (2) 4.6 x 250 mm 5 µm particle size column, at about 25.2 minutes, and
(j) a compound which elutes off a C-18 (2) 4.6 x 250 mm 5 µm particle size column, at about 26.5 minutes, where the retention times described are obtained in HPLC gradient conditions as follows:

| HPLC Gradient Conditions | | | |
|---|---|---|---|
| Time (min) | %A | %B | Flow (mL/min) |
| 0 | 100 | 0 | 1.5 |
| 4 | 100 | 0 | 1.5 |
| 10 | 85 | 15 | 1.5 |
| 12 | 75 | 25 | 1.5 |
| 17 | 70 | 30 | 1.5 |
| 28 | 25 | 75 | 1.5 |
| 33 | 10 | 90 | 1.5 |
| 34 | 10 | 90 | 1.5 |
| 35 | 100 | 0 | 1.5 |

| | | | |
|---|---|---|---|
| A = 20 mM ammonium formate in water B = 10 mM ammonium formate in methanol-water (90:10). | | | |

11. A purified metabolite of the compound having the formula 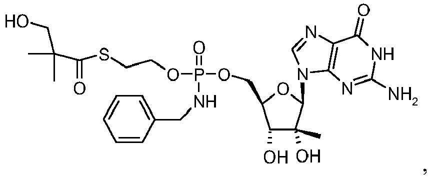 and wherein the metabolite elutes off a C-18 4.6 x 250 mm 5 µm particle size column, at about 16.4 minutes, in HPLC gradient conditions as follows:

| HPLC Gradient Conditions | | | |
|---|---|---|---|
| Time (min) | %A | %B | Flow (mL/min) |
| 0 | 95 | 5 | 1.0 |
| 15 | 70 | 30 | 1.0 |
| 20 | 70 | 30 | 1.0 |
| 30 | 50 | 50 | 1.0 |
| 50 | 50 | 50 | 1.0 |

| | | | |
|---|---|---|---|
| A = 25mM potassium phosphate + 5mM tetrabutylammonium dihydrogen phosphate pH 6.3 B = Methanol. | | | |

12. A purified metabolite of the compound having the formula 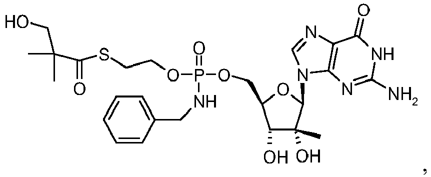 and wherein the metabolite is selected from the group consisting of:
(a) a compound which elutes off a C-18 4.6 x 250 mm 5 µm particle size column, at about 38.4 minutes;
(b) a compound which elutes off a C-18 4.6 x 250 mm 5 µm particle size column, at about 39.8 minutes,
(c) a compound which elutes off a C-18 4.6 x 250 mm 5 µm particle size column, at about 36.8 minutes,
(d) a compound which elutes off a C-18 4.6 x 250 mm 5 µm particle size column, at about 26.8 minutes,
(e) a compound which elutes off a C-18 4.6 x 250 mm 5 µm particle size column, at about 33.8 minutes,
(f) a compound which elutes off a C-18 4.6 x 250 mm 5 µm particle size column, at about 30.9 minutes,
(g) a compound which elutes off a C-18 4.6 x 250 mm 5 µm particle size column, at about 4.6 minutes, and
(h) a compound which elutes off a C-18 4.6 x 250 mm 5 µm particle size column, at about 28.1 minutes, where the retention times described are obtained in HPLC gradient conditions as follows:

| HPLC Gradient Conditions-Method 1 | | | |
|---|---|---|---|
| Time (min) | %MPA | %MPB | Flow (mL/min) |
| 0 | 95 | 5 | 1.0 |
| 15 | 70 | 30 | 1.0 |
| 20 | 70 | 30 | 1.0 |
| 30 | 50 | 50 | 1.0 |
| 50 | 50 | 50 | 1.0 |

| | | | |
|---|---|---|---|
| MPA = 25mM potassium phosphate + 5mM TBAP pH 6.3 MPB = Methanol. | | | |

13. An isotopically enriched compound having formula I, 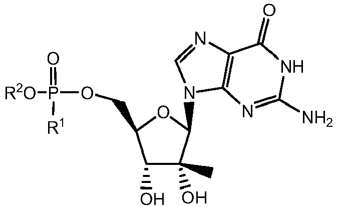 or a pharmaceutically acceptable salt, solvate, hydrate, a stereoisomeric or tautomeric form thereof, wherein R¹ is hydroxyl, amino or benzylamino; and R² is hydrogen, 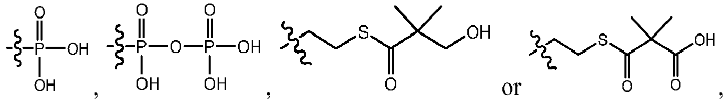 such that when R¹ is hydroxyl, R² is other than hydrogen, 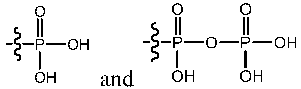 and
when R¹ is benzylamino, then R² is other than 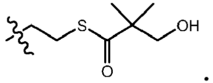 14. The isotopically enriched compound of item 13, wherein the compound is isotopically enriched compound 2, isotopically enriched compound 2a, isotopically enriched compound 2b, isotopically enriched compound 3, isotopically enriched compound 3a, isotopically enriched compound 3b, isotopically enriched compound 4, isotopically enriched compound 5, isotopically enriched compound 6, isotopically enriched compound 7, isotopically enriched compound 8, isotopically enriched compound 8a, isotopically enriched compound 8b, isotopically enriched compound 9, isotopically enriched compound 9a, isotopically enriched compound 9b, isotopically enriched compound 10, isotopically enriched compound 10a, isotopically enriched compound 10b, isotopically enriched compound 11, isotopically enriched compound 11a, and isotopically enriched compound 11b.
15. An isotopically enriched compound selected from the group consisting of isotopically enriched compound 1, isotopically enriched compound 1a, and isotopically enriched compound 1b.
16. A method for the treatment of a host infected with a *Flaviviridae* virus, comprising administering an effective treatment amount of a compound of any of items 1-15.
17. The method of item 16, wherein the virus is hepatitis C.
18. The method of item 17, wherein the host is a human.
19. The method of item 16, wherein said administration directs a substantial amount of said compound or pharmaceutically acceptable salt or stereoisomer thereof to the liver of said host.
20. The method of item 16, wherein the compound or pharmaceutically acceptable salt or stereoisomer thereof is administered in combination or alternation with a second anti-viral agent selected from the group consisting of an interferon, a ribavirin, an interleukin, a NS3 protease inhibitor, a cysteine protease inhibitor, a phenanthrenequinone, a thiazolidine derivative, a thiazolidine, a benzanilide, a helicase inhibitor, a polymerase inhibitor, a nucleotide analogue, a gliotoxin, a cerulenin, an antisense phosphorothioate oligodeoxynucleotide, an inhibitor of IRES-dependent translation, and a ribozyme.
21. The method of item 20, wherein the second agent is selected from the group consisting of pegylated interferon alpha 2a, interferon alphacon-1, natural interferon, albuferon, interferon beta-1a, omega interferon, interferon alpha, interferon gamma, interferon tau, interferon delta and interferon γ-1b.
22. The method of item 20, wherein the second agent is ribavirin.
23. The method of item 16, wherein the host is a human.
24. A pharmaceutical composition comprising a compound of any of items 1-15 and a pharmaceutically acceptable excipient, carrier or diluent.
25. The composition of item 18, wherein the composition is an oral formulation.
26. A method of preparing the purified compound of any one of items 3-9, comprising:
(a) contacting a precursor compound 1 having formula: 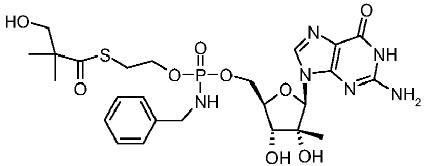 with hepatocytes or liver microsome compositions to yield a mixture; and
(b) isolating the compound from the mixture.
27. The method of item 24, wherein isolating comprises eluting the compound from a reverse phase media-containing column.
28. A compound having of formula VI 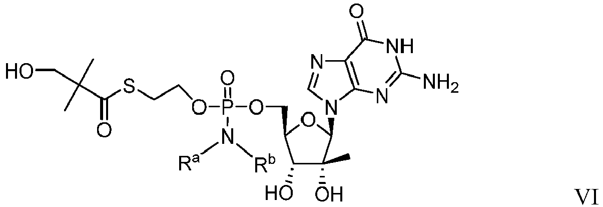 or a pharmaceutically acceptable salt, solvate, hydrate or stereoisomer thereof, wherein R^{a} and R^{b} are selected as follows:
i) R^{a} is hydrogen; and R^{b} is alkyl, carboxyalkyl, cycloalkyl, alkoxycarbonylalkyl or dialkylaminoalkyl; or
ii) R^{a} and R^{b} together with the nitrogen atom on which they are substituted form a 3-7 membered heterocyclic ring, optionally substituted with one or two alkyl groups.
29. The compound of item 28, wherein R^{a} and R^{b} are selected as follows:
i) R^{a} is hydrogen; and R^{b} is isopropyl, *t*-butyl, cyclohexyl, ethoxycarbonylmethyl, *t*-butyloxycarbonylmethyl, carboxymethyl or dimethylaminoethyl; or
ii) R^{a} and R^{b} together with the nitrogen atom on which they are substituted form a 4-methylpiperazine or morpholine ring.
30. The compound of item 28 selected from: 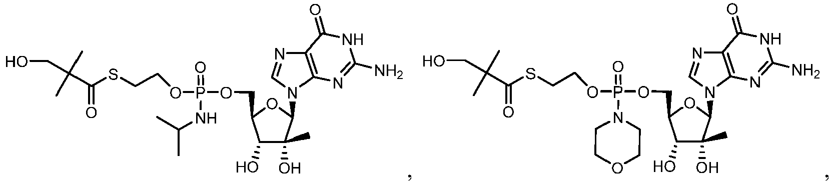 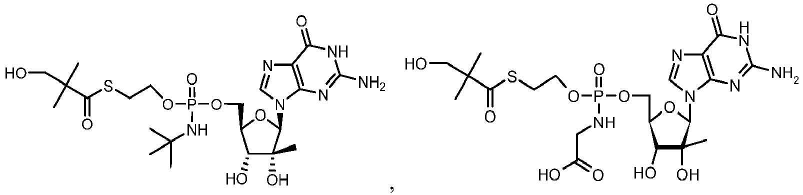 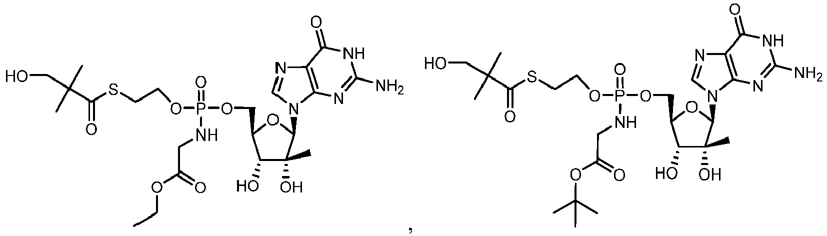 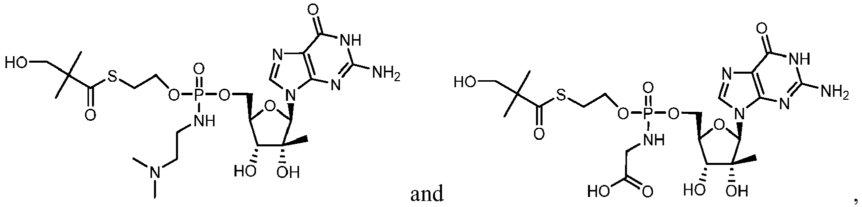 or
a pharmaceutically acceptable salt, solvate, hydrate or stereoisomer thereof.
31. The compound of item 28, wherein the compound is 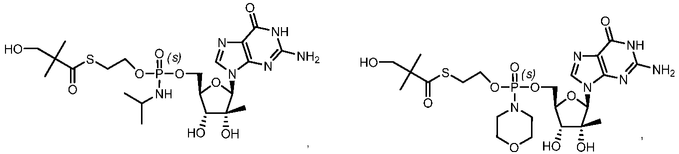 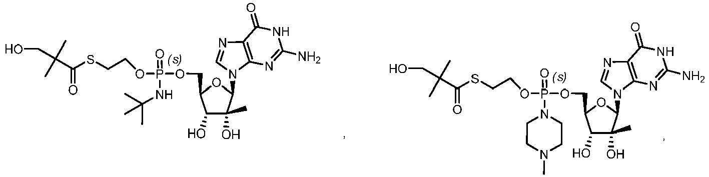 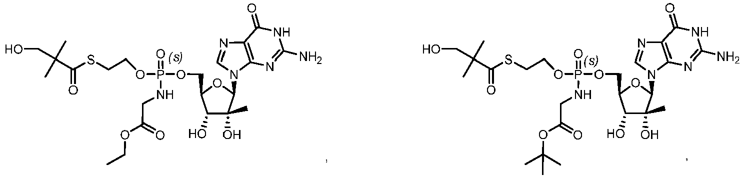 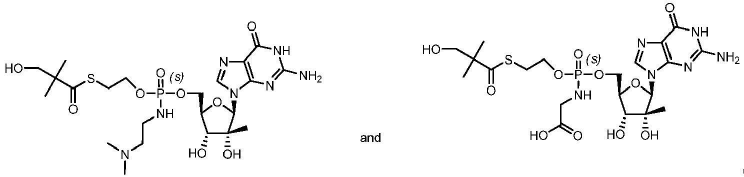 or
pharmaceutically acceptable salt, solvate, hydrate or stereoisomer thereof.
32. The compound of item 28, wherein the compound is: 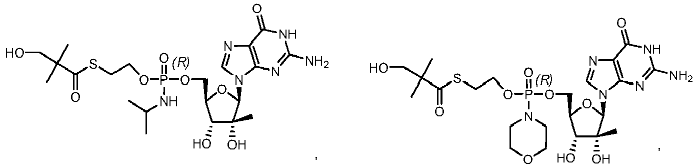 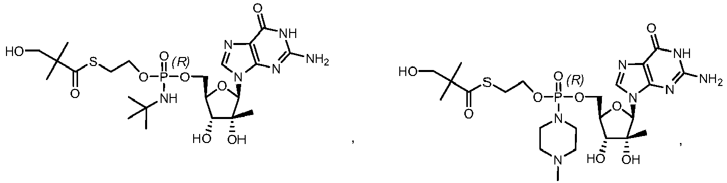 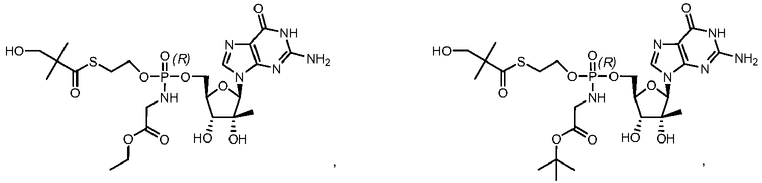 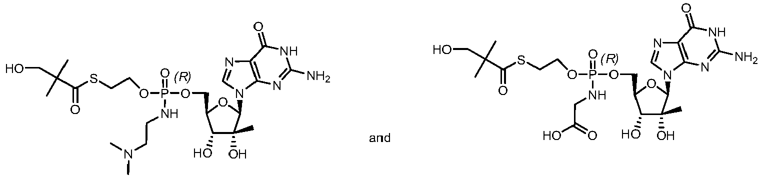 or
pharmaceutically acceptable salt, solvate, hydrate or stereoisomer thereof.
33. A method for the treatment of a host infected with a *Flaviviridae* virus, comprising administering an effective treatment amount of a compound of any of items 28-32.
34. A method for the treatment of a host infected with a *Flaviviridae* virus, comprising administering Compound **1** in an amount from about 1 mg/day to about 150 mg/day.
35. The method of item 34, wherein the amount is from about 5 mg/day to about 100 mg/day.
36. The method of item 34, wherein the amount is about 5, 10, 25, 50, 75, or 100 mg/day.
37. The method of any of items 33-36 further comprising administering a second anti-viral agent selected from the group consisting of an interferon, a ribavirin, an interleukin, a NS3 protease inhibitor, a cysteine protease inhibitor, a phenanthrenequinone, a thiazolidine derivative, a thiazolidine, a benzanilide, a helicase inhibitor, a polymerase inhibitor, a nucleotide analogue, a gliotoxin, a cerulenin, an antisense phosphorothioate oligodeoxynucleotide, an inhibitor of IRES-dependent translation, and a ribozyme.
38. The method of item 37, wherein the second agent is selected from the group consisting of pegylated interferon alpha 2a, interferon alphacon-1, natural interferon, albuferon, interferon bet-1a, omega interferon, interferon alpha, interferon gamma, interferon tau, interferon delta and interferon γ-1b.
39. The method of item 38, wherein the second agent is ribavirin.
40. The method of item 39, wherein Compound **1** is administered in an amount from about 1 mg/day to about 150 mg/day and the amount of ribavirin administered is from about 800 mg to about 1400 mg.
41. The method of item 40, wherein the amount of ribavirin administered is about 800 mg, 1000 mg, 1200 mg or 1400 mg.
42. The method of any of items 33-41, wherein the virus is hepatitis C.
43. The method of any of items 33-42, wherein the host is a human.
44. A pharmaceutical composition comprising a compound of any of items 28-32 and a pharmaceutically acceptable excipient, carrier or diluent.
45. The composition of item 34, wherein the composition is an oral formulation.
46. A pharmaceutical composition comprising Compound **1** in an amount from about 1 mg to about 150 mg.
47. The pharmaceutical composition of item 46, wherein the amount is about 5 mg or 25 mg.
48. The composition of item 47, wherein the composition is an oral capsule.
49. A process for preparing Compound **1** comprising:
i) reacting 2'-C-methyl guanosine with sodium sulphate and benzeneboronic acid to
   obtain the compound of formula **10-2** 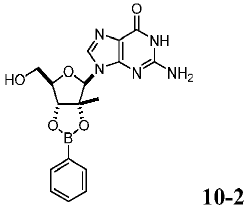
ii) reacting the compound of formula **10-2** with the phosphonate of formula **10-3** 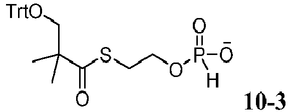 followed by reaction with benzylamine to obtain compound **10-4,** and 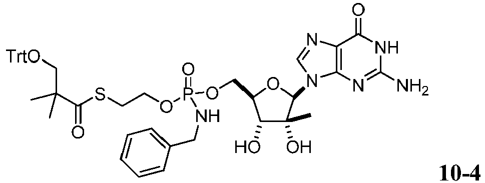
iii) deprotection of compound **10-4** to obtain Compound **1**

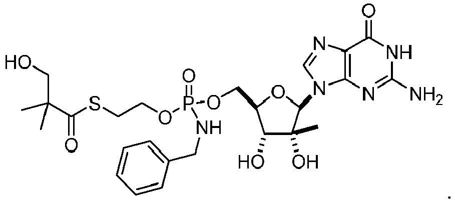

## Claims

1. A compound having formula VI 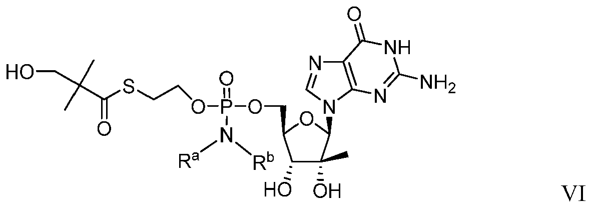 or a pharmaceutically acceptable salt, solvate, hydrate or stereoisomer thereof, wherein R^{a} and R^{b} are selected as follows:
i) R^{a} is hydrogen; and R^{b} is alkyl, carboxyalkyl, cycloalkyl, alkoxycarbonylalkyl or dialkylaminoalkyl; or
ii) R^{a} and R^{b} together with the nitrogen atom on which they are substituted form a 3-7 membered heterocyclic ring, optionally substituted with one or two alkyl groups.

2. The compound of claim 1, wherein R^{a} and R^{b} are selected as follows:
i) R^{a} is hydrogen; and R^{b} is isopropyl, *t*-butyl, cyclohexyl, ethoxycarbonylmethyl, *t*-butyloxycarbonylmethyl, carboxymethyl or dimethylaminoethyl; or
ii) R^{a} and R^{b} together with the nitrogen atom on which they are substituted form a 4-methylpiperazine or morpholine ring.

3. The compound of claim 1 selected from: 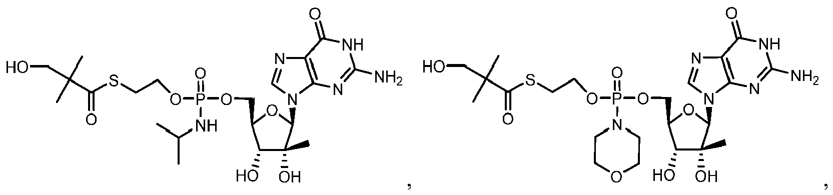 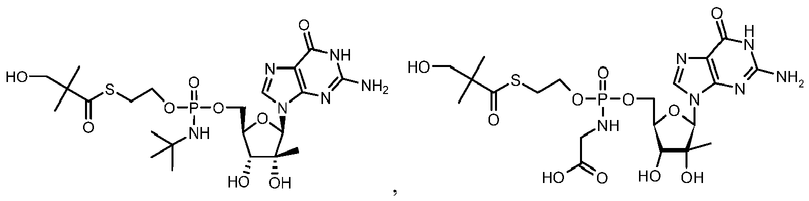 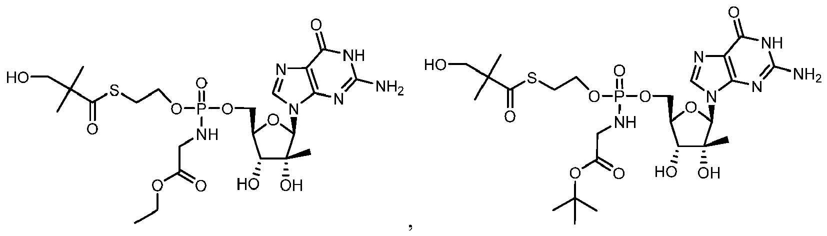 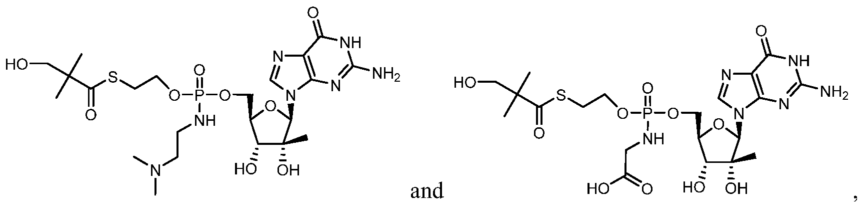 or
a pharmaceutically acceptable salt, solvate, hydrate or stereoisomer thereof.

4. The compound of claim 1 selected from: 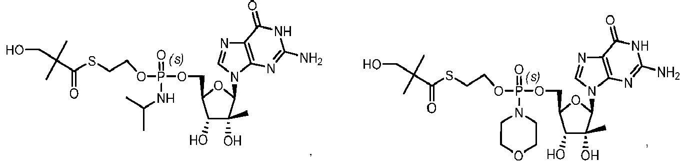 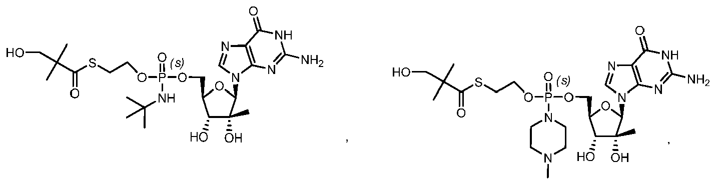 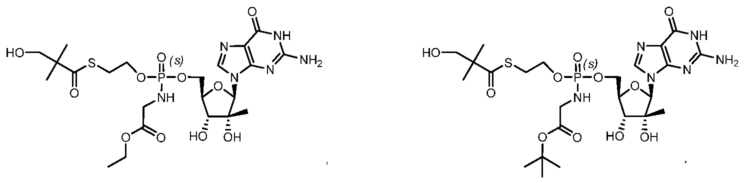 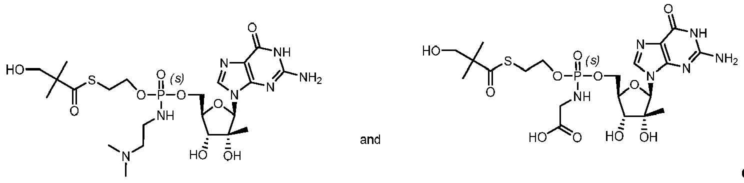 or
a pharmaceutically acceptable salt, solvate, hydrate or stereoisomer thereof.

5. The compound of claim 1 selected from: 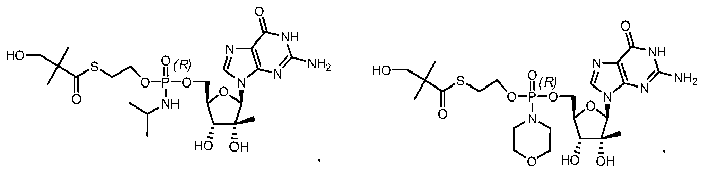 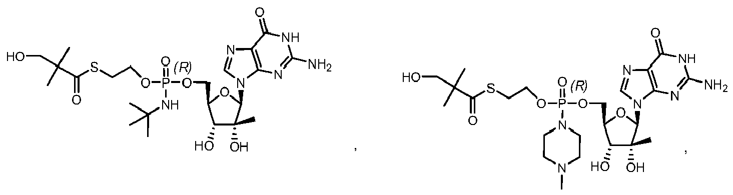 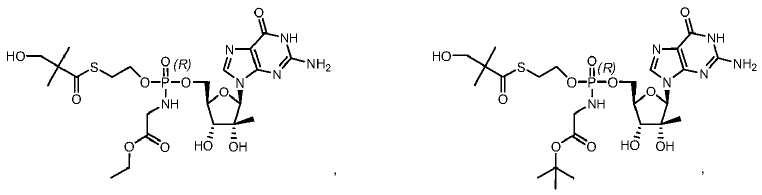 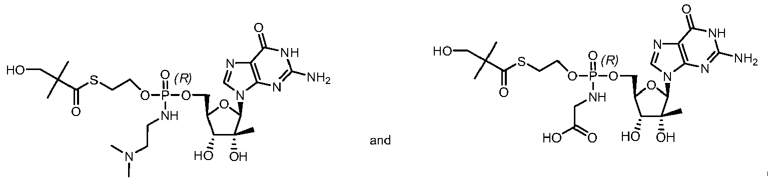 or
a pharmaceutically acceptable salt, solvate, hydrate or stereoisomer thereof.

6. A pharmaceutical composition comprising the compound of any of claims 1-5 and a pharmaceutically acceptable excipient, carrier or diluent.

7. The composition of claim 6, wherein the composition is an oral formulation.

8. A pharmaceutical composition comprising Compound 1, having formula:

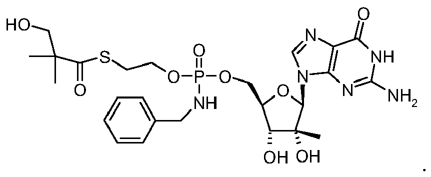

9. The compound of any of claims 1-5 for use in a method for the treatment of a host infected with a *Flaviviridae* virus.

10. Compound **1** having formula: 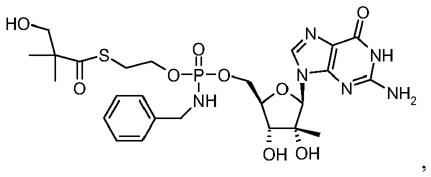 for use in a method for the treatment of a host infected with a *Flaviviridae* virus,
wherein the method comprises administering Compound **1** in an amount from about 1 mg/day to about 150 mg/day, or from about 5 mg/day to about 100 mg/day, or of about 5, 10, 25, 50, 75, or 100 mg/day.

11. The compound for use of claim 9 or 10, wherein the method comprises administering a second anti-viral agent,
wherein the second agent is selected from the group consisting of an interferon, a ribavirin, an interleukin, a NS3 protease inhibitor, a cysteine protease inhibitor, a phenanthrenequinone, a thiazolidine derivative, a thiazolidine, a benzanilide, a helicase inhibitor, a polymerase inhibitor, a nucleotide analogue, a gliotoxin, a cerulenin, an antisense phosphorothioate oligodeoxynucleotide, an inhibitor of IRES-dependent translation, and a ribozyme; or
wherein the second agent is selected from the group consisting of pegylated interferon alpha 2a, interferon alphacon-1, natural interferon, albuferon, interferon beta-1a, omega interferon, interferon alpha, interferon gamma, interferon tau, interferon delta and interferon γ-1b; or
wherein the second agent is ribavirin.

12. The compound for use of claim 11, wherein Compound 1 is administered in an amount from about 1 mg/day to about 150 mg/day and the amount of ribavirin administered is from about 800 mg to about 1400 mg, or the amount of ribavirin administered is about 800 mg, 1000 mg, 1200 mg or 1400 mg.

13. The compound for use of any of claims 9-12, wherein the virus is hepatitis C.

14. The compound for use of any of claims 9-13, wherein the host is a human.

15. A process for preparing Compound **1** comprising:
i) reacting 2'-C-methyl guanosine with sodium sulphate and benzeneboronic acid to
obtain the compound of formula **10-2** 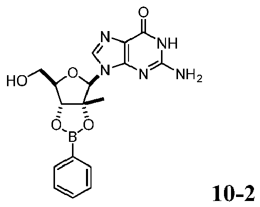
ii) reacting the compound of formula **10-2** with the phosphonate of formula **10-3** 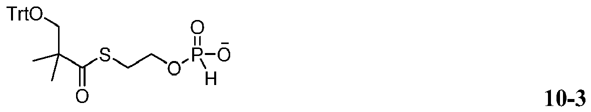 followed by reaction with benzylamine to obtain compound **10-4,** and 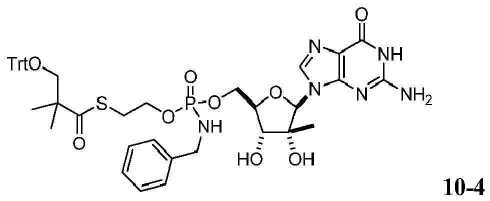
iii) deprotection of compound **10-4** to obtain Compound **1**
